(19) Europäisches Patentamt
European Patent Office
Office européen des brevets

(11) EP 2 825 161 B1

(12) **EUROPEAN PATENT SPECIFICATION**

(45) Date of publication and mention of the grant of the patent:
**02.01.2019 Bulletin 2019/01**

(21) Application number: **13711265.2**

(22) Date of filing: **12.03.2013**

(51) Int Cl.:
***A61K 31/00*** *(2006.01)*
***A61K 31/497*** *(2006.01)*
***A61K 31/4162*** *(2006.01)*
***C12Q 1/6886*** *(2018.01)*
***G01N 33/574*** *(2006.01)*
***A61P 35/00*** *(2006.01)*
***A61K 31/52*** *(2006.01)*

(86) International application number:
**PCT/US2013/030565**

(87) International publication number:
**WO 2013/138361 (19.09.2013 Gazette 2013/38)**

(54) **INHIBITORS OF HUMAN EZH2, AND METHODS OF USE THEREOF**

HEMMER VON MENSCHLICHEM EZH2 UND VERFAHREN ZUR VERWENDUNG DAVON

INHIBITEURS DE EZH2 HUMAIN ET PROCÉDÉS D'UTILISATION ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.03.2012 US 201213418242**

(43) Date of publication of application:
**21.01.2015 Bulletin 2015/04**

(73) Proprietor: **Epizyme, Inc.**
**Cambridge, MA 02139 (US)**

(72) Inventors:
- **KUNTZ, Kevin, Wayne**
  **Woburn, MA 01801 (US)**
- **KNUTSON, Sarah, Kathleen**
  **Cambridge, MA 02138 (US)**
- **WIGLE, Timothy James, Nelson**
  **Waltham, MA 02451 (US)**

(74) Representative: **Russell, Tim et al**
**Venner Shipley LLP**
**200 Aldersgate**
**London EC1A 4HD (GB)**

(56) References cited:
**WO-A2-2012/034132 WO-A2-2013/049770**

- **CHRISTINA R. MAJER ET AL: "A687V EZH2 is a gain-of-function mutation found in lymphoma patients", FEBS LETTERS, vol. 586, no. 19, 28 July 2012 (2012-07-28), pages 3448-3451, XP055075954, ISSN: 0014-5793, DOI: 10.1016/j.febslet.2012.07.066**
- **D. B. YAP ET AL: "Somatic mutations at EZH2 Y641 act dominantly through a mechanism of selectively altered PRC2 catalytic activity, to increase H3K27 trimethylation", BLOOD, vol. 117, no. 8, 29 December 2010 (2010-12-29), pages 2451-2459, XP055075957, ISSN: 0006-4971, DOI: 10.1182/blood-2010-11-321208**
- **M. T. MCCABE ET AL: "Mutation of A677 in histone methyltransferase EZH2 in human B-cell lymphoma promotes hypertrimethylation of histone H3 on lysine 27 (H3K27)", PROCEEDINGS OF THE NATIONAL ACADEMY OF SCIENCES, vol. 109, no. 8, 21 February 2012 (2012-02-21), pages 2989-2994, XP055075955, ISSN: 0027-8424, DOI: 10.1073/pnas.1116418109**
- **RUSSELL J. H. RYAN ET AL: "EZH2 Codon 641 Mutations are Common in BCL2-Rearranged Germinal Center B Cell Lymphomas", PLOS ONE, vol. 6, no. 12, 14 December 2011 (2011-12-14), page e28585, XP055075962, ISSN: 1932-6203, DOI: 10.1371/journal.pone.0028585**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- TIM J WIGLE ET AL: "The Y641C mutation of EZH2 alters substrate specificity for histone H3 lysine 27 methylation states", FEBS LETTERS, ELSEVIER, AMSTERDAM, NL, vol. 585, no. 19, 17 August 2011 (2011-08-17), pages 3011-3014, XP028300045, ISSN: 0014-5793, DOI: 10.1016/J.FEBSLET.2011.08.018 [retrieved on 2011-08-17]

## Description

### FIELD OF THE INVENTION

This invention relates to inhibition of wild-type and certain mutant forms of human histone methyltransferase EZH2, the catalytic subunit of the PRC2 complex which catalyzes the mono- through tri-methylation of lysine 27 on histone H3 (H3-K27), methods for treating cancers including follicular lymphoma and diffuse large B-cell lymphoma (DLBCL) and methods for determining responsiveness to an EZH2 inhibitor in a subject.

### BACKGROUND

In eukaryotic cells DNA is packaged with histones to form chromatin. Approximately 150 base pairs of DNA are wrapped twice around an octamer of histones (two each of histones 2A, 2B, 3 and 4) to form a nucleosome, the basic unit of chromatin. Changes in the ordered structure of chromatin can lead to alterations in transcription of associated genes. This process is highly controlled because changes in gene expression patterns can profoundly affect fundamental cellular processes, such as differentiation, proliferation and apoptosis. Control of changes in chromatin structure (and hence of transcription) is mediated by covalent modifications to histones, most notably of their N-terminal tails. These modifications are often referred to as epigenetic because they can lead to heritable changes in gene expression, but they do not affect the sequence of the DNA itself. Covalent modifications (for example, methylation, acetylation, phosphorylation and ubiquitination) of the side chains of amino acids are enzymatically mediated.

The selective addition of methyl groups to specific amino acid sites on histones is controlled by the action of a unique family of enzymes known as histone methyltransferases (HMTs). The level of expression of a particular gene is influenced by the presence or absence of one or more methyl groups at a relevant histone site. The specific effect of a methyl group at a particular histone site persists until the methyl group is removed by a histone demethylase, or until the modified histone is replaced through nucleosome turnover. In a like manner, other enzyme classes can decorate DNA and histones with other chemical species, and still other enzymes can remove these species to provide control of gene expression.

The orchestrated collection of biochemical systems behind transcriptional regulation must be tightly controlled in order for cell growth and differentiation to proceed optimally. Disease states result when these controls are disrupted by aberrant expression and/or activity of the enzymes responsible for DNA and histone modification. In human cancers, for example, there is a growing body of evidence to suggest that dysregulated epigenetic enzyme activity contributes to the uncontrolled cell proliferation associated with cancer as well as other cancer-relevant phenotypes such as enhanced cell migration and invasion. Beyond cancer, there is growing evidence for a role of epigenetic enzymes in a number of other human diseases, including metabolic diseases (such as diabetes), inflammatory diseases (such as Crohn's disease), neurodegenerative diseases (such as Alzheimer's disease), and cardiovascular diseases. Therefore, selectively modulating the aberrant action of epigenetic enzymes holds great promise for the treatment of a range of diseases.

#### Histone Methyltransferase EZH2

Polycomb group (PcG) and trithorax group (trxG) proteins are known to be part of the cellular memory system. Francis et al. (2001) Nat Rev Mol Cell Biol 2:409-21; Simon et al. (2002) Curr Opin Genet Dev 12:210-8. Both groups of proteins are involved in maintaining the spatial patterns of homeotic box (Hox) gene expression, which are established early in embryonic development by transiently expressed segmentation genes. In general, PcG proteins are transcriptional repressors that maintain the "off state," and trxG proteins are transcriptional activators that maintain the "on state." Because members of PcG and trxG proteins contain intrinsic histone methyltransferase (HMTase) activity, PcG and trxG proteins may participate in cellular memory through methylation of core histones. Beisel et al. (2002) Nature 419:857-62; Cao et al. (2002) Science 298:1039-43; Czermin et al. (2002) Cell 111:185-96; Kuzmichev et al. (2002) Genes Dev 16:2893-905; Milne et al. (2002) Mol Cell 10:1107-17; Muller et al. (2002) Cell 111:197-208; Nakamura et al. (2002) Mol Cell 10:1119-28.

Biochemical and genetic studies have provided evidence that *Drosophila* PcG proteins function in at least two distinct protein complexes, the Polycomb repressive complex 1 (PRC1) and the ESC-E(Z) complex (also known as Polycomb repressive complex 2 (PRC2)), although the compositions of the complexes may be dynamic. Otte et al. (2003) Curr Opin Genet Dev 13:448-54. Studies in *Drosophila* (Czermin et al. (*supra*); Muller et al. (*supra*)) and mammalian cells (Cao et al. (*supra*); Kuzmichev et al. (*supra*)) have demonstrated that the ESC-E(Z)/EED-EZH2 (i.e., PRC2) complexes have intrinsic histone methyltransferase activity. Although the compositions of the complexes isolated by different groups are slightly different, they generally contain EED, EZH2, SUZ12, and RbAp48 or *Drosophila* homologs thereof. However, a reconstituted complex comprising only EED, EZH2, and SUZ12 retains histone methyltransferase activity for lysine 27 of histone H3. US Patent 7,563,589.

Of the various proteins making up PRC2 complexes, EZH2 (Enhancer of Zeste Homolog 2) is the catalytic subunit. The catalytic site of EZH2 in turn is present within a SET domain, a highly conserved sequence motif (named after Su(var)3-9, Enhancer of Zeste, Trithorax) that is found in several chromatin-associated proteins, including members of both the Trithorax group and Polycomb group. SET domain is characteristic of all known histone lysine methyltransferases except the H3-K79 methyltransferase DOT1.

In addition to Hox gene silencing, PRC2-mediated histone H3-K27 methylation has been shown to participate in X-inactivation. Plath et al. (2003) Science 300:131-5; Silva et al. (2003) Dev Cell 4:481-95. Recruitment of the PRC2 complex to Xi and subsequent trimethylation on histone H3-K27 occurs during the initiation stage of X-inactivation and is dependent on Xist RNA. Furthermore, EZH2 and its associated histone H3-K27 methyltransferase activity was found to mark differentially the pluripotent epiblast cells and the differentiated trophectoderm. Erhardt et al. (2003) Development 130:4235-48).

Consistent with a role of EZH2 in maintaining the epigenetic modification patterns of pluripotent epiblast cells, Cre-mediated deletion of EZH2 results in loss of histone H3-K27 methylation in the cells. Erhardt et al. (*supra*). Further, studies in prostate and breast cancer cell lines and tissues have revealed a strong correlation between the levels of EZH2 and SUZ12 and the invasiveness of these cancers (Bracken et al. (2003) EMBO J 22:5323-35; Kirmizis et al. (2003) Mol Cancer Ther 2:113-21; Kleer et al. (2003) Proc Natl Acad Sci USA 100:11606-11; Varambally et al. (2002) Nature 419:624-9), indicating that dysfunction of the PRC2 complex may contribute to cancer.

Recently, somatic mutations of EZH2 were reported to be associated with follicular lymphoma (FL) and the germinal center B cell-like (GCB) subtype of diffuse large B-cell lymphoma (DLBCL). Morin et al. (2010) Nat Genet 42:181-5. In all cases, occurrence of the mutant EZH2 gene was found to be heterozygous, and expression of both wild-type and mutant alleles was detected in the mutant samples profiled by transcriptome sequencing. Currently, the standard of care for the treatment of most cases of DLBCL is the R-CHOP regimen. However, the outcome of this regimen is far from satisfactory. Therefore, there is a great medical need to identify novel and effective therapies, optionally based on the genetic profiles of the subject.

## SUMMARY OF THE INVENTION

The invention is based upon the discovery that cells expressing certain EZH2 mutants are more responsive to EZH2 inhibitors than cells expressing wild type EZH2.

The invention features an EZH2 inhibitor selected from compound 2 to compound 62 or a pharmaceutically acceptable salt thereof for use in a method of treating cancer or myelodysplastic syndromes (MDS, formerly known as preleukemia) in a subject expressing a mutant EZH2, wherein the mutant EZH2 comprises a substitution mutation at amino acid position 677, 687 or 641 of SEQ ID No:1.

The invention also features a method of determining a responsiveness of a subject having a cancer or myelodysplastic syndromes (MDS, formerly known as preleukemia) to an EZH2 inhibitor selected from compound 2 to compound 62 detecting a mutation in EZH2 in a sample obtained from the subject, wherein the mutation is or is expressed as a substitution mutation at amino acid position 677, 687 or 641 of SEQ ID No:1, wherein the presence of said mutation indicates the subject is responsive to the EZH2 inhibitor.

Described herein is a use of an EZH2 inhibitor in a therapeutically effective amount in the manufacture of a medicament for treating or alleviating a symptom of cancer or precancerous condition in a subject expressing a mutant EZH2 comprising a mutation in the substrate pocket domain as defined in SEQ ID NO: 6.

The invention further provides a use of a mutation in the EZH2 substrate pocket domain as defined in SEQ ID NO: 6 for determining a responsiveness of a subject having a cancer or a precancerous condition to an EZH2 inhibitor.

The mutant EZH2 of the present invention is a mutant EZH2 polypeptide or a nucleic acid sequence encoding a mutant EZH2 polypeptide. The mutant EZH2 comprises a mutation at amino acid position 677, 687, or 641 of SEQ ID NO: 1. More preferably, mutation is selected from the group consisting of a substitution of glycine (G) for the wild type residue alanine (A) at amino acid position 677 of SEQ ID NO: 1 (A677G); a substitution of valine (V) for the wild type residue alanine (A) at amino acid position 687 of SEQ ID NO: 1 (A687V); a substitution of phenylalanine (F) for the wild type residue tyrosine (Y) at amino acid position 641 of SEQ ID NO: 1 (Y641F); a substitution of histidine (H) for the wild type residue tyrosine (Y) at amino acid position 641 of SEQ ID NO: 1 (Y641H); a substitution of asparagine (N) for the wild type residue tyrosine (Y) at amino acid position 641 of SEQ ID NO: 1 (Y641N); a substitution of serine (S) for the wild type residue tyrosine (Y) at amino acid position 641 of SEQ ID NO: 1 (Y641S); and a substitution of cysteine (C) for the wild type residue tyrosine (Y) at amino acid position 641 of SEQ ID NO: 1 (Y641C).

The subject of the present invention includes any human subject who has been diagnosed with, has symptoms of, or is at risk of developing a cancer or myelodysplastic syndromes (MDS, formerly known as preleukemia). For example, the cancer is lymphoma, leukemia or melanoma. Preferably, the lymphoma is non-Hodgkin lymphoma, follicular lymphoma or diffuse large B-cell lymphoma. Alternatively, the leukemia is chronic myelogenous leukemia (CML)..

Unless otherwise defined, all technical and scientific terms used herein have the same meaning as commonly

understood by one of ordinary skill in the art to which this invention belongs. In the specification, the singular forms also include the plural unless the context clearly dictates otherwise. Although methods and materials similar or equivalent to those described herein can be used in the practice or testing of the present invention, suitable methods and materials are described below. The references cited herein are not admitted to be prior art to the claimed invention. In the case of conflict, the present specification, including definitions, will control. In addition, the materials, methods and examples are illustrative only and are not intended to be limiting.

Other features and advantages of the invention will be apparent from the following detailed description and claims.

## BRIEF DESCRIPTION OF THE FIGURES

**Figure 1** is two graphs establishing that B-cell lymphoma-associated mutants of EZH2 are active histone methyltransferases. *In vitro* methyltransferase activity of PRC2 complexes containing wild-type and various Y641 mutants of EZH2 was measured as (A) methyl transfer reactions using a peptide (H3 21-44) as substrate, and (B) methyl transfer reactions using avian nucleosomes as substrate. Symbols: wild-type (●), Y641F (○), Y641H (□), Y641N (■), and Y641S (▲). CPM is counts per minute, referring to scintillation counting as a result of $^3H$ radiation.

**Figure 2** is four graphs establishing that PRC2 complexes containing mutant EZH2 preferentially catalyze di- and tri-methylation of histone H3-K27. (A) Methyltransferase activity of mutant and wild-type (WT) complexes on unmethylated peptide (open bars), monomethylated peptide (hashed bars), and dimethylated peptide (closed bars). (B) Affinity for peptide substrates as judged by $K_{1/2}$ is similar across all peptide methylation states for PRC2 complexes containing wild-type (○), Y641F (●), Y641H (□), Y641N (■), and Y641S (▲) EZH2. Note that the variation in $K_{1/2}$ values across all substrates and all enzyme forms is less than 3.5-fold. For any particular methylation state of substrate the variation in $K_{1/2}$ value is less than 2-fold. (C) Enzyme turnover number ($k_{cat}$) varies with substrate methylation status in opposing ways for WT and Y641 mutants of EZH2. The $k_{cat}$ decreases with increasing K27 methylation states for wild-type (○), but increases for Y641F (●), Y641H (□),□ Y641N (■), and Y641S (▲) mutants of EZH2. (D) Catalytic efficiency ($k_{cat}/K_{1/2}$) decreases with increasing K27 methylation states for wild-type (○), but increases for Y641F (●), Y641H (□), Y641N (■), and Y641S (▲) mutants of EZH2. In panels B-D, the lines drawn to connect the data points are not intended to imply any mathematical relationship; rather, they are merely intended to serve as visual aides.

**Figure 3A** is a trio of graphs depicting predicted relative levels of H3-K27me3 (top panel), H3-K27me2 (middle panel), and H3-K27me1 (bottom panel) for cells containing different EZH2 mutants. Simulations were performed using a coupled enzyme steady state velocity equation and the steady state kinetic parameters shown in Table 2. All values are relative to the homozygous WT EZH2-containing cells and assume saturating concentrations of intracellular SAM, relative to Km and intracellular nucleosome concentrations similar to Km.

**Figure 3B** is a series of Western blot analyses of relative patterns of H3-K27 methylation status for lymphoma cell lines homozygous for WT EZH2, or heterozygous for the indicated EZH2 Y641 mutation. Panels from top to bottom depict the results of probing with antibodies specific for the following: total EZH2; H3-K27me3; H3-K27me2; H3-K27me1; and total histone H3 as loading control.

**Figure 4** depicts selected proposed mechanisms leading to aberrantly high levels of trimethylation on histone H3-K27 in cancer. These include: a) mutation of Y641 in EZH2 resulting in a change in substrate preference from the nonmethylated to the mono- and dimethylated histone H3-K27; b) overexpression of EZH2; c) mutations in *UTX* that inactivate enzyme function, causing a decrease in demethylation of H3-K27me3; and d) overexpression of the PRC2 complex subunit PHF19/PCL3 that leads to increases in recruitment of the PRC2 complex to specific genes and an increase in histone H3-K27 trimethylation. In all four models the alteration leads to aberrant histone H3-K27 trimethylation in the proximal promoter regions of genes resulting in transcriptional repression of key genes in cancer.

**Figure 5** depicts a SDS-PAGE gel showing that the expression levels of each of the five-component PRC2 complexes are similar with mutant and wild-type EZH2.

**Figure 6** is a pair of tables showing that mutant and wild-type (WT) PRC2 complexes display strong substrate preference for H3-K27-containing peptides. Each enzyme was tested against a panel of overlapping 15-mer peptides

covering all of H3 and H4. Activity was measured as velocity (CPM per minute), and the reported value represents the mean of two independent determinations for each reaction. For all the complexes the most favored peptide was H3:16-30. WT complex had greater than 6-fold more activity against this peptide than any of the mutant complexes.

**Figure 7** is a graph depicting inhibitory potency of S-adenosyl-L-homocysteine (SAH) against EZH2 WT and Y641 mutants of EZH2. The X axis shows log concentration of SAH; theY axis shows percent inhibition.

**Figure 8** is a graph depicting inhibitory potency of Compound 75 against EZH2 WT and Y641 mutants of EZH2. The X axis shows log concentration of Compound 75; the Y axis shows percent inhibition.

**Figure 9** depicts a Western Blot analysis of relative levels of H3-K27me1, me2 and me3 in a cell line pane, including multiple DLBCL lines expressing WT or Y641 mutatnt EZH2. Histones were extracted from the cell lines shown, fractionated by SDS-PAGE on a 4-20% gel, transferred to nitrocellulose membranes, and probed with antibodies to Histone H3, H3-K27me1, me2, or me3..

**Figure 10** depicts an immunocytochemistry analysis of H3 and H3-K27me3 levels in a panel of WT and Y641 mutant lymphoma cell lines. Cell pellets from the indicated cell lines were fixed and embedded in paraffin. Slides were prepared and levels of H3 and H3-K27me3 were evaluated by immunocytochemistry using antibodies to histone H3, or H3-K27me3.

**Figure 11** depicts an immunocytochemistry analysis of H3 and H3-K27me2 levels in a panel of WT and Y641 mutant lymphoma cell lines. Cell pellets from the indicated cell lines were fixed and embedded in paraffin. Slides were prepared and levels of H3 and H3-K27me2 were evaluated by immunocytochemistry using antibodies to histone H3, or H3-K27me2.

**Figure 12** is a graph depicting the inhibition of global H3-K27me3 levels by EZH2 inhibitor treatment in Y641 mutant WSU-DLCL2 cells. WSU-DLCL2 cells were treated for 4 days with the indicated concentrations of EZH2 inhibitor A or B. Following compound treatment, histones were extracted, fractionated by SDS-PAGE on a 4-20% gel, transferred to nitrocellulose membranes, and probed with antibodies to Histone H3, or H3-K27me3.

**Figure 13** is a graph showing that the EZH2 inhibitors can block proliferation of a Y641 mutant WSU-DLCL2 cells, but has little effect on non Y641 mutant OCI-LY19 cells. Cells were incubated in the presence of increasing concentrations of EZH2 inhibitor A or B for eleven days. Vehicle treated (DMSO) cells were included as controls. Cell number and viability was determined using the Guava Viacount assay in a Guava EasyCyte Plus instrument. Cells were split and media and compound was replenished every 3-4 days.

**Figure 14** is a graph showing the presence of an EZH2 (Y641) mutation and/or high H3-K27me3 and low H3-K27me2 levels predict sensitivity to EZH2 inhibitors. Cell lines were maintained in the presence of increasing concentrations of one EZH2 inhibitor up to 25 $\mu$M. Viable cells counts were used to derive $IC_{90}$ values after 11 days of treatment. Results are plotted with cell lines segregated according to EZH2 mutational status (A), or segregated according to H3-K27me2 and H3-K27me3 levels (B). In both plots, the line shows the average IC90 values from the indicated cell line group.

**Figure 15** is a panel of graphs showing velocity vs. enzyme concentration measured for wild-type, A677G or A687V EZH2. Biotinylated peptides representing histone H3 residues 21-44 containing un-, mono-, di- or trimethyl lysine 27 (H3K27me0 through H3K27me3) were assayed at a fixed concentration with a dilution series of the indicated EZH2 enzyme. Timepoints were sampled over the course of 90 minutes and 3H-SAM incorporation at lysine 27 of the H3 peptide was measured by capturing the peptide in a Flashplate and reading the counts per minute (CPM). Linear regression of the timecourse yielded enzyme velocity in CPM per minute (CPM/min) which was plotted as a function of enzyme concentration.

## DETAILED DESCRIPTION

Chromatin structure is important in gene regulation and epigenetic inheritance. Post-translational modifications of histones, such as methylation are involved in the establishment and maintenance of higher-order chromatin structure.

## EZH2 MUTANTS

EZH2 is a histone methyltransferase that is the catalytic subunit of the PRC2 complex which catalyzes the mono- through tri-methylation of lysine 27 on histone H3 (H3-K27).

Point mutations of the EZH2 gene at a single amino acid residue (e.g., Tyr641, herein referred to as Y641) of EZH2 have been reported to be linked to subsets of human B-cell lymphoma. Morin et al. (2010) Nat Genet 42(2):181-5. In particular, Morin et al. reported that somatic mutations of tyrosine 641 (Y641F, Y641H, Y641N, and Y641S) of EZH2 were associated with follicular lymphoma (FL) and the germinal center B cell-like (GCB) subtype of diffuse large B-cell lymphoma (DLBCL). The mutant allele is always found associated with a wild-type allele (heterozygous) in disease cells, and the mutations were reported to ablate the enzymatic activity of the PRC2 complex for methylating an unmodified peptide substrate.

The present invention is based in part upon the surprising discovery that cells expressing an EZH2 mutant are more sensitive to EZH2 inhibitors of the instant invention than cells expressing wild type EZH2. Accordingly, described herein are methods for treating or alleviating a symptom of cancer or precancerous condition in a subject by administering to a subject expressing a mutant EZH2 a therapeutically effective amount of an EZH2 inhibitor. The mutant EZH2 of the present invention refers to a mutant EZH2 polypeptide or a nucleic acid sequence encoding a mutant EZH2 polypeptide. The mutant EZH2 comprises a substitution mutation at amino acid position 677, 687, or 641 of SEQ ID NO: 1, such as, but is not limited to a substitution of glycine (G) for the wild type residue alanine (A) at amino acid position 677 of SEQ ID NO: 1 (A677G); a substitution of valine (V) for the wild type residue alanine (A) at amino acid position 687 of SEQ ID NO: 1 (A687V); a substitution of phenylalanine (F) for the wild type residue tyrosine (Y) at amino acid position 641 of SEQ ID NO: 1 (Y641F); a substitution of histidine (H) for the wild type residue tyrosine (Y) at amino acid position 641 of SEQ ID NO: 1 (Y641H); a substitution of asparagine (N) for the wild type residue tyrosine (Y) at amino acid position 641 of SEQ ID NO: 1 (Y641N); a substitution of serine (S) for the wild type residue tyrosine (Y) at amino acid position 641 of SEQ ID NO: 1 (Y641S); or a substitution of cysteine (C) for the wild type residue tyrosine (Y) at amino acid position 641 of SEQ ID NO: 1 (Y641C).

The mutation of the present invention may also include a substitution of serine (S) for the wild type residue asparagine (N) at amino acid position 322 of SEQ ID NO: 3 (N322S), a substitution of glutamine (Q) for the wild type residue arginine (R) at amino acid position 288 of SEQ ID NO: 3 (R288Q), a substitution of isoleucine (I) for the wild type residue threonine (T) at amino acid position 573 of SEQ ID NO: 3 (T573I), a substitution of glutamic acid (E) for the wild type residue aspartic acid (D) at amino acid position 664 of SEQ ID NO: 3 (D664E), a substitution of glutamine (Q) for the wild type residue arginine (R) at amino acid position 458 of SEQ ID NO: 5 (R458Q), a substitution of lysine (K) for the wild type residue glutamic acid (E) at amino acid position 249 of SEQ ID NO: 3 (E249K), a substitution of cysteine (C) for the wild type residue arginine (R) at amino acid position 684 of SEQ ID NO: 3 (R684C), a substitution of histidine (H) for the wild type residue arginine (R) at amino acid position 628 of SEQ ID NO: 21 (R628H), a substitution of histidine (H) for the wild type residue glutamine (Q) at amino acid position 501 of SEQ ID NO: 5 (Q501H), a substitution of asparagine (N) for the wild type residue aspartic acid (D) at amino acid position 192 of SEQ ID NO: 3 (D192N), a substitution of valine (V) for the wild type residue aspartic acid (D) at amino acid position 664 of SEQ ID NO: 3 (D664V), a substitution of leucine (L) for the wild type residue valine (V) at amino acid position 704 of SEQ ID NO: 3 (V704L), a substitution of serine (S) for the wild type residue proline (P) at amino acid position 132 of SEQ ID NO: 3 (P132S), a substitution of lysine (K) for the wild type residue glutamic acid (E) at amino acid position 669 of SEQ ID NO: 21 (E669K), a substitution of threonine (T) for the wild type residue alanine (A) at amino acid position 255 of SEQ ID NO: 3 (A255T), a substitution of valine (V) for the wild type residue glutamic acid (E) at amino acid position 726 of SEQ ID NO: 3 (E726V), a substitution of tyrosine (Y) for the wild type residue cysteine (C) at amino acid position 571 of SEQ ID NO: 3 (C571Y), a substitution of cysteine (C) for the wild type residue phenylalanine (F) at amino acid position 145 of SEQ ID NO: 3 (F145C), a substitution of threonine (T) for the wild type residue asparagine (N) at amino acid position 693 of SEQ ID NO: 3 (N693T), a substitution of serine (S) for the wild type residue phenylalanine (F) at amino acid position 145 of SEQ ID NO: 3 (F145S), a substitution of histidine (H) for the wild type residue glutamine (Q) at amino acid position 109 of SEQ ID NO: 21 (Q109H), a substitution of cysteine (C) for the wild type residue phenylalanine (F) at amino acid position 622 of SEQ ID NO: 21 (F622C), a substitution of arginine (R) for the wild type residue glycine (G) at amino acid position 135 of SEQ ID NO: 3 (G135R), a substitution of glutamine (Q) for the wild type residue arginine (R) at amino acid position 168 of SEQ ID NO: 5 (R168Q), a substitution of arginine (R) for the wild type residue glycine (G) at amino acid position 159 of SEQ ID NO: 3 (G159R), a substitution of cysteine (C) for the wild type residue arginine (R) at amino acid position 310 of SEQ ID NO: 5 (R310C), a substitution of histidine (H) for the wild type residue arginine (R) at amino acid position 561 of SEQ ID NO: 3 (R561H), a substitution of histidine (H) for the wild type residue arginine (R) at amino acid position 634 of SEQ ID NO: 21 (R634H), a substitution of arginine (R) for the wild type residue glycine (G) at amino acid position 660 of SEQ ID NO: 3 (G660R), a substitution of cysteine (C) for the wild type residue tyrosine (Y) at amino acid position 181 of SEQ ID NO: 3 (Y181C), a substitution of arginine (R) for the wild type residue histidine (H) at amino acid position 297 of SEQ ID NO: 3 (H297R), a substitution of serine (S) for the wild type residue cysteine (C) at amino acid position

612 of SEQ ID NO: 21 (C612S), a substitution of tyrosine (Y) for the wild type residue histidine (H) at amino acid position 694 of SEQ ID NO: 3 (H694Y), a substitution of alanine (A) for the wild type residue aspartic acid (D) at amino acid position 664 of SEQ ID NO: 3 (D664A), a substitution of threonine (T) for the wild type residue isoleucine (I) at amino acid position 150 of SEQ ID NO: 3 (I150T), a substitution of arginine (R) for the wild type residue isoleucine (I) at amino acid position 264 of SEQ ID NO: 3 (I264R), a substitution of leucine (L) for the wild type residue proline (P) at amino acid position 636 of SEQ ID NO: 3 (P636L), a substitution of threonine (T) for the wild type residue isoleucine (I) at amino acid position 713 of SEQ ID NO: 3 (I713T), a substitution of proline (P) for the wild type residue glutamine (Q) at amino acid position 501 of SEQ ID NO: 5 (Q501P), a substitution of glutamine (Q) for the wild type residue lysine (K) at amino acid position 243 of SEQ ID NO: 3 (K243Q), a substitution of aspartic acid (D) for the wild type residue glutamic acid (E) at amino acid position 130 of SEQ ID NO: 5 (E130D), a substitution of glycine (G) for the wild type residue arginine (R) at amino acid position 509 of SEQ ID NO: 3 (R509G), a substitution of histidine (H) for the wild type residue arginine (R) at amino acid position 566 of SEQ ID NO: 3 (R566H), a substitution of histidine (H) for the wild type residue aspartic acid (D) at amino acid position 677 of SEQ ID NO: 3 (D677H), a substitution of asparagine (N) for the wild type residue lysine (K) at amino acid position 466 of SEQ ID NO: 5 (K466N), a substitution of histidine (H) for the wild type residue arginine (R) at amino acid position 78 of SEQ ID NO: 3 (R78H), a substitution of methionine (M) for the wild type residue lysine (K) at amino acid position 1 of SEQ ID NO: 6 (K6M), a substitution of leucine (L) for the wild type residue serine (S) at amino acid position 538 of SEQ ID NO: 3 (S538L), a substitution of glutamine (Q) for the wild type residue leucine (L) at amino acid position 149 of SEQ ID NO: 3 (L149Q), a substitution of valine (V) for the wild type residue leucine (L) at amino acid position 252 of SEQ ID NO: 3 (L252V), a substitution of valine (V) for the wild type residue leucine (L) at amino acid position 674 of SEQ ID NO: 3 (L674V), a substitution of valine (V) for the wild type residue alanine (A) at amino acid position 656 of SEQ ID NO: 3 (A656V), a substitution of aspartic acid (D) for the wild type residue alanine (A) at amino acid position 731 of SEQ ID NO: 3 (Y731D), a substitution of threonine (T) for the wild type residue alanine (A) at amino acid position 345 of SEQ ID NO: 3 (A345T), a substitution of aspartic acid (D) for the wild type residue alanine (A) at amino acid position 244 of SEQ ID NO: 3 (Y244D), a substitution of tryptophan (W) for the wild type residue cysteine (C) at amino acid position 576 of SEQ ID NO: 3 (C576W), a substitution of lysine (K) for the wild type residue asparagine (N) at amino acid position 640 of SEQ ID NO: 3 (N640K), a substitution of lysine (K) for the wild type residue asparagine (N) at amino acid position 675 of SEQ ID NO: 3 (N675K), a substitution of tyrosine (Y) for the wild type residue aspartic acid (D) at amino acid position 579 of SEQ ID NO: 21 (D579Y), a substitution of isoleucine (I) for the wild type residue asparagine (N) at amino acid position 693 of SEQ ID NO: 3 (N693I), and a substitution of lysine (K) for the wild type residue asparagine (N) at amino acid position 693 of SEQ ID NO: 3 (N693K).

The mutant EZH2 of the present invention may include a frameshift at amino acid position 730, 391, 461, 441, 235, 254, 564, 662, 715, 405, 685, 64, 73, 656, 718, 374, 592, 505, 730, or 363 of SEQ ID NO: 3, 5 or 21 or the corresponding nucleotide position of the nucleic acid sequence encoding SEQ ID NO: 3, 5, or 21. The mutant EZH2 may also include an insertion of a glutamic acid (E) between amino acid positions 148 and 149 of SEQ ID NO: 3, 5 or 21. Another example of EZH2 mutation is a deletion of glutamic acid (E) and leucine (L) at amino acid positions 148 and 149 of SEQ ID NO: 3, 5 or 21. The mutant EZH2 may further comprise a nonsense mutation at amino acid position 733, 25, 317, 62, 553, 328, 58, 207, 123, 63, 137, or 60 of SEQ ID NO: 3, 5 or 21.

It has also been unexpectedly discovered that the wild-type (WT) EZH2 enzyme displays greatest catalytic efficiency (kcat/K) for the zero- to mono-methylation reaction of H3-K27 and lesser efficiency for subsequent (mono- to di- and di- to tri-methylation) reactions; whereas, in stark contrast, the disease-associated Y641 mutations display very limited ability to perform the first methylation reaction but have enhanced catalytic efficiency for the subsequent reactions relative to wild-type enzyme. These results imply that the malignant phenotype of disease exploits the combined activities of a H3-K27 mono-methylating enzyme (PRC2 containing WT EZH2 or EZH1) together with PRC2 containing mutant EZH2 for augmented conversion of H3-K27 to the tri-methylated form (H3-K27me3). Therefore, an aspect of the present disclosure relates to inhibiting the activity of EZH2, including certain mutant forms of EZH2. The present disclosure also relates to inhibiting selectively the activity of certain mutant forms of EZH2.

While not intending to be bound by any one theory, it is hypothesized that the mutation of EZH2 in its substrate pocket domain may facilitate multiple rounds of H3-K27 methylation by impacting the H-bonding pattern and/or steric crowding in the active site of the enzyme-bisubstrate ternary complex, affecting the formation of a proper water channel for deprotonation of the reacting lysine.

Human EZH2 nucleic acids and polypeptides have previously been described. See, e.g., Chen et al. (1996) Genomics 38:30-7 [746 amino acids]; Swiss-Prot Accession No. Q15910 [746 amino acids]; GenBank Accession Nos. NM_004456 and NP_004447 (isoform a [751 amino acids]); and GenBank Accession Nos. NM_152998 and NP_694543 (isoform b [707 amino acids]).

| Amino acid sequence of human EZH2 (Swiss-Prot Accession No. Q15910) (SEQ ID NO: 1) |
| --- |

(continued)

| |
|---|
| MGQTGKKSEKGPVCWRKRVKSEYMRLRQLKRFRRADEVKSMFSSNRQKILERTEILNQEW<br>KQRRIQPVHILTSVSSLRGTRECSVTSDLDFPTQVIPLKTLNAVASVPIMYSWSPLQQNF<br>MVEDETVLHNIPYMGDEVLDQDGTFIEELIKNYDGKVHGDRECGFINDEIFVELVNALGQ<br>YNDDDDDDDGDDPEEREEKQKDLEDHRDDKESRPPRKFPSDKIFEAISSMFPDKGTAEEL<br>KEKYKELTEQQLPGALPPECTPNIDGPNAKSVQREQSLHSFHTLFCRRCFKYDCFLHPFH<br>ATPNTYKRKNTETALDNKPCGPQCYQHLEGAKEFAAALTAERIKTPPKRPGGRRRGRLPN<br>NSSRPSTPTINVLESKDTDSDREAGTETGGENNDKEEEEKKDETSSSSEANSRCQTPIKM<br>KPNIEPPENVEWSGAEASMFRVLIGTYYDNFCAIARLIGTKTCRQVYEFRVKESSIIAPA<br>PAEDVDTPPRKKKRKHRLWAAHCRKIQLKKDGSSNHVYNYQPCDHPRQPCDSSCPCVIAQ<br>NFCEKFCQCSSECQNRFPGCRCKAQCNTKQCPCYLAVRECDPDLCLTCGAADHWDSKNVS<br>CKNCSIQRGSKKHLLLAPSDVAGWGIFIKDPVQKNEFISEYCGEIISQDEADRRGKVYDK<br>YMCSFLFNLNNDFVVDATRKGNKIRFANHSVNPNCYAKVMMVNGDHRIGIFAKRAIQTGE<br>ELFFDYRYSQADALKYVGIEREMEIP |

| mRNA sequence of human EZH2, transcript variant 1 (GenBank Accession No. NM_004456) (SEQ ID NO: 2) |
|---|
| ggcggcgcttgattgggctgggggggccaaataaaagcgatggcgattgggctgccgcgt<br>ttggcgctcggtccggtcgcgtccgacacccggtgggactcagaaggcagtggagccccg<br>gcggcggcggcggcggcgcgcgggggcgacgcgcgggaacaacgcgagtcggcgcgcggg<br>acgaagaataatcatgggccagactgggaagaaatctgagaagggaccagtttgttggcg<br>gaagcgtgtaaaatcagagtacatgcgactgagacagctcaagaggttcagacgagctga<br>tgaagtaaagagtatgtttagttccaatcgtcagaaaattttggaaagaacggaaatctt<br>aaaccaagaatggaaacagcgaaggatacagcctgtgcacatcctgacttctgtgagctc<br>attgcgcgggactagggagtgttcggtgaccagtgacttggattttccaacacaagtcat<br>cccattaaagactctgaatgcagttgcttcagtacccataatgtattcttggtctcccct<br>acagcagaattttatggtggaagatgaaactgttttacataacattccttatatgggaga<br>tgaagttttagatcaggatggtactttcattgaagaactaataaaaaattatgatgggaa<br>agtacacggggatagagaatgtgggtttataaatgatgaaatttttgtggagttggtgaa<br>tgcccttggtcaatataatgatgatgacgatgatgatgatggagacgatcctgaagaaag |

(continued)

agaagaaaagcagaaagatctggaggatcaccgagatgataaagaaagccgcccacctcg
gaaatttccttctgataaaatttttgaagccatttcctcaatgtttccagataagggcac
agcagaagaactaaaggaaaaatataaagaactcaccgaacagcagctcccaggcgcact
tcctcctgaatgtacccccaacatagatggaccaaatgctaaatctgttcagagagagca
aagcttacactcctttcatacgcttttctgtaggcgatgttttaaatatgactgcttcct
acatcgtaagtgcaattattcttttcatgcaacacccaacacttataagcggaagaacac
agaaacagctctagacaacaaaccttgtggaccacagtgttaccagcatttggagggagc
aaaggagtttgctgctgctctcaccgctgagcggataaagaccccaccaaaacgtccagg
aggccgcagaagaggacggcttcccaataacagtagcaggcccagcacccccaccattaa
tgtgctggaatcaaaggatacagacagtgatagggaagcagggactgaaacgggggaga
gaacaatgataaagaagaagaagagaagaaagatgaaacttcgagctcctctgaagcaaa
ttctcggtgtcaaacaccaataaagatgaagccaaatattgaacctcctgagaatgtgga
gtggagtggtgctgaagcctcaatgtttagagtcctcattggcacttactatgacaattt
ctgtgccattgctaggttaattgggaccaaaacatgtagacaggtgtatgagtttagagt
caaagaatctagcatcatagctccagctcccgctgaggatgtggatactcctccaaggaa
aaagaagaggaaacaccggttgtgggctgcacactgcagaaagatacagctgaaaaagga
cggctcctctaaccatgtttacaactatcaaccctgtgatcatccacggcagccttgtga
cagttcgtgcccttgtgtgatagcacaaaatttttgtgaaaagttttgtcaatgtagttc
agagtgtcaaaaccgctttccgggatgccgctgcaaagcacagtgcaacaccaagcagtg
cccgtgctacctggctgtccgagagtgtgaccctgacctctgtcttacttgtggagccgc
tgaccattgggacagtaaaaatgtgtcctgcaagaactgcagtattcagcggggctccaa
aaagcatctattgctggcaccatctgacgtggcaggctggggatttttatcaaagatcc
tgtgcagaaaaatgaattcatctcagaatactgtggagagattatttctcaagatgaagc
tgacagaagagggaaagtgtatgataaatacatgtgcagctttctgttcaacttgaacaa
tgattttgtggtggatgcaacccgcaagggtaacaaaattcgttttgcaaatcattcggt
aaatccaaactgctatgcaaaagttatgatggttaacggtgatcacaggataggtatttt
tgccaagagagccatccagactggcgaagagctgtttttttgattacagatacagccaggc
tgatgccctgaagtatgtcggcatcgaaagagaaatggaaatcccttgacatctgctacc
tcctcccccctcctctgaaacagctgccttagcttcaggaacctcgagtactgtgggcaa
tttagaaaaagaacatgcagtttgaaattctgaatttgcaaagtactgtaagaataattt
atagtaatgagtttaaaaatcaactttttattgccttctcaccagctgcaaagtgttttg
taccagtgaatttttgcaataatgcagtatggtacatttttcaactttgaataaagaata
cttgaacttgtccttgttgaatc

Full amino acid of EZH2, isoform a (GenBank Accession No. NP_004447) (SEQ ID NO: 3)

MGQTGKKSEKGPVCWRKRVKSEYMRLRQLKRFRRADEVKSMFSSNRQKILERTEILNQEW
KQRRIQPVHILTSVSSLRGTRECSVTSDLDFPTQVIPLKTLNAVASVPIMYSWSPLQQNF
MVEDETVLHNIPYMGDEVLDQDGTFIEELIKNYDGKVHGDRECGFINDEIFVELVNALGQ
YNDDDDDDDGDDPEEREEKQKDLEDHRDDKESRPPRKFPSDKIFEAISSMFPDKGTAEEL
KEKYKELTEQQLPGALPPECTPNIDGPNAKSVQREQSLHSFHTLFCRRCFKYDCFLHRKC
NYSFHATPNTYKRKNTETALDNKPCGPQCYQHLEGAKEFAAALTAERIKTPPKRPGGRRR
GRLPNNSSRPSTPTINVLESKDTDSDREAGTETGGENNDKEEEEKKDETSSSSEANSRCQ
TPIKMKPNIEPPENVEWSGAEASMFRVLIGTYYDNFCAIARLIGTKTCRQVYEFRVKESS
IIAPAPAEDVDTPPRKKKRKHRLWAAHCRKIQLKKDGSSNHVYNYQPCDHPRQPCDSSCP
CVIAQNFCEKFCQCSSECQNRFPGCRCKAQCNTKQCPCYLAVRECDPDLCLTCGAADHWD
SKNVSCKNCSIQRGSKKHLLLAPSDVAGWGIFIKDPVQKNEFISEYCGEIISQDEADRRG
KVYDKYMCSFLFNLNNDFVVDATRKGNKIRFANHSVNPNCYAKVMMVNGDHRIGIFAKRA
IQTGEELFFDYRYSQADALKYVGIEREMEIP

mRNA sequence of human EZH2, transcript variant 2 (GenBank Accession No. NM_152998) (SEQ ID NO: 4)

(continued)

| |
|---|
| ggcggcgcttgattgggctgggggggccaaataaaagcgatggcgattgggctgccgcgt ttggcgctcggtccggtcgcgtccgacacccggtgggactcagaaggcagtggagccccg gcggcggcggcggcggcgcgcgggggcgacgcgcgggaacaacgcgagtcggcgcgcggg acgaagaataatcatgggccagactgggaagaaatctgagaagggaccagtttgttggcg |
| gaagcgtgtaaaatcagagtacatgcgactgagacagctcaagaggttcagacgagctga tgaagtaaagagtatgtttagttccaatcgtcagaaaattttggaaagaacggaaatctt aaaccaagaatggaaacagcgaaggatacagcctgtgcacatcctgacttctgtgagctc attgcgcgggactagggaggtggaagatgaaactgttttacataacattccttatatggg agatgaagttttagatcaggatggtactttcattgaagaactaataaaaaattatgatgg gaaagtacacggggatagagaatgtgggtttataaatgatgaaatttttgtggagttggt gaatgcccttggtcaatataatgatgatgacgatgatgatgatggagacgatcctgaaga aagagaagaaaagcagaaagatctggaggatcaccgagatgataaagaaagccgcccacc tcggaaatttccttctgataaaatttttgaagccatttcctcaatgtttccagataaggg cacagcagaagaactaaaggaaaaatataaagaactcaccgaacagcagctcccaggcgc acttcctcctgaatgtacccccaacatagatggaccaaatgctaaatctgttcagagaga gcaaagcttacactcctttcatacgcttttctgtaggcgatgttttaaatatgactgctt cctacatccttttcatgcaacacccaacacttataagcggaagaacacagaaacagctct agacaacaaaccttgtggaccacagtgttaccagcatttggagggagcaaaggagtttgc tgctgctctcaccgctgagcggataaagaccccaccaaaacgtccaggaggccgcagaag aggacggcttcccaataacagtagcaggcccagcacccccaccattaatgtgctggaatc aaaggatacagacagtgatagggaagcagggactgaaacgggggggagagaacaatgataa agaagaagaagagaagaaagatgaaacttcgagctcctctgaagcaaattctcggtgtca aacaccaataaagatgaagccaaatattgaacctcctgagaatgtggagtggagtggtgc tgaagcctcaatgtttagagtcctcattggcacttactatgacaatttctgtgccattgc taggttaattgggaccaaaacatgtagacaggtgtatgagtttagagtcaaagaatctag catcatagctccagctcccgctgaggatgtggatactcctccaaggaaaaagaagaggaa acaccggttgtgggctgcacactgcagaaagatacagctgaaaaaggacggctcctctaa ccatgtttacaactatcaaccctgtgatcatccacggcagccttgtgacagttcgtgccc ttgtgtgatagcacaaaatttttgtgaaaagttttgtcaatgtagttcagagtgtcaaaa ccgctttccgggatgccgctgcaaagcacagtgcaacaccaagcagtgcccgtgctacct ggctgtccgagagtgtgaccctgacctctgtcttacttgtggagccgctgaccattggga cagtaaaaatgtgtcctgcaagaactgcagtattcagcggggctccaaaaagcatctatt gctggcaccatctgacgtggcaggctgggggatttttatcaaagatcctgtgcagaaaaa tgaattcatctcagaatactgtggagagattatttctcaagatgaagctgacagaagagg gaaagtgtatgataaatacatgtgcagctttctgttcaacttgaacaatgattttgtggt ggatgcaacccgcaagggtaacaaaattcgttttgcaaatcattcggtaaatccaaactg ctatgcaaaagttatgatggttaacggtgatcacaggataggtatttttgccaagagagc catccagactggcgaagagctgtttttgattacagatacagccaggctgatgccctgaa gtatgtcggcatcgaaagagaaatggaaatcccttgacatctgctacctcctcccccctc ctctgaaacagctgccttagcttcaggaacctcgagtactgtgggcaatttagaaaaaga acatgcagtttgaaattctgaatttgcaaagtactgtaagaataatttatagtaatgagt ttaaaaatcaacttttattgccttctcaccagctgcaaagtgttttgtaccagtgaatt tttgcaataatgcagtatggtacatttttcaactttgaataaagaatacttgaacttgtc cttgttgaatc |

| |
|---|
| Full amino acid of EZH2, isoform b (GenBank Accession No. NP_694543) (SEQ ID NO: 5) |

(continued)

| |
|---|
| MGQTGKKSEKGPVCWRKRVKSEYMRLRQLKRFRRADEVKSMFSSNRQKIL<br>ERTEILNQEWKQRRIQPVHILTSVSSLRGTREVEDETVLHNIPYMGDEVL<br>DQDGTFIEELIKNYDGKVHGDRECGFINDEIFVELVNALGQYNDDDDDDD<br>GDDPEEREEKQKDLEDHRDDKESRPPRKFPSDKIFEAISSMFPDKGTAEE<br>LKEKYKELTEQQLPGALPPECTPNIDGPNAKSVQREQSLHSFHTLFCRRC<br>FKYDCFLHPFHATPNTYKRKNTETALDNKPCGPQCYQHLEGAKEFAAALT<br>AERIKTPPKRPGGRRRGRLPNNSSRPSTPTINVLESKDTDSDREAGTETG<br>GENNDKEEEEKKDETSSSSEANSRCQTPIKMKPNIEPPENVEWSGAEASM<br>FRVLIGTYYDNFCAIARLIGTKTCRQVYEFRVKESSIIAPAPAEDVDTPP<br>RKKKRKHRLWAAHCRKIQLKKDGSSNHVYNYQPCDHPRQPCDSSCPCVIA<br>QNFCEKFCQCSSECQNRFPGCRCKAQCNTKQCPCYLAVRECDPDLCLTCG<br>AADHWDSKNVSCKNCSIQRGSKKHLLLAPSDVAGWGIFIKDPVQKNEFIS<br>EYCGEIISQDEADRRGKVYDKYMCSFLFNLNNDFVVDATRKGNKIRFANH<br>SVNPNCYAKVMMVNGDHRIGIFAKRAIQTGEELFFDYRYSQADALKYVGI |
| EREMEIP |

| Full amino acid of EZH2, isoform e (GenBank Accession No. NP_001190178.1) (SEQ ID NO: 21) |
|---|
| MGQTGKKSEKGPVCWRKRVKSEYMRLRQLKRFRRADEVKSMFSSNRQKILERTEILNQEWKQRRIQPVHI<br>LTSCSVTSDLDFPTQVIPLKTLNAVASVPIMYSWSPLQQNFMVEDETVLHNIPYMGDEVLDQDGTFIEEL<br>IKNYDGKVHGDRECGFINDEIFVELVNALGQYNDDDDDDDGDDPEEREEKQKDLEDHRDDKESRPPRKFP<br>SDKIFEAISSMFPDKGTAEELKEKYKELTEQQLPGALPPECTPNIDGPNAKSVQREQSLHSFHTLFCRRC<br>FKYDCFLHPFHATPNTYKRKNTETALDNKPCGPQCYQHLEGAKEFAAALTAERIKTPPKRPGGRRRGRLP<br>NNSSRPSTPTINVLESKDTDSDREAGTETGGENNDKEEEEKKDETSSSSEANSRCQTPIKMKPNIEPPEN<br>VEWSGAEASMFRVLIGTYYDNFCAIARLIGTKTCRQVYEFRVKESSIIAPAPAEDVDTPPRKKKRKHRLW<br>AAHCRKIQLKKGQNRFPGCRCKAQCNTKQCPCYLAVRECDPDLCLTCGAADHWDSKNVSCKNCSIQRGSK<br>KHLLLAPSDVAGWGIFIKDPVQKNEFISEYCGEIISQDEADRRGKVYDKYMCSFLFNLNNDFVVDATRKG<br>NKIRFANHSVNPNCYAKVMMVNGDHRIGIFAKRAIQTGEELFFDYRYSQADALKYVGIEREMEIP |

| Homo sapiens enhancer of zeste homolog 2 (Drosophila) (EZH2), transcript variant 5, mRNA (GenBank Accession No. NM_001203249.1) (SEQ ID NO: 22) |
|---|

(continued)

| |
|---|
| GACGACGTTCGCGGCGGGGAACTCGGAGTAGCTTCGCCTCTGACGTTTCCCCACGACGCACCCCGAAATC<br>CCCCTGAGCTCCGGCGGTCGCGGGCTGCCCTCGCCGCCTGGTCTGGCTTTATGCTAAGTTTGAGGGAAGA<br>GTCGAGCTGCTCTGCTCTCTATTGATTGTGTTTCTGGAGGGCGTCCTGTTGAATTCCCACTTCATTGTGT<br>ACATCCCCTTCCGTTCCCCCCAAAAATCTGTGCCACAGGGTTACTTTTTGAAAGCGGGAGGAATCGAGAA<br>GCACGATCTTTTGGAAAACTTGGTGAACGCCTAAATAATCATGGGCCAGACTGGGAAGAAATCTGAGAAG<br>GGACCAGTTTGTTGGCGGAAGCGTGTAAAATCAGAGTACATGCGACTGAGACAGCTCAAGAGGTTCAGAC<br>GAGCTGATGAAGTAAAGAGTATGTTTAGTTCCAATCGTCAGAAAATTTTGGAAAGAACGGAAATCTTAAA<br>CCAAGAATGGAAACAGCGAAGGATACAGCCTGTGCACATCCTGACTTCTTGTTCGGTGACCAGTGACTTG<br>GATTTTCCAACACAAGTCATCCCATTAAAGACTCTGAATGCAGTTGCTTCAGTACCCATAATGTATTCTT<br>GGTCTCCCCTACAGCAGAATTTTATGGTGGAAGATGAAACTGTTTTACATAACATTCCTTATATGGGAGA<br>TGAAGTTTTAGATCAGGATGGTACTTTCATTGAAGAACTAATAAAAAATTATGATGGGAAAGTACACGGG<br>GATAGAGAATGTGGGTTTATAAATGATGAAATTTTTGTGGAGTTGGTGAATGCCCTTGGTCAATATAATG<br>ATGATGACGATGATGATGATGGAGACGATCCTGAAGAAAGAGAAGAAAAGCAGAAAGATCTGGAGGATCA<br>CCGAGATGATAAAGAAAGCCGCCCACCTCGGAAATTTCCTTCTGATAAAATTTTTGAAGCCATTTCCTCA<br>ATGTTTCCAGATAAGGGCACAGCAGAAGAACTAAAGGAAAAATATAAAGAACTCACCGAACAGCAGCTCC<br>CAGGCGCACTTCCTCCTGAATGTACCCCCAACATAGATGGACCAAATGCTAAATCTGTTCAGAGAGAGCA<br>AAGCTTACACTCCTTTCATACGCTTTTCTGTAGGCGATGTTTTAAATATGACTGCTTCCTACATCCTTTT<br>CATGCAACACCCAACACTTATAAGCGGAAGAACACAGAAACAGCTCTAGACAACAAACCTTGTGGACCAC<br>AGTGTTACCAGCATTTGGAGGGAGCAAAGGAGTTTGCTGCTGCTCTCACCGCTGAGCGGATAAAGACCCC<br>ACCAAAACGTCCAGGAGGCCGCAGAAGAGGACGGCTTCCCAATAACAGTAGCAGGCCCAGCACCCCCACC<br>ATTAATGTGCTGGAATCAAAGGATACAGACAGTGATAGGGAAGCAGGGACTGAAACGGGGGGAGAGAACA<br>ATGATAAAGAAGAAGAAGAGAAGAAAGATGAAACTTCGAGCTCCTCTGAAGCAAATTCTCGGTGTCAAAC<br>ACCAATAAAGATGAAGCCAAATATTGAACCTCCTGAGAATGTGGAGTGGAGTGGTGCTGAAGCCTCAATG<br>TTTAGAGTCCTCATTGGCACTTACTATGACAATTTCTGTGCCATTGCTAGGTTAATTGGGACCAAAACAT<br>GTAGACAGGTGTATGAGTTTAGAGTCAAAGAATCTAGCATCATAGCTCCAGCTCCCGCTGAGGATGTGGA<br>TACTCCTCCAAGGAAAAAGAAGAGGAAACACCGGTTGTGGGCTGCACACTGCAGAAAGATACAGCTGAAA<br>AAGGGTCAAAACCGCTTTCCGGGATGCCGCTGCAAAGCACAGTGCAACACCAAGCAGTGCCCGTGCTACC<br>TGGCTGTCCGAGAGTGTGACCCTGACCTCTGTCTTACTTGTGGAGCCGCTGACCATTGGGACAGTAAAAA<br>TGTGTCCTGCAAGAACTGCAGTATTCAGCGGGGCTCCAAAAAGCATCTATTGCTGGCACCATCTGACGTG<br>GCAGGCTGGGGGATTTTTATCAAAGATCCTGTGCAGAAAAATGAATTCATCTCAGAATACTGTGGAGAGA<br>TTATTTCTCAAGATGAAGCTGACAGAAGAGGGAAAGTGTATGATAAATACATGTGCAGCTTTCTGTTCAA<br>CTTGAACAATGATTTTGTGGTGGATGCAACCCGCAAGGGTAACAAAATTCGTTTTGCAAATCATTCGGTA<br>AATCCAAACTGCTATGCAAAAGTTATGATGGTTAACGGTGATCACAGGATAGGTATTTTTGCCAAGAGAG<br>CCATCCAGACTGGCGAAGAGCTGTTTTTTGATTACAGATACAGCCAGGCTGATGCCCTGAAGTATGTCGG<br>CATCGAAAGAGAAATGGAAATCCCTTGACATCTGCTACCTCCTCCCCCCTCCTCTGAAACAGCTGCCTTA<br>GCTTCAGGAACCTCGAGTACTGTGGGCAATTTAGAAAAAGAACATGCAGTTTGAAATTCTGAATTTGCAA<br>AGTACTGTAAGAATAATTTATAGTAATGAGTTTAAAAATCAACTTTTTATTGCCTTCTCACCAGCTGCAA<br>AGTGTTTTGTACCAGTGAATTTTTGCAATAATGCAGTATGGTACATTTTTCAACTTTGAATAAAGAATAC |
| TTGAACTTGTCCTTGTTGAATC |

A structure model of partial EZH2 protein based on the A chain of nuclear receptor binding SET domain protein 1 (NSD1) is provided below. This model corresponds to amino acid residues 533-732 of EZH2 sequence of SEQ ID NO: 1.

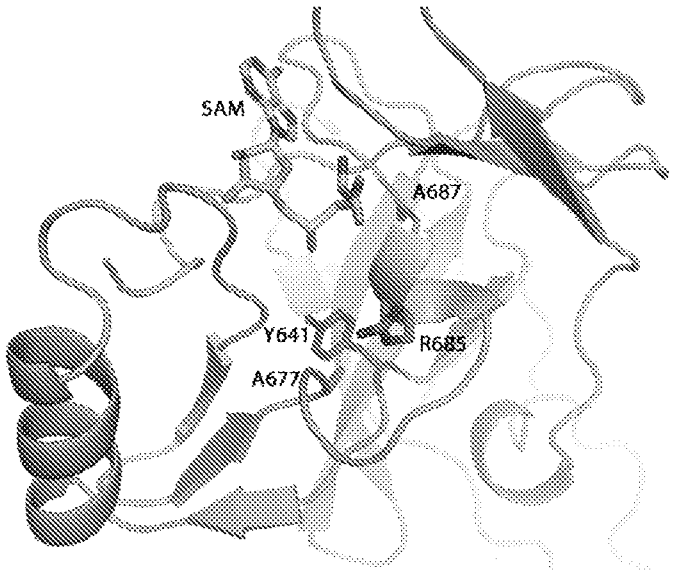


The corresponding amino acid sequence of this structure model is provided below. The residues in the substrate pocket domain are underlined. The residues in the SET domain are shown italic.

SCPCVIAQNFCEKFCQCSSECQNRFPGCRCKAQCNTKQCPCYLAVRECDPDLCLTCGAA DHWDSKNVSCKNCSIQRGSKK*HLLLAPSDVAGWGIFIKDPVQKNEFISEY*$^{641}$*CGEIISQDEA DRRGKVYDKYMCSFLFNLNNDFV*$^{674}$*VDA*$^{677}$*TRKGNKIR*$^{685}$*FA*$^{687}$*NHSVNPNCYAKVMMVNGD HRIGIFAKRAIQTGEELFFDY*RYSQAD (SEQ ID NO: 6)

The catalytic site of EZH2 is believed to reside in a conserved domain of the protein known as the SET domain. The amino acid sequence of the SET domain of EZH2 is provided by the following partial sequence spanning amino acid residues 613-726 of Swiss-Prot Accession No. Q15910 (SEQ ID NO: 1):

HLLLAPSDVAGWGIFIKDPVQKNEFISEYCGEIISQDEADRRGKVYDKYMCSFLFNLNNDFVVD ATRKGNKIRFANHSVNPNCYAKVMMVNGDHRIGIFAKRAIQTGEELFFDY (SEQ ID NO: 7).

The tyrosine (Y) residue shown underlined in SEQ ID NO: 7 is Tyr641 (Y641) in Swiss-Prot Accession No. Q15910 (SEQ ID NO: 1).

The SET domain of GenBank Accession No. NP_004447 (SEQ ID NO: 3) spans amino acid residues 618-731 and is identical to SEQ ID NO:6. The tyrosine residue corresponding to Y641 in Swiss-Prot Accession No. Q15910 shown underlined in SEQ ID NO: 7 is Tyr646 (Y646) in GenBank Accession No. NP_004447 (SEQ ID NO: 3).

The SET domain of GenBank Accession No. NP_694543 (SEQ ID NO: 5) spans amino acid residues 574-687 and is identical to SEQ ID NO: 7. The tyrosine residue corresponding to Y641 in Swiss-Prot Accession No. Q15910 shown underlined in SEQ ID NO: 7 is Tyr602 (Y602) in GenBank Accession No. NP_694543 (SEQ ID NO: 5).

The nucleotide sequence encoding the SET domain of GenBank Accession No. NP_004447 is

catctattgctggcaccatctgacgtggcaggctggggattttatcaaagatcctgtgcaga
aaaatgaattcatctcagaatactgtggagagattatttctcaagatgaagctgacagaagagg
gaaagtgtatgataaatacatgtgcagctttctgttcaacttgaacaatgattttgtggtggat
gcaacccgcaagggtaacaaaattcgttttgcaaatcattcggtaaatccaaactgctatgcaa
aagttatgatggttaacggtgatcacaggataggtatttttgccaagagagccatccagactgg
cgaagagctgtttttttgattac

(SEQ ID NO: 8),

where the codon encoding Y641 is shown underlined.

For purposes of this application, amino acid residue Y641 of human EZH2 is to be understood to refer to the tyrosine residue that is or corresponds to Y641 in Swiss-Prot Accession No. Q15910.

| Full amino acid sequence of Y641 mutant EZH2 (SEQ ID NO: 9) |
|---|
| MGQTGKKSEKGPVCWRKRVKSEYMRLRQLKRFRRADEVKSMFSSNRQKILERTEILNQEW<br>KQRRIQPVHILTSVSSLRGTRECSVTSDLDFPTQVIPLKTLNAVASVPIMYSWSPLQQNF<br>MVEDETVLHNIPYMGDEVLDQDGTFIEELIKNYDGKVHGDRECGFINDEIFVELVNALGQ<br>YNDDDDDDDGDDPEEREEKQKDLEDHRDDKESRPPRKFPSDKIFEAISSMFPDKGTAEEL<br>KEKYKELTEQQLPGALPPECTPNIDGPNAKSVQREQSLHSFHTLFCRRCFKYDCFLHPFH<br>ATPNTYKRKNTETALDNKPCGPQCYQHLEGAKEFAAALTAERIKTPPKRPGGRRRGRLPN<br>NSSRPSTPTINVLESKDTDSDREAGTETGGENNDKEEEEKKDETSSSSEANSRCQTPIKM<br>KPNIEPPENVEWSGAEASMFRVLIGTYYDNFCAIARLIGTKTCRQVYEFRVKESSIIAPA<br>PAEDVDTPPRKKKRKHRLWAAHCRKIQLKKDGSSNHVYNYQPCDHPRQPCDSSCPCVIAQ<br>NFCEKFCQCSSECQNRFPGCRCKAQCNTKQCPCYLAVRECDPDLCLTCGAADHWDSKNVS<br>CKNCSIQRGSKKHLLLAPSDVAGWGIFIKDPVQKNEFISEXCGEIISQDEADRRGKVYDK<br>YMCSFLFNLNNDFVVDATRKGNKIRFANHSVNPNCYAKVMMVNGDHRIGIFAKRAIQTGE<br>ELFFDYRYSQADALKYVGIEREMEIP |
| Wherein x can be any amino acid residue other than tyrosine (Y) |

Also for purposes of this application, a Y641 mutant of human EZH2, and, equivalently, a Y641 mutant of EZH2, is to be understood to refer to a human EZH2 in which the amino acid residue corresponding to Y641 of wild-type human EZH2 is substituted by an amino acid residue other than tyrosine.

In one embodiment the amino acid sequence of a Y641 mutant of EZH2 differs from the amino acid sequence of wild-type human EZH2 only by substitution of a single amino acid residue corresponding to Y641 of wild-type human EZH2 by an amino acid residue other than tyrosine.

In one embodiment the amino acid sequence of a Y641 mutant of EZH2 differs from the amino acid sequence of wild-type human EZH2 only by substitution of phenylalanine (F) for the single amino acid residue corresponding to Y641 of wild-type human EZH2. The Y641 mutant of EZH2 according to this embodiment is referred to herein as a Y641F mutant or, equivalently, Y641F.

| Y641F (SEQ ID NO: 10) |
|---|

(continued)

| |
|---|
| MGQTGKKSEKGPVCWRKRVKSEYMRLRQLKRFRRADEVKSMFSSNRQKILERTEILNQEW<br>KQRRIQPVHILTSVSSLRGTRECSVTSDLDFPTQVIPLKTLNAVASVPIMYSWSPLQQNF<br>MVEDETVLHNIPYMGDEVLDQDGTFIEELIKNYDGKVHGDRECGFINDEIFVELVNALGQ<br>YNDDDDDDDGDDPEEREEKQKDLEDHRDDKESRPPRKFPSDKIFEAISSMFPDKGTAEEL<br>KEKYKELTEQQLPGALPPECTPNIDGPNAKSVQREQSLHSFHTLFCRRCFKYDCFLHPFH<br>ATPNTYKRKNTETALDNKPCGPQCYQHLEGAKEFAAALTAERIKTPPKRPGGRRRGRLPN<br>NSSRPSTPTINVLESKDTDSDREAGTETGGENNDKEEEEKKDETSSSSEANSRCQTPIKM<br>KPNIEPPENVEWSGAEASMFRVLIGTYYDNFCAIARLIGTKTCRQVYEFRVKESSIIAPA<br>PAEDVDTPPRKKKRKHRLWAAHCRKIQLKKDGSSNHVYNYQPCDHPRQPCDSSCPCVIAQ<br>NFCEKFCQCSSECQNRFPGCRCKAQCNTKQCPCYLAVRECDPDLCLTCGAADHWDSKNVS<br>CKNCSIQRGSKKHLLLAPSDVAGWGIFIKDPVQKNEFISEFCGEIISQDEADRRGKVYDK<br>YMCSFLFNLNNDFVVDATRKGNKIRFANHSVNPNCYAKVMMVNGDHRIGIFAKRAIQTGE<br>ELFFDYRYSQADALKYVGIEREMEIP |

In one embodiment the amino acid sequence of a Y641 mutant of EZH2 differs from the amino acid sequence of wild-type human EZH2 only by substitution of histidine (H) for the single amino acid residue corresponding to Y641 of wild-type human EZH2. The Y641 mutant of EZH2 according to this embodiment is referred to herein as a Y641H mutant or, equivalently, Y641H.

| Y641H (SEQ ID NO: 11) |
|---|
| MGQTGKKSEKGPVCWRKRVKSEYMRLRQLKRFRRADEVKSMFSSNRQKILERTEILNQEW<br>KQRRIQPVHILTSVSSLRGTRECSVTSDLDFPTQVIPLKTLNAVASVPIMYSWSPLQQNF<br>MVEDETVLHNIPYMGDEVLDQDGTFIEELIKNYDGKVHGDRECGFINDEIFVELVNALGQ<br>YNDDDDDDDGDDPEEREEKQKDLEDHRDDKESRPPRKFPSDKIFEAISSMFPDKGTAEEL<br>KEKYKELTEQQLPGALPPECTPNIDGPNAKSVQREQSLHSFHTLFCRRCFKYDCFLHPFH<br>ATPNTYKRKNTETALDNKPCGPQCYQHLEGAKEFAAALTAERIKTPPKRPGGRRRGRLPN<br>NSSRPSTPTINVLESKDTDSDREAGTETGGENNDKEEEEKKDETSSSSEANSRCQTPIKM<br>KPNIEPPENVEWSGAEASMFRVLIGTYYDNFCAIARLIGTKTCRQVYEFRVKESSIIAPA<br>PAEDVDTPPRKKKRKHRLWAAHCRKIQLKKDGSSNHVYNYQPCDHPRQPCDSSCPCVIAQ<br>NFCEKFCQCSSECQNRFPGCRCKAQCNTKQCPCYLAVRECDPDLCLTCGAADHWDSKNVS<br>CKNCSIQRGSKKHLLLAPSDVAGWGIFIKDPVQKNEFISEHCGEIISQDEADRRGKVYDK<br>YMCSFLFNLNNDFVVDATRKGNKIRFANHSVNPNCYAKVMMVNGDHRIGIFAKRAIQTGE<br>ELFFDYRYSQADALKYVGIEREMEIP |

In one embodiment the amino acid sequence of a Y641 mutant of EZH2 differs from the amino acid sequence of wild-type human EZH2 only by substitution of asparagine (N) for the single amino acid residue corresponding to Y641 of wild-type human EZH2. The Y641 mutant of EZH2 according to this embodiment is referred to herein as a Y641N mutant or, equivalently, Y641N.

| Y641N (SEQ ID NO: 12) |
|---|
| MGQTGKKSEKGPVCWRKRVKSEYMRLRQLKRFRRADEVKSMFSSNRQKILERTEILNQEW<br>KQRRIQPVHILTSVSSLRGTRECSVTSDLDFPTQVIPLKTLNAVASVPIMYSWSPLQQNF<br>MVEDETVLHNIPYMGDEVLDQDGTFIEELIKNYDGKVHGDRECGFINDEIFVELVNALGQ<br>YNDDDDDDDGDDPEEREEKQKDLEDHRDDKESRPPRKFPSDKIFEAISSMFPDKGTAEEL<br>KEKYKELTEQQLPGALPPECTPNIDGPNAKSVQREQSLHSFHTLFCRRCFKYDCFLHPFH<br>ATPNTYKRKNTETALDNKPCGPQCYQHLEGAKEFAAALTAERIKTPPKRPGGRRRGRLPN<br>NSSRPSTPTINVLESKDTDSDREAGTETGGENNDKEEEEKKDETSSSSEANSRCQTPIKM<br>KPNIEPPENVEWSGAEASMFRVLIGTYYDNFCAIARLIGTKTCRQVYEFRVKESSIIAPA<br>PAEDVDTPPRKKKRKHRLWAAHCRKIQLKKDGSSNHVYNYQPCDHPRQPCDSSCPCVIAQ<br>NFCEKFCQCSSECQNRFPGCRCKAQCNTKQCPCYLAVRECDPDLCLTCGAADHWDSKNVS<br>CKNCSIQRGSKKHLLLAPSDVAGWGIFIKDPVQKNEFISENCGEIISQDEADRRGKVYDK<br>YMCSFLFNLNNDFVVDATRKGNKIRFANHSVNPNCYAKVMMVNGDHRIGIFAKRAIQTGE<br>ELFFDYRYSQADALKYVGIEREMEIP |

In one embodiment the amino acid sequence of a Y641 mutant of EZH2 differs from the amino acid sequence of wild-type human EZH2 only by substitution of serine (S) for the single amino acid residue corresponding to Y641 of wild-type human EZH2. The Y641 mutant of EZH2 according to this embodiment is referred to herein as a Y641S mutant or, equivalently, Y641S.

| Y641S (SEQ ID NO: 13) |
|---|
| MGQTGKKSEKGPVCWRKRVKSEYMRLRQLKRFRRADEVKSMFSSNRQKILERTEILNQEW<br>KQRRIQPVHILTSVSSLRGTRECSVTSDLDFPTQVIPLKTLNAVASVPIMYSWSPLQQNF<br>MVEDETVLHNIPYMGDEVLDQDGTFIEELIKNYDGKVHGDRECGFINDEIFVELVNALGQ<br>YNDDDDDDDGDDPEEREEKQKDLEDHRDDKESRPPRKFPSDKIFEAISSMFPDKGTAEEL |
| KEKYKELTEQQLPGALPPECTPNIDGPNAKSVQREQSLHSFHTLFCRRCFKYDCFLHPFH<br>ATPNTYKRKNTETALDNKPCGPQCYQHLEGAKEFAAALTAERIKTPPKRPGGRRRGRLPN<br>NSSRPSTPTINVLESKDTDSDREAGTETGGENNDKEEEEKKDETSSSSEANSRCQTPIKM<br>KPNIEPPENVEWSGAEASMFRVLIGTYYDNFCAIARLIGTKTCRQVYEFRVKESSIIAPA<br>PAEDVDTPPRKKKRKHRLWAAHCRKIQLKKDGSSNHVYNYQPCDHPRQPCDSSCPCVIAQ<br>NFCEKFCQCSSECQNRFPGCRCKAQCNTKQCPCYLAVRECDPDLCLTCGAADHWDSKNVS<br>CKNCSIQRGSKKHLLLAPSDVAGWGIFIKDPVQKNEFISESCGEIISQDEADRRGKVYDK<br>YMCSFLFNLNNDFVVDATRKGNKIRFANHSVNPNCYAKVMMVNGDHRIGIFAKRAIQTGE<br>ELFFDYRYSQADALKYVGIEREMEIP |

In one embodiment the amino acid sequence of a Y641 mutant of EZH2 differs from the amino acid sequence of wild-type human EZH2 only by substitution of cysteine (C) for the single amino acid residue corresponding to Y641 of wild-type human EZH2. The Y641 mutant of EZH2 according to this embodiment is referred to herein as a Y641C mutant or, equivalently, Y641C.

| Y641C (SEQ ID NO: 14) |
|---|
| MGQTGKKSEKGPVCWRKRVKSEYMRLRQLKRFRRADEVKSMFSSNRQKILERTEILNQEW<br>KQRRIQPVHILTSVSSLRGTRECSVTSDLDFPTQVIPLKTLNAVASVPIMYSWSPLQQNF<br>MVEDETVLHNIPYMGDEVLDQDGTFIEELIKNYDGKVHGDRECGFINDEIFVELVNALGQ<br>YNDDDDDDDGDDPEEREEKQKDLEDHRDDKESRPPRKFPSDKIFEAISSMFPDKGTAEEL<br>KEKYKELTEQQLPGALPPECTPNIDGPNAKSVQREQSLHSFHTLFCRRCFKYDCFLHPFH<br>ATPNTYKRKNTETALDNKPCGPQCYQHLEGAKEFAAALTAERIKTPPKRPGGRRRGRLPN<br>NSSRPSTPTINVLESKDTDSDREAGTETGGENNDKEEEEKKDETSSSSEANSRCQTPIKM<br>KPNIEPPENVEWSGAEASMFRVLIGTYYDNFCAIARLIGTKTCRQVYEFRVKESSIIAPA<br>PAEDVDTPPRKKKRKHRLWAAHCRKIQLKKDGSSNHVYNYQPCDHPRQPCDSSCPCVIAQ<br>NFCEKFCQCSSECQNRFPGCRCKAQCNTKQCPCYLAVRECDPDLCLTCGAADHWDSKNVS<br>CKNCSIQRGSKKHLLLAPSDVAGWGIFIKDPVQKNEFISECCGEIISQDEADRRGKVYDK<br>YMCSFLFNLNNDFVVDATRKGNKIRFANHSVNPNCYAKVMMVNGDHRIGIFAKRAIQTGE<br>ELFFDYRYSQADALKYVGIEREMEIP |

In one embodiment the amino acid sequence of a A677 mutant of EZH2 differs from the amino acid sequence of wild-type human EZH2 only by substitution of a non-alanine amino acid, preferably glycine (G) for the single amino acid residue corresponding to A677 of wild-type human EZH2. The A677 mutant of EZH2 according to this embodiment is referred to herein as an A677 mutant, and preferably an A677G mutant or, equivalently, A677G.

| A677 (SEQ ID NO: 15) |
|---|
| MGQTGKKSEKGPVCWRKRVKSEYMRLRQLKRFRRADEVKSMFSSNRQKILERTEILNQEW<br>KQRRIQPVHILTSVSSLRGTRECSVTSDLDFPTQVIPLKTLNAVASVPIMYSWSPLQQNF<br>MVEDETVLHNIPYMGDEVLDQDGTFIEELIKNYDGKVHGDRECGFINDEIFVELVNALGQ<br>YNDDDDDDDGDDPEEREEKQKDLEDHRDDKESRPPRKFPSDKIFEAISSMFPDKGTAEEL<br>KEKYKELTEQQLPGALPPECTPNIDGPNAKSVQREQSLHSFHTLFCRRCFKYDCFLHPFH<br>ATPNTYKRKNTETALDNKPCGPQCYQHLEGAKEFAAALTAERIKTPPKRPGGRRRGRLPN<br>NSSRPSTPTINVLESKDTDSDREAGTETGGENNDKEEEEKKDETSSSSEANSRCQTPIKM<br>KPNIEPPENVEWSGAEASMFRVLIGTYYDNFCAIARLIGTKTCRQVYEFRVKESSIIAPA |

(continued)

| |
|---|
| PAEDVDTPPRKKKRKHRLWAAHCRKIQLKKDGSSNHVYNYQPCDHPRQPCDSSCPCVIAQ NFCEKFCQCSSECQNRFPGCRCKAQCNTKQCPCYLAVRECDPDLCLTCGAADHWDSKNVS CKNCSIQRGSKKHLLLAPSDVAGWGIFIKDPVQKNEFISEYCGEIISQDEADRRGKVYDK YMCSFLFNLNNDFVVDXTRKGNKIRFANHSVNPNCYAKVMMVNGDHRIGIFAKRAIQTGE ELFFDYRYSQADALKYVGIEREMEIP |
| Wherein X is preferably a glycine (G). |

In one embodiment the amino acid sequence of a A687 mutant of EZH2 differs from the amino acid sequence of wild-type human EZH2 only by substitution of a non-alanine amino acid, preferably valine (V) for the single amino acid residue corresponding to A687 of wild-type human EZH2. The A687 mutant of EZH2 according to this embodiment is referred to herein as an A687 mutant and preferably an A687V mutant or, equivalently, A687V.

| A687 (SEQ ID NO: 16) |
|---|
| MGQTGKKSEKGPVCWRKRVKSEYMRLRQLKRFRRADEVKSMFSSNRQKILERTEILNQEW KQRRIQPVHILTSVSSLRGTRECSVTSDLDFPTQVIPLKTLNAVASVPIMYSWSPLQQNF MVEDETVLHNIPYMGDEVLDQDGTFIEELIKNYDGKVHGDRECGFINDEIFVELVNALGQ YNDDDDDDDGDDPEEREEKQKDLEDHRDDKESRPPRKFPSDKIFEAISSMFPDKGTAEEL KEKYKELTEQQLPGALPPECTPNIDGPNAKSVQREQSLHSFHTLFCRRCFKYDCFLHPFH ATPNTYKRKNTETALDNKPCGPQCYQHLEGAKEFAAALTAERIKTPPKRPGGRRRGRLPN NSSRPSTPTINVLESKDTDSDREAGTETGGENNDKEEEEKKDETSSSSEANSRCQTPIKM KPNIEPPENVEWSGAEASMFRVLIGTYYDNFCAIARLIGTKTCRQVYEFRVKESSIIAPA PAEDVDTPPRKKKRKHRLWAAHCRKIQLKKDGSSNHVYNYQPCDHPRQPCDSSCPCVIAQ NFCEKFCQCSSECQNRFPGCRCKAQCNTKQCPCYLAVRECDPDLCLTCGAADHWDSKNVS CKNCSIQRGSKKHLLLAPSDVAGWGIFIKDPVQKNEFISEYCGEIISQDEADRRGKVYDK YMCSFLFNLNNDFVVDATRKGNKIRFXNHSVNPNCYAKVMMVNGDHRIGIFAKRAIQTGE ELFFDYRYSQADALKYVGIEREMEIP |
| Wherein X is preferably a valine (V). |

The amino acid sequence of a R685 mutant of EZH2 may differ from the amino acid sequence of wild-type human EZH2 only by substitution of a non-arginine amino acid, preferably histidine (H) or cysteine (C) for the single amino acid residue corresponding to R685 of wild-type human EZH2. The R685 mutant of EZH2 is referred to herein as an R685 mutant and preferably an R685C mutant or an R685H mutant or, equivalently, R685H or R685C.

| A685 (SEQ ID NO: 17) |
|---|
| MGQTGKKSEKGPVCWRKRVKSEYMRLRQLKRFRRADEVKSMFSSNRQKILERTEILNQEW KQRRIQPVHILTSVSSLRGTRECSVTSDLDFPTQVIPLKTLNAVASVPIMYSWSPLQQNF MVEDETVLHNIPYMGDEVLDQDGTFIEELIKNYDGKVHGDRECGFINDEIFVELVNALGQ YNDDDDDDDGDDPEEREEKQKDLEDHRDDKESRPPRKFPSDKIFEAISSMFPDKGTAEEL KEKYKELTEQQLPGALPPECTPNIDGPNAKSVQREQSLHSFHTLFCRRCFKYDCFLHPFH ATPNTYKRKNTETALDNKPCGPQCYQHLEGAKEFAAALTAERIKTPPKRPGGRRRGRLPN NSSRPSTPTINVLESKDTDSDREAGTETGGENNDKEEEEKKDETSSSSEANSRCQTPIKM KPNIEPPENVEWSGAEASMFRVLIGTYYDNFCAIARLIGTKTCRQVYEFRVKESSIIAPA |
| PAEDVDTPPRKKKRKHRLWAAHCRKIQLKKDGSSNHVYNYQPCDHPRQPCDSSCPCVIAQ NFCEKFCQCSSECQNRFPGCRCKAQCNTKQCPCYLAVRECDPDLCLTCGAADHWDSKNVS CKNCSIQRGSKKHLLLAPSDVAGWGIFIKDPVQKNEFISEYCGEIISQDEADRRGKVYDK YMCSFLFNLNNDFVVDATRKGNKIXFANHSVNPNCYAKVMMVNGDHRIGIFAKRAIQTGE ELFFDYRYSQADALKYVGIEREMEIP |
| Wherein X is preferably a cysteine (C) or a histidine (H). |

The amino acid sequence of a mutant of EZH2 may differ from the amino acid sequence of wild-type human EZH2 in one or more amino acid residues in its substrate pocket domain as defined in SEQ ID NO: 6. This mutant of EZH2 is referred to herein as an EZH2 mutant.

| Mutant EZH2 comprising one or more mutations in the substrate pocket domain (SEQ ID NO: 18) |
|---|
| MGQTGKKSEKGPVCWRKRVKSEYMRLRQLKRFRRADEVKSMFSSNRQKILERTEILNQEW<br>KQRRIQPVHILTSVSSLRGTRECSVTSDLDFPTQVIPLKTLNAVASVPIMYSWSPLQQNF<br>MVEDETVLHNIPYMGDEVLDQDGTFIEELIKNYDGKVHGDRECGFINDEIFVELVNALGQ<br>YNDDDDDDDGDDPEEREEKQKDLEDHRDDKESRPPRKFPSDKIFEAISSMFPDKGTAEEL<br>KEKYKELTEQQLPGALPPECTPNIDGPNAKSVQREQSLHSFHTLFCRRCFKYDCFLHPFH<br>ATPNTYKRKNTETALDNKPCGPQCYQHLEGAKEFAAALTAERIKTPPKRPGGRRRGRLPN<br>NSSRPSTPTINVLESKDTDSDREAGTETGGENNDKEEEEKKDETSSSSEANSRCQTPIKM<br>KPNIEPPENVEWSGAEASMFRVLIGTYYDNFCAIARLIGTKTCRQVYEFRVKESSIIAPA<br>PAEDVDTPPRKKKRKHRLWAAHCRKIQLKKDGSSNHVYNYQPCDHPRQPCDSSCPCVIAQ<br>NFCEKFCQCSSECQNRFPGCRCKAQCNTKQCPCYLAVRECDPDLCLTCGAADHWDSKNVS<br>CKNCSIQRGSKKHLLLAPSDVAGWGIFIKDPVQKNEFISEXCGEIISQDEADRRGKVYDK<br>YMXXXLXNLNNDFXXDXTRKGNKXXXXHSVNPNCYAKVMMVNGDHRXGIFAKRAIQTGE<br>ELFXDXRYSXADALKYVGIEREMEIP<br>Wherein X can be any amino acid except the corresponding wild type residue. |

The implications of the present results for human disease are made clear by the data summarized in **Table 2** (see Example 5). Cells heterozygous for EZH2 would be expected to display a malignant phenotype due to the efficient formation of H3-K27me1 by the WT enzyme and the efficient, subsequent transition of this progenitor species to H3-K27me2, and, especially, H3-K27me3, by the mutant enzyme form(s).

It has been reported that H3-K27me1 formation is not exclusively dependent on WT-EZH2 catalysis. Knockout studies of EZH2 and of another PRC2 subunit, EED, have demonstrated H3-K27me1 formation can be catalyzed by PRC2 complexes containing either EZH2 or the related protein EZH1 as the catalytic subunit. Shen, X. et al. (2008) Mol Cell 32:491-502. Hence, catalytic coupling between the mutant EZH2 species and PRC2 complexes containing either WT-EZH2 or WT-EZH1 would suffice to augment H3-K27me2/3 formation, and thus produce the attendant malignant phenotype. The data therefore suggest that the malignant phenotype of follicular lymphoma (FL) and diffuse large B-cell lymphoma (DLBCL)of the germinal center B cell (GCB) subtype, associated with expression of mutant forms of EZH2, is the result of an overall gain of function with respect to formation of the trimethylated form of H3-K27. This interpretation of the data also helps to reconcile the existence of cancer-associated overexpression of EZH2 or PRC2 associated proteins (e.g., PHF19/PCL3) and also loss-of-function genotypes for the histone H3-K27 demethylase *UTX.* Loss of UTX activity would be enzymatically equivalent to a gain of function for EZH2, in either situation resulting in greater steady state levels of tri-methylated H3-K27 in cancer cells **(Figure 4).**

The mono-, di-, and tri- methylation states of histone H3-K27 are associated with different functions in transcriptional control. Histone H3-K27 monomethylation is associated with active transcription of genes that are poised for transcription. Cui et al. (2009) Cell Stem Cell 4:80-93; Barski (2007) Cell 129:823-37. In contrast, trimethylation of histone H3-K27 is associated with either transcriptionally repressed genes or genes that are poised for transcription when histone H3-K4 trimethylation is in *cis.* Cui et al. (*supra*); Kirmizis et al. (2007) Genes Dev 18:1592-1605; Bernstein et al. (2006) Cell 125:315-26. Taken together, alterations in the PRC2 complex activity reported in cancer, including the Y641 mutation of EZH2, are predicted to result in an increase in the trimethylated state of histone H3-K27 and thus to result in transcriptional repression.

Another discovery of the present invention is that cells expressing a mutant EZH2 comprising a mutation in the substrate pocket domain as defined in SEQ ID NO: 6 are, in general, more sensitive to small molecule EZH2 inhibitors than cells expressing wild type (WT) EZH2. Specifically, cells expressing Y641 mutant EZH2 show reduced growing, dividing or proliferation, or even undergo apoptosis or necrosis after the treatment of EZH2 inhibitors. In contrast, cells expressing WT EZH2 are not responsive to the anti-proliferative effect of the EZH2 inhibitors **(Figures 13 and 14).** Particularly, cells expressing a substitution mutation at amino acid position 677 of SEQ ID NO: 1 show greater sensitivity to EZH2 inhibitors than cells expressing other EZH2 mutants (Example 17).

EZH2 and other protein methyltransferases have been suggested to be attractive targets for drug discovery. Copeland et al. (2009) Nat Rev Drug Discov 8:724-32; Copeland et al. (2010) Curr Opin Chem Biol 14(4):505-10; Pollock et al. (2010) Drug Discovery Today: Therapeutic Strategies 6(1):71-9. The present data also suggest an experimental strategy for development of FL and GCB lymphoma-specific drugs. As the differences in substrate recognition between the WT and disease-associated mutants derive from transition state interactions, small molecule inhibitors that selectively mimic the transition state of the mutant EZH2 over that of the WT enzyme should prove to be effective in blocking H3-K27 methylation in mutation-bearing cells. Inhibitors of this type would be expected to display a large therapeutic index, as target-mediated toxicity would be minimal for any cells bearing only the WT enzyme. Transition state mimicry has proved to be an effective strategy for drug design in many disease areas. See, for example, Copeland, R. A. Enzymes:

A Practical Introduction to Structure, Mechanism and Data Analysis. 2nd ed, (Wiley, 2000).

The present results point to a previously unrecognized, surprising dependency on enzymatic coupling between enzymes that perform H3-K27 mono-methylation and certain mutant forms of EZH2 for pathogenesis in follicular lymphoma and diffuse large B-cell lymphoma. While not intending to be bound by any one theory, it is believed the data constitute the first example of a human disease that is dependent on such coupling of catalytic activity between normal (WT) and disease-associated mutant (such as Y641) enzymes.

Described herein is is a method for treating or alleviating a symptom of cancer or precancerous condition in a subject by administering to a subject expressing a mutant EZH2 comprising a mutation in the substrate pocket domain as defined in SEQ ID NO: 6 a therapeutically effective amount of an EZH2 inhibitor.

Described herein is a method for inhibiting in a subject conversion of H3-K27 to trimethylated H3-K27. The inhibition can involve inhibiting in a subject conversion of unmethylated H3-K27 to monomethylated H3-K27, conversion of monomethylated H3-K27 to dimethylated H3-K27, conversion of dimethylated H3-K27 to trimethylated H3-K27, or any combination thereof, including, for example, conversion of monomethylated H3-K27 to dimethylated H3-K27 and conversion of dimethylated H3-K27 to trimethylated H3-K27. As used herein, unmethylated H3-K27 refers to histone H3 with no methyl group covalently linked to the amino group of lysine 27. As used herein, monomethylated H3-K27 refers to histone H3 with a single methyl group covalently linked to the amino group of lysine 27. Monomethylated H3-K27 is also referred to herein as H3-K27me1. As used herein, dimethylated H3-K27 refers to histone H3 with two methyl groups covalently linked to the amino group of lysine 27. Dimethylated H3-K27 is also referred to herein as H3-K27me2. As used herein, trimethylated H3-K27 refers to histone H3 with three methyl groups covalently linked to the amino group of lysine 27. Trimethylated H3-K27 is also referred to herein as H3-K27me3.

Histone H3 is a 136 amino acid long protein, the sequence of which is known. See, for example, GenBank Accession No. CAB02546. As disclosed further herein, in addition to full-length histone H3, peptide fragments of histone H3 comprising the lysine residue corresponding to K27 of full-length histone H3 can be used as substrate for EZH2 (and likewise for mutant forms of EZH2) to assess conversion of H3-K27m1 to H3-K27m2 and conversion of H3-K27m2 to H3-K27m3. Such peptide fragment may correspond to amino acid residues 21-44 of histone H3. Such peptide fragment has the amino acid sequence LATKAARKSAPATGGVKKPHRYRP (SEQ ID NO: 19).

Described herein is a method involveing administering to a subject expressing a mutant EZH2 a therapeutically effective amount of an inhibitor of EZH2, wherein the inhibitor inhibits histone methyltransferase activity of EZH2, thereby inhibiting conversion of H3-K27 to trimethylated H3-K27 in the subject and thus treating or allievating a symptom of cancer or disorders associated with abnormal histon methylation levels in the subject.

A subject expressing a mutant EZH2 of the present invention refers to a subject having a detectable amount of a mutant EZH2 polypeptide. Preferably the mutant EZH2 polypeptide comprises one or more mutations in its substrate pocket domain as defined in SEQ ID NO: 6. The mutation includes a substitution at amino acid position 677, 687, or 641 of SEQ ID NO: 1, such as, but is not limited to a substitution of glycine (G) for the wild type residue alanine (A) at amino acid position 677 of SEQ ID NO: 1 (A677G); a substitution of valine (V) for the wild type residue alanine (A) at amino acid position 687 of SEQ ID NO: 1 (A687V); a substitution of phenylalanine (F) for the wild type residue tyrosine (Y) at amino acid position 641 of SEQ ID NO: 1 (Y641F); a substitution of histidine (H) for the wild type residue tyrosine (Y) at amino acid position 641 of SEQ ID NO: 1 (Y641H); a substitution of asparagine (N) for the wild type residue tyrosine (Y) at amino acid position 641 of SEQ ID NO: 1 (Y641N); a substitution of serine (S) for the wild type residue tyrosine (Y) at amino acid position 641 of SEQ ID NO: 1 (Y641S); or a substitution of cysteine (C) for the wild type residue tyrosine (Y) at amino acid position 641 of SEQ ID NO: 1 (Y641C). More preferablly, the subject has a detectable amount of a mutant EZH2 polypeptide comprising a substitution of glycine (G) for the wild type residue alanine (A) at amino acid position 677 of SEQ ID NO: 1 (A677G); a substitution of valine (V) for the wild type residue alanine (A) at amino acid position 687 of SEQ ID NO: 1 (A687V).

Alternatively a subject expressing a mutant EZH2 of the present invention refers to a subject having a detectable amount of a nucleic acid sequence encoding a mutant EZH2 polypeptide. Preferably a nucleic acid sequence encoding a mutant EZH2 polypeptide comprises one or more mutations in its substrate pocket domain as defined in SEQ ID NO: 6. The mutation includes a substitution at amino acid position 677, 687, or 641 of SEQ ID NO: 1, such as, but is not limited to a substitution of glycine (G) for the wild type residue alanine (A) at amino acid position 677 of SEQ ID NO: 1 (A677G); a substitution of valine (V) for the wild type residue alanine (A) at amino acid position 687 of SEQ ID NO: 1 (A687V); a substitution of phenylalanine (F) for the wild type residue tyrosine (Y) at amino acid position 641 of SEQ ID NO: 1 (Y641F); a substitution of histidine (H) for the wild type residue tyrosine (Y) at amino acid position 641 of SEQ ID NO: 1 (Y641H); a substitution of asparagine (N) for the wild type residue tyrosine (Y) at amino acid position 641 of SEQ ID NO: 1 (Y641N); a substitution of serine (S) for the wild type residue tyrosine (Y) at amino acid position 641 of SEQ ID NO: 1 (Y641S); or a substitution of cysteine (C) for the wild type residue tyrosine (Y) at amino acid position 641 of SEQ ID NO: 1 (Y641C). More preferablly, the subject has a detectable amount of nucleic acid sequence encoding a mutant EZH2 polypeptide comprising a substitution of glycine (G) for the wild type residue alanine (A) at amino acid position 677 of SEQ ID NO: 1 (A677G); a substitution of valine (V) for the wild type residue alanine (A) at amino acid

position 687 of SEQ ID NO: 1 (A687V).

## DETECTION OF EZH2 MUTANTS

A mutant EZH2 polypeptide can be detected using any suitable method. For example, a mutant EZH2 polypeptide can be detected using an antibody that binds specifically to the mutant EZH2 polypeptide or to a peptide fragment that is characteristic of the mutant EZH2 polypeptide. A peptide fragment that is characteristic of the mutant EZH2 polypeptide may include, for example, a SET domain as provided in SEQ ID NO: 7, except for substitution of one ore more residues in the substrate pocket domain as defined in SEQ ID NO: 6 by an amino acid residue other than the wild type residue. In another embodiment, a peptide fragment that is characteristic of the mutant EZH2 polypeptide may include, for example, a 10-113 amino acid fragment of the SET domain as provided in SEQ ID NO: 7, except for substitution of one ore more residues in the substrate pocket domain as defined in SEQ ID NO: 6 by an amino acid residue other than the wild type residue, provided that the fragment includes the amino acid residue corresponding to a mutation of EZH2. An antibody is considered to bind specifically to the mutant EZH2 polypeptide or to a peptide fragment that is characteristic of the mutant EZH2 polypeptide if it binds to that mutant EZH2 polypeptide or peptide fragment thereof but not to the corresponding wild-type EZH2 polypeptide or peptide fragment thereof. In one embodiment, such antibody is considered to bind specifically to the mutant EZH2 polypeptide or to a peptide fragment that is characteristic of the mutant EZH2 polypeptide if it binds to that mutant EZH2 polypeptide or peptide fragment thereof with an affinity that is at least ca. 100-fold greater than for the corresponding wild-type EZH2 polypeptide or peptide fragment thereof. In one embodiment, such antibody is considered to bind specifically to the mutant EZH2 polypeptide or to a peptide fragment that is characteristic of the mutant EZH2 polypeptide if it binds to that mutant EZH2 polypeptide or peptide fragment thereof with an affinity that is at least ca. 1000-fold greater than for the corresponding wild-type EZH2 polypeptide or peptide fragment thereof. The antibody can be used, for example, in an enzyme-linked immunosorbent assay (ELISA) or Western blot assay. The antibody may be monoclonal, polyclonal, chimeric, or an antibody fragment. The step of detecting the reaction product may be carried out with any suitable immunoassay.

In one embodiment the antibody is a monoclonal antibody. A monoclonal antibody can be prepared according to conventional methods well known in the art. See, for example, Kohler and Milstein (1975) Nature 256 (5517):495-7.

As another example, a mutant EZH2 polypeptide can be detected using mass spectrometry (MS), e.g., electrospray ionization coupled with time-of-flight (ESI-TOF) or matrix-assisted laser desorption/ionization coupled with time-of-flight (MALDI-TOF). Such methods are well known in the art. The analysis will involve identification of one or more peptide fragments comprising the mutation of interest, for example, a peptide 12 to 24 amino acids long comprising a sequence spanning the amino acid corresponding to a mutation in wild-type EZH2.

A nucleic acid sequence encoding a mutant EZH2 polypeptide or a peptide fragment that is characteristic of the mutant EZH2 polypeptide can be detected using any suitable method. For example, a nucleic acid sequence encoding a mutant EZH2 polypeptide can be detected using whole-genome resequencing or target region resequencing (the latter also known as targeted resequencing) using suitably selected sources of DNA and polymerase chain reaction (PCR) primers in accordance with methods well known in the art. See, for example, Bentley (2006) Curr Opin Genet Dev. 16:545-52, and Li et al. (2009) Genome Res 19:1124-32. The method typically and generally entails the steps of genomic DNA purification, PCR amplification to amplify the region of interest, cycle sequencing, sequencing reaction cleanup, capillary electrophoresis, and data analysis. High quality PCR primers to cover region of interest are designed using *in silico* primer design tools. Cycle sequencing is a simple method in which successive rounds of denaturation, annealing, and extension in a thermal cycler result in linear amplification of extension products. The products are typically terminated with a fluorescent tag that identifies the terminal nucleotide base as G, A, T, or C. Unincorporated dye terminators and salts that may compete for capillary eletrophoretic injection are removed by washing. During capillary electrophoresis, the products of the cycle sequencing reaction migrate through capillaries filled with polymer. The negatively charged DNA fragments are separated by size as they move through the capillaries toward the positive electrode. After electrophoresis, data collection software creates a sample file of the raw data. Using downstream software applications, further data analysis is performed to translate the collected color data images into the corresponding nucleotide bases. Alternatively or in addition, the method may include the use of microarray-based targeted region genomic DNA capture and/or sequencing. Kits, reagents, and methods for selecting appropriate PCR primers and performing resequencing are commercially available, for example, from Applied Biosystems, Agilent, and NimbleGen (Roche Diagnostics GmbH). Methods such as these have been used to detect *JAK*2 and myeloproliferative leukemia gene (*MPL*) mutations and to diagnose polycythemia vera, essential thrombocythemia, and idiopathic myelofibrosis. For use in the instant invention, PCR primers may be selected so as to amplify, for example, at least a relevant portion of SEQ ID NO: 8 (above).

Alternatively or in addition, a nucleic acid sequence encoding a mutant EZH2 polypeptide may be detected using a Southern blot in accordance with methods well known in the art. In one embodiment a DNA sequence encoding a mutant EZH2 polypeptide is detected using nucleic acid hybridization performed under highly stringent conditions. A nucleic acid probe is selected such that its sequence is complementary to a target nucleic acid sequence that includes

a codon for the mutant amino acid corresponding to a mutation of wild-type EZH2. Such mutation includes any mutation of EZH2, preferably one or more mutations in the substrate pocket domain as defined in SEQ ID NO: 6 of EZH2, such as, but are not limited to a substitution of glycine (G) for the wild type residue alanine (A) at amino acid position 677 of SEQ ID NO: 1 (A677G); a substitution of valine (V) for the wild type residue alanine (A) at amino acid position 687 of SEQ ID NO: 1 (A687V); a substitution of methionine (M) for the wild type residue valine (V) at amino acid position 674 of SEQ ID NO: 1 (V674M); a substitution of histidine (H) for the wild type residue arginine (R) at amino acid position 685 of SEQ ID NO: 1 (R685H); a substitution of cysteine (C) for the wild type residue arginine (R) at amino acid position 685 of SEQ ID NO: 1 (R685C); a substitution of phenylalanine (F) for the wild type residue tyrosine (Y) at amino acid position 641 of SEQ ID NO: 1 (Y641F); a substitution of histidine (H) for the wild type residue tyrosine (Y) at amino acid position 641 of SEQ ID NO: 1 (Y641H); a substitution of asparagine (N) for the wild type residue tyrosine (Y) at amino acid position 641 of SEQ ID NO: 1 (Y641N); a substitution of serine (S) for the wild type residue tyrosine (Y) at amino acid position 641 of SEQ ID NO: 1 (Y641S); or a substitution of cysteine (C) for the wild type residue tyrosine (Y) at amino acid position 641 of SEQ ID NO: 1 (Y641C).

A sequence-specific probe is combined with a sample to be tested under highly stringent conditions. The term "highly stringent conditions" as used herein refers to parameters with which the art is familiar. Nucleic acid hybridization parameters may be found in references that compile such methods, e.g., J. Sambrook, et al., eds., Molecular Cloning: A Laboratory Manual, Second Edition, Cold Spring Harbor Laboratory Press, Cold Spring Harbor, New York, 1989, or F. M. Ausubel, et al., eds., Current Protocols in Molecular Biology, John Wiley & Sons, Inc., New York. More specifically, highly stringent conditions, as used herein, refers, for example, to hybridization at 65° C in hybridization buffer (3.5 × SSC, 0.02% Ficoll, 0.02% polyvinyl pyrrolidone, 0.02% bovine serum albumin (BSA), 2.5 mM $NaH_2PO_4$ (pH 7), 0.5% SDS, 2 mM EDTA). SSC is 0.15 M sodium chloride/0.015 M sodium citrate, pH 7; SDS is sodium dodecyl sulphate; and EDTA is ethylenediaminetetracetic acid. After hybridization, the membrane upon which the DNA is transferred is washed, for example, in 2 × SSC at room temperature and then at 0.1-0.5 × SSC/0.1 × SDS at temperatures up to 68° C.

There are other conditions, reagents, and so forth that can be used, which result in a similar degree of stringency. The skilled artisan will be familiar with such conditions, and thus they are not given here. It will be understood, however, that the skilled artisan will be able to manipulate the conditions in a manner to permit the clear identification of EZH2-associated nucleic acids of the invention, including, in particular, nucleic acids encoding mutants of EZH2 (e.g., by using lower stringency conditions). The skilled artisan also is familiar with the methodology for screening cells and libraries for expression of such molecules, which then are routinely isolated, followed by isolation of the pertinent nucleic acid molecule and sequencing.

The subject is administered a therapeutically effective amount of an inhibitor of EZH2. As used herein, an inhibitor of EZH2 refers, generally, to a small molecule, i.e., a molecule of molecular weight less than about 1.5 kilodaltons (kDa), which is capable of interfering with the histone methyltransferase enzymatic activity of EZH2.

In one embodiment the inhibitor of EZH2 inhibits histone methyltransferase activity of wild-type EZH2. In one embodiment the inhibitor of EZH2 inhibits histone methyltransferase activity of the mutant EZH2. In one embodiment the inhibitor of EZH2 inhibits histone methyltransferase activity of wild-type EZH2 and histone methyltransferase activity of the mutant EZH2. In one embodiment the inhibitor of EZH2 selectively inhibits histone methyltransferase activity of the mutant EZH2.

As disclosed herein, certain mutants of EZH2 (such as Y641) are relatively poor catalysts for conversion of unmethylated H3-K27 to H3-K27me1 and yet unexpectedly effective catalysts for conversion of H3-K27me2 to H3-K27me3. Certain mutants of EZH2 (such as A687) prefer monomethyl H3-K27 substrate. In contrast, certain mutants of EZH2 (such as A677) show equal preference among unmethylated, monomethylated and dimethylated H3-K27. Conversely, wild-type EZH2 is a relatively effective catalyst for conversion of unmethylated H3-K27 to H3-K27me1 and yet unexpectedly ineffective catalyst for conversion of H3-K27me2 to H3-K27me3. This is important because mono-, di- and tri-methylated states of H3-K27 exhibit different functions in transcriptional control. For example, H3-K27me1 is associated with active transcription of genes that are poised for transcription, while H3-K27me3 is associated with transcriptionally repressed genes or genes that are poised for transcription when H3-K4 trimethylation is in *cis.* Thus, selective inhibition of histone methyltransferase activity of the mutant of EZH2 affects selective inhibition of production of the different methylated forms of H3-K27, thereby modifying transcription associated with H3-K27 methylation levels.

An inhibitor of EZH2 "selectively inhibits" histone methyltransferase activity of the mutant EZH2 when it inhibits histone methyltransferase activity of the mutant EZH2 more effectively than it inhibits histone methyltransferase activity of wild-type EZH2. For example, in one embodiment the selective inhibitor has an IC50 for the mutant EZH2 that is at least 40 percent lower than the IC50 for wild-type EZH2. In one embodiment the selective inhibitor has an IC50 for the mutant EZH2 that is at least 50 percent lower than the IC50 for wild-type EZH2. In one embodiment the selective inhibitor has an IC50 for the mutant EZH2 that is at least 60 percent lower than the IC50 for wild-type EZH2. In one embodiment the selective inhibitor has an IC50 for the mutant EZH2 that is at least 70 percent lower than the IC50 for wild-type EZH2. In one embodiment the selective inhibitor has an IC50 for the mutant EZH2 that is at least 80 percent lower than the IC50 for wild-type EZH2. In one embodiment the selective inhibitor has an IC50 for the mutant EZH2 that is at least 90

percent lower than the IC50 for wild-type EZH2.

In one embodiment, the selective inhibitor of a mutant EZH2 exerts essentially no inhibitory effect on wild-type EZH2.

The inhibitor inhibits conversion of H3-K27me2 to H3-K27me3. In one embodiment the inhibitor is said to inhibit trimethylation of H3-K27. Since conversion of H3-K27me1 to H3-K27me2 precedes conversion of H3-K27me2 to H3-K27me3, an inhibitor of conversion of H3-K27me1 to H3-K27me2 naturally also inhibits conversion of H3-K27me2 to H3-K27me3, i.e., it inhibits trimethylation of H3-K27. It is also possible to inhibit conversion of H3-K27me2 to H3-K27me3 without inhibition of conversion of H3-K27me1 to H3-K27me2. Inhibition of this type would also result in inhibition of trimethylation of H3-K27, albeit without inhibition of dimethylation of H3-K27.

In one embodiment the inhibitor inhibits conversion of H3-K27me1 to H3-K27me2 and the conversion of H3-K27me2 to H3-K27me3. Such inhibitor may directly inhibit the conversion of H3-K27me1 to H3-K27me2 alone. Alternatively, such inhibitor may directly inhibit both the conversion of H3-K27me1 to H3-K27me2 and the conversion of H3-K27me2 to H3-K27me3.

The inhibitor inhibits histone methylase activity. Inhibition of histone methylase activity can be detected using any suitable method. The inhibition can be measured, for example, either in terms of rate of histone methylase activity or as product of histone methylase activity. Methods suitable for either of these readouts are included in the Examples below.

The inhibition is a measurable inhibition compared to a suitable negative control. In one embodiment, inhibition is at least 10 percent inhibition compared to a suitable negative control. That is, the rate of enzymatic activity or the amount of product with the inhibitor is less than or equal to 90 percent of the corresponding rate or amount made without the inhibitor. In various other embodiments, inhibition is at least 20, 25, 30, 40, 50, 60, 70, 75, 80, 90, or 95 percent inhibition compared to a suitable negative control. In one embodiment, inhibition is at least 99 percent inhibition compared to a suitable negative control. That is, the rate of enzymatic activity or the amount of product with the inhibitor is less than or equal to 1 percent of the corresponding rate or amount made without the inhibitor.

Described herein is the inhibitor S-adenosyl-L-homocysteine (SAH). SAH has the structural formula

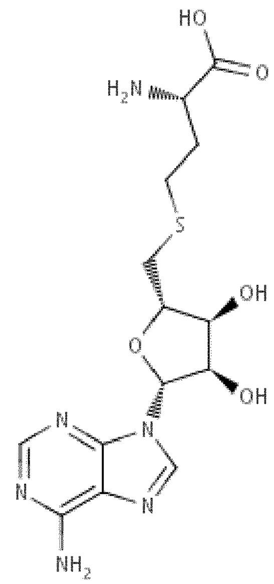

(SAH)

and is commercially available from a number of suppliers, including, for example, Sigma-Aldrich, St. Louis, MO. SAH has been described as an inhibitor of transmethylation by S-adenosylmethionine-dependent methyltransferases.

Described herein is the inhibitor Compound 75

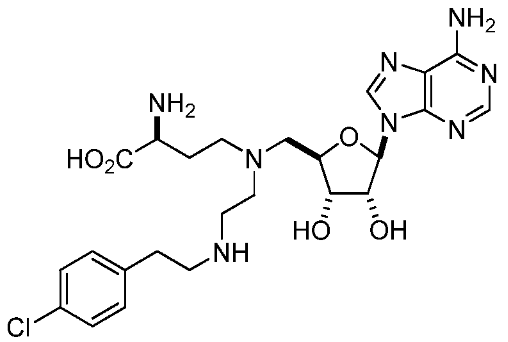

(75)

or a pharmaceutically acceptable salt thereof.

In certain embodiments the invention comprises the step of performing an assay to detect a mutant of EZH2 in a sample from a subject. Assays of this type are described above. As used herein, a "sample from a subject" refers to any suitable sample containing cells or components of cells obtained or derived from a subject. In one embodiment the sample includes cells suspected to express mutant EZH2, e.g., cancer cells. In one embodiment the sample is a blood sample. In one embodiment the sample is a biopsy sample obtained from, for example, a lymphatic tissue (e.g., lymph node) or bone marrow. In one embodiment the sample is a biopsy sample obtained from a tissue other than or in addition to a lymphatic tissue (e.g., lymph node) or bone marrow. For example, in one embodiment the sample is a biopsy from a cancer, e.g., a tumor composed of cancer cells. Cells in the sample can be isolated from other components of the sample. For example, peripheral blood mononuclear cells (PBMCs) can be isolated as a buffy coat from a blood sample that has been centrifuged in accordance with methods familiar to those of skill in the art.

When the result of the assay on a sample from a subject indicates that a mutant EZH2 is present in the sample, the subject is said to express mutant EZH2. Indeed, in one embodiment, when the result of the assay on a sample from a subject indicates that a mutant EZH2 is present in the sample, the subject is identified as a candidate for treatment with an inhibitor of EZH2, wherein the inhibitor selectively inhibits histone methyltransferase activity of the mutant EZH2.

When the result of the assay on a sample from a cancer indicates that a mutant EZH2 is present in the cancer, the cancer is said to express the mutant EZH2.

Similarly, when the result of the assay on a sample comprising cancer cells from a subject having a cancer indicates that a mutant EZH2 is present in the sample, the subject is said to express the mutant EZH2.

Detection of dimethylated H3-K27 or trimethlated H3-K27 can be accomplished using any suitable method in the art. In one embodiment, the methylation level is detected using antibodies specific for dimethylated H3-K27 or trimethlated H3-K27. For example, the isolated tissue is formalin fixed and embedded in paraffin blocks for long term preservation. The blocks can be used to prepare slides for immunohistochemical staining or fluorescent staining with antibodies against methylated H3-K27. Alternatively, whole cell lysates or histone extracts can be prepared from the isolated tissue sample and subsequently used for immunohistochemical staining, western blot analysis or fluorescent staining. In another embodiment the methylation level is detected using a polypeptide or an aptamer specific for dimethylated H3-K27 or trimethlated H3-K27. In another embodiment, the methylation level is detected using mass spectrometry (MS).

A control dimethylated H3-K27 or a control trimethlated H3-K27 can be established from a control sample, *e.g.,* an adjacent non-tumor tissue isolated from the subject or a healthy tissue from a healthy subject. Alternatively, the control methylation level of H3-K27me2 or H3-K27me3 can be established by a pathologist with known methods in the art.

## SCREENING METHODS

Described herein is a method for identifying a test compound as an inhibitor of a mutant EZH2. The method may include combining an isolated mutant EZH2 with a histone substrate, a methyl group donor (such as S-adenosyl methionine (SAM)), and a test compound, wherein the histone substrate comprises a form of H3-K27 selected from the group consisting of unmethylated H3-K27, monomethylated H3-K27, dimethylated H3-K27, and any combination thereof; and performing an assay to detect methylation of H3-K27 in the histone substrate, thereby identifying the test compound as an inhibitor of the mutant EZH2 when methylation of H3-K27 in the presence of the test compound is less than methylation of H3-K27 in the absence of the test compound. The assay to detect methylation of H3-K27 can be selected to measure the rate of methylation, the extent of methylation, or both the rate and extent of methylation.

The mutant EZH2 is isolated as a PRC2 complex or functional equivalent thereof. As used herein, the term "isolated" means substantially separated from other components with which the complex may be found as it occurs in nature. A compound can be isolated without necessarily being purified. The mutant of EZH2 may be isolated as a complex

of a mutant EZH2 together with EED and SUZ12. The mutant of EZH2 may be isolated as a complex of a mutant EZH2 together with EED, SUZ12, and RbAp48. Under appropriate conditions, a PRC2 complex or functional equivalent thereof exhibits histone methyltransferase activity for H3-K27. The complex may be composed of recombinantly expressed component polypeptides, e.g., EZH2, EED, SUZ12, with or without RbAp48.

The isolated mutant EZH2 is combined with a histone substrate. A histone substrate includes any suitable source of histone polypeptides or fragments thereof that can serve as substrate for EZH2. The histone substrate may include histones isolated from a subject. The histones can be isolated from cells of a subject using any suitable method; such methods are well known to persons skilled in the art and need not be further specified here. See, for example, Fang et al. (2004) Methods Enzymol 377:213-26. The histone substrate may be provided as nucleosomes. The histone substrate may be provided as avian (chicken) erythrocyte nucleosomes.

Histone substrate so provided may include an admixture of states of histone modification, including various states of H3-K27 methylation as judged by Western blotting with H3-K27 methylation state-specific antibodies. The histone substrate may be provided as purified full-length histone H3. Such purified full-length histone H3 may be provided as a homogeneous preparation in respect of states of H3-K27 methylation, or as an admixture of various states of H3-K27 methylation. Homogeneous preparations of isolated histone H3 in respect of states of H3-K27 methylation may be prepared in part by passage over an immunoaffinity column loaded with suitable H3-K27 methylation state-specific antibodies or by immunoprecipitation using magnetic beads coated with suitable H3-K27 methylation state-specific antibodies. Alternatively or in addition, the methylation state of H3-K27 can be characterized as part of performing the assay. For example, the starting material histone substrate might be characterized as containing 50 percent unmethylated H3-K27, 40 percent monomethylated H3-K27, 10 percent dimethylated H3-K27, and 0 percent trimethylated H3-K27.

The histone substrate may include a peptide library or a suitable peptide comprising one or more amino acid sequences related to histone H3, including, in particular, a sequence that encompasses H3-K27. The histone substrate may be a peptide fragment that corresponds to amino acid residues 21-44 of histone H3. Such peptide fragment has the amino acid sequence LATKAARKSAPATGGVKKPHRYRP (SEQ ID NO: 19). The peptide library or peptide can be prepared by peptide synthesis according to techniques well known in the art and optionally modified so as to incorporate any desired degree of methylation of lysine corresponding to H3-K27. As described in the Examples below, such peptides can also be modified to incorporate a label, such as biotin, useful in performing downstream assays. The label may be appended to the amino (N)-terminus of the peptide(s). The label may be appended to the carboxy (C)-terminus of the peptide(s).

H3-K27 methylation-specific antibodies are available from a variety of commercial sources, including, for example, Cell Signaling Technology (Danvers, MA) and Active Motif (Carlsbad, CA).

The isolated mutant EZH2 is combined with a test compound. As used herein, a "test compound" refers to a small organic molecule having a molecular weight of less than about 1.5 kDa. A test compound may be a known compound. A test compound may be a novel compound. A test compound can be provided as part of a library of such compounds, wherein the library includes, for example, tens, hundreds, thousands, or even more compounds. A library of compounds may advantageously be screened in a high throughput screening assay, for example, using arrays of test compounds and robotic manipulation in accordance with general techniques well known in the art.

A test compound may be a compound that is a derivative of SAH or a derivative of Compound 75.

Detection of methylation of H3-K27 can be accomplished using any suitable method. The source of donor methyl groups may include methyl groups that are labeled with a detectable label. The detectable label may be an isotopic label, e.g., tritium. Other types of labels may include, for example, fluorescent labels.

Detection of formation of trimethylated H3-K27 can be accomplished using any suitable method. For example, detection of formation of trimethylated H3-K27 can be accomplished using an assay to detect incorporation of labeled methyl groups, such as described above, optionally combined with a chromatographic or other method to separate labeled products by size, e.g., polyacrylamide gel electrophoresis (PAGE), capillary electrophoresis (CE), or high pressure liquid chromatography (HPLC). Alternatively or in addition, detection of formation of trimethylated H3-K27 can be accomplished using antibodies that are specific for trimethylated H3-K27.

Detection of conversion of monomethylated H3-K27 to dimethylated H3-K27 can be accomplished using any suitable method. The conversion may be measured using antibodies specific for monomethylated H3-K27 and dimethylated H3-K27. For example, starting amounts or concentrations of monomethylated H3-K27 and dimethylated H3-K27 may be determined using appropriate antibodies specific for monomethylated H3-K27 and dimethylated H3-K27. Following the combination of enzyme, substrate, methyl group donor, and test compound, resulting amounts or concentrations of monomethylated H3-K27 and dimethylated H3-K27 may then be determined using appropriate antibodies specific for monomethylated H3-K27 and dimethylated H3-K27. The beginning and resulting amounts or concentrations of monomethylated H3-K27 and dimethylated H3-K27can then be compared. Alternatively or in addition, beginning and resulting amounts or concentrations of monomethylated H3-K27 and dimethylated H3-K27 can then be compared to corresponding amounts of concentrations from a negative control. A negative control reaction, in which no test agent is included in the assay, can be run in parallel or as a historical control. Results of such control reaction can optionally be subtracted from

corresponding results of the experimental reaction prior to or in conjunction with making the comparison mentioned above.

Because the dimethylated form of H3-K27 may be further methylated in the same assay, a reduction in the amount or concentration of monomethylated H3-K27 may not appear to correspond directly to an increase in dimethylated H3-K27. In this instance, it may be presumed, however, that a reduction in the amount or concentration of monomethylated H3-K27 is, by itself, reflective of conversion of monomethylated H3-K27 to dimethylated H3-K27.

Detection of conversion of dimethylated H3-K27 to trimethylated H3-K27 can be accomplished using any suitable method. The conversion may be measured using antibodies specific for dimethylated H3-K27 and trimethylated H3-K27. For example, starting amounts or concentrations of dimethylated H3-K27 and trimethylated H3-K27 may be determined using appropriate antibodies specific for dimethylated H3-K27 and trimethylated H3-K27. Following the combination of enzyme, substrate, and test compound, resulting amounts or concentrations of dimethylated H3-K27 and trimethylated H3-K27 may then be determined using appropriate antibodies specific for dimethylated H3-K27 and trimethylated H3-K27. The beginning and resulting amounts or concentrations of dimethylated H3-K27 and trimethylated H3-K27can then be compared. Alternatively or in addition, beginning and resulting amounts or concentrations of dimethylated H3-K27 and trimethylated H3-K27 can then be compared to corresponding amounts of concentrations from a negative control. A negative control reaction, in which no test agent is included in the assay, can be run in parallel or as a historical control. Results of such control reaction can optionally be subtracted from corresponding results of the experimental reaction prior to or in conjunction with making the comparison mentioned above.

A test agent is identified as an inhibitor of the mutant EZH2 when methylation of H3-K27 with the test compound is less than methylation of H3-K27 without the test compound. A test agent may be identified as an inhibitor of the mutant EZH2 when formation of trimethylated H3-K27 in the presence of the test compound is less than formation of trimethylated H3-K27 in the absence of the test compound.

Described herein is a method for identifying a selective inhibitor of a mutant EZH2. The method may include combining an isolated mutant EZH2 with a histone substrate, a methyl group donor (e.g., SAM), and a test compound, wherein the histone substrate comprises a form of H3-K27 selected from the group consisting of monomethylated H3-K27, dimethylated H3-K27, and a combination of monomethylated H3-K27 and dimethylated H3-K27, thereby forming a test mixture; combining an isolated wild-type EZH2 with a histone substrate, a methyl group donor (e.g., SAM), and a test compound, wherein the histone substrate comprises a form of H3-K27 selected from the group consisting of monomethylated H3-K27, dimethylated H3-K27, and a combination of monomethylated H3-K27 and dimethylated H3-K27, thereby forming a control mixture; performing an assay to detect trimethylation of the histone substrate in each of the test mixture and the control mixture; calculating the ratio of (a) trimethylation with the mutant EZH2 and the test compound (M+) to (b) trimethylation with the mutant EZH2 without the test compound (M-); calculating the ratio of (c) trimethylation with wild-type EZH2 and the test compound (WT+) to (d) trimethylation with wild-type EZH2 without the test compound (WT-); comparing the ratio (a)/(b) with the ratio (c)/(d); and identifying the test compound as a selective inhibitor of the mutant EZH2 when the ratio (a)/(b) is less than the ratio (c)/(d). The method may further include taking into account a negative control without test compound for either or both of the test mixture and the control mixture.

The present invention also provides a previously unrecognized, surprising correlation of a patient's responsiveness to an EZH2 inhibitor. Accordingly, an aspect of the invention is a method for determining responsiveness of a subject having cancer or myelodysplastic syndromes (MDS, formerly known as preleukemia) to an EZH2 inhibitor selected from compound 2 to compound 62. The method involves detecting the presence of mutation in EZH2 in a sample obtained from a subject wherein the mutation is or is expressed as a substitution mutation at amino acid position 677, 687 or 641 of SEQ ID No:1 wherein the presence of said mutation indicates the subject is responsive to the EZH2 inhibitor. Preferred substitution amino acid mutation includes a substitution at amino acid position 677, 687, or 641 of SEQ ID NO: 1, such as, but is not limited to a substitution of glycine (G) for the wild type residue alanine (A) at amino acid position 677 of SEQ ID NO: 1 (A677G); a substitution of valine (V) for the wild type residue alanine (A) at amino acid position 687 of SEQ ID NO: 1 (A687V); a substitution of phenylalanine (F) for the wild type residue tyrosine (Y) at amino acid position 641 of SEQ ID NO: 1 (Y641F); a substitution of histidine (H) for the wild type residue tyrosine (Y) at amino acid position 641 of SEQ ID NO: 1 (Y641H); a substitution of asparagine (N) for the wild type residue tyrosine (Y) at amino acid position 641 of SEQ ID NO: 1 (Y641N); a substitution of serine (S) for the wild type residue tyrosine (Y) at amino acid position 641 of SEQ ID NO: 1 (Y641S); or a substitution of cysteine (C) for the wild type residue tyrosine (Y) at amino acid position 641 of SEQ ID NO: 1 (Y641C).

In a preferred embodiment, the subject has cancer or a cancerous condition. For example, the cancer is lymphoma, leukemia or melanoma. Preferably, the lymphoma is non-Hodgkin lymphoma, follicular lymphoma or diffuse large B-cell lymphoma. Alternatively, the leukemia is chronic myelogenous leukemia (CML).

In another preferred embodiment, the mutant EZH2 polypeptide or the nucleic acid sequence encoding the mutant EZH2 polypeptide of the present invention comprises a substitution at amino acid position 677, 687, or 641 of SEQ ID NO: 1, such as, but is not limited to a substitution of glycine (G) for the wild type residue alanine (A) at amino acid position 677 of SEQ ID NO: 1 (A677G); a substitution of valine (V) for the wild type residue alanine (A) at amino acid position 687 of SEQ ID NO: 1 (A687V); a substitution of phenylalanine (F) for the wild type residue tyrosine (Y) at

amino acid position 641 of SEQ ID NO: 1 (Y641F); a substitution of histidine (H) for the wild type residue tyrosine (Y) at amino acid position 641 of SEQ ID NO: 1 (Y641H); a substitution of asparagine (N) for the wild type residue tyrosine (Y) at amino acid position 641 of SEQ ID NO: 1 (Y641N); a substitution of serine (S) for the wild type residue tyrosine (Y) at amino acid position 641 of SEQ ID NO: 1 (Y641S); or a substitution of cysteine (C) for the wild type residue tyrosine (Y) at amino acid position 641 of SEQ ID NO: 1 (Y641C).

**PHARMACEUTICAL COMPOSITIONS**

One or more EZH2 antagonists can be administered alone to a human patient or in pharmaceutical compositions where they are mixed with suitable carriers or excipient(s) at doses to treat or ameliorate a disease or condition as described herein. Mixtures of these EZH2 antagonists can also be administered to the patient as a simple mixture or in suitable formulated pharmaceutical compositions. For example, one aspect of the invention relates to pharmaceutical composition comprising a therapeutically effective dose of an EZH2 antagonist, or a pharmaceutically acceptable salt, hydrate, enantiomer or stereoisomer thereof; and a pharmaceutically acceptable diluent or carrier.

Techniques for formulation and administration of EZH2 antagonists may be found in references well known to one of ordinary skill in the art, such as Remington's "The Science and Practice of Pharmacy," 21st ed., Lippincott Williams & Wilkins 2005.

Suitable routes of administration may, for example, include oral, rectal, or intestinal administration; parenteral delivery, including intravenous, intramuscular, intraperitoneal, subcutaneous, or intramedullary injections, as well as intrathecal, direct intraventricular, or intraocular injections; topical delivery, including eyedrop and transdermal; and intranasal and other transmucosal delivery.

Alternatively, one may administer an EZH2 antagonist in a local rather than a systemic manner, for example, via injection of the EZH2 antagonist directly into an edematous site, often in a depot or sustained release formulation.

An EZH2 antagonist may be administered by direct injection into a tumor or lymph node.

Furthermore, one may administer an EZH2 antagonist in a targeted drug delivery system, for example, in a liposome coated with cancer cell-specific antibody.

The pharmaceutical compositions of the present invention may be manufactured, e.g., by conventional mixing, dissolving, granulating, dragee-making, levigating, emulsifying, encapsulating, entrapping or lyophilizing processes.

Pharmaceutical compositions for use in accordance with the present invention thus may be formulated in a conventional manner using one or more physiologically acceptable carriers comprising excipients and auxiliaries which facilitate processing of the active EZH2 antagonists into preparations which can be used pharmaceutically. Proper formulation is dependent upon the route of administration chosen.

For injection, the agents of the invention may be formulated in aqueous solutions, preferably in physiologically compatible buffers such as Hanks' solution, Ringer's solution, or physiological saline buffer. For transmucosal administration, penetrants are used in the formulation appropriate to the barrier to be permeated. Such penetrants are generally known in the art.

For oral administration, the EZH2 antagonists can be formulated readily by combining the active EZH2 antagonists with pharmaceutically acceptable carriers well known in the art. Such carriers enable the EZH2 antagonists of the invention to be formulated as tablets, pills, dragees, capsules, liquids, gels, syrups, slurries, suspensions and the like, for oral ingestion by a patient to be treated. Pharmaceutical preparations for oral use can be obtained by combining the active EZH2 antagonist with a solid excipient, optionally grinding a resulting mixture, and processing the mixture of granules, after adding suitable auxiliaries, if desired, to obtain tablets or dragee cores. Suitable excipients include fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active EZH2 antagonist doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active EZH2 antagonists may be dissolved or suspended in suitable liquids, such as fatty oils, liquid paraffin, or liquid polyethylene glycols. In addition, stabilizers may be added.

For buccal administration, the compositions may take the form of tablets or lozenges formulated in conventional manner.

For administration by inhalation, the EZH2 antagonists for use according to the present invention are conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of pressurized aerosol the dosage unit may be determined by providing a valve to deliver a metered amount. Capsules and cartridges of e.g., gelatin for use in an inhaler or insufflator may be formulated containing a powder mix of the EZH2 antagonist and a suitable powder base such as lactose or starch.

The EZH2 antagonists can be formulated for parenteral administration by injection, e.g., bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active EZH2 antagonists in water-soluble form. Additionally, suspensions of the active EZH2 antagonists may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the EZH2 antagonists to allow for the preparation of highly concentrated solutions.

Alternatively, the active ingredient may be in powder form for reconstitution before use with a suitable vehicle, e.g., sterile pyrogen-free water.

The EZH2 antagonists may also be formulated in rectal compositions such as suppositories or retention enemas, e.g., containing conventional suppository bases, such as cocoa butter or other glycerides.

In addition to the formulations described previously, the EZH2 antagonists may also be formulated as a depot preparation. Such long acting formulations may be administered by implantation (for example, subcutaneously or intramuscularly or by intramuscular injection). Thus, for example, the EZH2 antagonists may be formulated with suitable polymeric or hydrophobic materials (for example as an emulsion in an acceptable oil) or ion exchange resins, or as sparingly soluble derivatives (for example, as a sparingly soluble salt).

Alternatively, other delivery systems for hydrophobic pharmaceutical EZH2 antagonists may be employed. Liposomes and emulsions are examples of delivery vehicles or carriers for hydrophobic drugs. Certain organic solvents such as dimethysulfoxide also may be employed. Additionally, the EZH2 antagonists may be delivered using a sustained-release system, such as semi-permeable matrices of solid hydrophobic polymers containing the therapeutic agent. Various sustained-release materials have been established and are well known by those skilled in the art. Sustained-release capsules may, depending on their chemical nature, release the EZH2 antagonists for a few weeks up to over 100 days. Depending on the chemical nature and the biological stability of the therapeutic reagent, additional strategies for protein stabilization may be employed.

The pharmaceutical compositions may also comprise suitable solid or gel phase carriers or excipients. Examples of such carriers or excipients include but are not limited to calcium carbonate, calcium phosphate, various sugars, starches, cellulose derivatives, gelatin, and polymers, such as polyethylene glycols.

## METHODS OF TREATMENT

Described herein are methods of treating, preventing or alleviating a symptom of conditions and diseases, such as cancers and precancerous conditions, the course of which can be influenced by modulating the methylation status of histones or other proteins, wherein said methylation status is mediated at least in part by the activity of EZH2. Modulation of the methylation status of histones can in turn influence the level of expression of target genes activated by methylation, and/or target genes suppressed by methylation.

Described herein is a method for treating or alleviating a symptom of cancer or precancerous condition. The method comprises the step of administering to a subject having a cancer or a precancerous condition and expressing a mutant EZH2 a therapeutically effective amount of an inhibitor of EZH2. Preferably, a mutant EZH2 polypeptide or a nucleic acid sequence encoding a mutant EZH2 polypeptide comprises a mutation in its substrate pocket domain as defined in SEQ ID NO: 6. More preferablly, a mutant EZH2 polypeptide or a nucleic acid sequence encoding a mutant EZH2 polypeptide comprises a substitution mutation at amino acid position 677, 687, 674, 685, or 641 of SEQ ID NO: 1, such as, but is not limited to a substitution of glycine (G) for the wild type residue alanine (A) at amino acid position 677 of SEQ ID NO: 1 (A677G); a substitution of valine (V) for the wild type residue alanine (A) at amino acid position 687 of SEQ ID NO: 1 (A687V); a substitution of methionine (M) for the wild type residue valine (V) at amino acid position 674 of SEQ ID NO: 1 (V674M); a substitution of histidine (H) for the wild type residue arginine (R) at amino acid position 685 of SEQ ID NO: 1 (R685H); a substitution of cysteine (C) for the wild type residue arginine (R) at amino acid position 685 of SEQ ID NO: 1 (R685C); a substitution of phenylalanine (F) for the wild type residue tyrosine (Y) at amino acid position 641 of SEQ ID NO: 1 (Y641F); a substitution of histidine (H) for the wild type residue tyrosine (Y) at amino acid

position 641 of SEQ ID NO: 1 (Y641H); a substitution of asparagine (N) for the wild type residue tyrosine (Y) at amino acid position 641 of SEQ ID NO: 1 (Y641N); a substitution of serine (S) for the wild type residue tyrosine (Y) at amino acid position 641 of SEQ ID NO: 1 (Y641S); or a substitution of cysteine (C) for the wild type residue tyrosine (Y) at amino acid position 641 of SEQ ID NO: 1 (Y641C).

The inhibitor may inhibit histone methyltransferase activity of the mutant EZH2. The inhibitor may selectively inhibit histone methyltransferase activity of the mutant EZH2. Optionally, the cancer is a lymphoma. Preferably, the cancer is a non-Hodgkin lymphoma, a follicular lymphoma or a diffuse large B-cell lymphoma (DLBCL). Alternatively, the cancer is leukemia (such as CML) or melanoma. The precancerous condition includes, but is not limited to, myelodysplastic syndromes (MDS, formerly known as preleukemia).

Diseases such as cancers and neurological disease can be treated by administration of modulators of protein (e.g., histone) methylation, e.g., modulators of histone methyltransferase, or histone demethylase enzyme activity. Histone methylation has been reported to be involved in aberrant expression of certain genes in cancers, and in silencing of neuronal genes in non-neuronal cells. Modulators described herein can be used to treat such diseases, i.e., to inhibit methylation of histones in affected cells.

Based at least on the fact that abnormal histone methylation has been found to be associated with certain cancers and precancerous conditions, a method for treating cancer or a precancerous condition with a mutant EZH2 in a subject comprises administering to the subject in need thereof a therapeutically effective amount of a compound that inhibits methylation or restores methylation to roughly its level in counterpart normal cells. As described herein, a method for treating cancer or a precancerous condition in a subject comprises administering to the subject in need thereof a therapeutically effective amount of a compound that inhibits conversion of unmethylated H3-K27 to monomethylated H3-K27 (H3-K27me1). As described herein, a method for treating cancer or a precancerous condition in a subject comprises administering to the subject in need thereof a therapeutically effective amount of a compound that inhibits conversion of monomethylated H3-K27 (H3-K27me1) to dimethylated H3-K27 (H3-K27me2). As described herein, a method for treating cancer or a precancerous condition in a subject comprises administering to the subject in need thereof a therapeutically effective amount of a compound that inhibits conversion of H3-K27me2 to trimethylated H3-K27 (H3-K27me3). As described herein, a method for treating cancer or a precancerous condition in a subject comprises administering to the subject in need thereof a therapeutically effective amount of a compound that inhibits both conversion of H3-K27me1 to H3-K27me2 and conversion of H3-K27me2 to H3-K27me3. It is important to note that disease-specific increase in methylation can occur at chromatin in key genomic loci in the absence of a global increase in cellular levels of histone or protein methylation. For example, it is possible for aberrant hypermethylation at key disease-relevant genes to occur against a backdrop of global histone or protein hypomethylation.

Modulators of methylation can be used for modulating cell proliferation, generally. For example, in some cases excessive proliferation may be reduced with agents that decrease methylation, whereas insufficient proliferation may be stimulated with agents that increase methylation. Accordingly, diseases that may be treated include hyperproliferative diseases, such as benign cell growth and malignant cell growth (cancer).

Exemplary cancers that may be treated include lymphomas, including non-Hodgkin lymphoma, follicular lymphoma (FL) and diffuse large B-cell lymphoma (DLBCL); melanoma; and leukemia, including CML. Exemplary precancerous condition includes myelodisplastic syndrome (MDS; formerly known as preleukemia).

Other cancers include Acute Lymphoblastic Leukemia; Acute Myeloid Leukemia; Adrenocortical Carcinoma; AIDS-Related Cancers; AIDS-Related Lymphoma; Anal Cancer; Astrocytoma, Childhood Cerebellar; Astrocytoma, Childhood Cerebral; Basal Cell Carcinoma, see Skin Cancer (non-Melanoma); Bile Duct Cancer, Extrahepatic; Bladder Cancer; Bone Cancer, osteosarcoma/Malignant Fibrous Histiocytoma; Brain Stem Glioma; Brain Tumor; Brain Tumor, Cerebellar Astrocytoma; Brain Tumor, Cerebral Astrocytoma/Malignant Glioma; Brain Tumor, Ependymoma; Brain Tumor, Medulloblastoma; Brain Tumor, Supratentorial Primitive Neuroectodermal Tumors; Brain Tumor, Visual Pathway and Hypothalamic Glioma; Breast Cancer; Bronchial Adenomas/Carcinoids; Burkitt's Lymphoma; Carcinoid Tumor; Carcinoid Tumor, Gastrointestinal; Carcinoma of Unknown Primary; Central Nervous System Lymphoma, Primary; Cerebellar Astrocytoma; Cervical Cancer; Childhood Cancers; Chronic Lymphocytic Leukemia; Chronic Myelogenous Leukemia; Chronic Myelogenous Leukemia, Hairy Cell; Chronic Myeloproliferative Disorders; Colon Cancer; Colorectal Cancer; Cutaneous T-Cell Lymphoma, see Mycosis Fungoides and Sezary Syndrome; Endometrial Cancer; Esophageal Cancer; Ewing's Family of Tumors; Extrahepatic Bile Duct Cancer; Eye Cancer, Intraocular Melanoma; Eye Cancer, Retinoblastoma; Gallbladder Cancer; Gastric (Stomach) Cancer; Gastrointestinal Carcinoid Tumor; Germ Cell Tumor, Extracranial; Germ Cell Tumor, Extragonadal; Germ Cell Tumor, Ovarian; Gestational Trophoblastic Tumor; Glioma; Glioma, Childhood Brain Stem; Glioma, Childhood Cerebral Astrocytoma; Glioma, Childhood Visual Pathway and Hypothalamic; Hairy Cell Leukemia; Head and Neck Cancer; Hepatocellular (Liver) Cancer, Adult (Primary); Hepatocellular (Liver) Cancer, Childhood (Primary); Hodgkin's Lymphoma; Hodgkin's Lymphoma During Pregnancy; Hypopharyngeal Cancer; Hypothalamic and Visual Pathway Glioma; Intraocular Melanoma; Islet Cell Carcinoma (Endocrine Pancreas); Kaposi's Sarcoma; Kidney (Renal Cell) Cancer; Kidney Cancer; Laryngeal Cancer; Leukemia; Lip and Oral Cavity Cancer; Liver Cancer, Adult (Primary); Liver Cancer, Childhood (Primary); Lung Cancer, Non-Small Cell; Lung Cancer, Small Cell; Lymphoma,

Primary Central Nervous System; Macroglobulinemia, Waldenstrom's; Malignant Fibrous Histiocytoma of Bone/Osteosarcoma; Medulloblastoma; Melanoma; Merkel Cell Carcinoma; Mesothelioma; Mesothelioma, Adult Malignant; Metastatic Squamous Neck Cancer with Occult Primary; Multiple Endocrine Neoplasia Syndrome; Multiple Myeloma; Multiple Myeloma/Plasma Cell Neoplasm Mycosis Fungoides; Myelodysplastic Syndromes; Myelodysplastic/Myeloproliferative Diseases; Myeloid Leukemia, Adult Acute; Myeloid Leukemia, Childhood Acute; Myeloproliferative Disorders, Chronic; Nasal Cavity and Paranasal Sinus Cancer; Nasopharyngeal Cancer; Neuroblastoma; Non-Hodgkin's Lymphoma; Non-Hodgkin's Lymphoma During Pregnancy; Oral Cancer; Oral Cavity Cancer, Lip and; Oropharyngeal Cancer; Osteosarcoma/Malignant Fibrous Histiocytoma of Bone; Ovarian Cancer; Ovarian Epithelial Cancer; Ovarian Low Malignant Potential Tumor; Pancreatic Cancer; Pancreatic Cancer, Islet Cell; Paranasal Sinus and Nasal Cavity Cancer; Parathyroid Cancer; Penile Cancer; Pheochromocytoma; Pineoblastoma and Supratentorial Primitive Neuroectodermal Tumors; Pituitary Tumor; Plasma Cell Neoplasm/Multiple Myeloma; Pleuropulmonary Blastoma; Pregnancy and Breast Cancer; Prostate Cancer; Rectal Cancer; Retinoblastoma; Rhabdomyosarcoma; Salivary Gland Cancer; Sarcoma, Ewing's Family of Tumors; Sarcoma, Soft Tissue; Sarcoma, Uterine; Sezary Syndrome; Skin Cancer; Skin Cancer (non-Melanoma); Small Intestine Cancer; Soft Tissue Sarcoma; Squamous Cell Carcinoma, see Skin Cancer (non-Melanoma); Squamous Neck Cancer with Occult Primary, Metastatic; Stomach (Gastric) Cancer; Testicular Cancer; Thymoma; Thymoma and Thymic Carcinoma; Thyroid Cancer; Transitional Cell Cancer of the Renal Pelvis and Ureter; Trophoblastic Tumor, Gestational; Unknown Primary Site, Cancer of; Unusual Cancers of Childhood; Urethral Cancer; Uterine Cancer, Endometrial; Uterine Sarcoma; Vaginal Cancer; Visual Pathway and Hypothalamic Glioma; Vulvar Cancer; Waldenstrom's Macroglobulinemia; Wilms' Tumor; and Women's Cancers.

Any other disease in which epigenetic methylation, which is mediated by EZH2, plays a role may be treatable or preventable using compounds and methods described herein.

For example, neurologic diseases that may be treated include epilepsy, schizophrenia, bipolar disorder or other psychological and/or psychiatric disorders, neuropathies, skeletal muscle atrophy, and neurodegenerative diseases, e.g., a neurodegenerative disease. Exemplary neurodegenerative diseases include: Alzheimer's, Amyotrophic Lateral Sclerosis (ALS), and Parkinson's disease. Another class of neurodegenerative diseases includes diseases caused at least in part by aggregation of poly-glutamine. Diseases of this class include: Huntington's Diseases, Spinalbulbar Muscular Atrophy (SBMA or Kennedy's Disease), Dentatorubropallidoluysian Atrophy (DRPLA), Spinocerebellar Ataxia 1 (SCA1), Spinocerebellar Ataxia 2 (SCA2), Machado-Joseph Disease (MJD; SCA3), Spinocerebellar Ataxia 6 (SCA6), Spinocerebellar Ataxia 7 (SCA7), and Spinocerebellar Ataxia 12 (SCA12).

## COMBINATION THERAPY

In one aspect of the invention, an EZH2 antagonist, or a pharmaceutically acceptable salt thereof, can be used in combination with another therapeutic agent to treat diseases such as cancer and/or neurological disorders. For example, the additional agent can be a therapeutic agent that is art-recognized as being useful to treat the disease or condition being treated by the compound of the present invention. The additional agent also can be an agent that imparts a beneficial attribute to the therapeutic composition (e.g., an agent that affects the viscosity of the composition).

The combination therapy contemplated by the invention includes, for example, administration of a compound of the invention, or a pharmaceutically acceptable salt thereof, and additional agent(s) in a single pharmaceutical formulation as well as administration of a compound of the invention, or a pharmaceutically acceptable salt thereof, and additional agent(s) in separate pharmaceutical formulations. In other words, co-administration shall mean the administration of at least two agents to a subject so as to provide the beneficial effects of the combination of both agents. For example, the agents may be administered simultaneously or sequentially over a period of time.

The agents set forth below are for illustrative purposes and not intended to be limiting. The combinations, which are part of this invention, can be the compounds of the present invention and at least one additional agent selected from the lists below. The combination can also include more than one additional agent, e.g., two or three additional agents if the combination is such that the formed composition can perform its intended function.

For example, one aspect of the invention relates to an EZH2 antagonist for use in combination with another agent for the treatment of cancer and/or a neurological disorder. In one embodiment, an additional agent is an anticancer agent that is a compound that affects histone modifications, such as an HDAC inhibitor. In certain embodiments, an additional anticancer agent is selected from the group consisting of chemotherapetics (such as 2CdA, 5-FU, 6-Mercaptopurine, 6-TG, Abraxane™, Accutane®, Actinomycin-D, Adriamycin®, Alimta®, all-trans retinoic acid, amethopterin, Ara-C, Azacitadine, BCNU, Blenoxane®, Camptosar®, CeeNU®, Clofarabine, Clolar™, Cytoxan®, daunorubicin hydrochloride, DaunoXome®, Dacogen®, DIC, Doxil®, Ellence®, Eloxatin®, Emcyt®, etoposide phosphate, Fludara®, FUDR®, Gemzar®, Gleevec®, hexamethylmelamine, Hycamtin®, Hydrea®, Idamycin®, Ifex®, ixabepilone, Ixempra®, L-asparaginase, Leukeran®, liposomal Ara-C, L-PAM, Lysodren, Matulane®, mithracin, Mitomycin-C, Myleran®, Navelbine®, Neutrexin®, nilotinib, Nipent®, Nitrogen Mustard, Novantrone®, Oncaspar®, Panretin®, Paraplatin®, Platinol®, prolifeprospan 20 with carmustine implant, Sandostatin®, Targretin®, Tasigna®, Taxotere®, Temodar®, TESPA,

Trisenox®, Valstar®, Velban®, Vidaza™, vincristine sulfate, VM 26, Xeloda® and Zanosar®); biologics (such as Alpha Interferon, Bacillus Calmette-Guerin, Bexxar®, Campath®, Ergamisol®, Erlotinib, Herceptin®, Interleukin-2, Iressa®, lenalidomide, Mylotarg®, Ontak®, Pegasys®, Revlimid®, Rituxan®, Tarceva™, Thalomid®, Velcade® and Zevalin™); small molecules (such as Tykerb®); corticosteroids (such as dexamethasone sodium phosphate, DeltaSone® and Delta-Cortef®); hormonal therapies (such as Arimidex®, Aromasin®, Casodex®, Cytadren®, Eligard®, Eulexin®, Evista®, Faslodex®, Femara®, Halotestin®, Megace®, Nilandron®, Nolvadex®, Plenaxis™ and Zoladex®); and radiopharmaceuticals (such as Iodotope®, Metastron®, Phosphocol® and Samarium SM-153).

## DOSAGE

As used herein, a "therapeutically effective amount" or "therapeutically effective dose" is an amount of an EZH2 antagonist or a combination of two or more such compounds, which inhibits, totally or partially, the progression of the condition or alleviates, at least partially, one or more symptoms of the condition. A therapeutically effective amount can also be an amount which is prophylactically effective. The amount which is therapeutically effective will depend upon the patient's size and gender, the condition to be treated, the severity of the condition and the result sought. In one embodiment, a therapeutically effective dose refers to that amount of the EZH2 antagonists that result in amelioration of symptoms in a patient. For a given patient, a therapeutically effective amount may be determined by methods known to those of skill in the art.

Toxicity and therapeutic efficacy of EZH2 antagonists can be determined by standard pharmaceutical procedures in cell cultures or experimental animals, e.g., for determining the maximum tolerated dose (MTD) and the $ED_{50}$ (effective dose for 50% maximal response). The dose ratio between toxic and therapeutic effects is the therapeutic index and it can be expressed as the ratio between MTD and $ED_{50}$. The data obtained from these cell culture assays and animal studies can be used in formulating a range of dosage for use in humans. Dosage may also be guided by monitoring the EZH2 antagonist's effect on pharmacodynamic markers of enzyme inhibition (e.g., histone methylation or target gene expression) in diseased or surrogate tissue. Cell culture or animal experiments can be used to determine the relationship between doses required for changes in pharmacodynamic markers and doses required for therapeutic efficacy can be determined in cell culture or animal experiments or early stage clinical trials. The dosage of such EZH2 antagonists lies preferably within a range of circulating concentrations that include the $ED_{50}$ with little or no toxicity. The dosage may vary within this range depending upon the dosage form employed and the route of administration utilized. The exact formulation, route of administration and dosage can be chosen by the individual physician in view of the patient's condition. In the treatment of crises, the administration of an acute bolus or an infusion approaching the MTD may be required to obtain a rapid response.

Dosage amount and interval may be adjusted individually to provide plasma levels of the active moiety which are sufficient to maintain the methyltransferase modulating effects, or minimal effective concentration (MEC) for the required period of time to achieve therapeutic efficacy. The MEC will vary for each EZH2 antagonist but can be estimated from *in vitro* data and animal experiments. Dosages necessary to achieve the MEC will depend on individual characteristics and route of administration. However, high pressure liquid chromatography (HPLC) assays or bioassays can be used to determine plasma concentrations.

Dosage intervals can also be determined using the MEC value. EZH2 antagonists should be administered using a regimen which maintains plasma levels above the MEC for 10-90% of the time, preferably between 30-90% and most preferably between 50-90% until the desired amelioration of symptoms is achieved. Alternatively, different MEC plasma levels may be maintained for differing amounts of time. In cases of local administration or selective uptake, the effective local concentration of the drug may not be related to plasma concentration.

One of skill in the art can select from a variety of administration regimens and the amount of EZH2 antagonist administered will, of course, be dependent on the subject being treated, on the subject's weight, the severity of the affliction, the manner of administration and the judgment of the prescribing physician.

## COMPOUNDS AND PHARMACEUTICAL COMPOSITIONS

Aspects of the invention concern compounds which are useful according to the methods described herein. These compounds are referred to herein as "inhibitors of EZH2" and, equivalently, "EZH2 antagonists". The compounds can be presented as the compounds *per se,* pharmaceutically acceptable salts of the compounds, or as pharmaceutical compositions.

Other compounds are described in PCT/US2012/026953, filed on February 28, 2012; U.S. Provisional Application Serial Nos: 61/474821, filed on April 13, 2011; 61/474825, filed on April 13, 2011; 61/499595, filed on June 21, 2011; and 61/505676, filed on July 8, 2011.

Exemplary EZH2 inhibitor compounds of the present invention are shown in Table 1. In the table below, each occurrence of

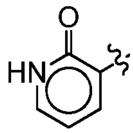

should be construed as

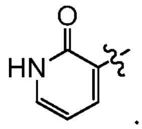

**Table 1**

| Compound Number | Structure |
|---|---|
| 2 | 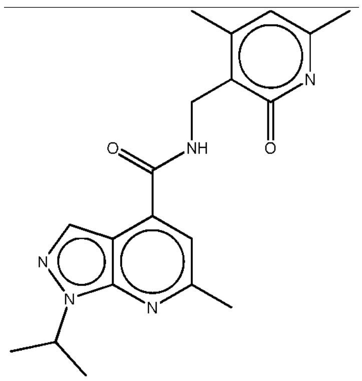 |
| 3 | 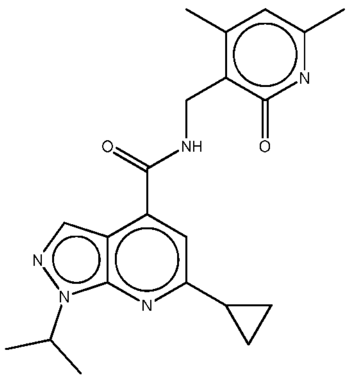 |
| 4 | 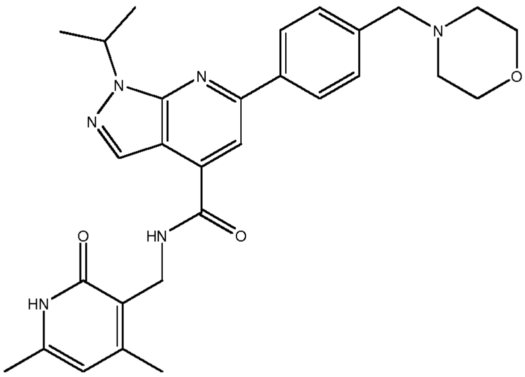 |

(continued)

| Compound Number | Structure |
|---|---|
| 5 | 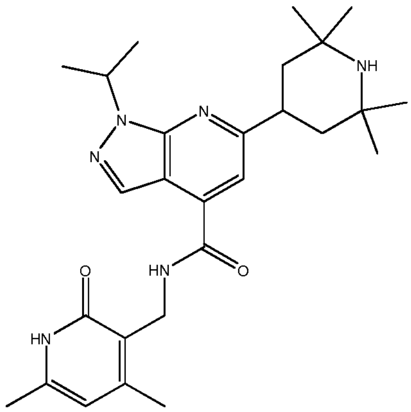 |
| 6 | 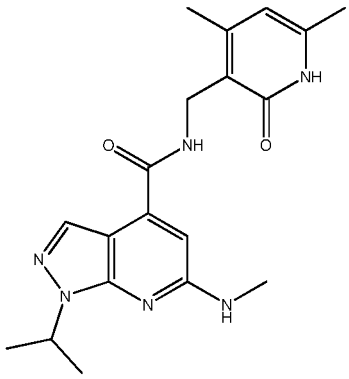 |
| 7 | 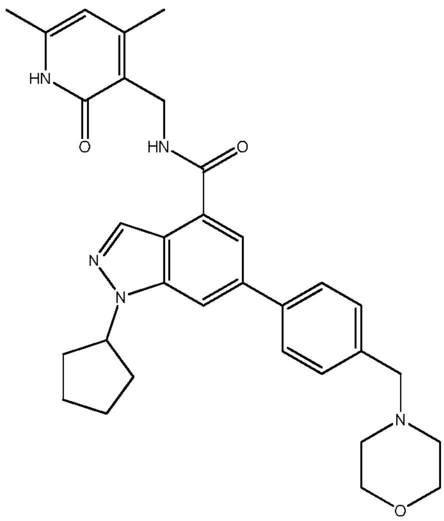 |

(continued)

| Compound Number | Structure |
|---|---|
| 8 | 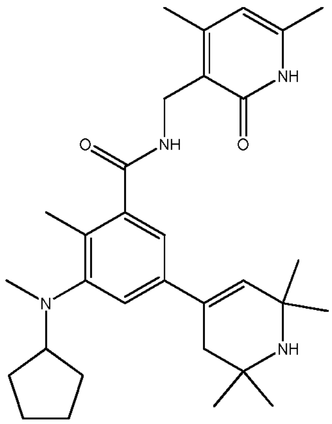 |
| 9 | 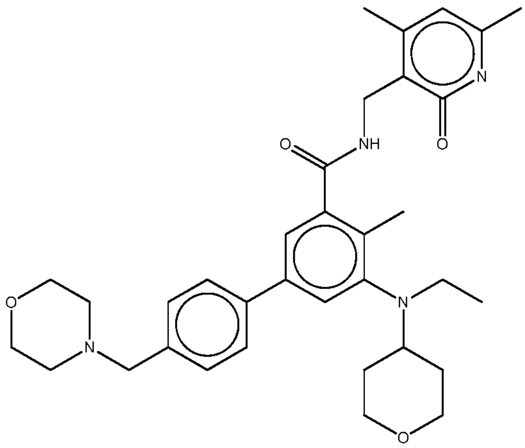 |
| 10 | 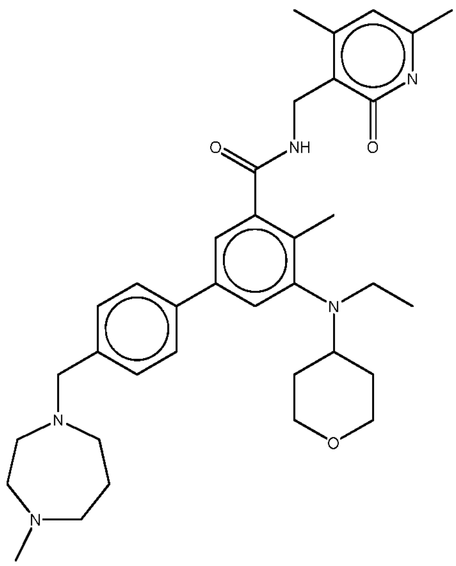 |

(continued)

| Compound Number | Structure |
|---|---|
| 11 | 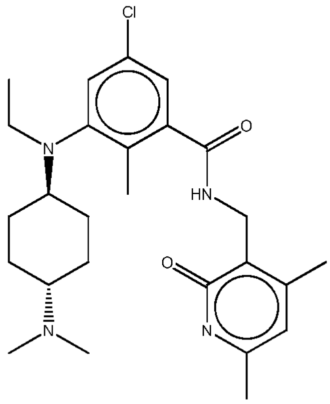 |
| 12 | 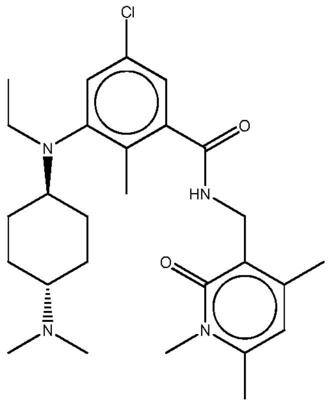 |
| 13 | 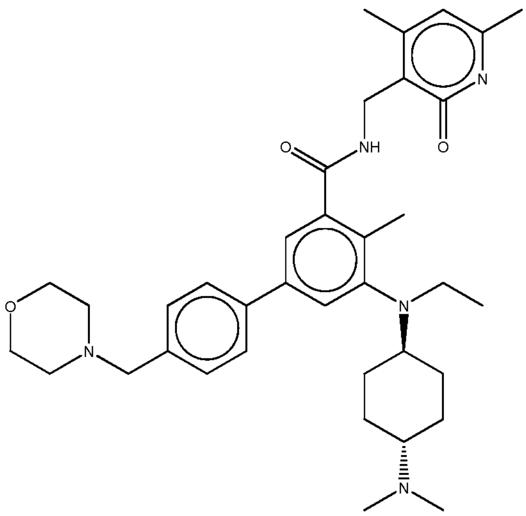 |

(continued)

| Compound Number | Structure |
|---|---|
| 14 | 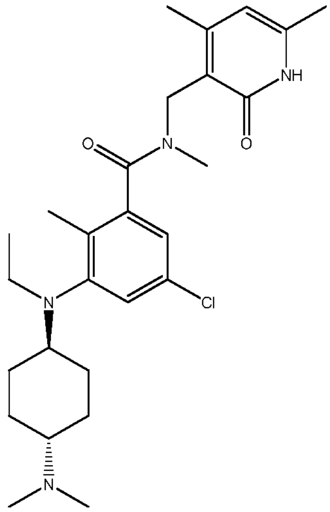 |
| 15 | 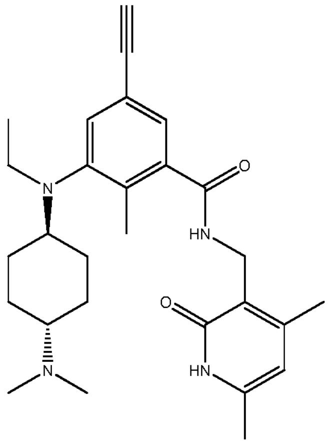 |
| 16 | 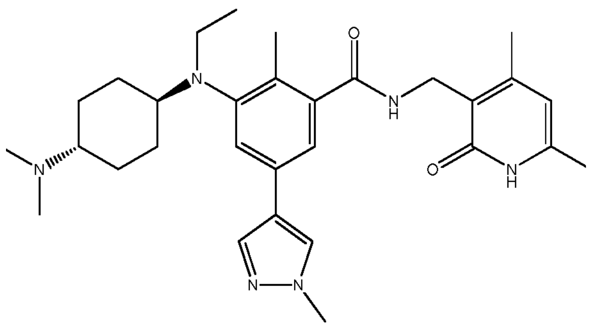 |

(continued)

| Compound Number | Structure |
|---|---|
| 17 | 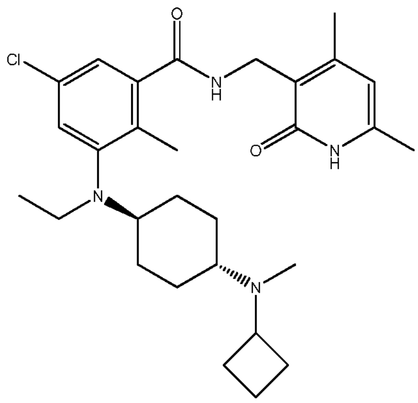 |
| 18 | 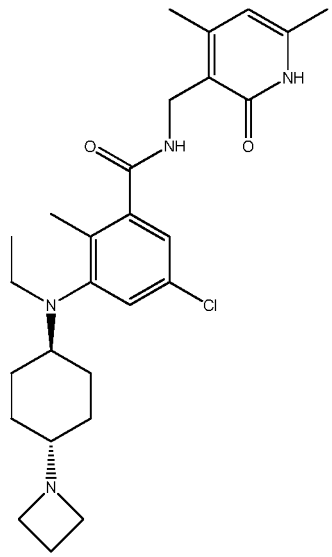 |
| 19 | 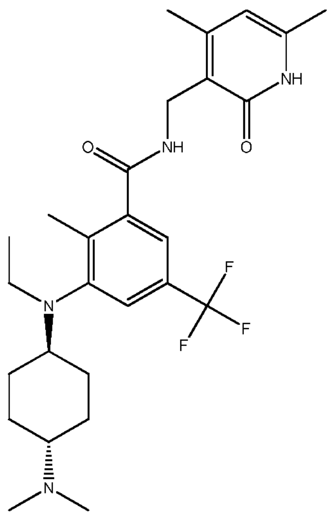 |

(continued)

| Compound Number | Structure |
|---|---|
| 20 | 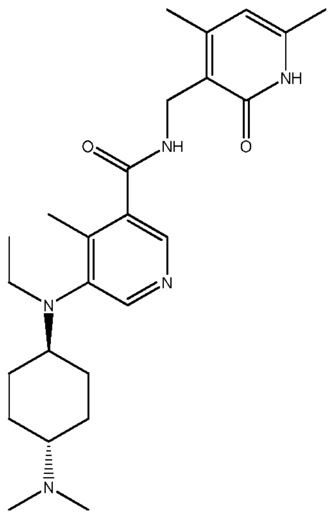 |
| 21 | 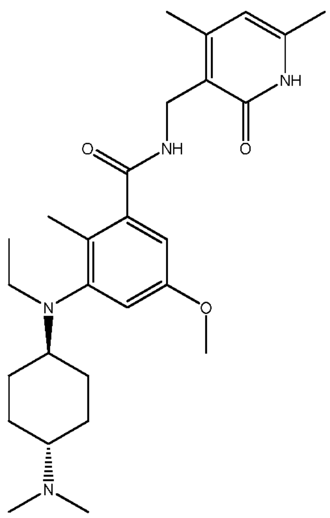 |
| 22 | 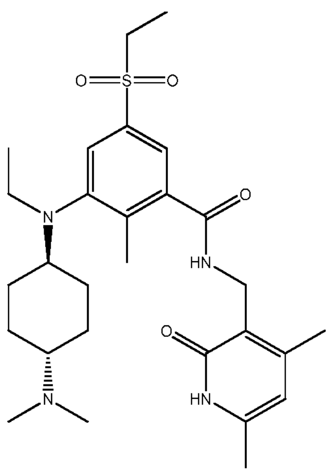 |

(continued)

| Compound Number | Structure |
|---|---|
| 23 | 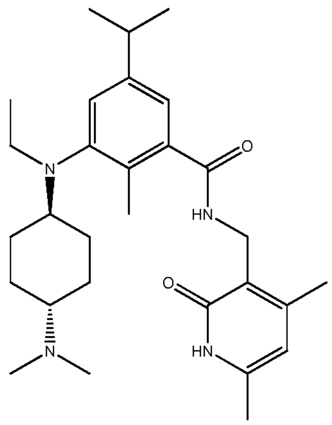 |
| 24 | 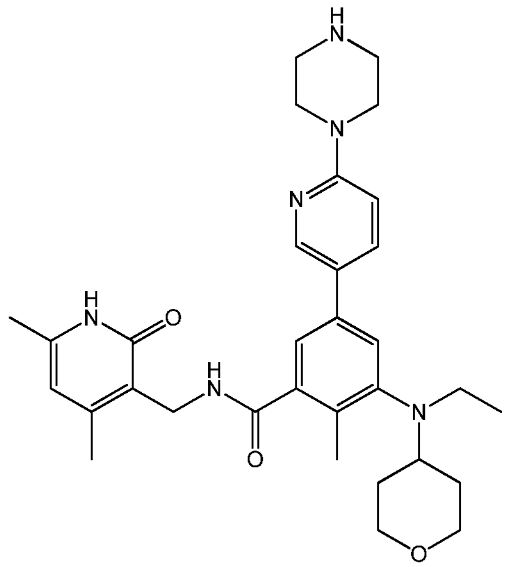 |
| 25 | 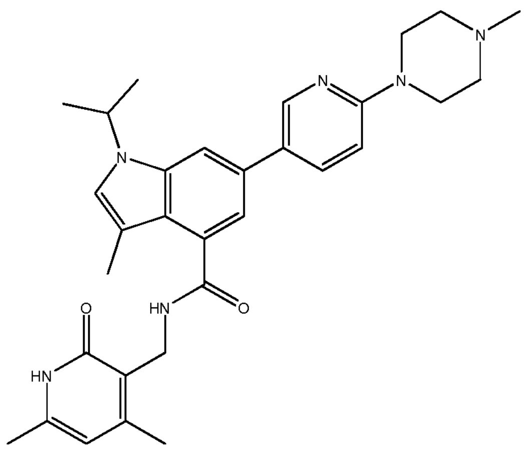 |

(continued)

| Compound Number | Structure |
|---|---|
| 26 | 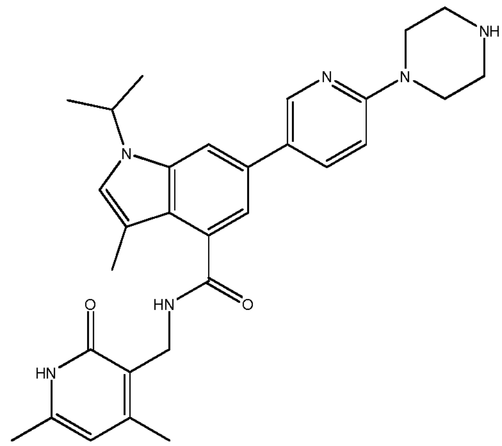 |
| 27 | 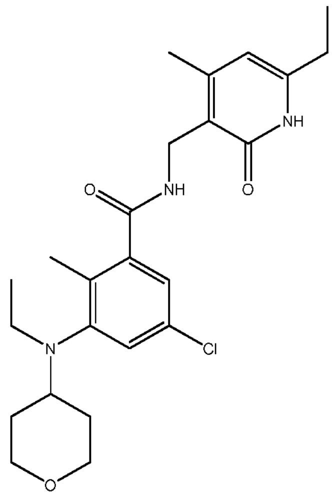 |
| 28 | 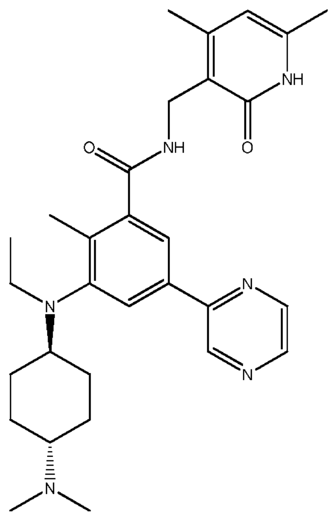 |

(continued)

| Compound Number | Structure |
|---|---|
| 29 | 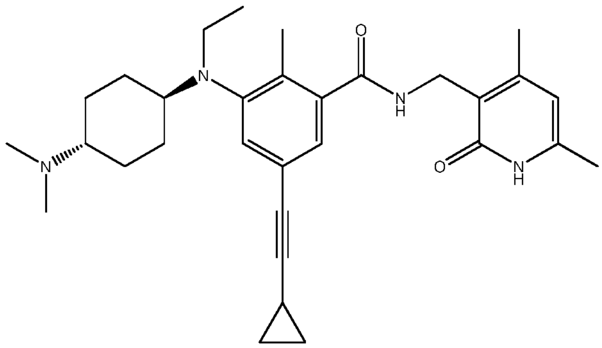 |
| 30 | 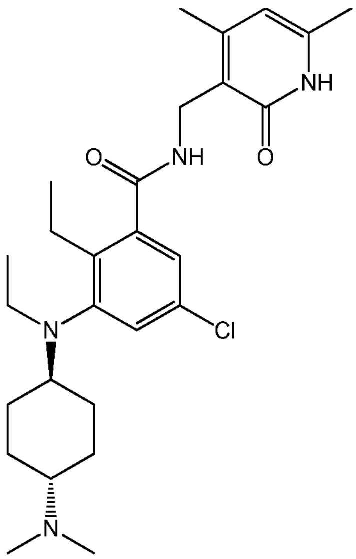 |
| 31 | 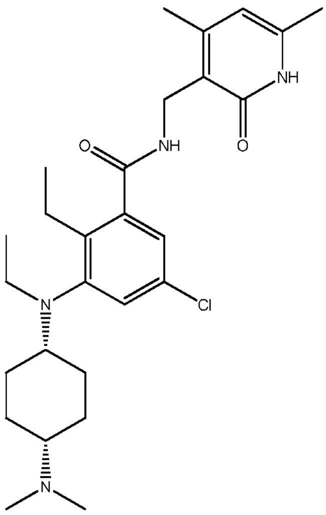 |

(continued)

| Compound Number | Structure |
|---|---|
| 32 | 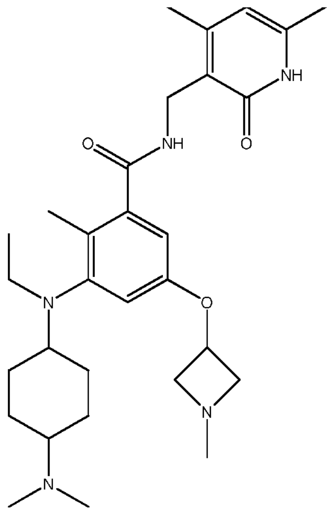 |
| 33 | 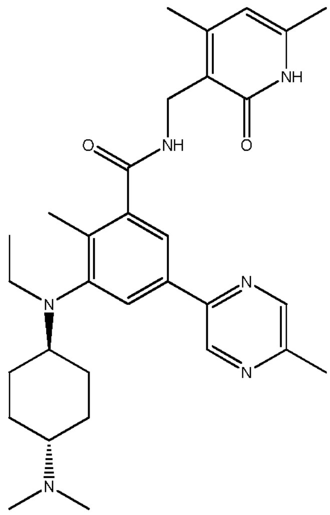 |
| 34 | 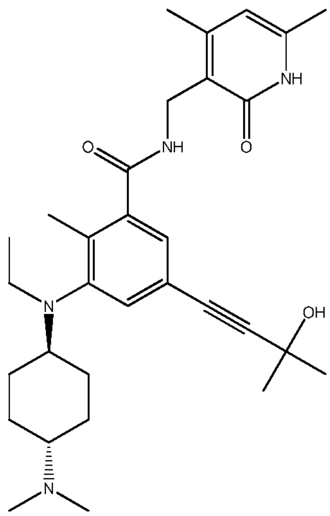 |

(continued)

| Compound Number | Structure |
|---|---|
| 35 | 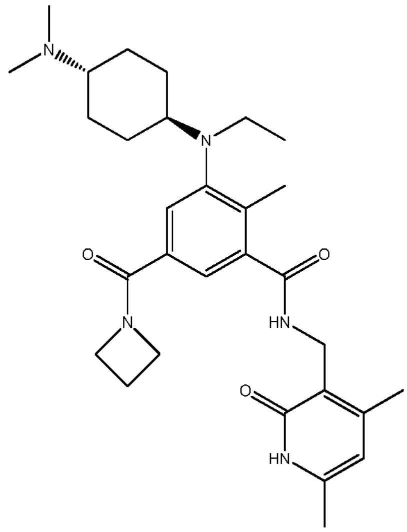 |
| 36 | 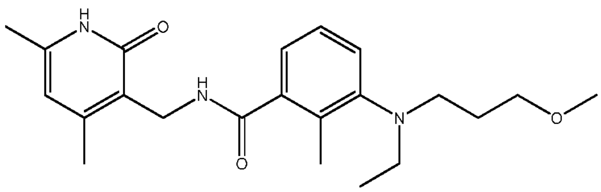 |
| 37 | 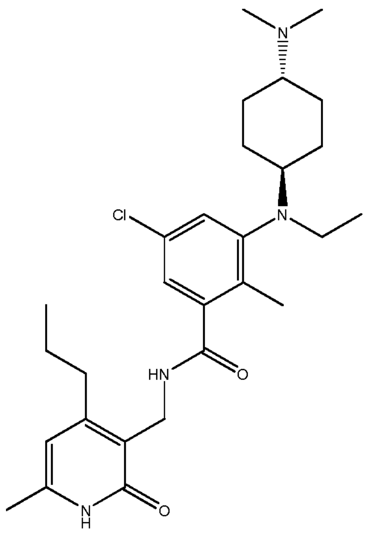 |
| 38 | 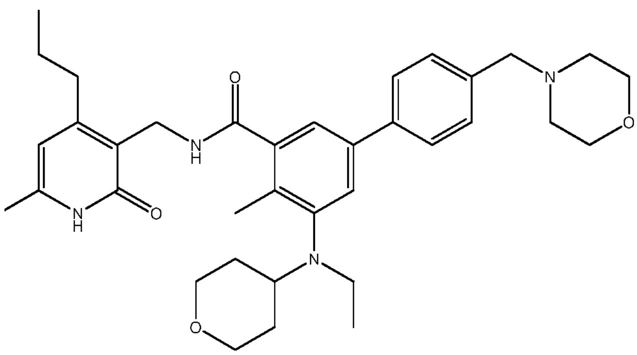 |

(continued)

| Compound Number | Structure |
|---|---|
| 39 | 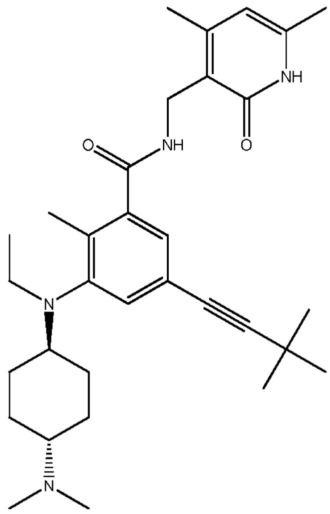 |
| 40 | 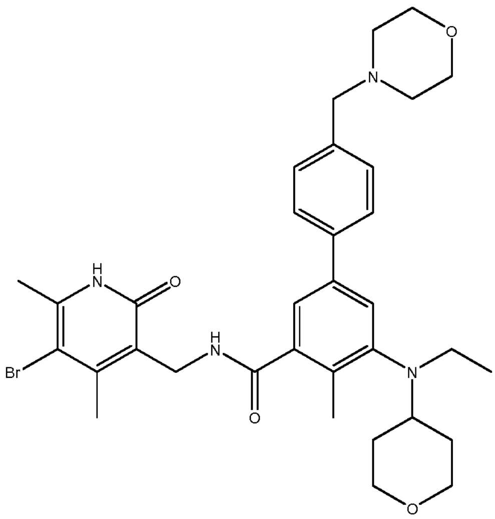 |
| 41 | 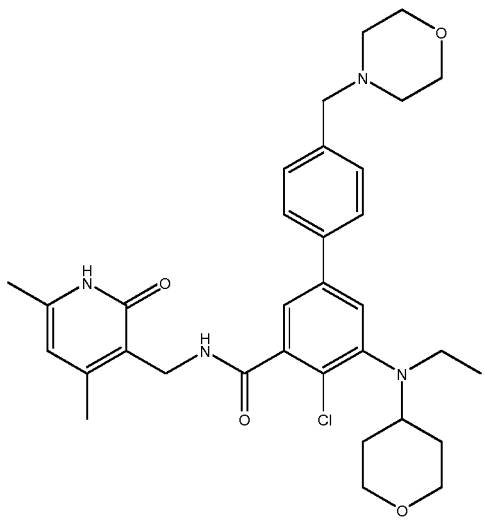 |

(continued)

| Compound Number | Structure |
|---|---|
| 42 | 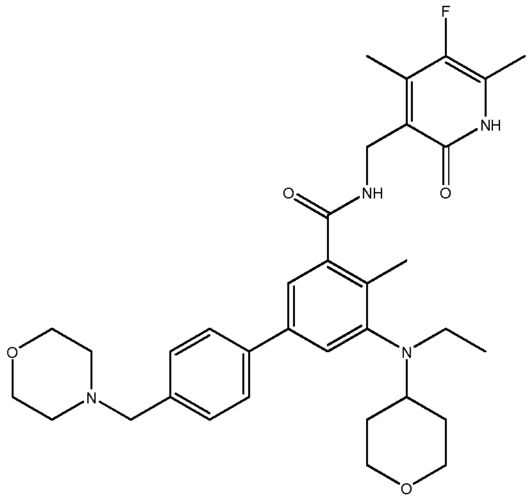 |
| 43 | 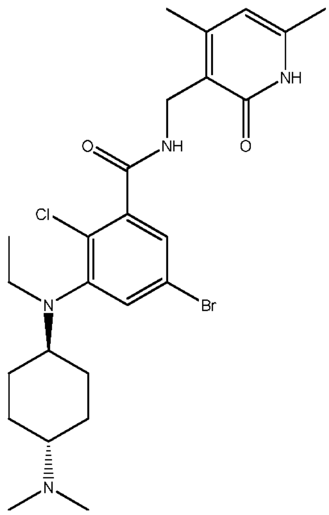 |
| 44 | 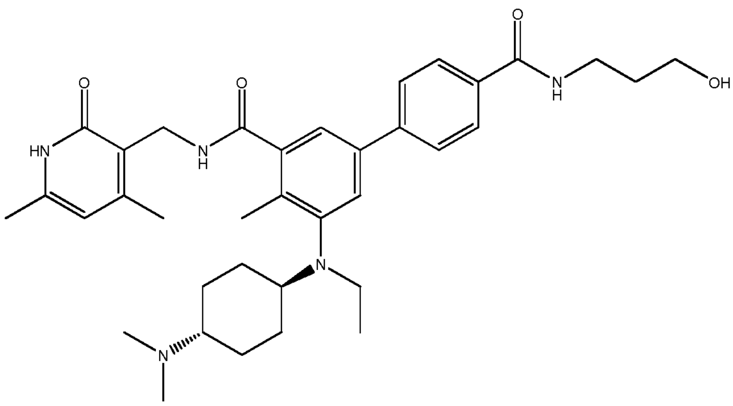 |

(continued)

| Compound Number | Structure |
|---|---|
| 45 | 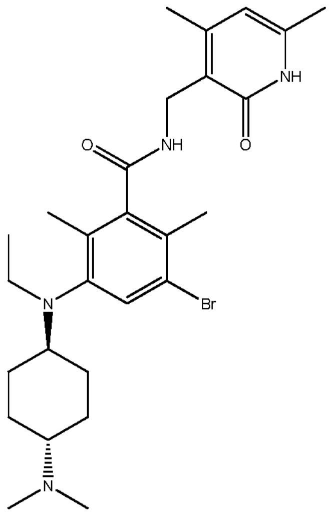 |
| 46 | 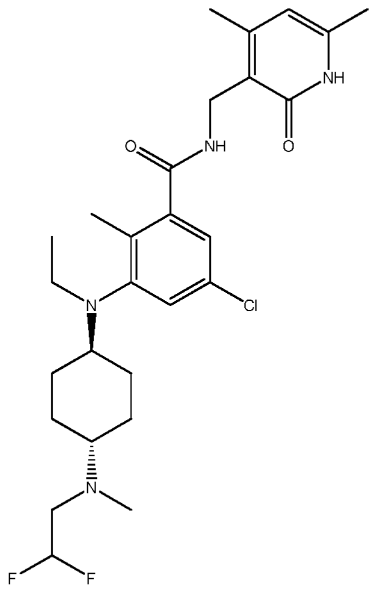 |

(continued)

| Compound Number | Structure |
|---|---|
| 47 | 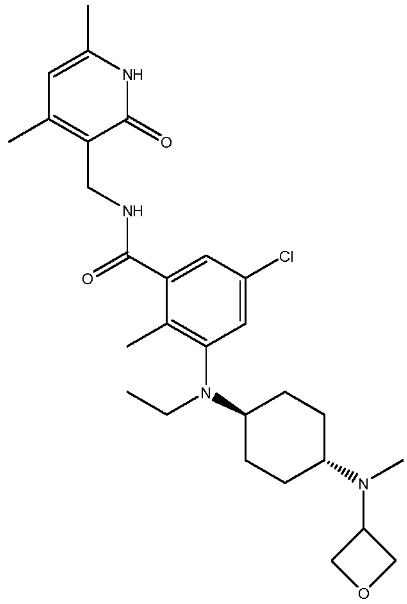 |
| 48 | 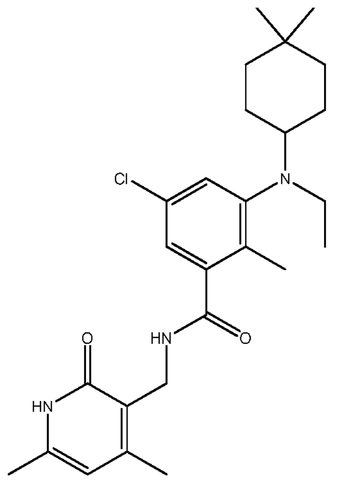 |
| 49 | 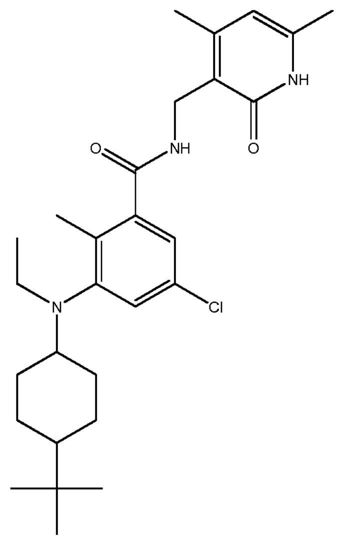 |

(continued)

| Compound Number | Structure |
|---|---|
| 50 | 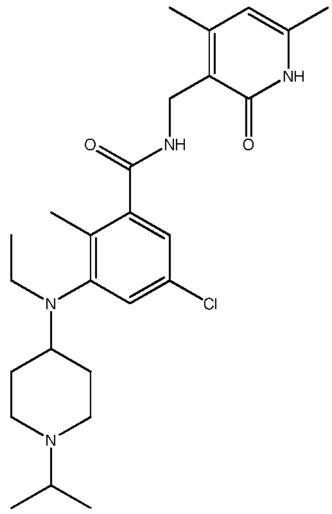 |
| 51 | 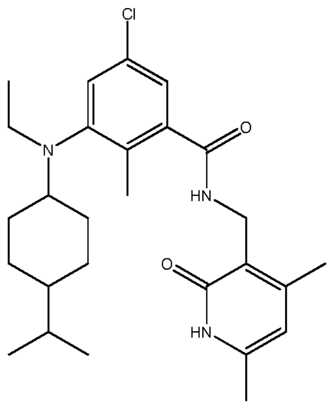 |
| 52 | 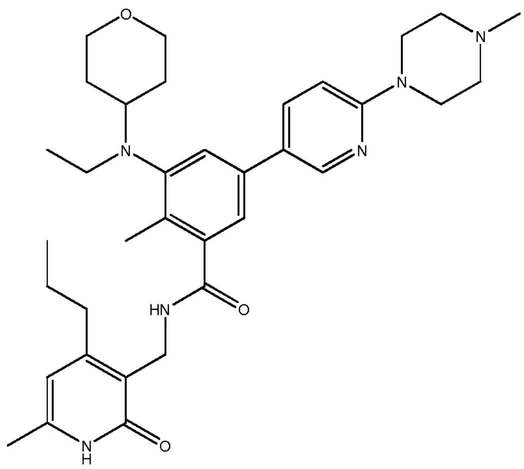 |

(continued)

| Compound Number | Structure |
|---|---|
| 53 | 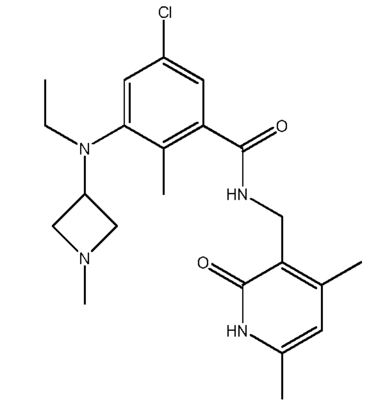 |
| 54 | 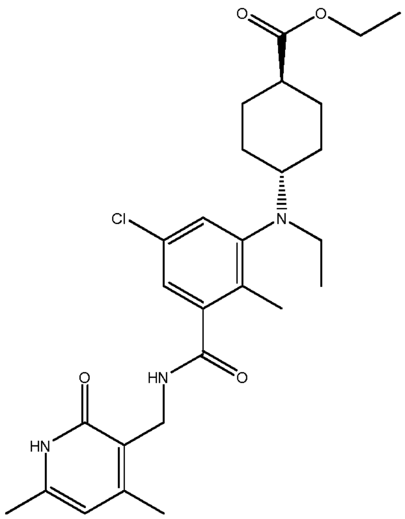 |
| 55 | 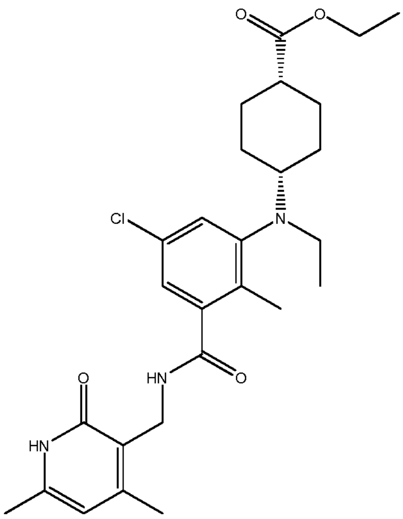 |

(continued)

| Compound Number | Structure |
|---|---|
| 56 | 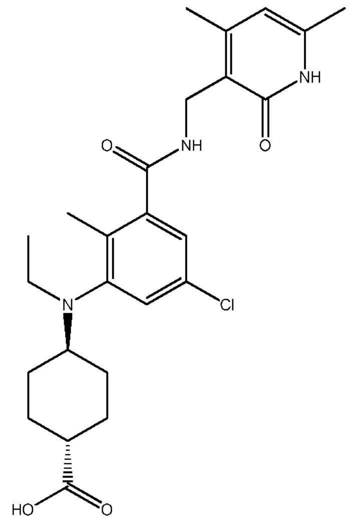 |
| 57 | 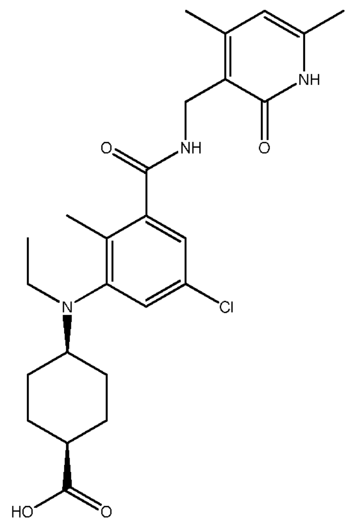 |

(continued)

| Compound Number | Structure |
|---|---|
| 58 | 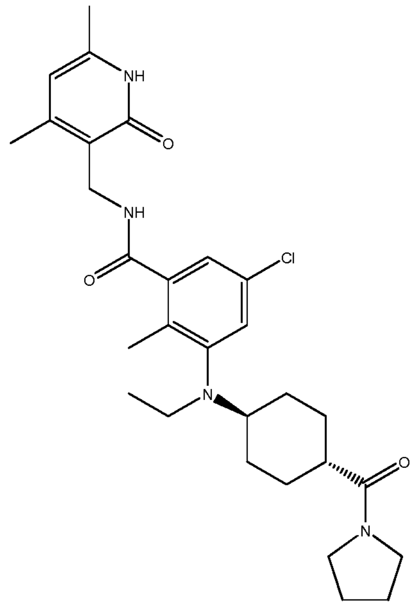 |
| 59 | 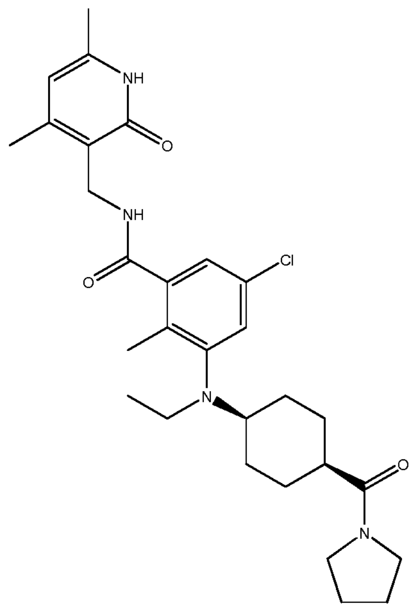 |

(continued)

| Compound Number | Structure |
|---|---|
| 60 | 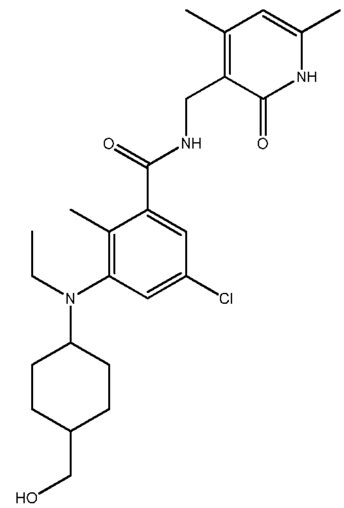 |
| 61 | 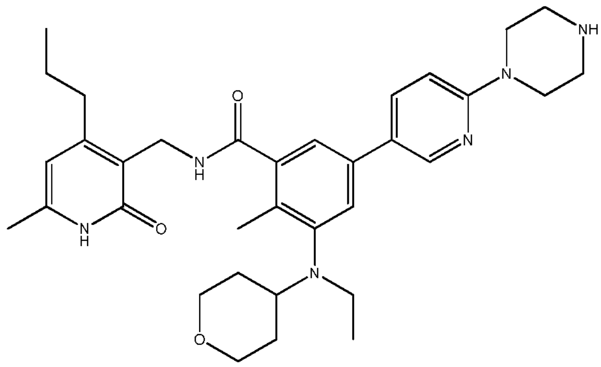 |
| 62 | 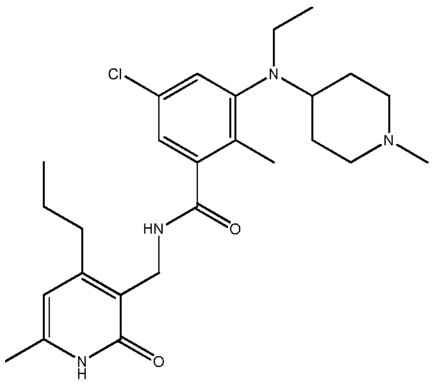 |

An "effective amount" means that amount of a drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

The term "optionally" means that the subsequently described event(s) may or may not occur, and includes both event(s), which occur, and events that do not occur.

Herein, the term "pharmaceutically-acceptable salts" refers to salts that retain the desired biological activity of the subject compound and exhibit minimal undesired toxicological effects. These pharmaceutically-acceptable salts may be prepared in situ during the final isolation and purification of the compound, or by separately reacting the purified

compound in its free acid or free base form with a suitable base or acid, respectively.

By the term "co-administering" and derivatives thereof as used herein is meant either simultaneous administration or any manner of separate sequential administration of one or more additional pharmaceutically active compounds, whether for treating cancer, the side effects of cancer or cancer therapy, or some other disease. Preferably, if the administration is not simultaneous, the compounds are administered in a close time proximity to each other. Furthermore, it does not matter if the compounds are administered in the same dosage form, e.g. one compound may be administered topically and another compound may be administered orally.

The compounds decribed herein include the compounds themselves, as well as their pharmaceutically acceptable salts, their solvates, their N-oxides, and their prodrugs, if applicable. In certain embodiments, compounds decribed herein may contain an acidic functional group, one acidic enough to form salts. Representative salts include pharmaceutically acceptable metal salts such as sodium, potassium, lithium, calcium, magnesium, aluminum, and zinc salts; carbonates and bicarbonates of a pharmaceutically-acceptable metal salts such as sodium, potassium, lithium, calcium, magnesium, aluminum, and zinc; pharmaceutically-acceptable organic primary, secondary, and tertiary amines including aliphatic amines, aromatic amines, aliphatic diamines, and hydroxy alkylamines such as methylamine, ethylamine, 2-hydroxyethylamine, diethylamine, triethylamine, ethylenediamine, ethanolamine, diethanolamine, and cyclohexylamine.

In certain embodiments, compounds decribed herein may contain a basic functional group and are therefore capable of forming pharmaceutically-acceptable acid addition salts by treatment with a suitable acid. Suitable acids include pharmaceutically-acceptable inorganic acids and pharmaceutically-acceptable organic acids. Representative pharmaceutically acceptable acid addition salts include hydrochloride, hydrobromide, nitrate, methylnitrate, sulfate, bisulfate, sulfamate, phosphate, acetate, hydroxyacetate, phenylacetate, propionate, butyrate, isobutyrate, valerate, maleate, hydroxymaleate, acrylate, fumarate, malate, tartrate, citrate, salicylate, *p*-aminosalicylate, glycollate, lactate, heptanoate, phthalate, oxalate, succinate, benzoate, o-acetoxybenzoate, chlorobenzoate, methylbenzoate, dinitrobenzoate, hydroxybenzoate, methoxybenzoate, mandelate, tannate, formate, stearate, ascorbate, palmitate, oleate, pyruvate, pamoate, malonate, laurate, glutarate, glutamate, estolate, methanesulfonate (mesylate), ethanesulfonate (esylate), 2-hydroxyethanesulfonate, benzenesulfonate (besylate), *p*-aminobenzenesulfonate, *p*-toluenesulfonate (tosylate) and napthalene-2-sulfonate.

Compounds of the present invention that contain nitrogens can be converted to N-oxides by treatment with an oxidizing agent (*e.g.,* 3-chloroperoxybenzoic acid (*m*CPBA) and/or hydrogen peroxides) to afford other compounds of the present invention. Thus, all shown and claimed nitrogen-containing compounds are considered, when allowed by valency and structure, to include both the compound as shown and its N-oxide derivative (which can be designated as $N \rightarrow O$ or $N^+-O^-$). Furthermore, in other instances, the nitrogens in the compounds of the present invention can be converted to N-hydroxy or N-alkoxy compounds. For example, N-hydroxy compounds can be prepared by oxidation of the parent amine by an oxidizing agent such as *m*-CPBA. All shown and claimed nitrogen-containing compounds are also considered, when allowed by valency and structure, to cover both the compound as shown and its N-hydroxy (*i.e.,* N-OH) and N-alkoxy (*i.e.,* N-OR, wherein R is substituted or unsubstituted $C_1$-$C_6$ alkyl, $C_1$-$C_6$ alkenyl, $C_1$-$C_6$ alkynyl, 3-14-membered carbocycle or 3-14-membered heterocycle) derivatives.

In the present specification, the structural formula of the compound represents a certain isomer for convenience in some cases, but the present invention includes all isomers, such as geometrical isomers, optical isomers based on an asymmetrical carbon, stereoisomers, tautomers, and the like. In addition, a crystal polymorphism may be present for the compounds represented by the formula. It is noted that any crystal form, crystal form mixture, or anhydride or hydrate thereof is included in the scope of the present invention. Furthermore, so-called metabolite which is produced by degradation of the present compound *in vivo* is included in the scope of the present invention.

"Isomerism" means compounds that have identical molecular formulae but differ in the sequence of bonding of their atoms or in the arrangement of their atoms in space. Isomers that differ in the arrangement of their atoms in space are termed "stereoisomers." Stereoisomers that are not mirror images of one another are termed "diastereoisomers," and stereoisomers that are non-superimposable mirror images of each other are termed "enantiomers" or sometimes optical isomers. A mixture containing equal amounts of individual enantiomeric forms of opposite chirality is termed a "racemic mixture."

A carbon atom bonded to four nonidentical substituents is termed a "chiral center."

"Chiral isomer" means a compound with at least one chiral center. Compounds with more than one chiral center may exist either as an individual diastereomer or as a mixture of diastereomers, termed "diastereomeric mixture." When one chiral center is present, a stereoisomer may be characterized by the absolute configuration (R or S) of that chiral center. Absolute configuration refers to the arrangement in space of the substituents attached to the chiral center. The substituents attached to the chiral center under consideration are ranked in accordance with the *Sequence Rule* of Cahn, Ingold and Prelog. (Cahn et al., Angew. Chem. Inter. Edit. 1966, 5, 385; errata 511; Cahn et al., Angew. Chem. 1966, 78, 413; Cahn and Ingold, J. Chem. Soc. 1951 (London), 612; Cahn et al., Experientia 1956, 12, 81; Cahn, J. Chem. Educ. 1964, 41, 116).

"Geometric isomer" means the diastereomers that owe their existence to hindered rotation about double bonds

or a cycloalkyl linker (e.g., 1,3-cylcobutyl). These configurations are differentiated in their names by the prefixes cis and trans, or Z and E, which indicate that the groups are on the same or opposite side of the double bond in the molecule according to the Cahn-Ingold-Prelog rules.

It is to be understood that the compounds of the present invention may be depicted as different chiral isomers or geometric isomers. It should also be understood that when compounds have chiral isomeric or geometric isomeric forms, all isomeric forms are intended to be included in the scope of the present invention, and the naming of the compounds does not exclude any isomeric forms.

Furthermore, the structures and other compounds discussed in this invention include all atropic isomers thereof. "Atropic isomers" are a type of stereoisomer in which the atoms of two isomers are arranged differently in space. Atropic isomers owe their existence to a restricted rotation caused by hindrance of rotation of large groups about a central bond. Such atropic isomers typically exist as a mixture, however as a result of recent advances in chromatography techniques, it has been possible to separate mixtures of two atropic isomers in select cases.

"Tautomer" is one of two or more structural isomers that exist in equilibrium and is readily converted from one isomeric form to another. This conversion results in the formal migration of a hydrogen atom accompanied by a switch of adjacent conjugated double bonds. Tautomers exist as a mixture of a tautomeric set in solution. In solutions where tautomerization is possible, a chemical equilibrium of the tautomers will be reached. The exact ratio of the tautomers depends on several factors, including temperature, solvent and pH. The concept of tautomers that are interconvertable by tautomerizations is called tautomerism.

Of the various types of tautomerism that are possible, two are commonly observed. In keto-enol tautomerism a simultaneous shift of electrons and a hydrogen atom occurs. Ring-chain tautomerism arises as a result of the aldehyde group (-CHO) in a sugar chain molecule reacting with one of the hydroxy groups (-OH) in the same molecule to give it a cyclic (ring-shaped) form as exhibited by glucose.

Common tautomeric pairs are: ketone-enol, amide-nitrile, lactam-lactim, amide-imidic acid tautomerism in heterocyclic rings (*e.g.,* in nucleobases such as guanine, thymine and cytosine), imine-enamine and enamine-enamine. An example of keto-enol equilibria is between pyridin-2(1H)-ones and the corresponding pyridin-2-ols, as shown below.

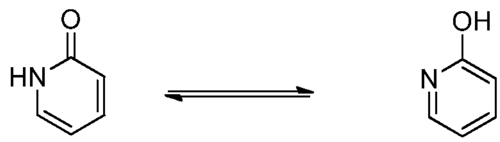

pyridin-2(1*H*)-one pyridin-2-ol

It is to be understood that the compounds of the present invention may be depicted as different tautomers. It should also be understood that when compounds have tautomeric forms, all tautomeric forms, including mixtures thereof, are intended to be included in the scope of the present invention, and the naming of the compounds does not exclude any tautomer form.

The compounds decribed herein may be prepared in crystalline or non-crystalline form, and, if crystalline, may optionally be solvated, e.g., as the hydrate. This invention includes within its scope stoichiometric solvates (e.g., hydrates) as well as compounds containing variable amounts of solvent (e.g., water).

Certain of the compounds described herein may contain one or more chiral atoms, or may otherwise be capable of existing as two enantiomers. The compounds claimed below include mixtures of enantiomers as well as purified enantiomers or enantiomerically enriched mixtures. Also included within the scope of the invention are the individual isomers of the compounds decribed herein, or claimed below, as well as any wholly or partially equilibrated mixtures thereof. The present invention also covers the individual isomers of the claimed compounds as mixtures with isomers thereof in which one or more chiral centers are inverted.

Where there are different isomeric forms they may be separated or resolved one from the other by conventional methods, or any given isomer may be obtained by conventional synthetic methods or by stereospecific or asymmetric syntheses.

While it is possible that, for use in therapy, a compound decribed herein, as well as salts, solvates, N-oxides, and the like, may be administered as a neat preparation, i.e., no additional carrier, the more usual practice is to present the active ingredient confected with a carrier or diluent. Accordingly, decribed herein are pharmaceutical compositions, which include a compound decribed herein and salts, solvates and the like, and one or more pharmaceutically acceptable carriers, diluents, or excipients. The compounds of decribed herein and salts, solvates, N-oxides, etc, are as described above. The carrier(s), diluent, or excipient(s) must be acceptable in the sense of being compatible with the other ingredients of the formulation and not deleterious to the recipient thereof. Described herein there is also provided a process for the preparation of a pharmaceutical formulation including admixing a compound decribed herein, or salts, solvates,

N-oxides, etc, with one or more pharmaceutically acceptable carriers, diluents or excipients.

It will be appreciated by those skilled in the art that certain protected derivatives of compounds decribed herein, which may be made prior to a final deprotection stage, may not possess pharmacological activity as such, but may, in certain instances, be administered orally or parenterally and thereafter metabolized in the body to form compounds of the invention which are pharmacologically active. Such derivatives may therefore be described as "prodrugs". Further, certain compounds of the invention may act as prodrugs of other compounds of the invention. It will further be appreciated by those skilled in the art, that certain moieties, known to those skilled in the art as "pro-moieties" may be placed on appropriate functionalities when such functionalities are present within compounds of the invention. Preferred prodrugs for compounds of the invention include: esters, carbonate esters, hemi-esters, phosphate esters, nitro esters, sulfate esters, sulfoxides, amides, carbamates, azo-compounds, phosphamides, glycosides, ethers, acetals, and ketals.

Additionally, the compounds of the present invention, for example, the salts of the compounds, can exist in either hydrated or unhydrated (the anhydrous) form or as solvates with other solvent molecules. Nonlimiting examples of hydrates include monohydrates, dihydrates, etc. Nonlimiting examples of solvates include ethanol solvates, acetone solvates, etc.

"Solvate" means solvent addition forms that contain either stoichiometric or non stoichiometric amounts of solvent. Some compounds have a tendency to trap a fixed molar ratio of solvent molecules in the crystalline solid state, thus forming a solvate. If the solvent is water the solvate formed is a hydrate; and if the solvent is alcohol, the solvate formed is an alcoholate. Hydrates are formed by the combination of one or more molecules of water with one molecule of the substance in which the water retains its molecular state as $H_2O$.

As used herein, the term "analog" refers to a chemical compound that is structurally similar to another but differs slightly in composition (as in the replacement of one atom by an atom of a different element or in the presence of a particular functional group, or the replacement of one functional group by another functional group). Thus, an analog is a compound that is similar or comparable in function and appearance, but not in structure or origin to the reference compound.

As defined herein, the term "derivative" refers to compounds that have a common core structure, and are substituted with various groups as described herein.

The term "bioisostere" refers to a compound resulting from the exchange of an atom or of a group of atoms with another, broadly similar, atom or group of atoms. The objective of a bioisosteric replacement is to create a new compound with similar biological properties to the parent compound. The bioisosteric replacement may be physicochemically or topologically based. Examples of carboxylic acid bioisosteres include, but are not limited to, acyl sulfonimides, tetrazoles, sulfonates and phosphonates. See, *e.g.,* Patani and LaVoie, Chem. Rev. 96, 3147-3176, 1996.

The present invention is intended to include all isotopes of atoms occurring in the present compounds. Isotopes include those atoms having the same atomic number but different mass numbers. By way of general example and without limitation, isotopes of hydrogen include tritium and deuterium, and isotopes of carbon include C-13 and C-14.

An EZH2 antagonist and optionally other therapeutics can be administered *per se* (neat) or in the form of a pharmaceutically acceptable salt. When used in medicine the salts should be pharmaceutically acceptable, but non-pharmaceutically acceptable salts can conveniently be used to prepare pharmaceutically acceptable salts thereof.

Compounds useful in accordance with the invention may be provided as salts with pharmaceutically compatible counterions (i.e., pharmaceutically acceptable salts). A "pharmaceutically acceptable salt" means any non-toxic salt that, upon administration to a recipient, is capable of providing, either directly or indirectly, a compound or a prodrug of a compound useful in accordance with this invention. A "pharmaceutically acceptable counterion" is an ionic portion of a salt that is not toxic when released from the salt upon administration to a subject. Pharmaceutically compatible salts may be formed with many acids, including but not limited to hydrochloric, sulfuric, acetic, lactic, tartaric, malic, and succinic acids. Salts tend to be more soluble in water or other protic solvents than their corresponding free base forms. The present invention includes the use of such salts.

Pharmaceutically acceptable acid addition salts include those formed with mineral acids such as hydrochloric acid and hydrobromic acid, and also those formed with organic acids such as maleic acid. For example, acids commonly employed to form pharmaceutically acceptable salts include inorganic acids such as hydrogen bisulfide, hydrochloric, hydrobromic, hydroiodic, sulfuric and phosphoric acid, as well as organic acids such as para-toluenesulfonic, salicylic, tartaric, bitartaric, ascorbic, maleic, besylic, fumaric, gluconic, glucuronic, formic, glutamic, methanesulfonic, ethanesulfonic, benzenesulfonic, lactic, oxalic, para-bromophenylsulfonic, carbonic, succinic, citric, benzoic and acetic acid, and related inorganic and organic acids. Such pharmaceutically acceptable salts thus include sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, terephathalate, sulfonate, xylenesulfonate, phenylacetate, phenylpropionate, phenylbutyrate, citrate, lactate, β-hydroxybutyrate, glycolate, maleate, tartrate, methanesulfonate, propanesulfonate, naphthalene-1-sulfonate, naphthalene-2-sulfonate,

mandelate and the like.

Suitable bases for forming pharmaceutically acceptable salts with acidic functional groups include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or trialkylamines; dicyclohexylamine; tributyl amine; pyridine; N-methyl,N-ethylamine; diethylamine; triethylamine; mono-, bis-, or tris-(2-hydroxy-lower alkyl amines), such as mono-, bis-, or tris-(2-hydroxyethyl)amine, 2-hydroxy-tert-butylamine, or tris-(hydroxymethyl)methylamine, N,N-di alkyl-N-(hydroxy alkyl)-amines, such as N,N-dimethyl-N-(2-hydroxyethyl)amine, or tri-(2-hydroxyethyl)amine; N-methyl-D-glucamine; and amino acids such as arginine, lysine, and the like.

Certain compounds useful in accordance with the invention and their salts may exist in more than one crystalline form (i.e., polymorph); the present invention includes the use of each of the crystal forms and mixtures thereof.

Certain compounds useful in accordance with the invention may contain one or more chiral centers, and exist in different optically active forms. When compounds useful in accordance with the invention contain one chiral center, the compounds exist in two enantiomeric forms and the present invention includes the use of both enantiomers and mixtures of enantiomers, such as racemic mixtures thereof. The enantiomers may be resolved by methods known to those skilled in the art; for example, enantiomers may be resolved by formation of diastereoisomeric salts which may be separated, for example, by crystallization; formation of diastereoisomeric derivatives or complexes which may be separated, for example, by crystallization, gas-liquid or liquid chromatography; selective reaction of one enantiomer with an enantiomer-specific reagent, for example, via enzymatic esterification; or gas-liquid or liquid chromatography in a chiral environment, for example, on a chiral support (e.g., silica with a bound chiral ligand) or in the presence of a chiral solvent. Where the desired enantiomer is converted into another chemical entity by one of the separation procedures described above, a further step may be used to liberate the desired purified enantiomer. Alternatively, specific enantiomers may be synthesized by asymmetric synthesis using optically active reagents, substrates, catalysts or solvents, or by converting one enantiomer into the other by asymmetric transformation.

When a compound useful in accordance with the invention contains more than one chiral center, it may exist in diastereoisomeric forms. The diastereoisomeric compounds may be separated by methods known to those skilled in the art (for example, chromatography or crystallization) and the individual enantiomers may be separated as described above. The present invention includes the use of various diastereoisomers of compounds useful in accordance with the invention, and mixtures thereof. Compounds useful in accordance with the invention may exist in different tautomeric forms or as different geometric isomers, and the present invention includes the use of each tautomer and/or geometric isomer of compounds useful in accordance with the invention, and mixtures thereof. Compounds useful in accordance with the invention may exist in zwitterionic form. The present invention includes the use of each zwitterionic form of compounds useful in accordance with the invention, and mixtures thereof.

## KITS

An EZH2 antagonist may, if desired, be presented in a kit (e.g., a pack or dispenser device) which may contain one or more unit dosage forms containing the EZH2 antagonist. The pack may for example comprise metal or plastic foil, such as a blister pack. The pack or dispenser device may be accompanied by instructions for administration. Compositions comprising an EZH2 antagonist of the invention formulated in a compatible pharmaceutical carrier may also be prepared, placed in an appropriate container, and labeled for treatment of an indicated condition. Instructions for use may also be provided.

Also provided herein are kits comprising a plurality of methylation detection reagents that detect the methylated H3-K27. For example, the kit includes mono-methylated H3-K27, di-methylated H3-K27 and tri-methylated H3-K27 detection reagents. The detection reagent is for example antibodies or fragments thereof, polypeptide or aptamers.

A kit may also include an EZH2 mutant detection reagent, e.g., nucleic acids that specifically identify a mutant EZH2 nucleic acid sequence by having homologous nucleic acid sequences, such as oligonucleotide sequences, complementary to a portion of the mutant EZH2 nucleic acid sequence or antibodies to proteins encoded by the mutant EZH2 nucleic acids packaged together in the form of a kit. The oligonucleotides can be fragments of the EZH2 gene. For example the oligonucleotides can be 200, 150, 100, 50, 25, 10 or less nucleotides in length. The kit may contain in separate containers an aptamer or an antibody, control formulations (positive and/or negative), and/or a detectable label such as fluorescein, green fluorescent protein, rhodamine, cyanine dyes, Alexa dyes, luciferase, radiolabels, among others. Instructions (*e.g.,* written, tape, VCR, CD-ROM, etc.) for carrying out the assay may be included in the kit. The assay may for example be in the form of a Western Blot analysis, Immunohistochemistry (IHC), immunofluorescence (IF), sequencing and Mass spectrometry (MS) as known in the art.

## DEFINITIONS

For convenience, certain terms employed in the specification, examples, and appended claims are collected here. All definitions, as defined and used herein, supersede dictionary definitions, definitions in documents incorporated by reference, and/or ordinary meanings of the defined terms.

The articles "a" and "an" are used herein to refer to one or to more than one (i.e., to at least one) of the grammatical object of the article. By way of example, "an element" means one element or more than one element.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods described herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively, as set forth in the United States Patent Office Manual of Patent Examining Procedures, Section 2111.03.

The terms "co-administration" and "co-administering" refer to both concurrent administration (administration of two or more therapeutic agents at the same time) and time varied administration (administration of one or more therapeutic agents at a time different from that of the administration of an additional therapeutic agent or agents), as long as the therapeutic agents are present in the patient to some extent at the same time.

The term "precancerous condition" or "premalignant condition" refers to a disease, syndrome, or finding that, if left untreated, may lead to cancer. It is a generalized state associated with a significantly increased risk of cancer.

The term "treating" as used herein refers to alleviate of at least one symptom of the disease, disorder or condition. The term encompasses the administration and/or application of one or more compounds described herein, to a subject, for the purpose of providing management of, or remedy for a condition. "Treatment" for the purposes of this disclosure, may, but does not have to, provide a cure; rather, "treatment" may be in the form of management of the condition. When the compounds described herein are used to treat unwanted proliferating cells, including cancers, "treatment" includes partial or total destruction of the undesirable proliferating cells with minimal destructive effects on normal cells. A desired mechanism of treatment of unwanted rapidly proliferating cells, including cancer cells, at the

cellular level is apoptosis.

The term "preventing" as used herein includes either preventing or slowing the onset of a clinically evident disease progression altogether or preventing or slowing the onset of a preclinically evident stage of a disease in individuals at risk. This includes prophylactic treatment of those at risk of developing a disease.

The term "subject" as used herein for purposes of treatment includes any human subject who has been diagnosed with, has symptoms of, or is at risk of developing a cancer or a precancerous condition. For methods of prevention the subject is any human subject. To illustrate, for purposes of prevention, a subject may be a human subject who is at risk of or is genetically predisposed to obtaining a disorder characterized by unwanted, rapid cell proliferation, such as cancer. The subject may be at risk due to exposure to carcinogenic agents, being genetically predisposed to disorders characterized by unwanted, rapid cell proliferation, and so on.

Except as otherwise indicated, standard methods can be used for the production of recombinant and synthetic polypeptides, fusion proteins, antibodies or antigen-binding fragments thereof, manipulation of nucleic acid sequences, production of transformed cells, and the like. Such techniques are known to those skilled in the art. See, e.g., Sambrook et al., Molecular Cloning: A Laboratory Manual, 3rd Ed. (Cold Spring Harbor, N.Y., 2001); F.M. Ausubel et al. Current Protocols in Molecular Biology (Green Publishing Associates, Inc. and John Wiley & Sons, Inc., New York).

The term "EZH2 polypeptide" encompasses functional fragments of the full-length polypeptides and functional equivalents of either of the foregoing that have substantially similar or substantially identical amino acid sequences (at least about 75%, 80%, 85%, 90%, 95% 98% or more amino acid sequence similarity or identity), where the functional fragment or functional equivalent retains one or more of the functional properties of the native polypeptide.

By "functional" it is meant that the polypeptide (or nucleic acid) has the same or substantially similar activity with respect to one or more of the biological properties of the native polypeptide (or nucleic acid), e.g., at least about 50%, 75%, 85%, 90%, 95% or 98% or more of the activity of the native polypeptide (or nucleic acid).

The term "modulate" (and grammatical equivalents) refers to an increase or decrease in activity. The term "increase" or "enhance" (and grammatical equivalents) may mean an elevation by at least about 25%, 50%, 75%, 2-fold, 3-fold, 5-fold, 10-fold, 15-fold, 20-fold or more. The terms "decrease" or "reduce" (and grammatical equivalents) means a diminishment by at least about 25%, 40%, 50%, 60%, 75%, 80%, 85%, 90%, 95%, 98% or more. The indicated activity, substance or other parameter may not be detectable. Specifically provided are antagonists of EZH2.

The term "pharmacodynamic marker" refers to a molecular marker of drug response that can be measured in patients receiving the drug. The marker should be a direct measure of modulation of the drug target and be able to show quantitative changes in response to dose. A potential pharmacodynamic marker for EZH2 antagonists could be levels of histone H3-K27 methylation in disease or surrogate tissue.

As used herein, the term "responsiveness" is interchangeable with terms "responsive", "sensitive", and "sensitivity", and it is meant that a subject showing therapeutic response when administered an EZH inhibitor, *e.g.,* tumor cells or tumor tissues of the subject undergo apoptosis and/or necrosis, and/or display reduced growing, dividing, or proliferation..

The term "control" or "reference" refers to methylation levels (*e.g.,* monomethylation level, dimethylation level or trimethylation level) detected in an adjacent non-tumor tissue isolated from the subject, detected in a healthy tissue from a healthy subject, or established by a pathologist with standard methods in the art.

By "sample" it means any biological sample derived from the subject, includes but is not limited to, cells, tissues samples and body fluids (including, but not limited to, mucus, blood, plasma, serum, urine, saliva, and semen).

Throughout the description, where compositions are described as having, including, or comprising specific components, it is contemplated that compositions also consist essentially of, or consist of, the recited components. Similarly, where methods or processes are described as having, including, or comprising specific process steps, the processes also consist essentially of, or consist of, the recited processing steps. Further, it should be understood that the order of steps or order for performing certain actions is immaterial so long as the invention remains operable. Moreover, two or more steps or actions can be conducted simultaneously.

The synthetic processes of the invention can tolerate a wide variety of functional groups; therefore various substituted starting materials can be used. The processes generally provide the desired final compound at or near the end of the overall process, although it may be desirable in certain instances to further convert the compound to a pharmaceutically acceptable salt, ester or prodrug thereof.

Compounds of the present invention can be prepared in a variety of ways using commercially available starting materials, compounds known in the literature, or from readily prepared intermediates, by employing standard synthetic methods and procedures either known to those skilled in the art, or which will be apparent to the skilled artisan in light of the teachings herein. Standard synthetic methods and procedures for the preparation of organic molecules and functional group transformations and manipulations can be obtained from the relevant scientific literature or from standard textbooks in the field. Although not limited to any one or several sources, classic texts such as Smith, M. B., March, J., March’s Advanced Organic Chemistry: Reactions, Mechanisms, and Structure, 5th edition, John Wiley & Sons: New York, 2001; and Greene, T.W., Wuts, P.G. M., Protective Groups in Organic Synthesis, 3rd edition, John Wiley & Sons:

New York, 1999, are useful and recognized reference textbooks of organic synthesis known to those in the art. The following descriptions of synthetic methods are designed to illustrate, but not to limit, general procedures for the preparation of compounds of the present invention.

Compounds of the present invention can be conveniently prepared by a variety of methods familiar to those skilled in the art. The compounds of this invention with each of the formulae described herein may be prepared according to the following procedures from commercially available starting materials or starting materials which can be prepared using literature procedures. These procedures show the preparation of representative compounds of this invention.

Compounds designed, selected and/or optimized by methods described above, once produced, can be characterized using a variety of assays known to those skilled in the art to determine whether the compounds have biological activity. For example, the molecules can be characterized by conventional assays, including but not limited to those assays described below, to determine whether they have a predicted activity, binding activity and/or binding specificity.

Furthermore, high-throughput screening can be used to speed up analysis using such assays. As a result, it can be possible to rapidly screen the molecules described herein for activity, using techniques known in the art. General methodologies for performing high-throughput screening are described, for example, in Devlin (1998) High Throughput Screening, Marcel Dekker; and U.S. Patent No. 5,763,263. High-throughput assays can use one or more different assay techniques including, but not limited to, those described below.

Citation of publications and patent documents is not intended as an admission that any is pertinent prior art, nor does it constitute any admission as to the contents or date of the same. The invention having now been described by way of written description, those of skill in the art will recognize that the invention can be practiced in a variety of embodiments and that the foregoing description and examples below are for purposes of illustration and not limitation of the claims that follow.

## EXAMPLES

The invention now being generally described, it will be more readily understood by reference to the following examples, which are included merely for purposes of illustration of certain aspects and embodiments of the present invention, and are not intended to limit the invention.

### **Example** 1 - **Recombinant Five-Component PRC2 Complex**

Wild-type EZH2 (GenBank Accession No. NM_004456) or Tyr641 mutants were co-expressed with wild-type AEBP2 (GenBank Accession No. NM_153207), EED (GenBank Accession No. NM_003797), SUZ12 (GenBank Accession No. NM_015355) and RbAp48 (GenBank Accession No. NM_005610) in *Spodoptera frugiperda* (Sf9) cells using a baculovirus expression system. An N-terminal FLAG tag on the EED was used to purify active PRC2 complex from cell lysates (BPS Bioscience, catalog number 51004). The purity of the final PRC2 preparations was assessed by SDS-PAGE with Coomassie blue staining.

### Example 2 - H3, H4 Peptide Panel

A library consisting of 44 peptides of 15 amino acids each was synthesized by $21^{st}$ Century Biochemicals (Marlboro, MA). This peptide panel encompassed all of the amino acids of human histones H3 and H4 with 5 residue overlaps between consecutive peptide sequences. The N-terminus of each peptide was appended with biotin, and the C-termini were represented as the amide. Purity (> 95%) and identity were confirmed by liquid chromatography/mass spectral analysis.

For study of the H3-K27 methylation status dependence of enzyme activity, peptides were synthesized representing the amino acid sequence of human H3 from residues 21-44 (H3:21-44) with lysine 27 represented as the unmodified, mono-methylated, di-methylated or tri-methylated side chain amine. These peptides were purchased from New England Peptide (Gardner, MA) with biotin appended to the C-terminus of each peptide.

### Example 3 - Evaluation of H3-K27 Methylation Status in Cells

The cell lines OCI-LY19 (ACC 528), KARPAS-422 (ACC 32), and WSU-DLCL2 (ACC 575) were obtained from DSMZ. The cell lines DB (CRL-2289) and SU-DHL6 (CRL-2959) were obtained from ATCC. OCI-LY19, WSU-DLCL2, and DB cell lines were grown in RPMI-1640 with 10% FBS, and KARPAS-422 and SU-DHL6 cell lines were grown in RPMI-1640 plus 20% FBS. Cells were grown to a density of 1.5-2 x$10^6$ cells/mL and 1 x$10^7$ cells were harvested by centrifugation at 264 x g, washed in ice cold PBS and lysed by resuspension in a 10X pellet volume of RIPA lysis buffer containing 50 mM Tris-HCl, 15 0mM NaCl, 0.25% DOC, 1% NP-40, and 1 mM EDTA (Millipore #20-188), plus 0.1% SDS and protease inhibitor tablets (Roche # 1836153). Lysates were sonicated by 2 rounds of 10 1-second bursts at

setting 3 with a Misonix XL-2000 to ensure efficient histone extraction, and cleared by centrifugation at 4° C using a bench top centrifuge at 14,000 rpm for 10 minutes. Protein concentration was determined by BCA assay (Pierce). Four micrograms of each lysate was fractionated on 4-20% Tris-Glycine gel (Invitrogen), transferred to PVDF, and probed with the following antibodies in Odyssey blocking buffer: mouse anti-EZH2 (CST 3147; 1:2000 dilution), rabbit anti-H3-K27me3 (CST 9733; 1:10000 dilution), rabbit anti-H3-K27me2 (CST 9755; 1:5000 dilution), rabbit anti-H3-K27me1 (Active Motif 39377; 1:5000 dilution), and mouse anti-Total H3 (CST 3638; 1:20000 dilution). Following primary Ab incubation, membranes were probed with IRDye 800CW donkey-anti-mouse IgG (LiCOR #926-32212) or Alexa Fluor 680 goat-anti-rabbit IgG (Invitrogen #A-21076) secondary Ab and imaged using the LiCOR Odyssey system.

**Example 4 - Enzymology**

As noted above, it had previously been concluded that the disease-associated changes at Tyr641 resulted in loss of function with respect to EZH2-catalyzed H3-K27 methylation. However, a presumptive reduction in the rate of H3-K27 methylation due to enzyme heterozygosity was difficult to rationalize as the basis for a malignant phenotype, especially in light of previous data indicating that overexpression of EZH2, loss-of-function mutations in the corresponding H3-K27 demethylase *UTX,* or overexpression of components of the PRC2, such as PHF19/PCL3, involved in increased H3-K27 trimethylation, all result in malignant phenotypes in specific human cancers. Morin et al. (2010) Nat Genet 42:181-5; Martinez-Garcia et al. (2010) Nat Genet 42:100-1; Bracken et al. (2003) EMBO J 22:5323-35; Kleer et al. (2003) Proc Natl Acad Sci USA 100:11606-11; Varambally et al. (2002) Nature 419:624-9; Simon et al. (2008) Mutat Res 647:21-9; van Haaften et al. (2009) Nat Genet 41:521-3; Wang et al. (2004) Gene 343:69-78; Cao et al. (2008) Mol Cell Biol 28:1862-72; and Sarma et al. (2008) Mol Cell Biol 28:2718-31). Therefore, the enzymology of these mutations was explored in greater detail.

Recombinant PRC2 complexes were prepared with WT and Tyr641 mutant versions of human EZH2 (see Example 1 above; Cao et al. (2004) Mol Cell 15:57-67). Equal concentrations (nominally 8 nM, based on protein determinations) of each complex were initially tested for the ability to catalyze $^3$H-methyl transfer from labeled S-adenosyl methionine (SAM) to an unmodified peptide representing the amino acid sequence surrounding H3-K27 (H3:21-44) or to native avian erythrocyte oligonucleosomes. As previously reported (Morin et al. (2010) Nat Genet 42:181-5), it was found that the WT enzyme displayed robust activity for methyl transfer to this unmethylated peptidic substrate, but that none of the mutant enzymes displayed significant methyltransferase activity **(Figure 1A).** In contrast to the previously reported data and that in Fig. 1A, it was found that all of the mutant EZH2 constructs were active methyltransferases against the avian nucleosome substrate **(Figure 1B).** The nucleosomes isolated from the avian natural source represent an admixture of states of histone modification, including various states of H3-K27 methylation as judged by Western blotting with H3-K27 methylation-specific antibodies.

There are several potential explanations for the discordant activity of the mutant PRC2 complexes on peptide and nucleosome substrates. One possibility is that substrate recognition sites distal to the enzyme active site (i.e., exosites) are important determinants of substrate binding and turnover; these sites would engage complementary recognition elements on the nucleosome that are not available on small peptidic substrates. However, when *E. coli*-expressed, recombinant human histone H3 was tested as a substrate for the WT and mutant PRC2 complexes, the resulting pattern of activity was identical to that seen for the peptide substrate; that is, the WT enzyme demonstrated robust methyltransferase activity against the H3 substrate, the Y641F mutant showed 7% the activity of WT complex, and all other mutants displayed ≤ 1% the activity of WT complex. Hence, exosite engagement seems an unlikely explanation for the current results. The nucleosome presents many lysine residues beyond H3-K27 as potential sites of methylation that would not be present in the small peptidic substrate. Thus, another possibility is that mutation of Y641 alters the substrate specificity of EZH2 to result in methylation of lysine residues other than H3-K27. This possibility is unlikely given the excellent agreement between mutant activity on small peptide and recombinant H3 protein substrates.

The apparent discordance between the present results and those previously reported was resolved when the enzymatic activity of the WT and mutant PRC2 complexes were tested against a panel of peptidic substrates that represent all possible lysine (K) residues of histone H3 and histone H4 (see Example 2 above). All of the enzyme forms showed significant activity only against peptides containing the equivalent of residue H3-K27. The specific activity of the mutants, however, was greatly reduced relative to WT in the order WT >> Y641F > Y641S ~ Y641H > Y641N, again consistent with previous reported findings.

**Example 5 - Enzymology**

To understand further the enzymatic activity of these mutants, and to reconcile the apparent discrepancy between activity against peptidic and nucleosome substrates, the ability of the enzyme forms to catalyze further methylation of various H3-K27 methylation states in the context of the H3:21-44 peptide was studied. As stated above, it was found that all of the mutant enzymes were deficient catalysts of unmodified H3-K27 peptide methylation, relative to the

WT enzyme. Remarkably, however, all of the mutant enzymes were found to be superior to WT enzyme in catalyzing further methylation of the mono- and especially the di-methylated H3-K27 peptides **(Figure 2).** Thus, the data suggest that the WT enzyme is most efficient in catalyzing the zero- to mono-methylation reaction. The mutant enzymes are defective in catalyzing this initial step, but are more efficient than the WT enzyme in catalyzing the subsequent steps leading from mono-methyl to di- and tri-methyl H3-K27.

The origins of the differential substrate specificities of WT and mutant EZH2 were explored through steady state enzyme kinetics. As summarized in **Table 2,** the mutations have minimal effects on ground-state substrate recognition, as demonstrated by the similar values of $K_m$ for nucleosome and of $K_{1/2}$ for peptide substrates. In all cases the peptidic substrates displayed sigmoidal binding behavior; hence the concentration of peptide resulting in half-maximal velocity is reported here as $K_{1/2}$ instead of the more common Michaelis constant, $K_m$. Copeland (2005) Evaluation of Enzyme Inhibitors in Drug Discovery: A Guide to Medicinal Chemists and Pharmacologists, Wiley. The SAM $K_m$ likewise displayed minimal variation among the enzyme forms, ranging from 208 ± 50 to 304 ± 64 nM. Instead, the differences in substrate utilization appear to have their origin in transition state recognition, as demonstrated by differences in $k_{cat}$ values among the enzymes for various substrates **(Table 2).** As a result, the catalytic efficiency, quantified as the ratio $k_{cat}/K$ (where K is either $K_m$ or $K_{1/2}$, depending on substrate identity; *vide supra*), varies between the WT and mutant enzymes for different states of H3-K27 methylation **(Table 2).**

**Table 2.** Steady state kinetic parameters for methylation reactions catalyzed by PRC2 containing wild-type or Y641 mutants of EZH2.

| Enzyme | Substrate H3-K27 Methylation Status | K (nM) | $k_{cat}$ ($h^{-1}$ x $10^{-2}$) | $k_{cat}/K$ ($h^{-1}\cdot nM^{-1}$ x $10^{-4}$) |
|---|---|---|---|---|
| WT | 0 | 184 ± 10 | 84.0 ± 3.0 | 45.7 ± 3.0 |
| | 1 | 436 ± 42 | 65.4 ± 5.8 | 15.0 ± 2.0 |
| | 2 | 178 ± 16 | 6.0 ± 0.3 | 3.4 ± 0.3 |
| | Nucleosome | 141 ± 31 | 42.6 ± 2.6 | 30.2 ± 6.9 |
| Y641F | 0 | 240 ± 19 | 4.8 ± 0.3 | 2.0 ± 0.2 |
| | 1 | 404 ± 124 | 15.0 ± 4.3 | 3.7 ± 1.6 |
| | 2 | 191 ± 10 | 84.0 ± 2.8 | 44.0 ± 2.7 |
| | Nucleosome | 176 ± 19 | 65.4 ± 2.0 | 37.2 ± 4.2 |
| Y641H | 0 | -[a] | - | - |
| | 1 | 319 ± 57 | 28.2 ± 3.7 | 8.8 ± 2.0 |
| | 2 | 148 ± 9 | 22.8 ± 0.9 | 15.4 ± 1.1 |
| | Nucleosome | 140 ± 22 | 23.4 ± 1.0 | 16.7 ± 2.7 |
| Y641N | 0 | - | - | - |
| | 1 | 280 ± 11 | 23.4 ± 0.8 | 8.4 ± 0.4 |
| | 2 | 157 ± 11 | 96.0 ± 4.0 | 61.1 ± 5.0 |
| | Nucleosome | 191 ± 34 | 23.4 ± 1.3 | 12.3 ± 2.3 |
| Y641S | 0 | - | - | - |
| | 1 | 249 ± 8 | 27.6 ± 0.8 | 11.1 ± 0.5 |
| | 2 | 136 ± 8 | 59.4 ± 2.0 | 43.7 ± 3.0 |
| | Nucleosome | 137 ± 28 | 23.4 ± 1.4 | 17.1 ± 3.6 |
| [a] Activity too low to measure. | | | | |

**Example 6** - **Enzymology**

The steady state kinetic parameters listed in **Table 2** made it possible to calculate the expected levels of different H3-K27 methylation states for cells heterozygous for the various mutant EZH2 forms, relative to cells homozygous for

the WT enzyme. To perform these simulations, a number of simplifying assumptions were made: (1) that steady state enzyme kinetics are relevant to PRC2-catalyzed H3-K27 methylation in the cellular context and that all measurements are made at the same time point in cell growth; (2) that the mutant and WT enzyme are expressed at equal levels in heterozygous cells and that the total EZH2 level is equal in all cells; (3) that the cellular concentration of SAM, relative to its $K_m$ is saturating and does not change among the cells; (4) that the cellular concentration of nucleosome, is similar to its $K_m$ and likewise does not change among cells; (5) that EZH1 catalyzed methylation of H3-K27 was insignificant and constant among the cells; and (6) that any H3-K27 demethylase activity was also constant among the cells.

With these assumptions in place, the predictions illustrated in **Figure 3A** were obtained for relative levels of H3-K27me3 (top panel), H3-K27me2 (middle panel) and H3-K27me1 (bottom panel). A clear pattern emerges from these simulations. The level of H3-K27me3 increases relative to WT cells for all mutant-harboring cells, ranging from a 30% increase for the Y641H mutant to > 400% for the Y641N mutant. At the same time, the levels of H3-K27me2 decreases to < 50% of WT for all of the mutants, and the levels of H3-K27me1 are reduced by approximately half for all mutants, relative to WT.

The relative levels of the H3-K27 methylation states in B-cell lymphoma cell lines that are known to be homozygous for WT EZH2 (OCI-LY19) or heterozygous for EZH2 Y641N (DB, KARPAS 422, and SU-DHL-6) or EZH2 Y641F (WSU-DLCL2) were then measured by Western blotting **(Figure 3B).** The pattern of relative H3-K27 methylation states seen in Figure 3b is in excellent agreement with the results of the simulations based on in vitro steady state kinetic parameters, despite the assumptions used in the simulations and the use of a non-physiological peptide surrogate as substrate.

Thus, increased H3-K27me3 was observed for all Y641 mutant-harboring cells relative to WT, decreased H3-K27me2 was observed for all Y641 mutant-harboring cells relative to WT, and decreased H3-K27me1 was observed for at least two of the four mutant cell lines. The near-comparable levels of H3-K27me1 in WT and KARPAS 422 and SU-DHL-6 cells may reflect different expression levels of WT and mutant EZH2, different contributions of EZH1, or other factors not accounted for in the simulations. Nevertheless, the concordance between the predicted and experimental patterns of H3-K27 methylation status is remarkable and supports the view that enzymatic coupling between WT and mutant EZH2 leads to increased H3-K27me3, thus resulting in the malignant phenotype of cells that are heterozygous for these mutants.

**Example 7 - *In Vitro* Assays of PRC2 Methyltransferase Activity**

*Flashplate assay with peptide substrate.* For initial comparison of WT and Y641 mutants of EZH2, biotinylated histone H3:21-44 peptide containing unmethylated K27 (New England Peptide), monomethylated K27 (Millipore) or dimethylated K27 (Millipore) at a concentration of 800 nM was combined with a mixture of S-adenosylmethionine-Cl (SAM) at 1,700 nM, and 300 nM tritiated SAM (Perkin Elmer). This substrate combination was then added to the PRC2 in assay buffer (20 mM BICINE, 1 mM DTT, 0.002% Tween 20, 0.005% bovine skin gelatin (BSG), pH 7.6). Reactions were allowed to proceed for the indicated time interval and then quenched by addition of excess cold SAM (600 μM final concentration). Quenched reaction mixtures were transferred to a streptavidin-coated Flashplate (Perkin Elmer, catalog number SMP410), allowed to bind for one hour, and then detected on a TopCount NXT HTS scintillation and luminescence counter (Perkin Elmer). Each time point represented the average of six individual reactions. Steady state kinetic parameters were determined under identical reaction conditions except that the concentration of peptide or SAM was varied, while at saturating conditions of the other substrate. Velocity was plotted as a function of varied substrate concentration and the data were fitted to the untransformed version of the Michaelis-Menten equation or the untransformed version of a sigmoidal kinetic equation to calculate values of K and $k_{cat}$. Standard errors of fitted parameters are listed in **Table 2** and were used to construct the error bars illustrated in **Figure 2** panels B and C. Error associated with $k_{cat}/K$ **(Table 2)** were calculated according to standard methods of error propagation; the fractional error of $k_{cat}/K$ was determined as:

$$\mu\frac{k_{cat}}{K}=\sqrt{\left(\frac{\mu k_{cat}}{k_{cat}}\right)^2+\left(\frac{\mu K}{K}\right)^2} \quad (1)$$

where $\mu k_{cat}$ is the standard error of $k_{cat}$ and $\mu K$ is the standard error of K.

*Filterplate assay with oligonucleosome.* Chicken erythrocyte oligonucleosomes were purified as previously described. Fang et al. (2004) Methods Enzymol 377:213-26. Nucleosomes were combined with a mixture of SAM and tritiated SAM, and added to PRC2 in assay buffer (20 mM BICINE, 100 mM KCl, 1 mM DTT, 0.002% Tween 20, 0.005% BSG, pH 7.6). Reactions were run and quenched as above. Quenched reaction mixture was transferred to a glass fiber

filterplate (Millipore, catalog number MSFBN6B) and washed three times with 10% trichloroacetic acid and allowed to dry. Microscint Zero (30 μL) was added and tritium incorporation was detected on a TopCount scintillation and luminescence counter. Steady state parameters were determined under identical reaction conditions except that the concentration of nucleosome or SAM was varied while at saturating conditions of the other substrate. Velocity was plotted as a function of varied substrate concentration and fitted to the untransformed version of the Michaelis-Menten equation to derive the values of $K_m$ and $k_{cat}$ as described above.

**Example 8 - Compound Preparation**

**A. PREPARATION OF REFERENCE COMPOUND 63**

| | |
|---|---|
| 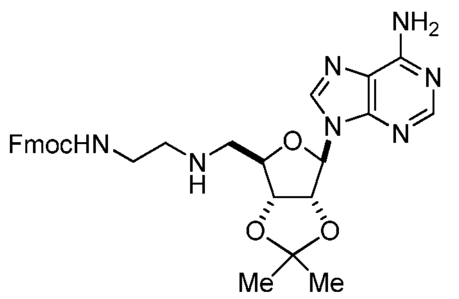 | **63** |

To a solution of 9-((3aR,4R,6R,6aR)-6-(aminomethyl)-2,2-dimethyltetrahydrofuro[3,4-d][1,3]dioxol-4-yl)-9H-purin-6-amine (Townsend, A. P. et al. (2009) Org. Let. 11:2976-2979) (3.05 g, 9.96 mmol) in DCE (250 mL) was added (9H-fluoren-9-yl)methyl (2-oxoethyl)carbamate (2.8 g, 9.96 mmol) and $NaB(OAc)_3H$ (2.96 g, 13.95 mmol), the mixture stirred for 4 h at room temperature. $K_2CO_3$ solution was added to pH at 8-9. DCM was added, the organic layer was dried with $Na_2SO4$, concentrated and purified by SGC (DCM : MeOH = 30 : 1) to give **63** (2.9 g, yield: 50.9%).

**B. PREPARATION OF REFERENCE COMPOUND 65**

| | |
|---|---|
| 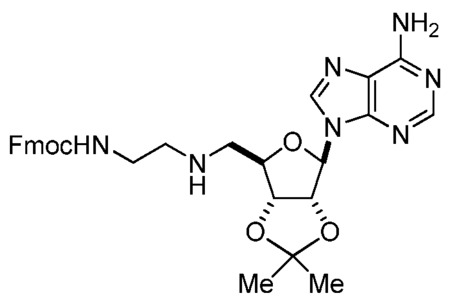 | **63** |
| 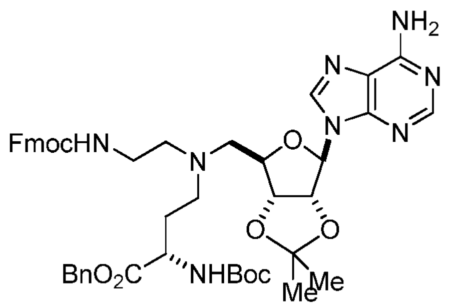 | **65** |

To a solution of **63** (2.9 g, 5.08 mmol) in DCE (250 mL), (S)-benzyl 2-((tert-butoxycarbonyl)amino)-4-oxobutanoate (1.56 g, 5.08 mmol) and $NaB(OAc)_3H$ (1.51 g, 7.11 mmol) were added, the mixture stirred for 4h at room temperature. $K_2CO_3$ solution was added to pH at 8-9. DCM was added, the organic layer was dried with $Na_2SO_4$, concentrated and purified with SGC (DCM: MeOH =100:1) to give **65** (2.8 g, yield: 63.9%).

## C. PREPARATION OF REFERENCE COMPOUND 75

| | |
|---|---|
| 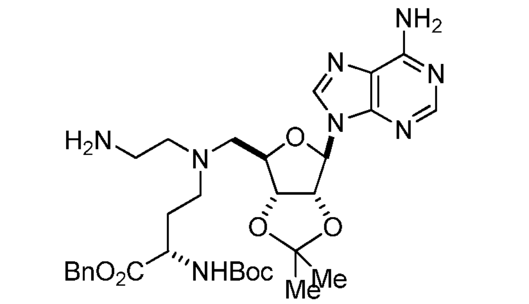 | **72** |
| 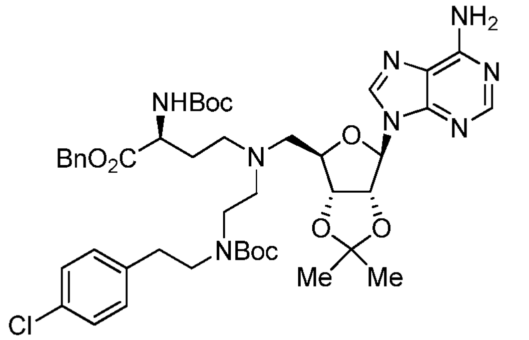 | **73** |
| 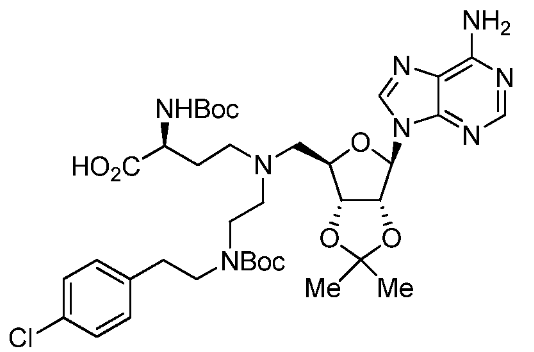 | **74** |
| 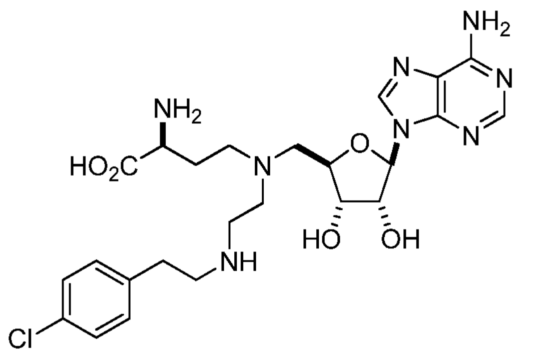 | **75** |

**Step 1.** To a solution **of 65B** (2.2 g, 2.55 mmol) in DCM (10 mL), $Et_2NH$ (1.1 g, 15.3 mmol) were added, the

mixture stirred for 4 h at room temperature. The mixture was concentrated to give crude **72** (2.2 g).

**Step 2.** To a stirred solution of **72** (167 mg, 0.26 mmol) in MeOH (4 mL), 2-(4-chlorophenyl) acetaldehyde (40 mg, 0.26 mmol) was added and stirred at room temperature for 20 min. Then $Na(OAc)_3BH$ (83 mg, 0.39 mmol) and HOAc (0.4 mL) was added and stirred overnight. Then $NaHCO_3$ (aq) was added and extracted with DCM (25 mL x 3), washed with brine, dried with $Na_2SO_4$ and concentrated. The crude product was purified by preparative TLC (DCM / MeOH = 10: 1) to afford **73** (30 mg, yield: 14%) as white powder. LC/MS (m/z): 779.7 $[M+1]^+$.

**Step 3.** A mixture of **73** (30 mg, 0.038 mmol) and 10% Pd/C (15 mg) in MeOH (2 mL) was stirred at room temperature under $H_2$ overnight. The mixture was filtered and the filtrate was concentrated to give crude product. The crude product was purified by preparative TLC (DCM / MeOH = 8: 1) to afford **74** (20 mg, yield: 69%) as white powder. LC/MS (m/z): 689.7 $[M+1]^+$.

**Step 4.** A solution of **74** (20 mg, 0.028 mmol) in 90% TFA (1 mL) was stirred at room temperature for 1h, and concentrated as a solid to remove TFA to give the compound **75** (TFA salt) as a colorless oil without purification. LC/MS (m/z): 549.7 $[M+1]^+$.

**Reference Example 9 - Inhibition of EZH2 Wild-Type and Y641 Mutants by SAH**

S-Adenosyl-L-homocysteine (SAH) was serially diluted 3 fold in DMSO for 10 points and 1 μL was plated in a 384 well microtiter plate. Positive control (100% inhibition standard) was 100 μM final concentration of SAH and negative control (0% inhibition standard) contained 1 μL of DMSO. SAH was then incubated for 30 minutes with 40 μL per well of EZH2 wild-type and mutants at 8 nM in pH 7.6 assay buffer (20 mM BICINE, 100 mM KCl, 1 mM DTT, 0.002% Tween 20, 0.005% BSG). A substrate mix at 10 μL per well was added which contained S-adenosylmethionine-Cl (SAM) at 150 nM and tritiated SAM at 100 nM, and biotinylated oligonucleosome at 150 nM in pH 7.6 assay buffer. Quenched enzyme reaction was transferred to a streptavidin-coated Flashplate (Perkin Elmer, catalog number SMP410), allowed to bind for one hour, and detected on a TopCount NXT HTS (Perkin Elmer).

Results are shown in **Figure 7**. IC50 values are shown in **Table 3.**

**Table 3.** Inhibition of WT EZH2 and Y641 mutants of EZH2 by SAH.

| | WT | Y641H | Y641S | Y641N | Y641F |
|---|---|---|---|---|---|
| IC50, μM | 0.467 | 0.263 | 0.283 | 0.380 | 4.80 |

**Reference Example 10-Inhibition of EZH2 Wild-Type and Y641 Mutants by Compound 75**

Compound 75 was serially diluted 3 fold in DMSO for 10 points and 1 μL was plated in a 384 well microtiter plate. Positive control (100% inhibition standard) was 100 μM final concentration of SAH and negative control (0% inhibition standard) contained 1 μL of DMSO. Compound 75 was then incubated for 30 minutes with 40 μL per well of EZH2 wild-type and mutants at 8 nM in pH 7.6 assay buffer (20 mM BICINE, 100 mM KCl, 1 mM DTT, 0.002% Tween 20, 0.005% BSG). A substrate mix at 10 μL per well was added which contained S-adenosylmethionine-Cl (SAM) at 150 nM and tritiated SAM at 100 nM, and biotinylated oligonucleosome at 150 nM in pH 7.6 assay buffer. Quenched enzyme reaction was transferred to a streptavidin-coated Flashplate (Perkin Elmer, catalog number SMP410), allowed to bind for one hour, and detected on a TopCount NXT HTS (Perkin Elmer).

Results are shown in **Figure 8.** IC50 values are shown in **Table 4.**

**Table 4.** Inhibition of WT EZH2 and Y641 mutants of EZH2 by Compound 75.

| | WT | Y641S | Y641N | Y641F | Y641H |
|---|---|---|---|---|---|
| IC50, μM | 8.95 | 2.50 | 4.10 | 7.18 | 7.56 |

**Example 11 - H3-K27me2/me3 ratios predict sensitivity to an EZH2 inhibitor**

Tumor cell lines heterozygous for the EZH2 (Y641) mutation display increased levels of H3-K27me3, the methylation state of H3-K27 thought to be important in tumorigenesis. Levels of the mono (H3-K27me1), di (H3-K27me2), or trimethylated (H3-K27me3) forms of H3-K27 in a panel of cell lines that were WT for EZH2, or heterozygous for EZH2 (Y641) mutations were evaluated. Cell lines used are listed in Table 5. The majority of lines are B-cell lymphoma lines, however two melanoma lines were also included. IGR1 is a melanoma line that has recently been found to contain a Y641N mutation in EZH2, and A375 cells were included as a WT EZH2 melanoma control line. Figures 9A and B show the results of western blot analysis of histones isolated from this cell line panel probed with antibodies recognizing H3-K27me1, H3-K27me2, or H3-K27me3. In general, global H3-K27me3 levels are higher in Y641 mutant containing cell lines than in cell lines expressing WT EZH2 exclusively. The exception is Farage cells, where H3-K27me3 levels were similar to those in WT lines. More striking are the dramatically lower levels of H3-K27me2 in EZH2 Y641 mutant cell lines relative to wild type cell lines. Little or no H3-K27me2 signal was observed in western blot of histones extracted from Y641 mutant cell lines, whereas the signal observed with the same antibody in WT cell lines was more intense than that observed with the antibody specific for H3-K27me3. Overall, in WT cell lines the western blot signal with an HK27me2 antibody was higher than the signal observed with the H3-K27me3 antibody, whereas the opposite was true in Y641 mutant cell lines. Thus the ratio of H3-K27me3/me2 signal in Y641 lines is higher than that observed in WT lines.

The H3-K27 methylation state can also be examined by Mass spectrometry (MS), an independent method that does not rely on antibody reagents. The MS analysis demonstrated that H3-K27me3 levels are higher in Y641 mutant and Pfeiffer lines (A677G) than in the other WT lines, whereas the opposite is true for H3-K27me2 levels. In the Y641 mutant and Pfeiffer lines (A677G), H3-K27me3 levels were higher than H3-K27me2 levels, whereas the opposite was true in the other WT lines. These results are consistent with those observed by western blot analysis in Figure 9A and B.

The differences in H3-K27 methylation state was also detected by immunocytochemistry using antibodies to H3-K27me2 or H3-K27me3. This immunohistochemistry assay is used for detecting aberrant H3-K27me2/3 ratios associated with Y641 mutant EZH2 in formalin fixed paraffin embedded patient tumor tissue samples. A panel of five WT and five Y641 mutant lymphoma cell line pellets were fixed and embedded in paraffin blocks and stained with anti-H3-K27me2 or H3-K27me3 antibodies. An antibody to histone H3 was included as a positive control, since all cells should contain nuclear histone H3. Figure 10 shows that all cell lines were positive in 100% of cells for both H3-K27me3 and H3 staining. Under these conditions, no clear difference in H3-K27me3 staining intensity was observed between WT and Y641 mutant cell lines. This may reflect the limited dynamic range of chromogenic immunocytochemistry staining compared to other methods of detection. However, as shown in Figure 11, cell lines could be clearly segregated into those staining positive or negative for H3-K27me2. All WT cell lines stained positive for H3-K27me2, whereas all Y641 mutant cell lines and Pfeiffer cells (A677G) showed no staining with the H3-K27me2 antibody. These results are consistent with those obtained by western and MS analysis.

Without wishing to be bound by theory, the increased levels of H3-K27me3 associated with the gain of function EZH2 (Y641) mutations may render cells bearing EZH2 mutations more sensitive to small molecule EZH2 inhibitors. To evaluate whether the increased H3-K27me3 and/or decreased H3-K27me2 levels observed in Pfeiffer cells in the absence of an EZH2 Y641 mutation would also correlate with sensitivity to EZH2 inhibitors, two compounds that demonstrate potent inhibition of EZH2 in biochemical assays with IC50s of 85 and 16 nM respectively were tested. Treatment of WSU-DLCL2 cells with either compound led to inhibition of global H3-K27me3 levels, confirming their ability to enter cells and inhibit cellular EZH2 methyltransferase activity (Figure 12).

The sensitivity of a panel of WT and Y641 mutant cell lines to each compound was evaluated in proliferation assays. Because the anti-proliferative activity of EZH2 inhibitors takes several days to manifest, compounds were assessed in 11-day proliferation assays. Figure 13 shows representative growth curves for WT (OCI-LY19), or Y641 mutant (WSU-DLCL2) cell lines treated with the test compounds. Both compounds demonstrated anti-proliferative activity against WSU-DLCL2 cells, but little activity against OCI-LY19 cells. Inhibitor A was a more potent inhibitor of WSU-DLCL2 proliferation than Inhibitor B and this is consistent with Inhibitor A being a more potent inhibitor of EZH2 in biochemical assays. Proliferation assays were performed in a panel of WT and Y641 mutant lymphoma cell lines, with Inhibitor B, and day 11 IC90 values were derived. Figure 14A shows IC90 values of lymphoma cell lines grouped by EZH2 Y641 status. Overall, Y641 mutant cell lines demonstrated increased sensitivity to EZH2 inhibitors relative to WT cell lines, although RL and SUDHL4 cells were significantly less sensitive than other mutant lines. Pfeiffer cells (A677G) demonstrate high H3-K27me3 and low H3-K27me2 levels, and so grouping cell lines according to high H3-K27me3 and low H3-K27me2 gives better discrimination of EZH2 inhibitor sensitivity as shown for Inhibitor B in Figure 14B. Thus, high H3-K27me3 and low H3-K27me2 levels can be used to predict sensitivity to EZH2 inhibitors, independent of knowledge of mutational status.

These results demonstrates that identifying EZH2 Y641 mutations in patient tumors and/or detecting low levels of H3-K27me2 relative to H3-K27me3 through use of techniques such as western blot, MS or IHC in a patient can be used to identify which patient will respond to EZH2 inhibitor treatment.

**Table 5.** Cell lines used in this study.

| Cancer | EZH2 Status | Cell Line |
|---|---|---|
| **Lymphoma:** DLBCL (Diffuse Large Cell B Cell Lymphoma) and other B-cell Lymphoma | Wild Type | OCI-LY19 |
| | | HT |
| | | MC116 |
| | | BC-1 |
| | | BC-3 |
| | | Toledo |
| | | DOHH-2 |
| | | Farage |
| | | SR |
| | | NU-DHL-1 |
| | | NU-DUL-1 |
| | | |
| | Y641 Mutation | SU-DHL-10 (Y641F) |
| | | DB (Y641N) |
| | | KARPAS 422 (Y641N) |
| | | SU-DHL-6 (Y641N) |
| | | WSU-DLCL-2 (Y641F) |
| | | RL (Y641N) |
| | | SU-DHL-4 (Y641S) |
| Melanoma | Wild Type | A375 |
| | Y641 Mutation | IGR-1 (Y641N) |

**Example 12 - Recombinant 4-Component PRC2 Complexes**

Wild-type (WT) EZH2 (GenBank Accession No. NM_004456) or A677G and A687V mutants were co-expressed with wild-type EED (GenBank Accession No. NM_003797), SUZ12 (GenBank Accession No. NM_015355) and RbAp48 (GenBank Accession No. NM_005610) in *Spodoptera frugiperda* (Sf9) cells using a baculovirus expression system. An N-terminal FLAG tag on the EED was used to purify active PRC2 complex from cell lysates. The purity of the final PRC2 preparations was assessed by SDS-PAGE with Coomassie blue staining and protein concentration was determined using a bovine serum albumin standard curve in a Bradford assay.

**Example 13 - *In vitro* Assays of PRC2 Methyltransferase Activity**

*Standard Procedure for Flashplate assay with peptide substrates.* Activity of the wild-type or mutant EZH2-containing PRC2 complexes was investigated using a series of four peptides representing the amino acid sequence of human H3 from residues 21-44 (H3:21-44) with lysine 27 represented as the unmodified, monomethylated, dimethylated or trimethylated side chain amine, consisting of the following sequence, with the H3-K27 lysine subject to modification underlined, ATKAARKSAPATGGVKKPHRYRPGG[K-Ahx-Biot]-amide (SEQ ID NO: 20). Biotin (Biot) was appended to a C-terminal lysine (K) residue through an aminohexyl linker (AHX) attached to the lysine side chain amine (21st Century Biochemicals). For comparison of mutant of WT and mutant EZH2 activity, biotinylated histone H3:21-44 peptides were

combined with a mixture of S-adenosylmethionine (SAM; Sigma-Aldrich) and tritiated SAM ($^3$H-SAM; American Radiolabeled Chemicals) and recombinant 4-component PRC2 in assay buffer (20 mM BICINE, 1 mM DTT, 0.002% Tween 20, 0.005% bovine skin gelatin (BSG), pH 7.6). Reactions were allowed to proceed for the indicated time interval and then quenched by addition of excess cold SAM (100 μM final concentration). Quenched reaction mixtures were transferred to a streptavidin-coated Flashplate (Perkin Elmer, catalog number SMP410), and allowed to bind for one hour before the plates were washed in a Biotek EL-405x platewasher and read on a TopCount NXT HTS scintillation and luminescence counter (PerkinElmer) .

**Example 14 - Enzymology**

Recombinant 4-component PRC2 complexes were prepared with wild-type and either A677G or A687V mutant versions of EZH2 (see Example 12 above; Cao et al. (2004) Mol Cell 15:57-67). Each complex was initially tested for the ability to catalyze $^3$H-methyl transfer from labeled S-adenosyl methionine (SAM) to each of the four H3:21-44 peptides. Enzyme was serially diluted, and a mixture of peptide (200 nM) and SAM (200 nM $^3$H-SAM and 800 nM unlabeled SAM) was added. Reactions were quenched at 15 minute intervals by the addition of an excess of unlabeled SAM and reaction velocity was calculated based on the linear regression of raw counts per minute (CPM) vs. time. As shown in Figure 15, the mutant enzymes displayed a different pattern of activity than the wild-type enzyme. The A677G and A687V mutants had robust activity on all of the unmethylated, monomethylated and dimethylated H3:21-44 peptides, whereas the wild-type enzyme only showed robust activity on the unmethylated and monomethylated peptides. The control peptide, containing fully trimethylated H3-K27 was not methylated in the assay, indicating that H3-K27 was the target lysine.

**Example 15 - Enzymology**

To further understand the enzymatic activity of these mutants, the origins of the differential substrate specificities of wild-type and mutant EZH2 were explored through steady-state enzyme kinetics. Reactions containing a titration of the H3-K27 peptides with fixed enzyme (4 nM) and SAM (200 nM $^3$H-SAM and 800 nM unlabeled SAM) were performed. In all cases the peptidic substrates displayed sigmoidal binding behavior; hence the concentration of peptide resulting in half-maximal velocity is reported here as $K_{1/2}$ instead of the more common Michaelis-Menten constant, $K_m$ (Copeland (2005) Evaluation of Enzyme Inhibitors in Drug Discovery: A Guide to Medicinal Chemists and Pharmacologists, Wiley). As summarized in Table 6, the mutations have an effect on ground-state substrate recognition as demonstrated by lower $K_{1/2}$ for the unmethylated peptide substrates and higher $K_{1/2}$ values for the dimethylated peptide substrates. Additionally, the maximal velocities of the enzymes are affected by the mutations. The A677G mutation leads to 2.9-, 3.7- and 22-fold increases in $k_{cat}$ on respective un-, mono-, and dimethyl H3-K27 peptide susbtrates, while the A687V mutation results in a 3-fold reduction in $k_{cat}$ on the unmethyl H3-K27 peptide, but produces respective 3.5- and 2.5-fold increases in $k_{cat}$ on the mono- and dimethyl H3-K27 peptides. The SAM $K_m$ displayed minimal variation among the enzyme forms, 403 ± 64 nM to 899 ± 89 nM on the substrate containing the preferred methylation state at the H3-K27 residue.

**Table 6. Summary of Steady-State Enzyme Kinetics for WT and Mutant EZH2 Enzymes**

| Enzyme | Peptide Substrate H3-K27 Methylation Status | $K_{1/2}$ (nM) | $k_{cat}$ ($h^{-1}$) | $k_{cat}/K_{1/2}$ ($h^{-1} \cdot nM^{-1}$ X $10^{-4}$) |
|---|---|---|---|---|
| *WT | 0 | 154 ± 12 | 4.80 ± 0.20 | 305 ± 26 |
| | 1 | 337 ± 26 | 3.33 ± 0.21 | 99 ± 9 |
| | 2 | 144 ± 11 | 1.08 ± 0.04 | 75 ± 6 |
| A677G | 0 | 88 ± 6 | 14.05 ± 0.54 | 1590 ± 120 |
| | 1 | 222 ± 51 | 12.25 ± 1.67 | 570 ± 150 |
| | 2 | 522 ± 117 | 23.85 ± 3.60 | 450 ± 120 |

(continued)

| Enzyme | Peptide Substrate H3-K27 Methylation Status | $K_{1/2}$ (nM) | $k_{cat}$ ($h^{-1}$) | $k_{cat}/K_{1/2}$ ($h^{-1}\cdot nM^{-1}$ X $10^{-4}$) |
|---|---|---|---|---|
| A687V | 0 | 43 ± 3 | 1.58 ± 0.08 | 370 ± 30 |
| | 1 | 176 ± 13 | 11.49 ± 0.50 | 650 ± 60 |
| | 2 | 352 ± 155 | 2.70 ± 0.65 | 80 ± 40 |
| *Wild-type data was previously published in Wigle et al., Febs Lett (2011) Oct 3;585(19):3011-4 using 4-component EZH2. | | | | |

**Example 16 - Inhibition of Wild-Type EZH2 and EZH2 mutants by EZH2 inhibitors**

Test compounds were serially diluted 3-fold in DMSO in a 10 point-curve and 1 μL was spotted into a 384-well microplate in duplicate using a Platemate Plus equipped with 384-channel head (Thermo Scientific). The final top concentration of test compounds in the assay was 10 μM. Positive control (100% inhibition standard) was 1 mM final concentration of SAH and negative control (0% inhibition standard) contained 1 μL of DMSO. Test compounds were then incubated for 30 minutes with 40 μL per well of wild-type EZH2 (final concentration was 4 nM), Y641F EZH2 (final concentration was 0.1 nM) and A677G and A687V EZH2 (for each, final concentration was 2 nM) and peptide in IX assay buffer (20 mM BICINE pH = 7.6, 1 mM DTT, 0.002% Tween 20, 0.005% BSG). For the wild-type EZH2 and A677G EZH2 assays, biotinylated peptide H3:21-44 with unmethylated K27 was present at a final concentration of 200 nM, while in the A687V EZH2 assay, biotinylated peptide H3:21-44 with monomethylated K27 was present at a final concentration of 200 nM and in the Y641F EZH2 assay biotinylated peptide H3:21-44 with dimethylated K27 was present at a final concentration of 200 nM. To initiate the reaction containing the wild-type EZH2 enzyme, a substrate mix of 10 μL per well was added that contained unlabeled SAM (final concentration was 1800 nM) and $^3$H-SAM (final concentration was 200 nM) in IX assay buffer. To initiate the reaction containing the Y641F EZH2 enzyme a substrate mix of 10 μL per well was added that contained unlabeled SAM (final concentration was 700 nM), and $^3$H-SAM (final concentration was 300 nM) in IX assay buffer. To initiate the reactions containing the A677G or A687V EZH2, a substrate mix of 10 μL per well was added which contained unlabeled SAM (final concentration was 400 nM) and $^3$H-SAM (final concentration was 100 nM). Reactions proceeded for 90 min, then were quenched with excess unlabeled SAM (167 μM), then were transferred to a streptavidin-coated Flashplate (PerkinElmer, catalog number SMP410), allowed to bind for one hour, and detected on a TopCount NXT HTS (PerkinElmer). The $IC_{50}$ values are obtained from 4-parameter fits of the % inhibition of enzyme activity and are tabulated in Table 7. The formulae used to derive $IC_{50}$ values are indicated below.

**% inhibition calculation**

$$\%\ inh = 100 - \left(\frac{dpm_{cmpd} - dpm_{min}}{dpm_{max} - dpm_{min}}\right) \times 100$$

Where dpm = disintegrations per minute, cmpd = signal in assay well, and min and max are the respective minimum and maximum signal controls.

**Four-parameter IC50 fit**

$$Y = Bottom + \frac{(Top - Bottom)}{(1 + (\frac{X}{IC_{50}})^{Hill\ Coefficient}}$$

Where top and bottom are the normally allowed to float, but may be fixed at 100 or 0 respectively in a 3-parameter fit. The Hill Coefficient normally allowed to float but may also be fixed at 1 in a 3-parameter fit. Y is the %

inhibition and X is the compound concentration.

**Table 7. Inhibition of wild-type and mutant EZH2 by EZH2 inhibitors**

| Compound Number | EZH2 Inhibitor | WT (uM) | Y641F (uM) | A677G (uM) | A687V (uM) |
|---|---|---|---|---|---|
| 1. (reference) | SAH | 6.9082 | 16.6193 | 6.2379 | 5.9034 |
| 2. | EPZ004710 | 2.9758 | 3.7887 | 0.3187 | 0.5378 |
| 3. | EPZ004744 | 1.5203 | 0.7432 | 0.1128 | 0.1354 |
| 4. | EPZ005030 | 0.2600 | 0.1846 | 0.0418 | 0.0504 |
| 5. | EPZ005100 | 0.3579 | 0.2923 | 0.0316 | 0.0599 |
| 6. | EPZ005260 | 2.3755 | 1.5781 | 0.2130 | 0.3189 |
| 7. | EPZ005687 | 0.0950 | 0.0750 | 0.0113 | 0.0082 |
| 8. | EPZ006089 | 0.2300 | 0.3110 | 0.0190 | 0.0277 |
| 9. | EPZ006438 | 0.0062 | 0.0111 | 0.0022 | 0.0015 |
| 10. | EPZ006632 | 0.0025 | 0.0034 | 0.0040 | 0.0032 |
| 11. | EPZ007038 | 0.0099 | 0.0123 | 0.0060 | 0.0032 |
| 12. | EPZ007209 | 0.0065 | 0.0074 | 0.0086 | 0.0068 |
| 13. | EPZ007210 | 0.0043 | 0.0044 | 0.0038 | 0.0036 |
| 14. | EPZ007227 | 0.0143 | 0.0207 | 0.0122 | 0.0128 |
| 15. | EPZ007426 | 0.0181 | 0.0088 | 0.0034 | 0.0040 |
| 16. | EPZ007428 | 0.0014 | 0.0055 | 0.0019 | 0.0021 |
| 17. | EPZ007478 | 0.0088 | 0.0114 | 0.0042 | 0.0071 |
| 18. | EPZ007648 | 0.0025 | 0.0079 | 0.0058 | 0.0067 |
| 19. | EPZ007649 | 0.0094 | 0.0092 | 0.0096 | 0.0082 |
| 20. | EPZ007655 | 0.0125 | 0.0104 | 0.0192 | 0.0171 |
| 21. | EPZ007692 | 0.0100 | 0.0117 | 0.0116 | 0.0103 |
| 22. | EPZ007789 | 0.0108 | 0.0114 | 0.0048 | 0.0051 |
| 23. | EPZ007790 | 0.0169 | 0.0158 | 0.0073 | 0.0065 |
| 24. | EPZ008205 | 0.0129 | 0.0093 | 0.0112 | 0.0101 |
| 25. | EPZ008277 | 0.0333 | 0.0092 | 0.0023 | 0.0055 |
| 26. | EPZ008278 | 0.0384 | 0.0106 | 0.0093 | 0.0191 |
| 27. | EPZ008279 | 0.0223 | 0.0182 | 0.0022 | 0.0051 |
| 28. | EPZ008280 | 0.0067 | 0.0029 | 0.0028 | 0.0039 |
| 29. | EPZ008286 | 0.0043 | 0.0025 | 0.0015 | 0.0018 |
| 30. | EPZ008335 | 0.0065 | 0.0033 | 0.0015 | 0.0029 |
| 31. | EPZ008336 | 0.0057 | 0.0036 | 0.0013 | 0.0024 |
| 32. | EPZ008337 | 0.0087 | 0.0015 | 0.0008 | 0.0014 |
| 33. | EPZ008338 | 0.0120 | 0.0096 | 0.0031 | 0.0072 |
| 34. | EPZ008344 | 0.0124 | 0.0036 | 0.0016 | 0.0046 |
| 35. | EPZ008491 | 0.0091 | 0.0014 | 0.0029 | 0.0029 |
| 36. | EPZ008493 | 0.1320 | | 0.0127 | 0.0882 |
| 37. | EPZ008494 | 0.0079 | 0.0046 | 0.0028 | 0.0038 |

(continued)

| Compound Number | EZH2 Inhibitor | WT (uM) | Y641F (uM) | A677G (uM) | A687V (uM) |
|---|---|---|---|---|---|
| 38. | EPZ008495 | 0.0134 | 0.0104 | 0.0063 | 0.0084 |
| 39. | EPZ008496 | 0.0154 | 0.0104 | 0.0040 | 0.0076 |
| 40. | EPZ008497 | > 10.0 uM | 3.2876 | 0.9735 | 1.2184 |
| 41. | EPZ008592 | 0.0145 | 0.0051 | 0.0035 | 0.0075 |
| 42. | EPZ008623 | 0.2440 | 0.1835 | 0.0922 | 0.1067 |
| 43. | EPZ008630 | 0.0034 | 0.0029 | 0.0035 | 0.0032 |
| 44. | EPZ008681 | 0.0029 | 0.0015 | 0.0027 | 0.0047 |
| 45. | EPZ008686 | 0.0073 | 0.0055 | 0.0045 | 0.0096 |
| 46. | EPZ008989 | 0.0094 | 0.0069 | 0.0022 | 0.0028 |
| 47. | EPZ008990 | 0.0061 | 0.0067 | 0.0016 | 0.0017 |
| 48. | EPZ008991 | 0.0348 | 0.0293 | 0.0094 | 0.0193 |
| 49. | EPZ008992 | 0.3333 | 0.1678 | 0.0638 | 0.1188 |
| 50. | EPZ008994 | 0.0715 | 0.0275 | 0.0121 | 0.0205 |
| 51. | EPZ009090 | 0.0300 | 0.0111 | 0.0120 | 0.0227 |
| 52. | EPZ009097 | 0.0047 | 0.0039 | 0.0046 | 0.0036 |
| 53. | EPZ009099 | 0.0765 | 0.0255 | 0.0057 | 0.0222 |
| 54. | EPZ009152 | 0.0030 | 0.0031 | 0.0029 | 0.0033 |
| 55. | EPZ009153 | 0.0052 | 0.0032 | 0.0056 | 0.0037 |
| 56. | EPZ009154 | 0.0278 | 0.0420 | 0.0141 | 0.0438 |
| 57. | EPZ009155 | 0.0563 | 0.0524 | 0.0251 | 0.0623 |
| 58. | EPZ009156 | 0.0034 | 0.0217 | 0.0051 | 0.0132 |
| 59. | EPZ009157 | 0.0397 | 0.0443 | 0.0214 | 0.0436 |
| 60. | EPZ009158 | 0.0021 | 0.0016 | 0.0027 | 0.0027 |
| 61. | EPZ009161 | 0.0009 | | 0.0008 | 0.0011 |
| 62. | EPZ009162 | 0.0657 | 0.0351 | 0.0146 | 0.0178 |

**Example 17 - A677 mutant shows the highest sensitivity to the EZH2 inhibitors**

Pfeiffer cells were obtained from ATCC (CRL-2632). WSU-DLCL2 (ACC 575) and OCI-Ly19 (ACC 528) cells were obtained from DSMZ. All cells were maintained in RPMI + 10% FBS. For cell proliferation analysis, exponentially growing Pfeiffer, WSU-DLC2, or OCI-Ly19 cells were plated, in triplicate, in 96-well plates at a density of $1x10^5$ cells/mL, $5x10^4$ cells/mL, or $2.5x10^5$ cells/mL (respectively) in a final volume of 150uL. Cells were incubated with Compound #7 at final concentrations ranging from 0.011 to 25uM for $IC_{50}$ determination over an 11-day time course. Cells were incubated with Compound #13 at final concentrations ranging from 0.00004 to 10uM for $IC_{50}$ determination over an 11-day time course. Every 3-4 days, viable cell numbers were determined using the Guava Viacount assay (Millipore #4000-0040) and analyzed on a Guava EasyCyte Plus instrument. After cell counts, growth media and EPZ2 inhibitor (Compound #7 or Compound #13) were replaced, and cells were split back to the original plating density. The final split-adjusted number of viable cells/mL from day 11 of the time course was used to calculate the proliferation $IC_{50}$ values, using Graphpad Prism software.

The Pfeiffer cell line, containing the heterozygous EZH2 mutation A677G, is shown to be sensitive to EZH2 inhibition by a small molecule inhibitor Compound #7. Proliferation inhibition is seen as early as 96hr post-inhibitor treatment. The proliferation $IC_{50}$ after 11 days for Pfeiffer cells treated with Compound #7is 5.2nM, compared to WSU-DLCL2 cells, which contain the Y641F heterozygous mutation, and have an $IC_{50}$ of 270nM or OCI-Ly19 cells, which are

WT for EZH2, and have an $IC_{50}$ of 3000nM. These results show that in a cellular context, the sensitivity of WT and mutant EZH2 to inhibition by small molecule inhibitor is A677G>>>Y641F>>WT, as measured by proliferation.

The Pfeiffer cell line, containing the heterozygous EZH2 mutation A677G, is shown to be sensitive to EZH2 inhibition by a small molecule inhibitor Compound #13. Proliferation inhibition is seen as early as 96hr post-inhibitor treatment. The proliferation $IC_{50}$ after 11 days for Pfeiffer cells treated with Compound #13is 0.4 nM, compared to WSU-DLCL2 cells, which contain the Y641F heterozygous mutation, and have an $IC_{50}$ of 4.9 nM or OCI-Ly19 cells, which are WT for EZH2, and have an $IC_{50}$ of 430 nM. These results show that in a cellular context, the sensitivity of WT and mutant EZH2 to inhibition by small molecule inhibitor is A677G>>>Y641F>>WT, as measured by proliferation.

## EQUIVALENTS

While several embodiments of the present invention have been described and illustrated herein, those of ordinary skill in the art will readily envision a variety of other means and/or structures for performing the functions and/or obtaining the results and/or one or more of the advantages described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used. Those skilled in the art will recognize, or be able to ascertain using no more than routine experimentation, many equivalents to the specific embodiments of the invention described herein. It is, therefore, to be understood that the foregoing embodiments are presented by way of example only and that, within the scope of the appended claims and equivalents thereto, the invention may be practiced otherwise than as specifically described. The present invention is directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present invention.

## Claims

1. An EZH2 inhibitor selected from compound 2 to compound 62,

| | |
|---|---|
| 2 | 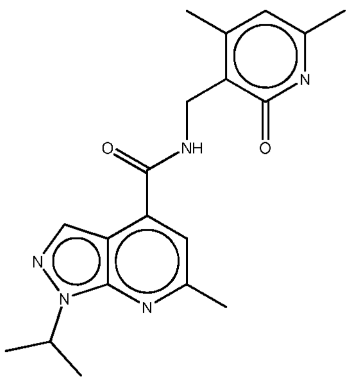 |
| 3 | 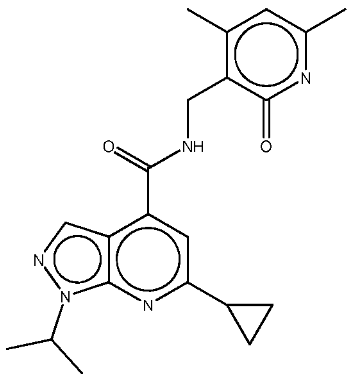 |

(continued)

| | |
|---|---|
| 4 | 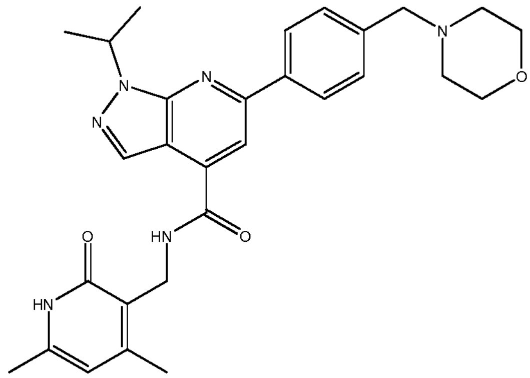 |
| 5 | 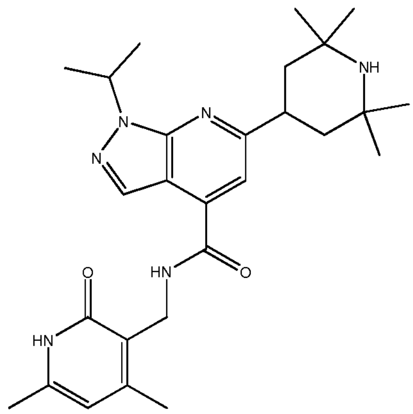 |
| 6 | 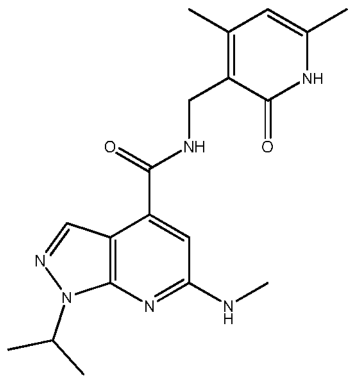 |

(continued)

| | |
|---|---|
| 7 | 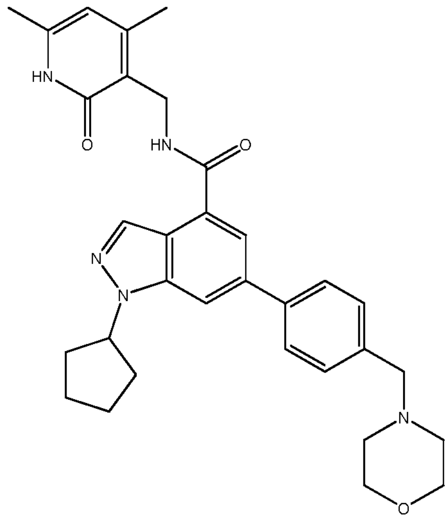 |
| 8 | 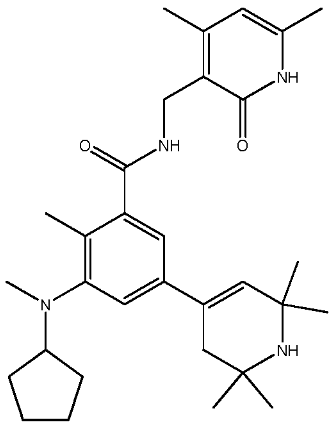 |
| 9 | 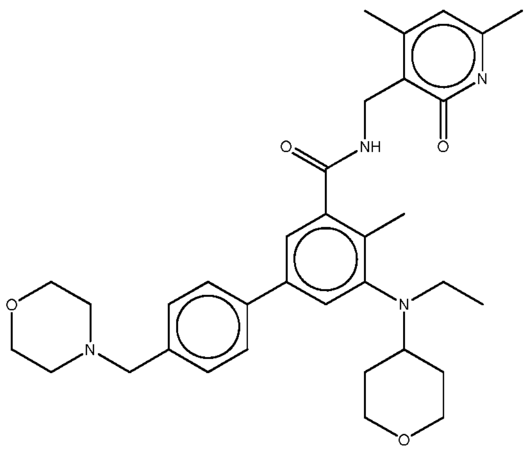 |

(continued)

| | |
|---|---|
| 10 | 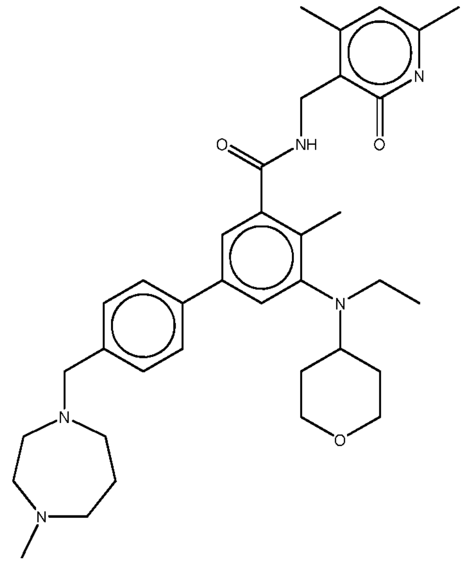 |
| 11 | 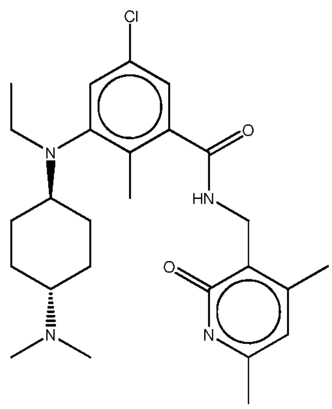 |
| 12 | 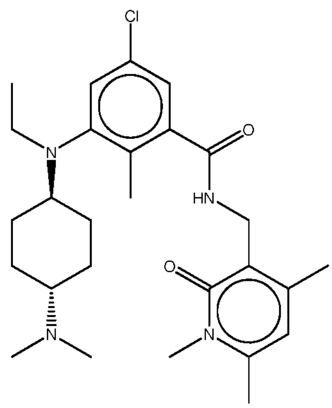 |

(continued)

| | |
|---|---|
| 13 | 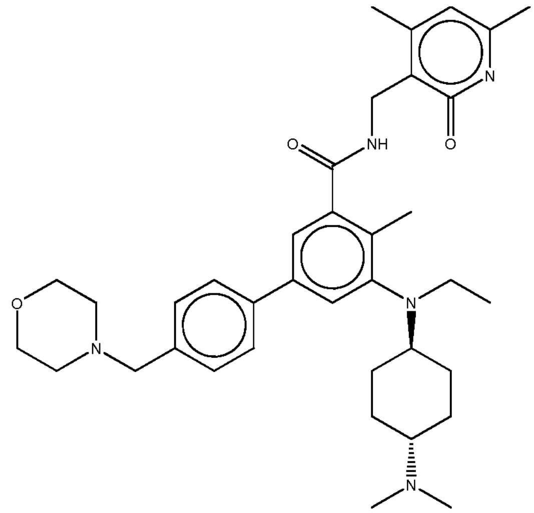 |
| 14 | 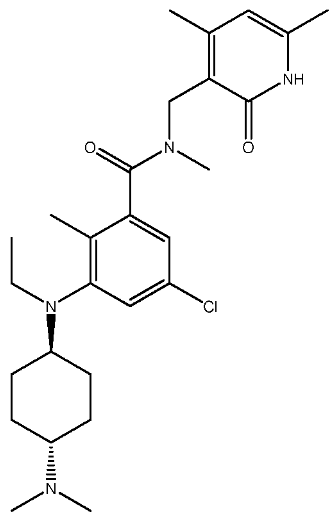 |
| 15 | 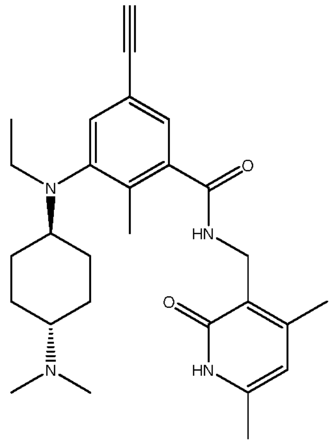 |

(continued)

| | |
|---|---|
| 16 | 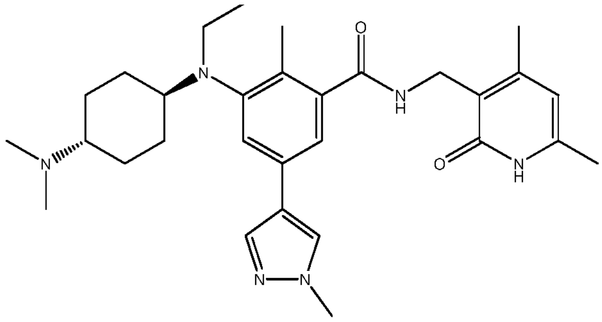 |
| 17 | 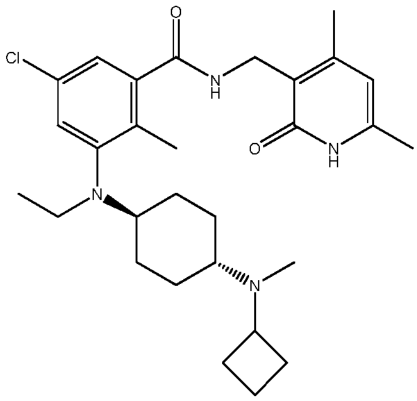 |
| 18 | 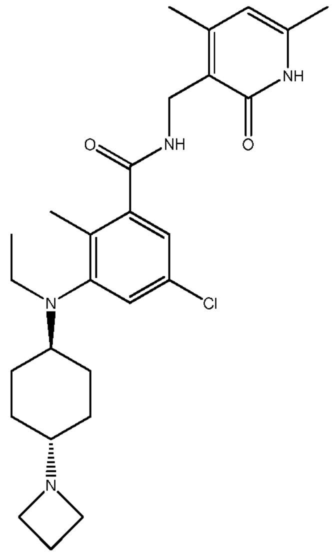 |

(continued)

| | |
|---|---|
| 19 | 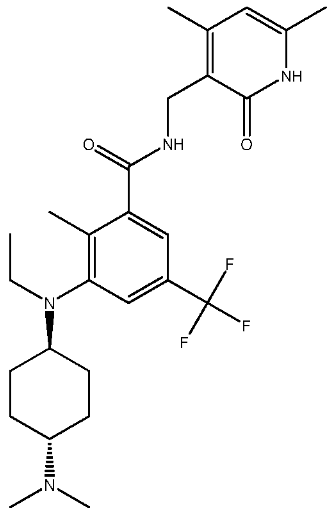 |
| 20 | 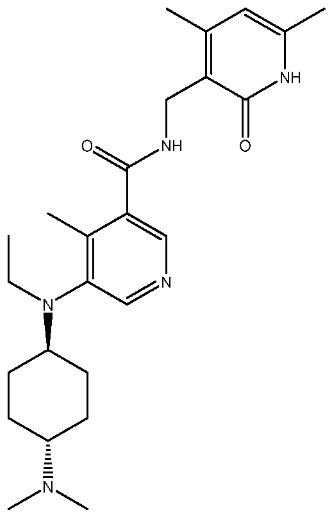 |
| 21 | 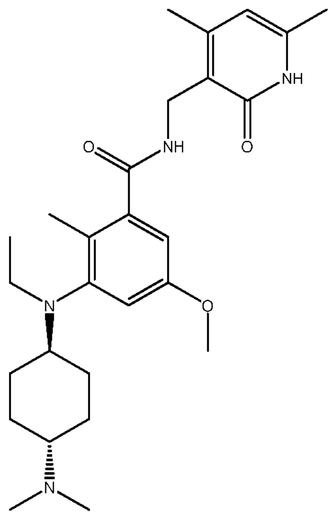 |

(continued)

| | |
|---|---|
| 22 | 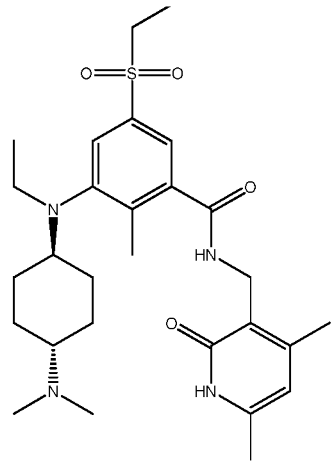 |
| 23 | 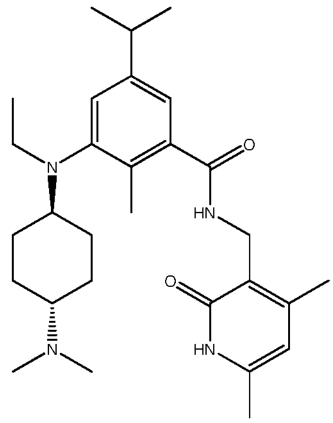 |
| 24 | 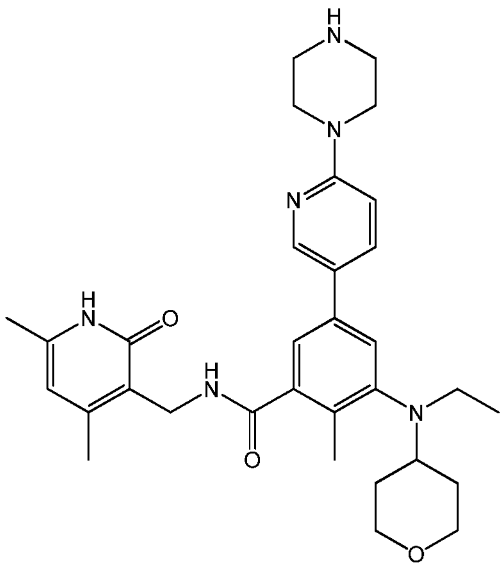 |

(continued)

| | |
|---|---|
| 25 | 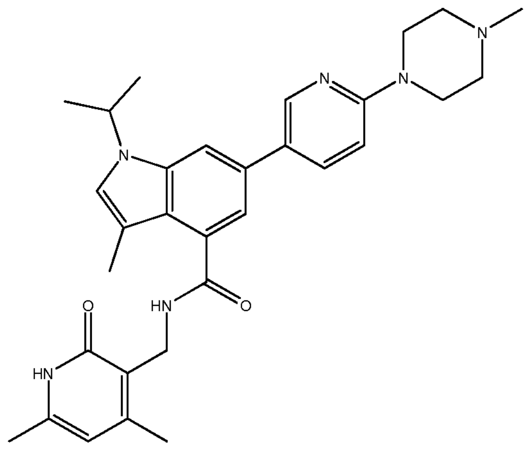 |
| 26 | 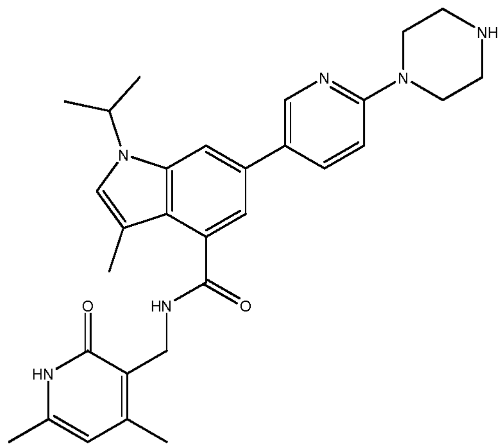 |
| 27 | 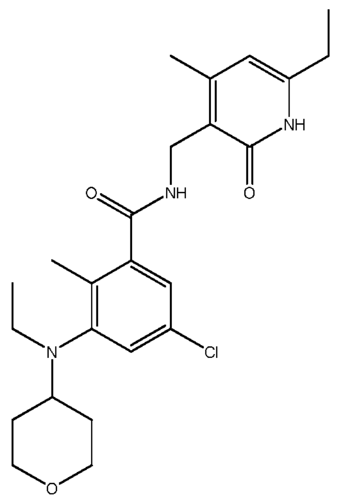 |

(continued)

| | |
|---|---|
| 28 | 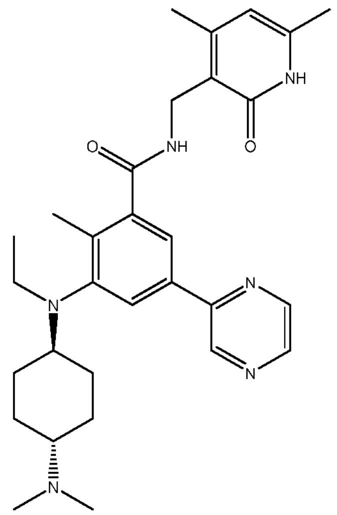 |
| 29 | 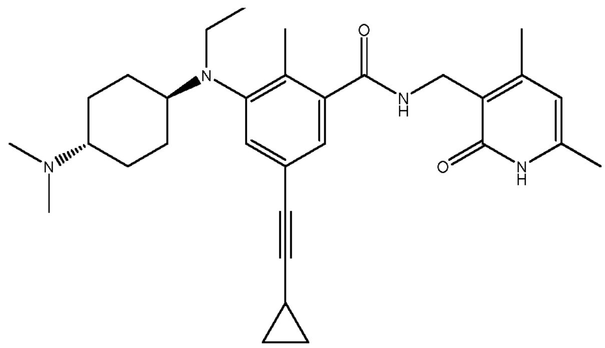 |
| 30 | 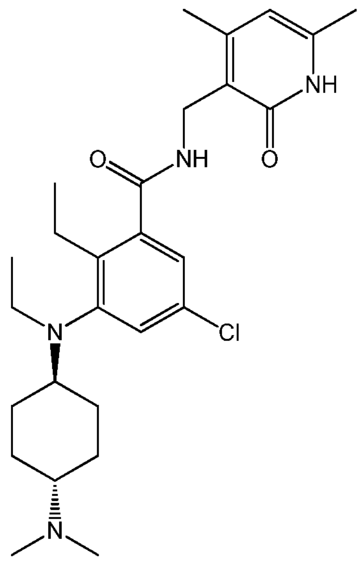 |

(continued)

| | |
|---|---|
| 31 | 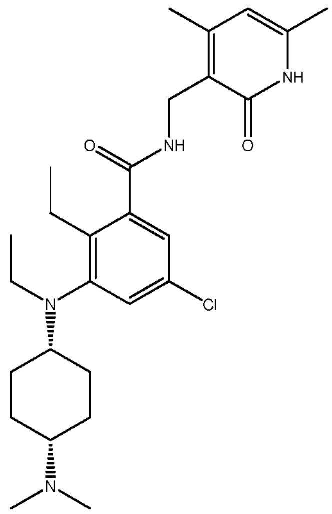 |
| 32 | 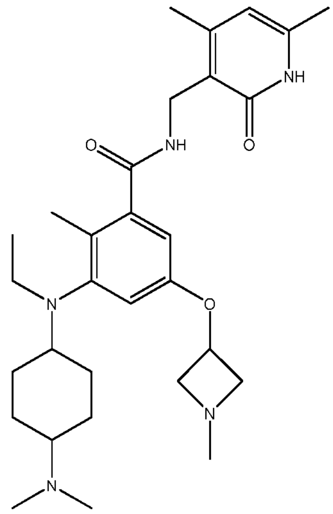 |
| 33 | 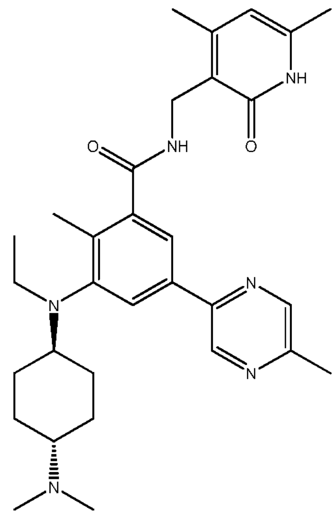 |

(continued)

| | |
|---|---|
| 34 | 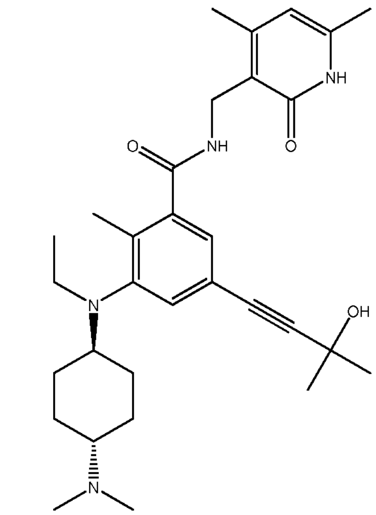 |
| 35 | 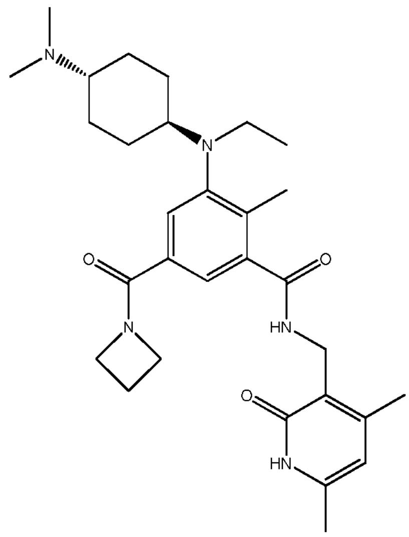 |
| 36 | 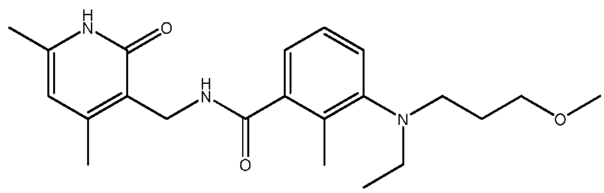 |

(continued)

| | |
|---|---|
| 37 | 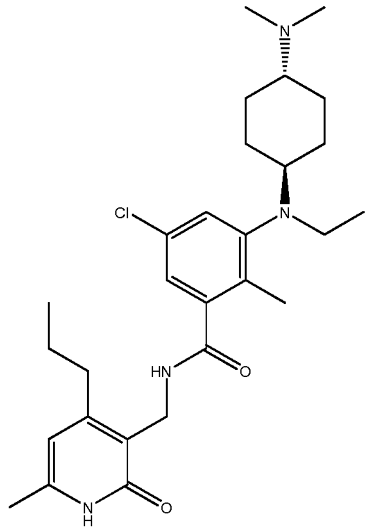 |
| 38 | 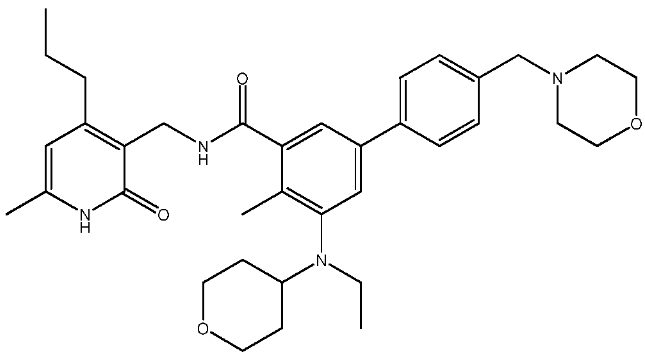 |
| 39 | 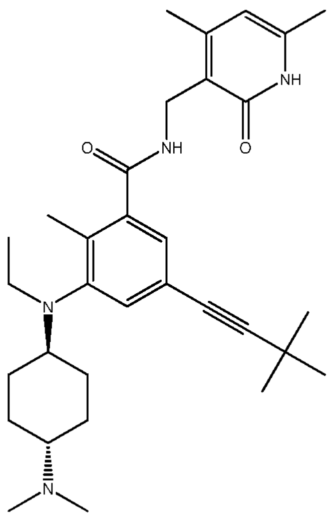 |

(continued)

| | |
|---|---|
| 40 | 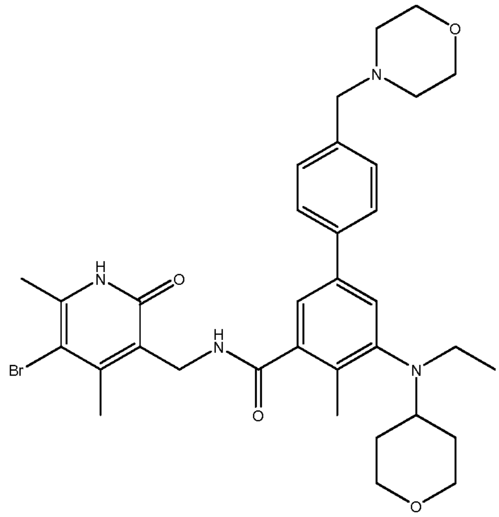 |
| 41 | 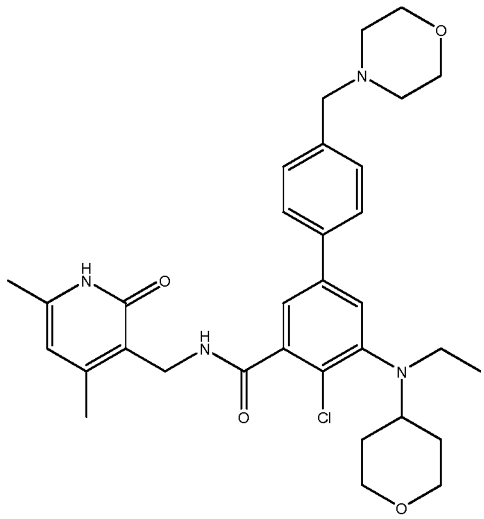 |
| 42 | 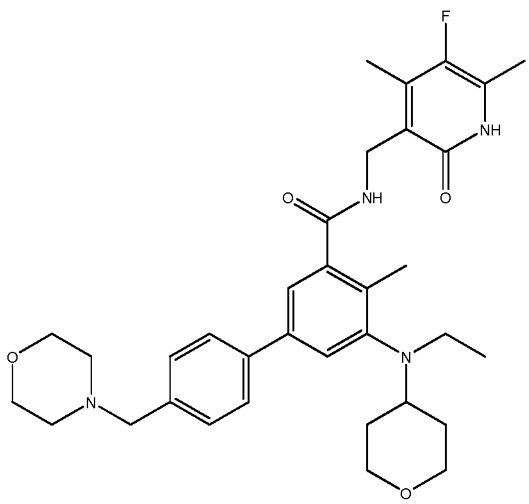 |

(continued)

| | |
|---|---|
| 43 | 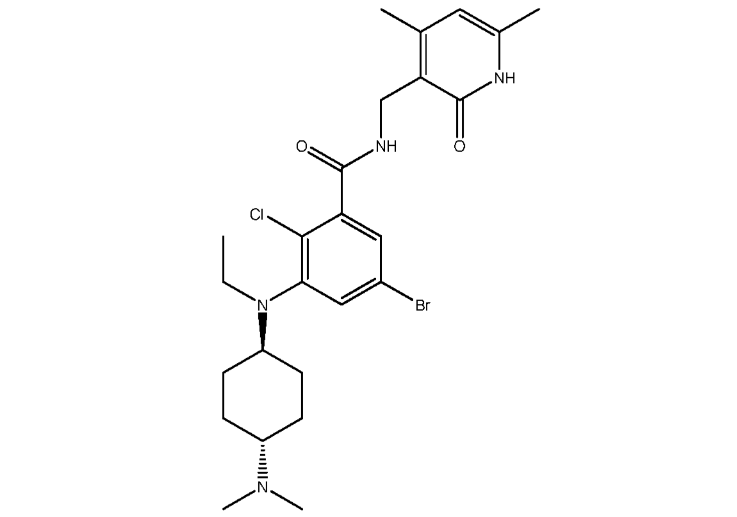 |
| 44 | 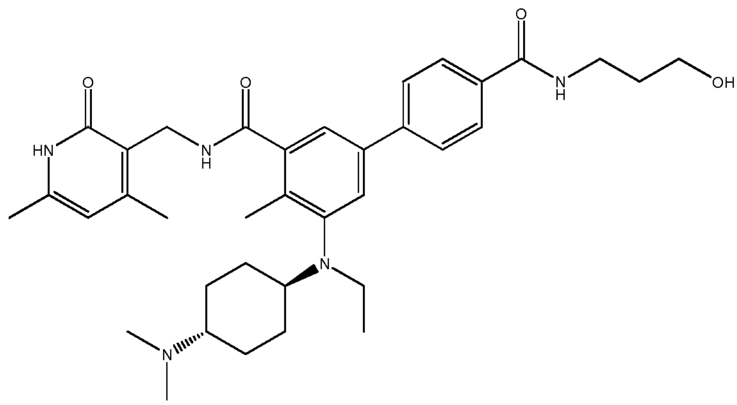 |
| 45 | 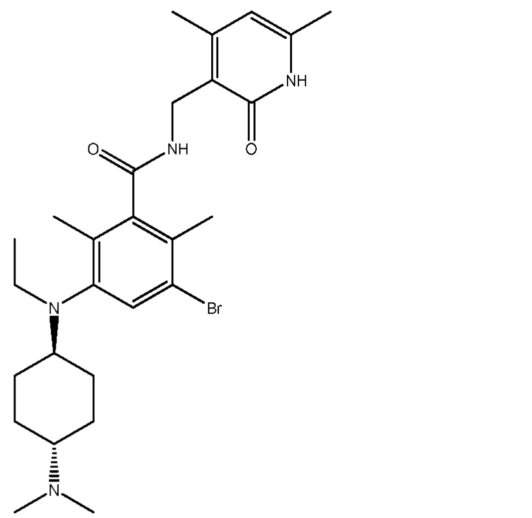 |

(continued)

| | |
|---|---|
| 46 | 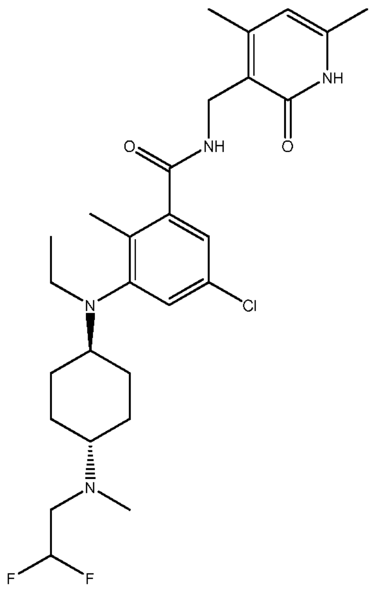 |
| 47 | 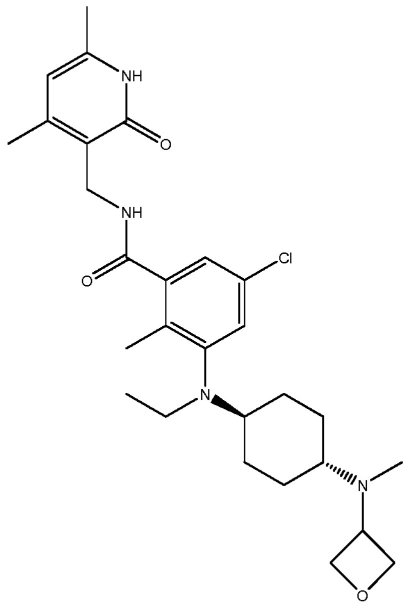 |
| 48 | 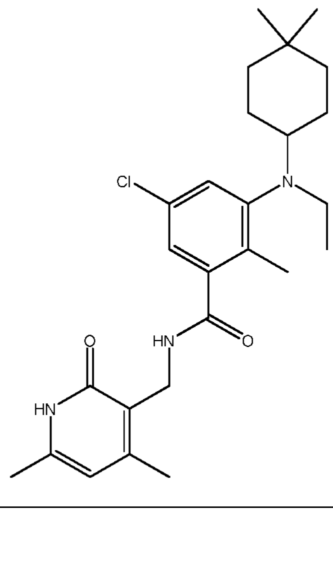 |

(continued)

| | |
|---|---|
| 49 | 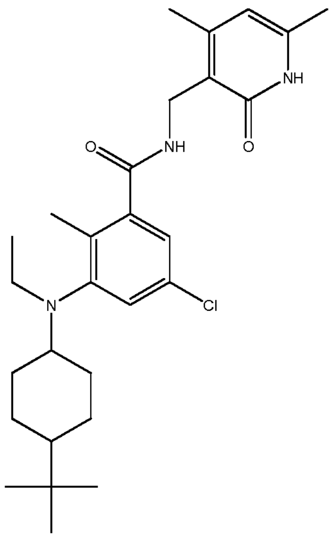 |
| 50 | 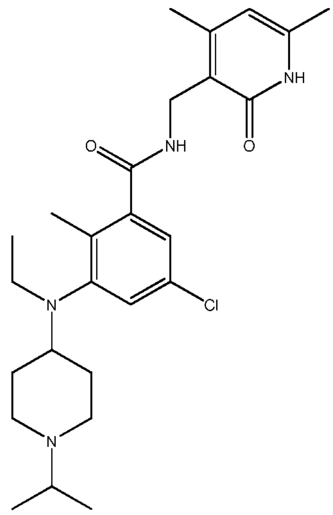 |
| 51 | 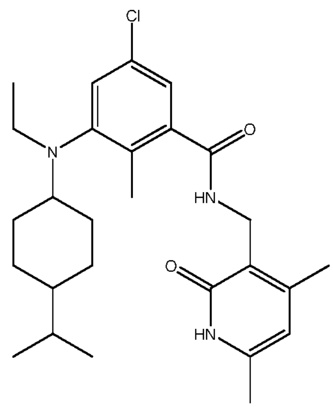 |

(continued)

| | |
|---|---|
| 52 | 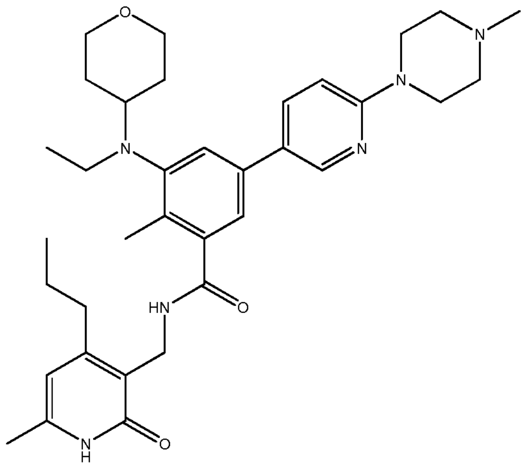 |
| 53 | 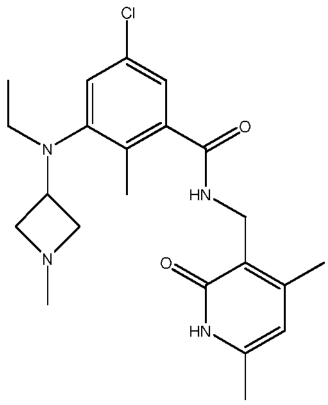 |
| 54 | 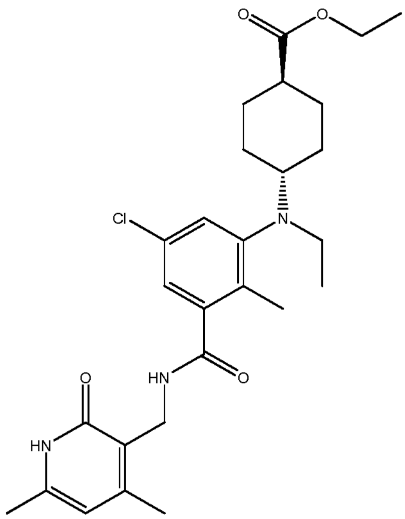 |

(continued)

| | |
|---|---|
| 55 | 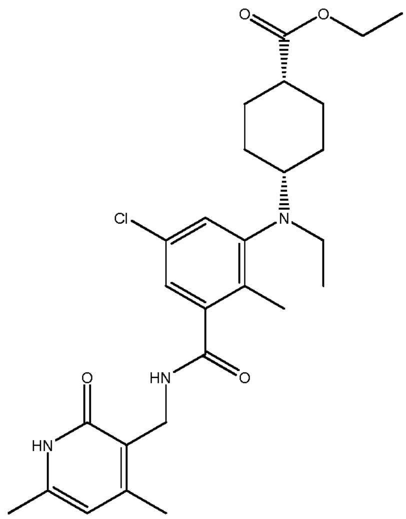 |
| 56 | 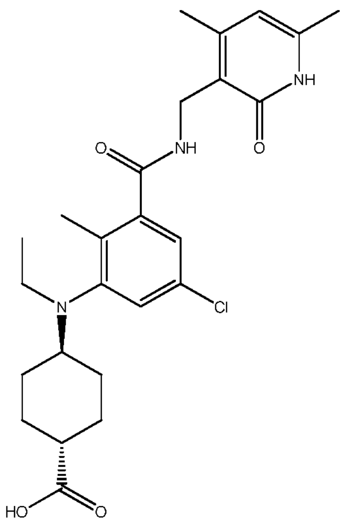 |
| 57 | 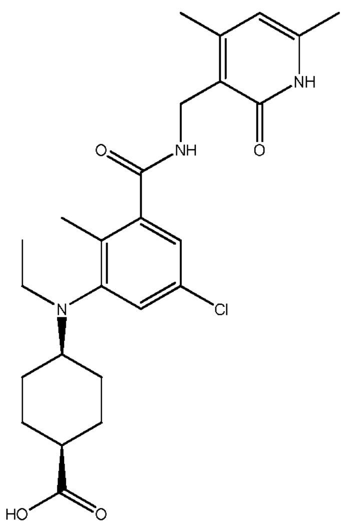 |

(continued)

| | |
|---|---|
| 58 | 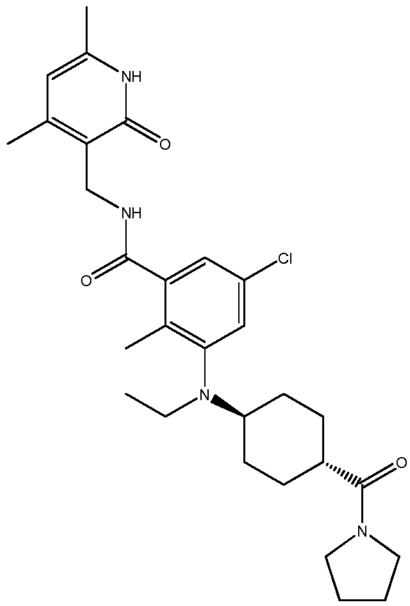 |
| 59 | 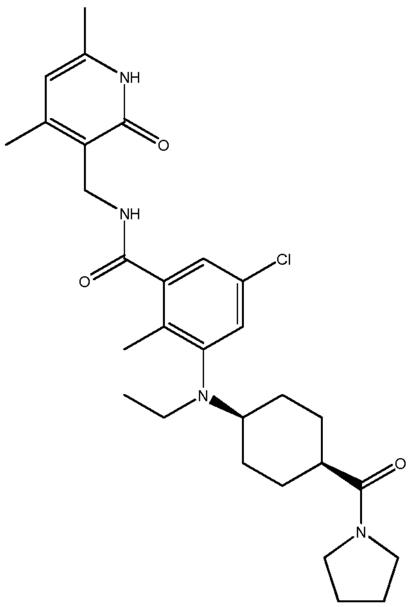 |

(continued)

| | |
|---|---|
| 60 | 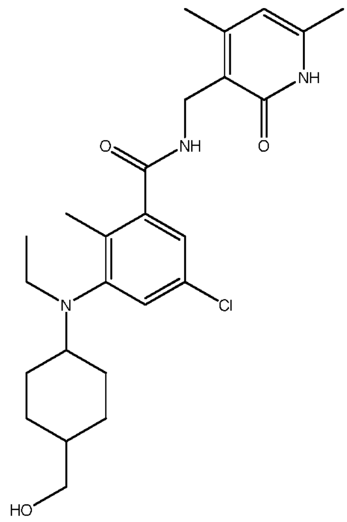 |
| 61 | 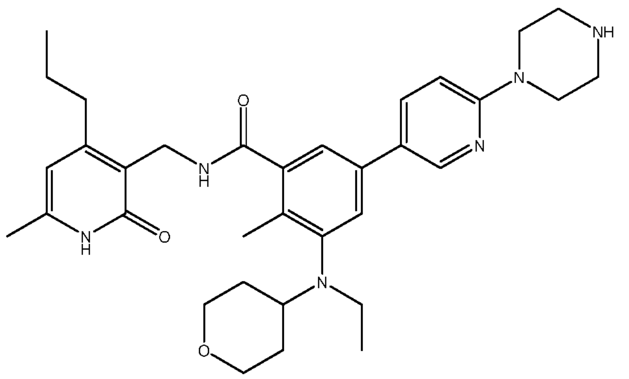 |
| 62 | 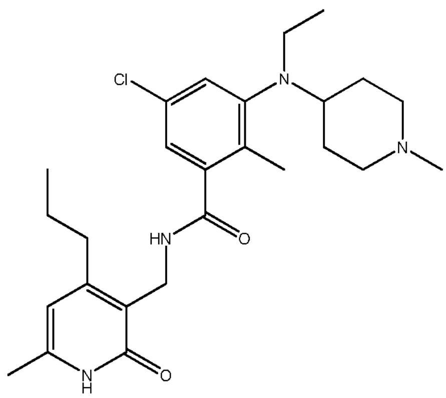 |

or a pharmaceutically acceptable salt thereof for use in a method of treating cancer or myelodysplastic syndromes (MDS, formerly known as preleukemia) in a subject expressing a mutant EZH2, wherein the mutant EZH2 comprises a substitution mutation at amino acid position 677, 687 or 641 of SEQ ID No:1.

2. An EZH2 inhibitor for the use according to claim 1, wherein the mutant EZH2 comprises more than one substitution mutation at amino acid position 677, 687 or 641 of SEQ ID No:1.

3. An EZH2 inhibitor for the use according to claim 1, wherein the EZH2 inhibitor is

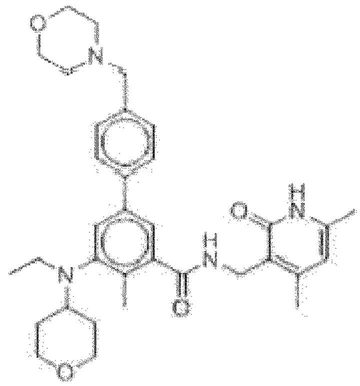

4. An EZH2 inhibitor for the use according to claim 1, wherein the cancer is selected from

a) lymphoma, optionally wherein the lymphoma is selected from the group consisting of non-Hodgkin lymphoma, follicular lymphoma and diffuse large B-cell lymphoma, or
b) leukemia, optionally wherein the leukemia is chronic myelogenous leukemia (CML),
c) melanoma.

5. An EZH2 inhibitor for the use according to claim 1, wherein said mutation is selected from the group consisting of a substitution of glycine (G) for the wild type residue alanine (A) at amino acid position 677 of SEQ ID NO: 1 (A677G); a substitution of valine (V) for the wild type residue alanine (A) at amino acid position 687 of SEQ ID NO: 1 (A687V); a substitution of phenylalanine (F) for the wild type residue tyrosine (Y) at amino acid position 641 of SEQ ID NO: 1 (Y641F); a substitution of histidine (H) for the wild type residue tyrosine (Y) at amino acid position 641 of SEQ ID NO: 1 (Y641H); a substitution of asparagine (N) for the wild type residue tyrosine (Y) at amino acid position 641 of SEQ ID NO: 1 (Y641N); a substitution of serine (S) for the wild type residue tyrosine (Y) at amino acid position 641 of SEQ ID NO: 1 (Y641S); and a substitution of cysteine (C) for the wild type residue tyrosine (Y) at amino acid position 641 of SEQ ID NO: 1 (Y641C).

6. A method of determining in vitro the responsiveness of a subject, having cancer or myelodysplastic syndromes (MDS, formerly known as preleukemia), to an EZH2 inhibitor selected from compound 2 to compound 62,

| | |
|---|---|
| 2 | 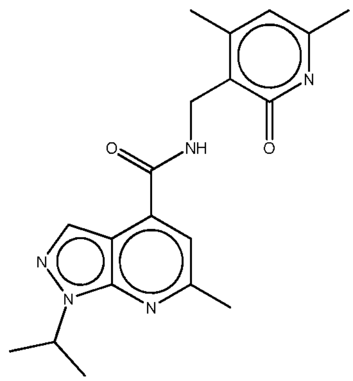 |

(continued)

| | |
|---|---|
| 3 | 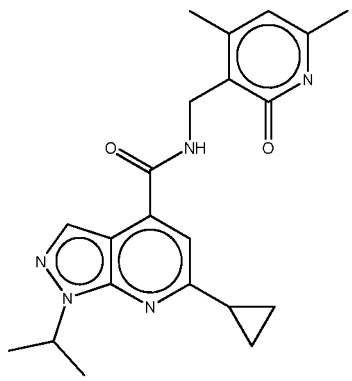 |
| 4 | 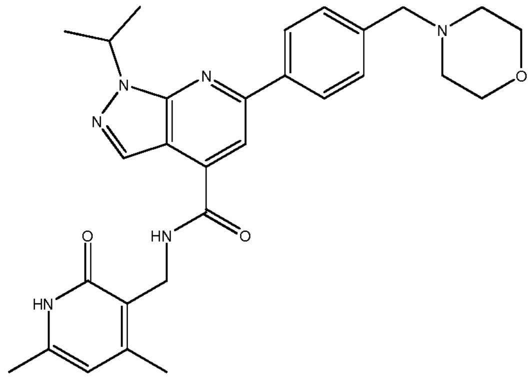 |
| 5 | 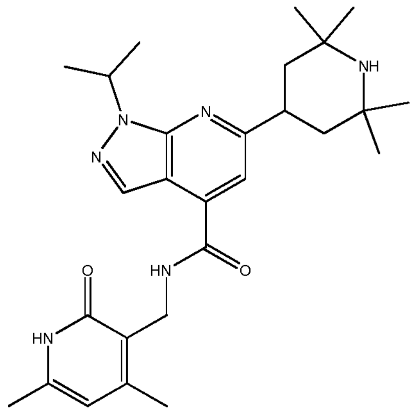 |
| 6 | 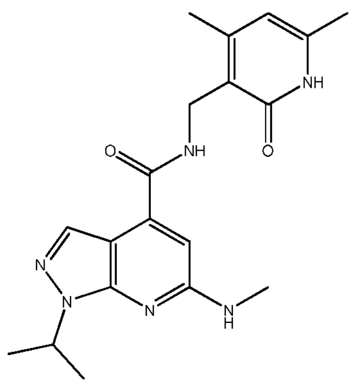 |

(continued)

| | |
|---|---|
| 7 | 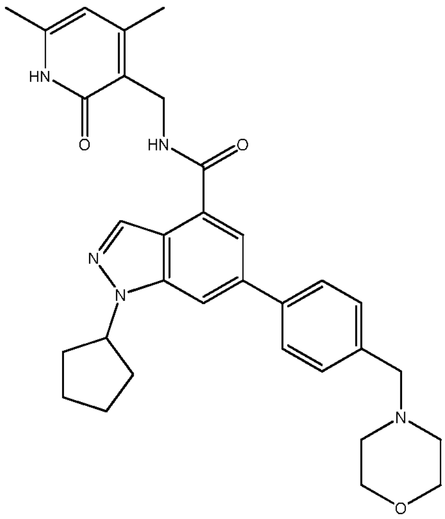 |
| 8 | 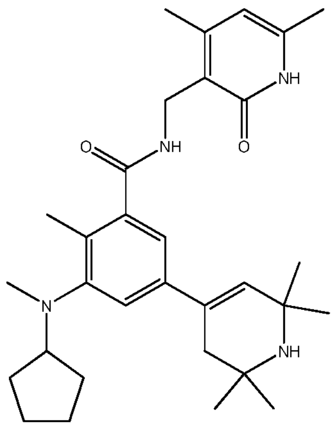 |
| 9 | 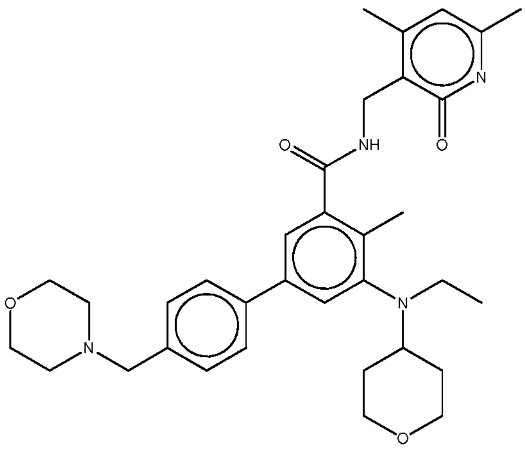 |

(continued)

| | |
|---|---|
| 10 | 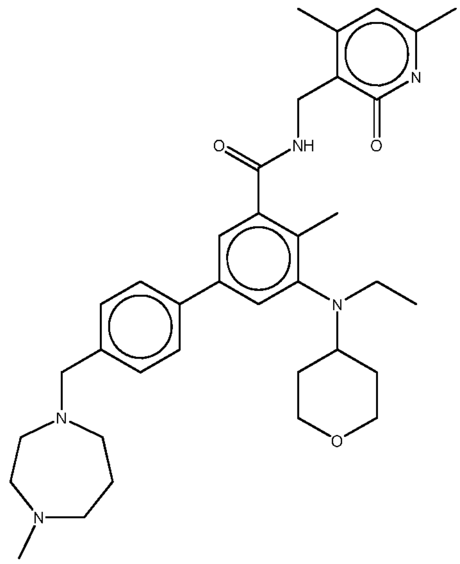 |
| 11 | 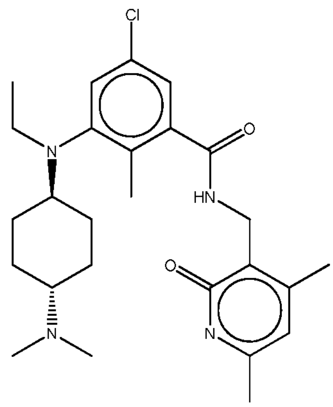 |
| 12 | 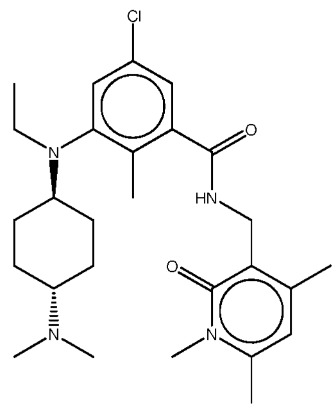 |

(continued)

| | |
|---|---|
| 13 | 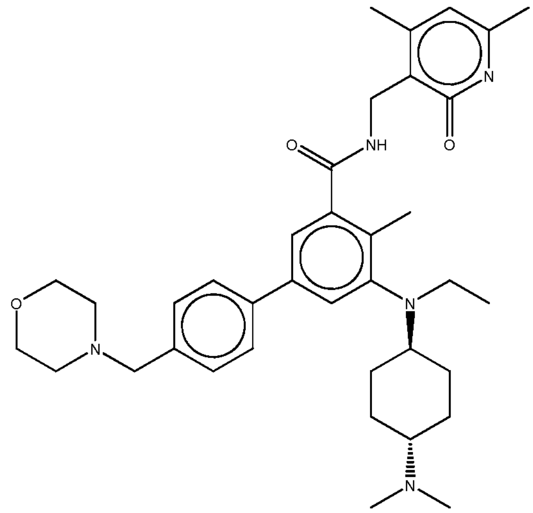 |
| 14 | 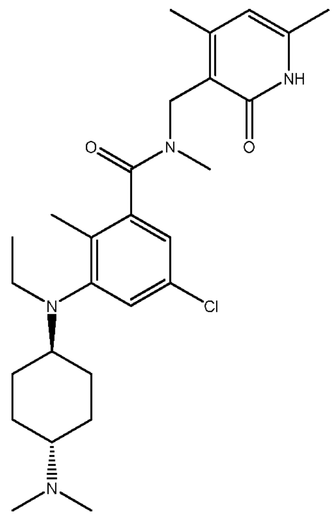 |
| 15 | 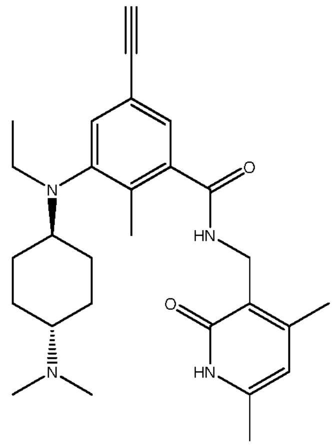 |

(continued)

| | |
|---|---|
| 16 | 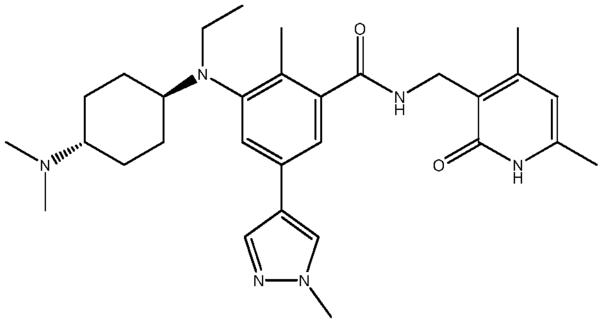 |
| 17 | 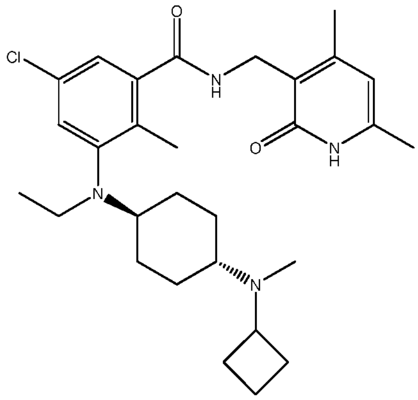 |
| 18 | 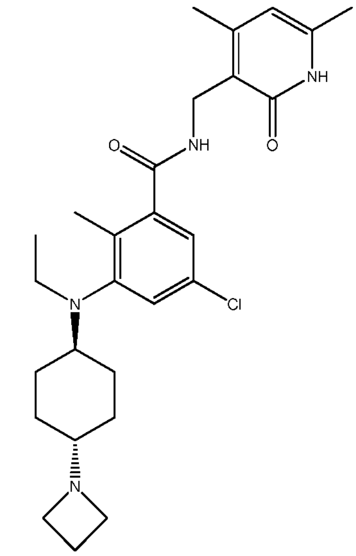 |

(continued)

| | |
|---|---|
| 19 | 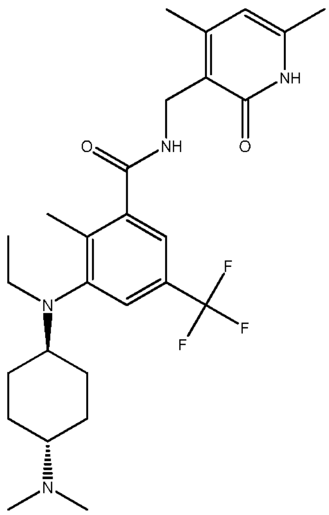 |
| 20 | 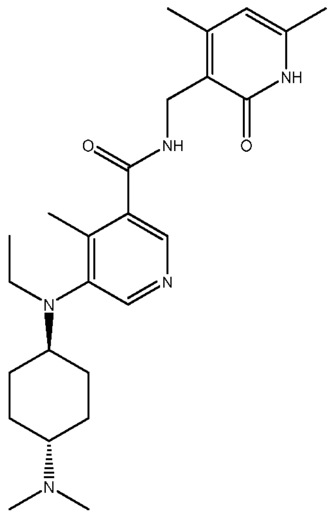 |
| 21 | 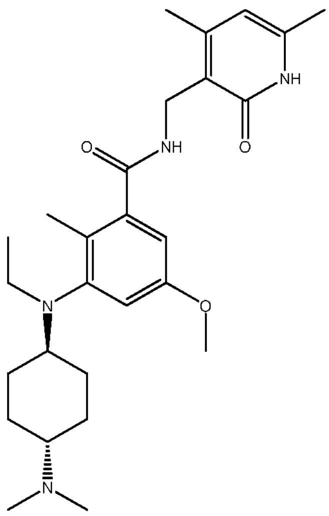 |

(continued)

| | |
|---|---|
| 22 | 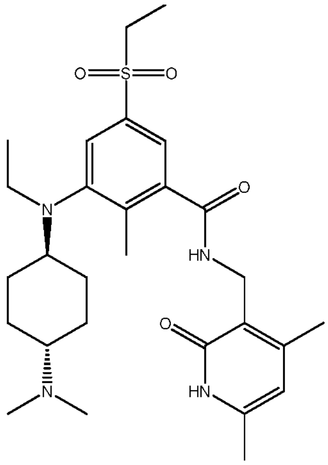 |
| 23 | 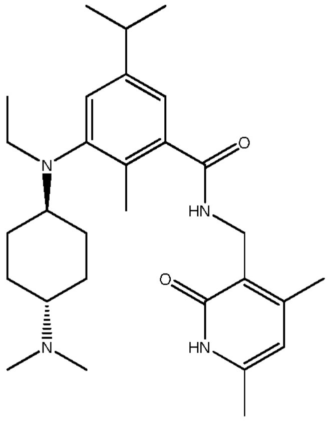 |
| 24 | 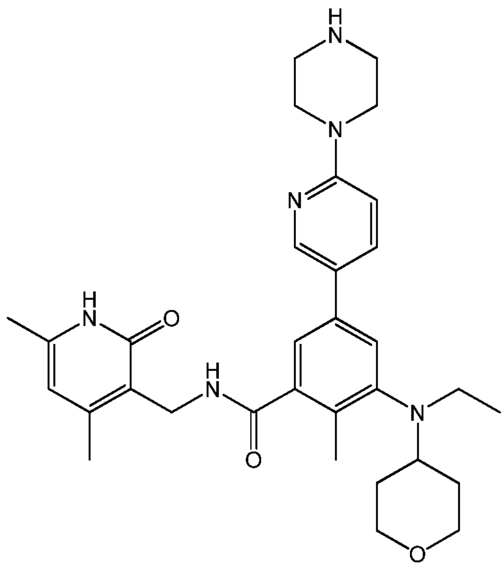 |

(continued)

| | |
|---|---|
| 25 | 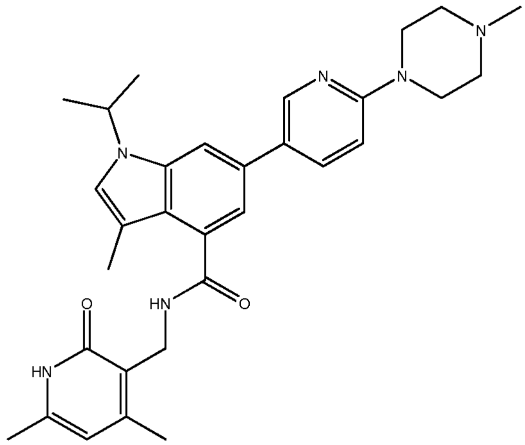 |
| 26 | 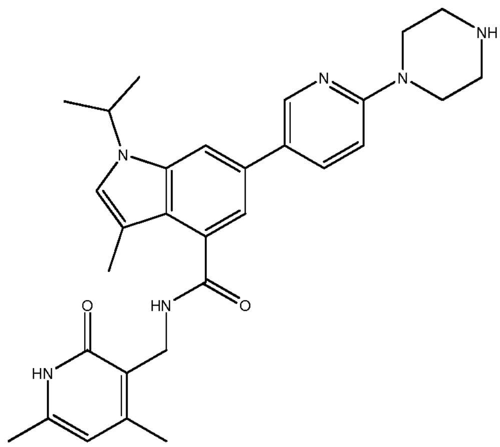 |
| 27 | 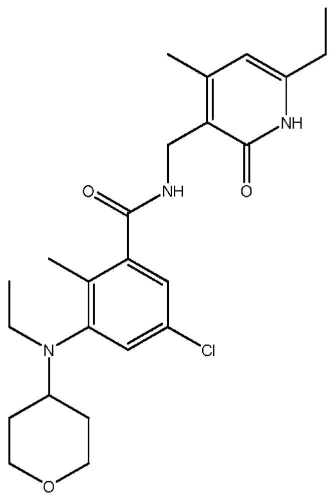 |

(continued)

| | |
|---|---|
| 28 | 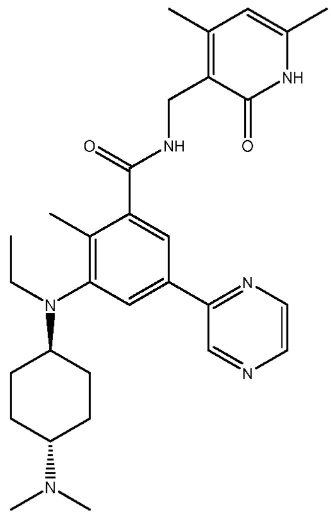 |
| 29 | 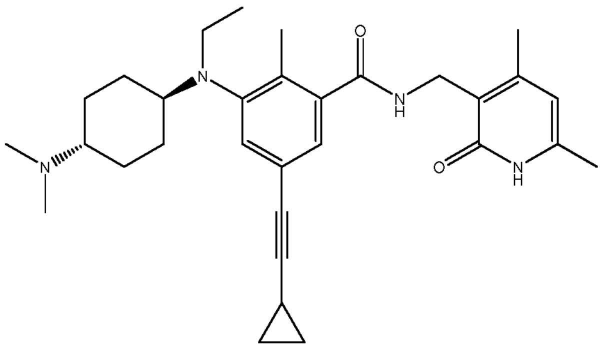 |
| 30 | 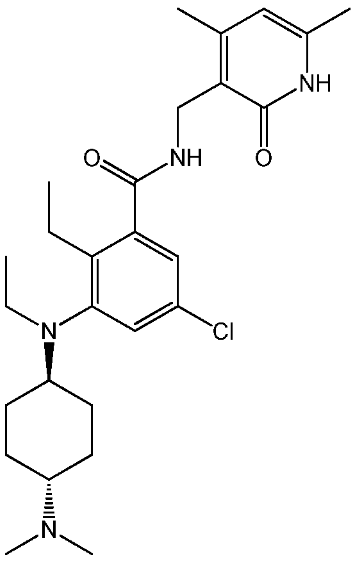 |

(continued)

| | |
|---|---|
| 31 | 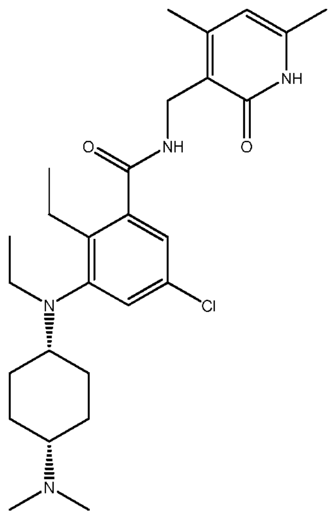 |
| 32 | 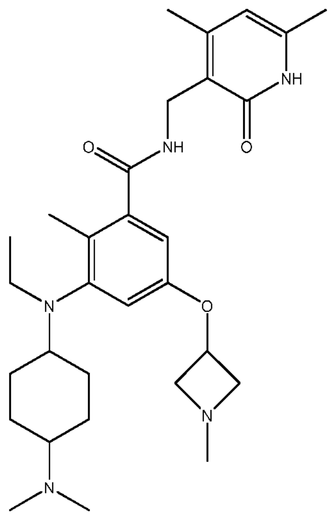 |
| 33 | 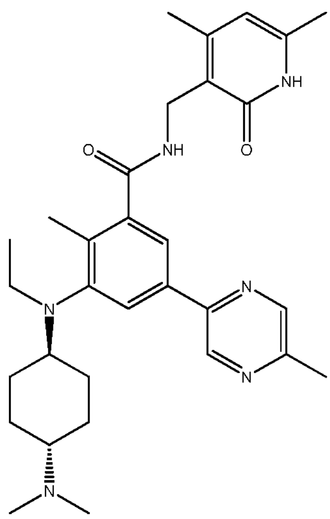 |

(continued)

| | |
|---|---|
| 34 | 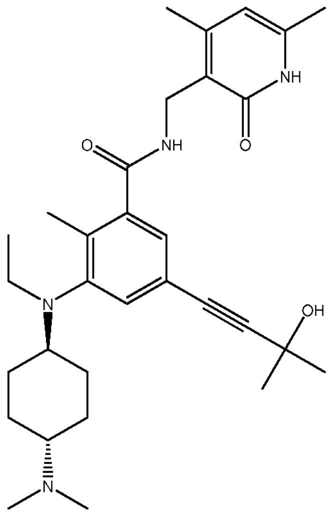 |
| 35 | 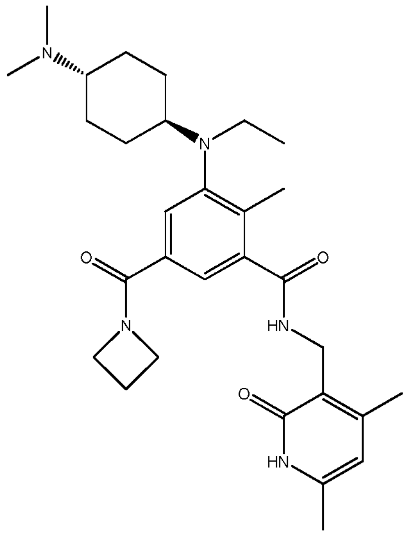 |
| 36 | 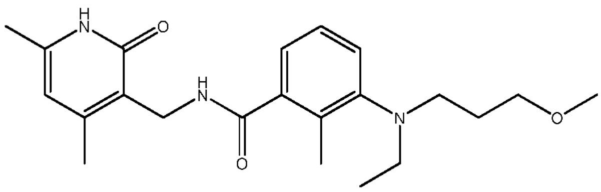 |

(continued)

| | |
|---|---|
| 37 | 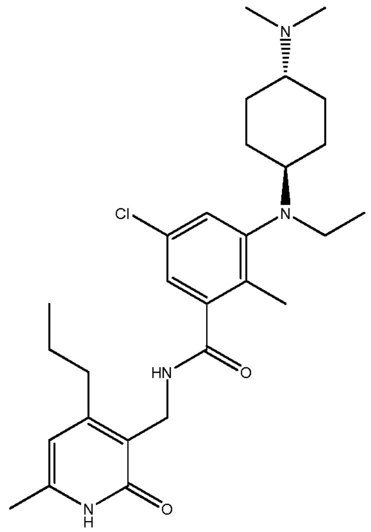 |
| 38 | 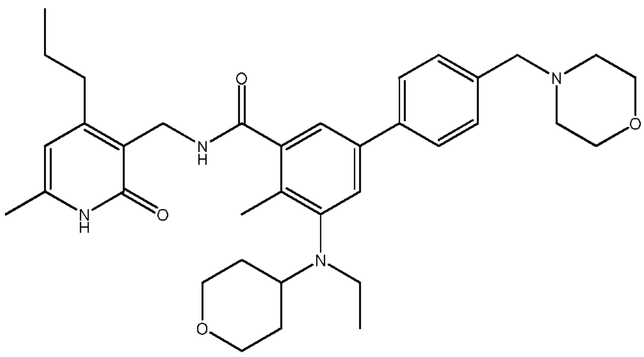 |
| 39 | 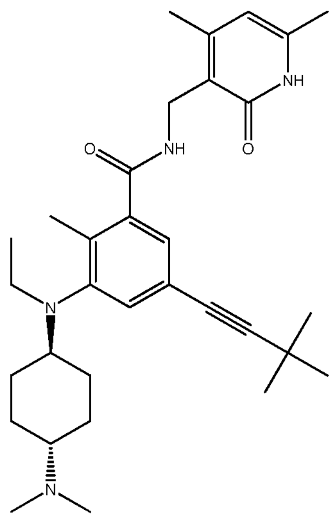 |

(continued)

| | |
|---|---|
| 40 | 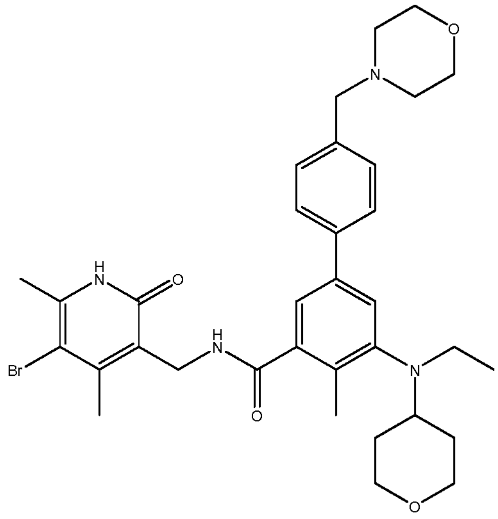 |
| 41 | 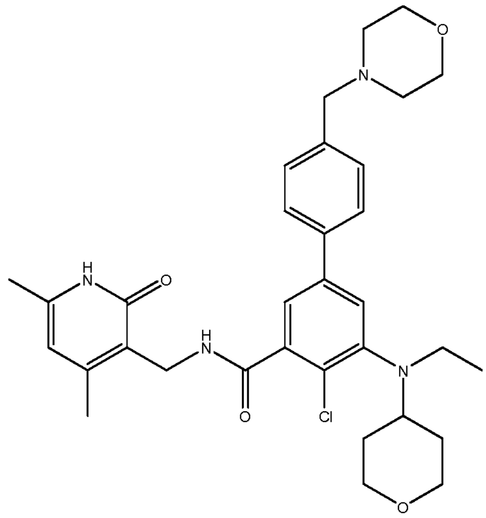 |
| 42 | 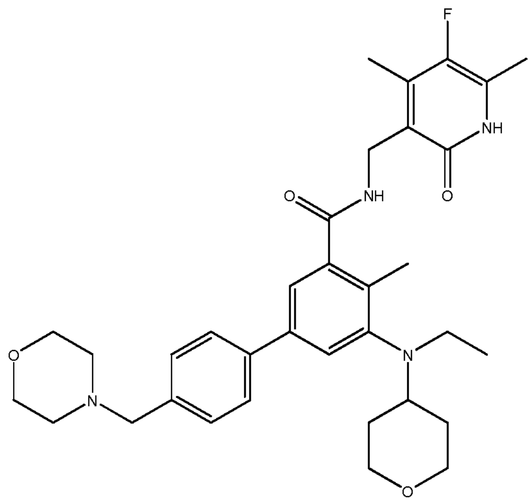 |

(continued)

| | |
|---|---|
| 43 | 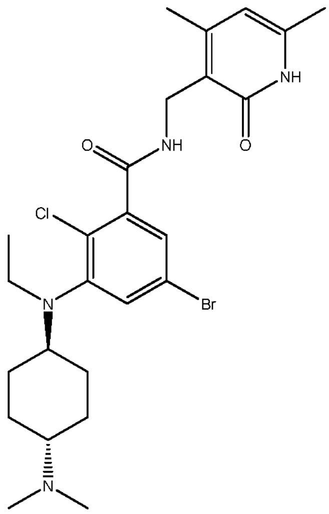 |
| 44 | 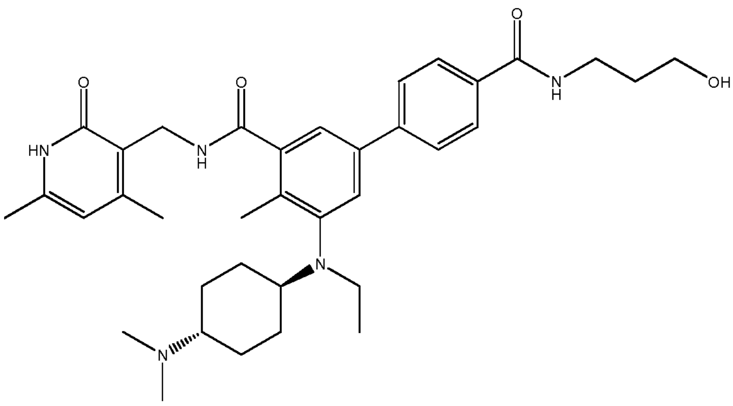 |
| 45 | 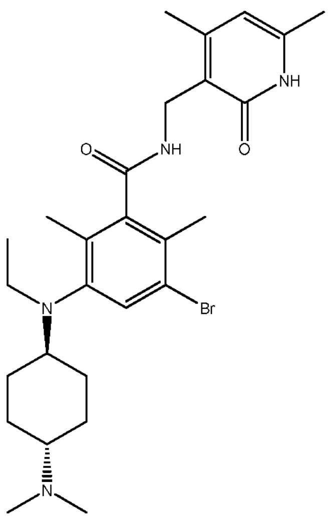 |

(continued)

| | |
|---|---|
| 46 | 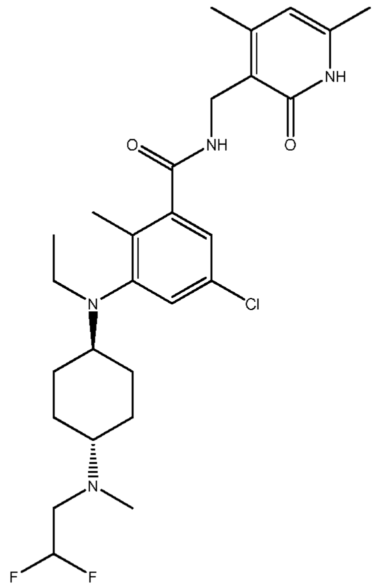 |
| 47 | 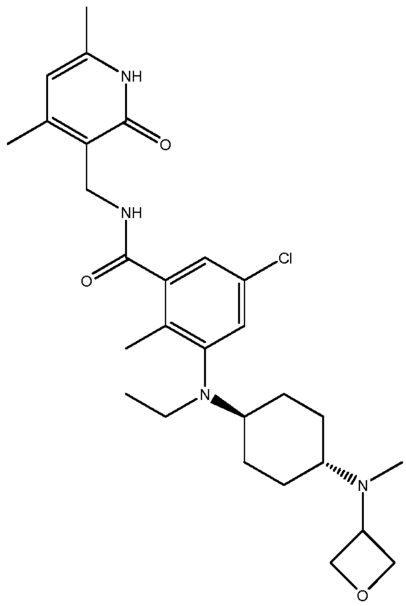 |

(continued)

| | |
|---|---|
| 48 | 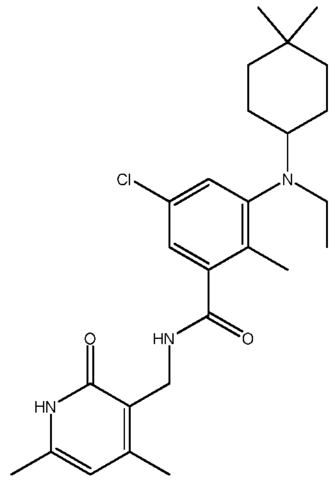 |
| 49 | 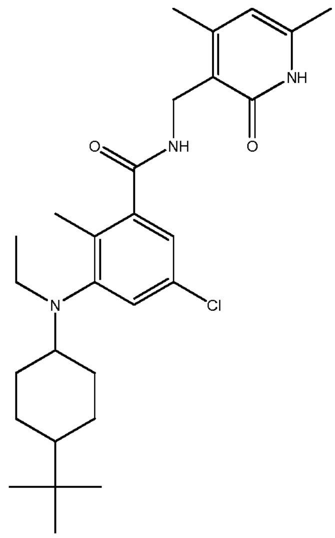 |
| 50 | 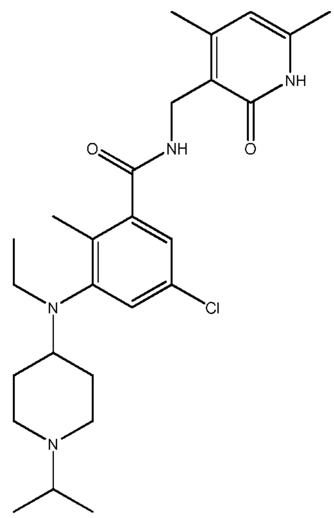 |

(continued)

| | |
|---|---|
| 51 | 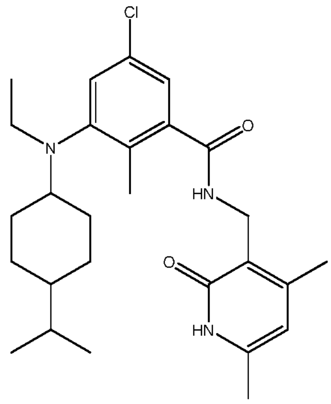 |
| 52 | 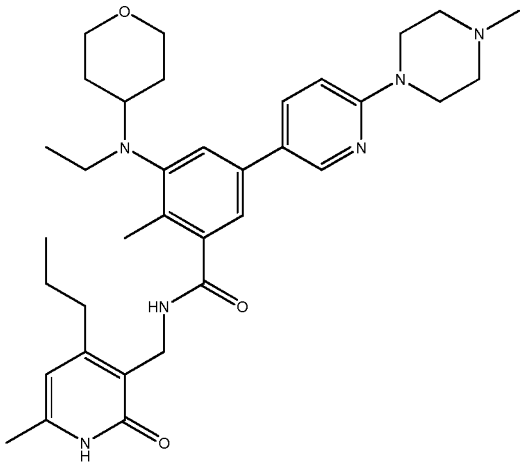 |
| 53 | 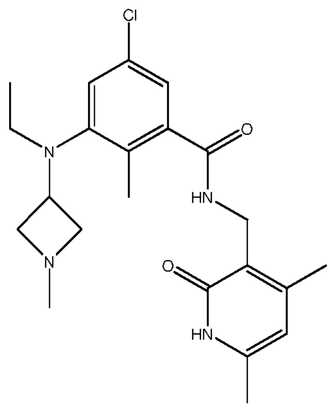 |

(continued)

| | |
|---|---|
| 54 | 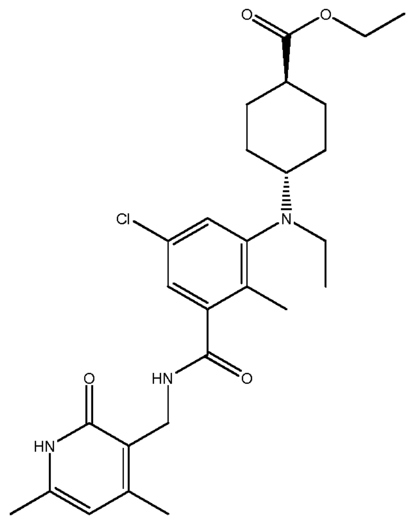 |
| 55 | 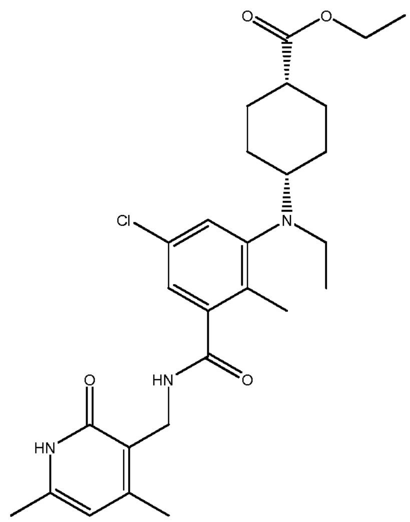 |
| 56 | 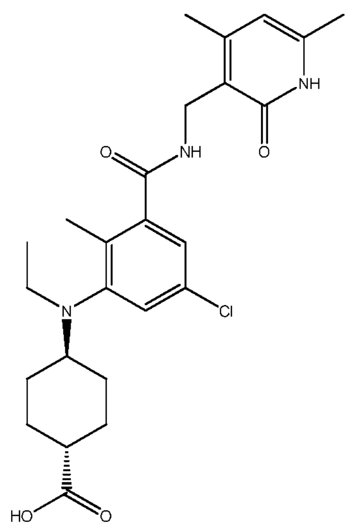 |

(continued)

| | |
|---|---|
| 57 | 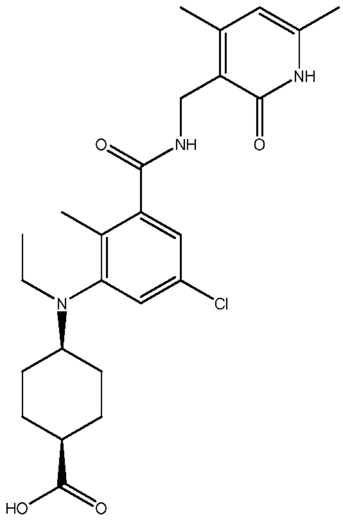 |
| 58 | 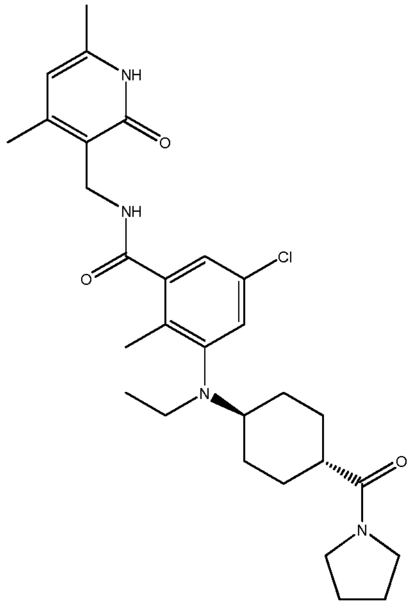 |

(continued)

| | |
|---|---|
| 59 | 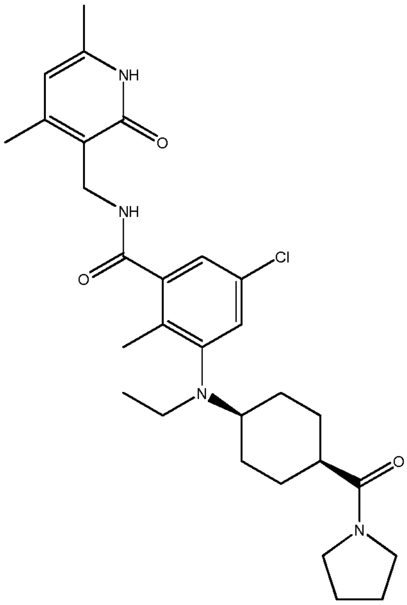 |
| 60 | 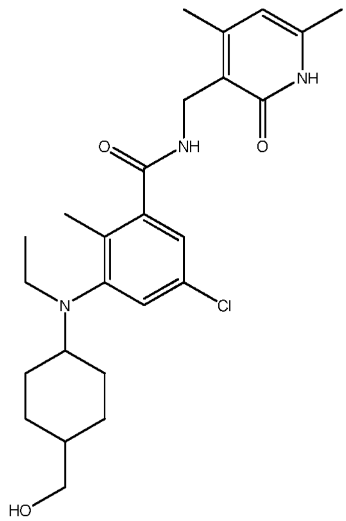 |
| 61 | 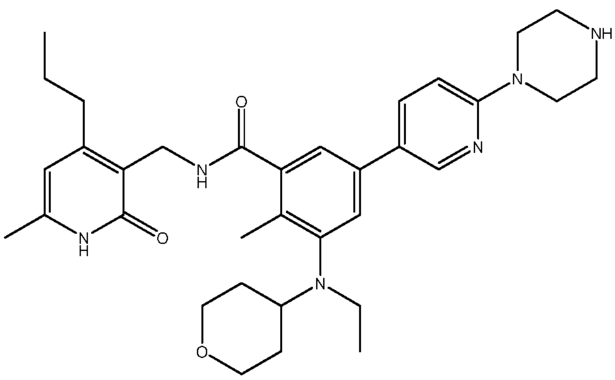 |

(continued)

| | |
|---|---|
| 62 | 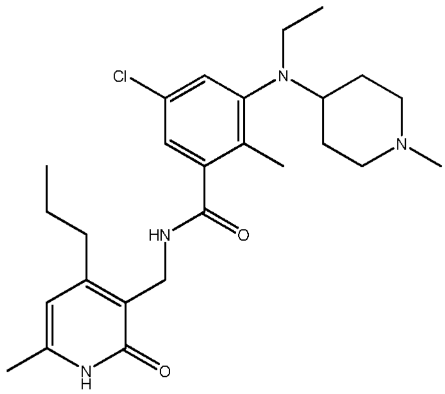 |

comprising detecting the presence of mutation in EZH2 in a sample obtained from the subject, wherein the mutation is or is expressed as a substitution mutation at amino acid position 677, 687 or 641 of SEQ ID No:1, wherein the presence of said mutation indicates the subject is responsive to the EZH2 inhibitor.

**7.** A method according to claim 6, wherein the method includes detecting the presence of more than one substitution mutation at amino acid position 677, 687 or 641 of SEQ ID No:1.

**8.** A method according to claim 6, wherein the cancer is selected from

a) lymphoma, optionally wherein the lymphoma is selected from the group consisting of non-Hodgkin lymphoma, follicular lymphoma and diffuse large B-cell lymphoma, or
b) leukemia, optionally wherein the leukemia is chronic myelogenous leukemia (CML), or
c) melanoma.

**9.** A method of claim 6, wherein said mutation is selected from the group consisting of a substitution of glycine (G) for the wild type residue alanine (A) at amino acid position 677 of SEQ ID NO: 1 (A677G); a substitution of valine (V) for the wild type residue alanine (A) at amino acid position 687 of SEQ ID NO: 1 (A687V); a substitution of phenylalanine (F) for the wild type residue tyrosine (Y) at amino acid position 641 of SEQ ID NO: 1 (Y641F); a substitution of histidine (H) for the wild type residue tyrosine (Y) at amino acid position 641 of SEQ ID NO: 1 (Y641H); a substitution of asparagine (N) for the wild type residue tyrosine (Y) at amino acid position 641 of SEQ ID NO: 1 (Y641N); a substitution of serine (S) for the wild type residue tyrosine (Y) at amino acid position 641 of SEQ ID NO: 1 (Y641S); and a substitution of cysteine (C) for the wild type residue tyrosine (Y) at amino acid position 641 of SEQ ID NO: 1 (Y641C).

## Patentansprüche

**1.** EZH2-Hemmstoff, ausgewählt aus Verbindung 2 bis Verbindung 62

| | |
|---|---|
| 2 | 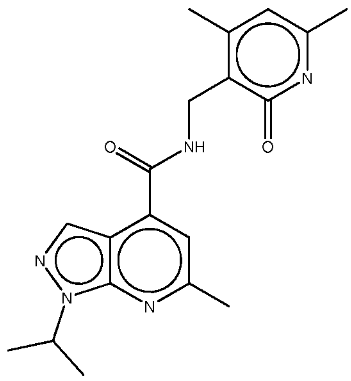 |
| 3 | 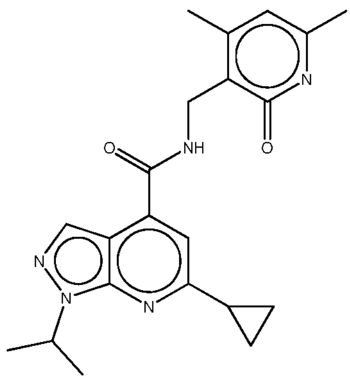 |
| 4 | 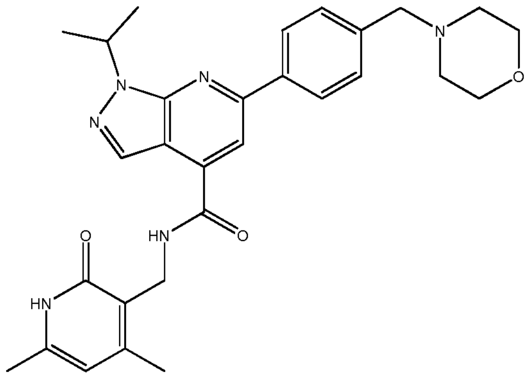 |
| 5 | 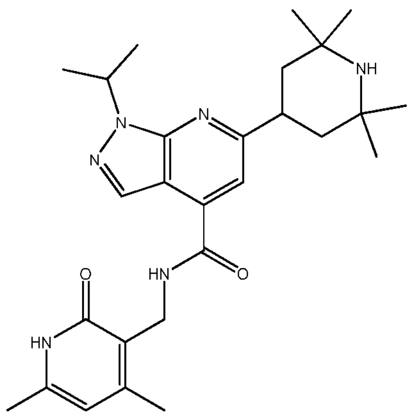 |

(fortgesetzt)

| | |
|---|---|
| 6 | 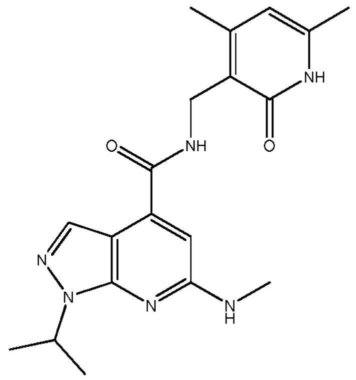 |
| 7 | 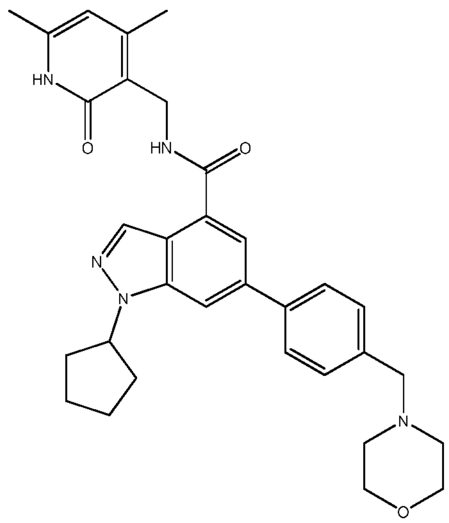 |
| 8 | 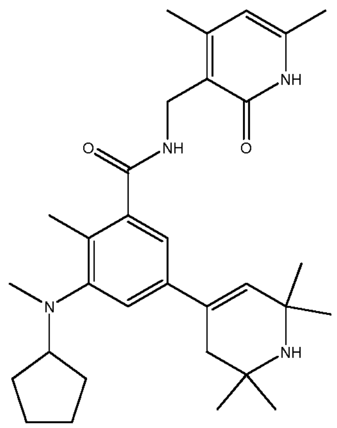 |

(fortgesetzt)

| | |
|---|---|
| 9 | 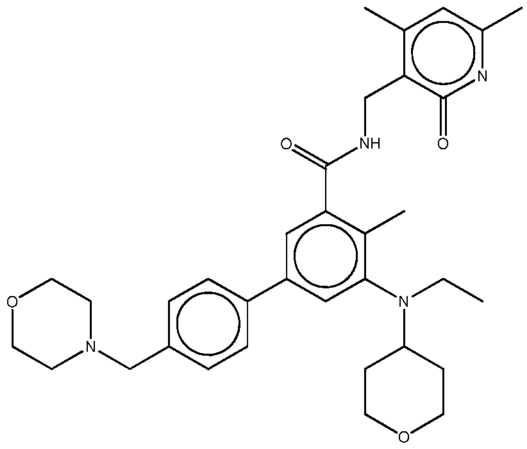 |
| 10 | 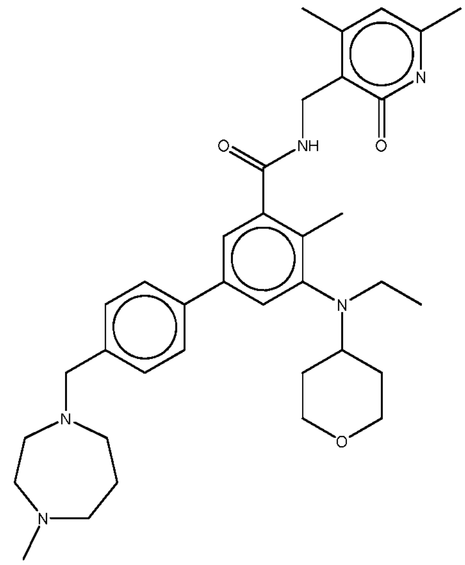 |
| 11 | 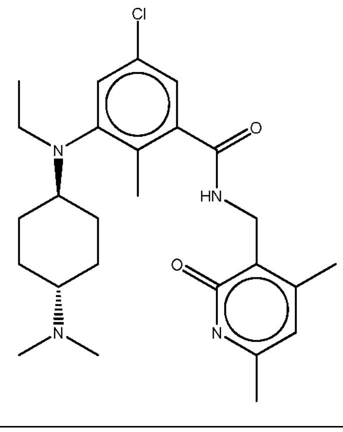 |

(fortgesetzt)

| | |
|---|---|
| 12 | 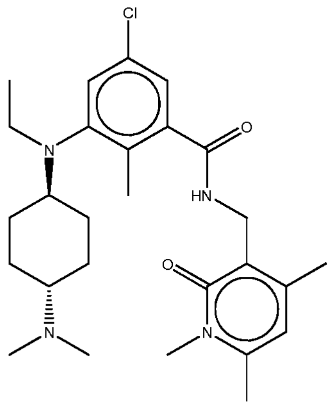 |
| 13 | 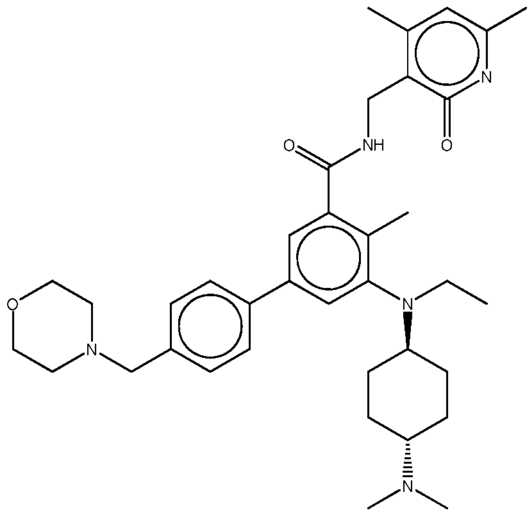 |
| 14 | 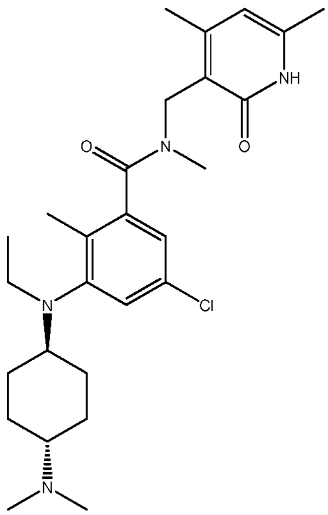 |

(fortgesetzt)

| | |
|---|---|
| 15 | 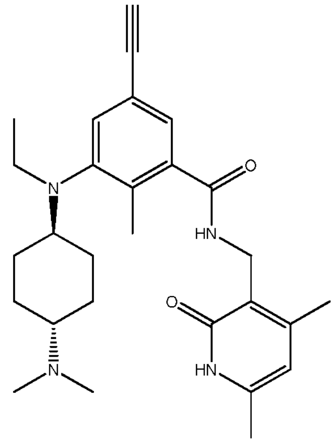 |
| 16 | 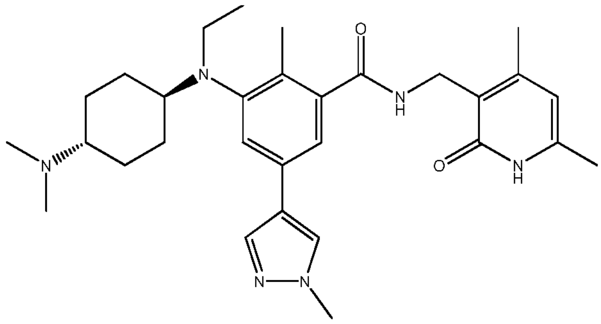 |
| 17 | 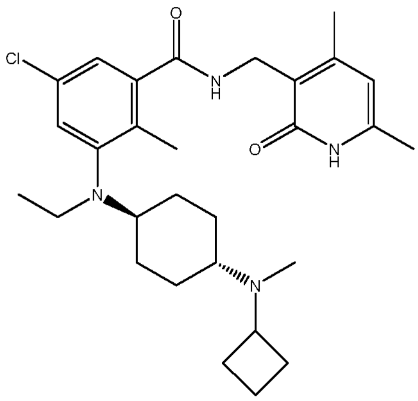 |

(fortgesetzt)

| | |
|---|---|
| 18 | 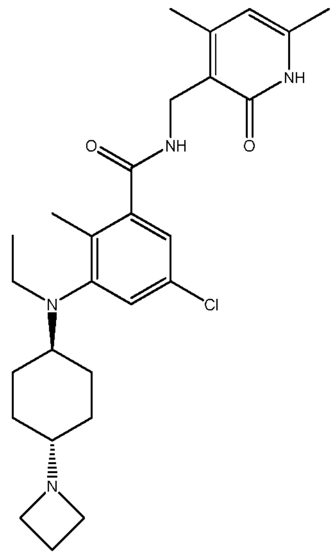 |
| 19 | 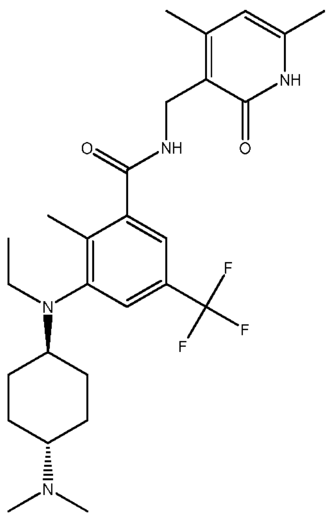 |
| 20 | 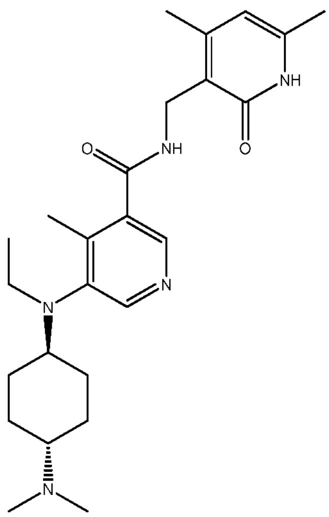 |

(fortgesetzt)

| | |
|---|---|
| 21 | 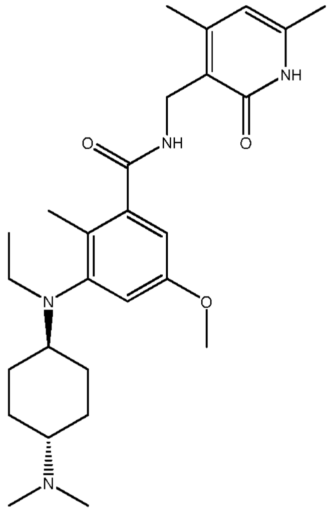 |
| 22 | 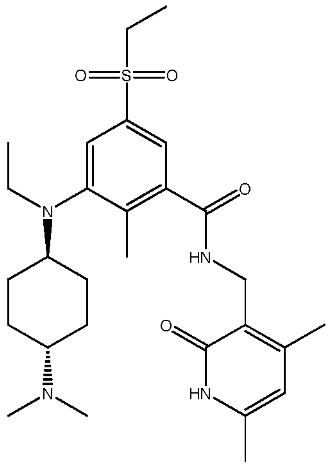 |
| 23 | 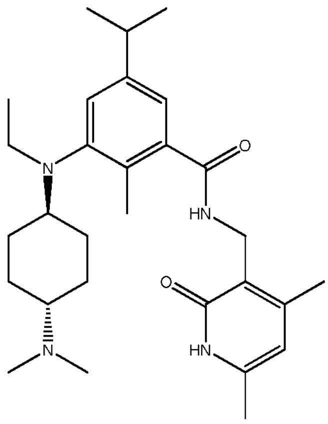 |

(fortgesetzt)

| | |
|---|---|
| 24 | 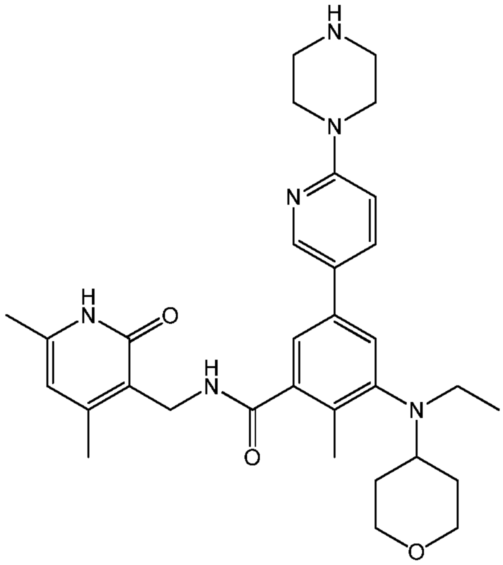 |
| 25 | 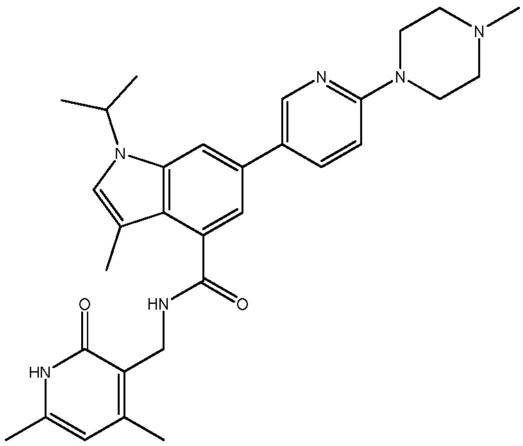 |
| 26 | 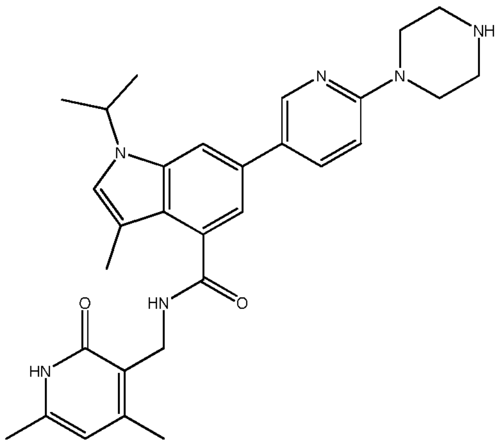 |

(fortgesetzt)

| | |
|---|---|
| 27 | 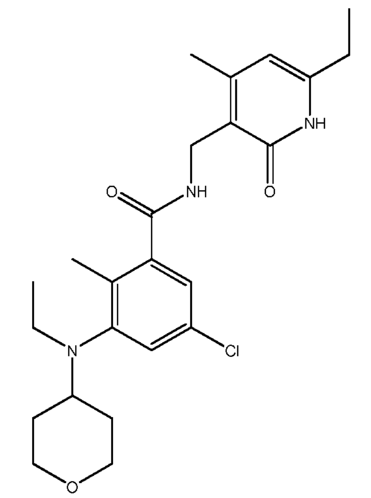 |
| 28 | 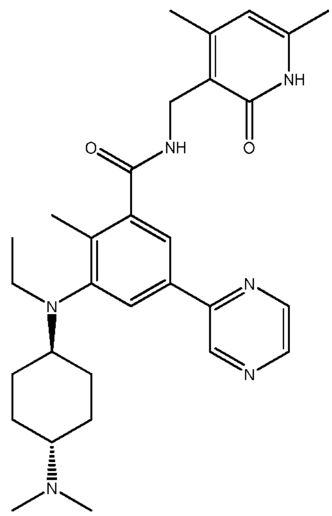 |
| 29 | 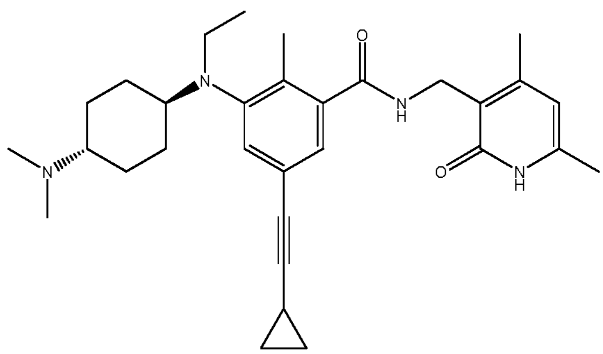 |

(fortgesetzt)

| | |
|---|---|
| 30 | 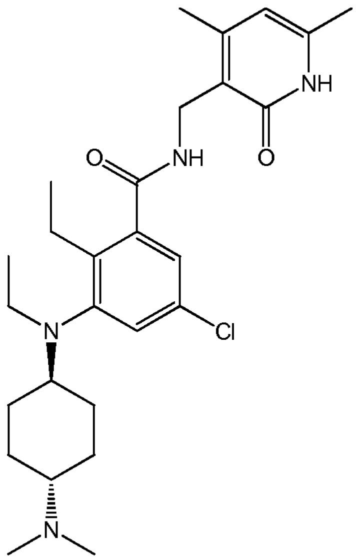 |
| 31 | 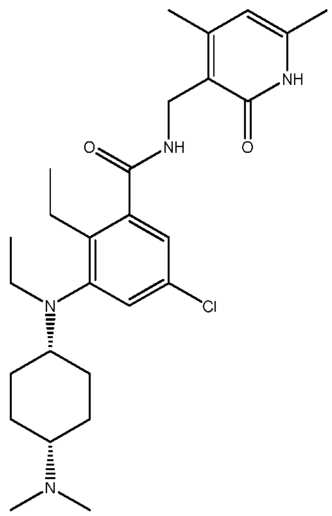 |
| 32 | 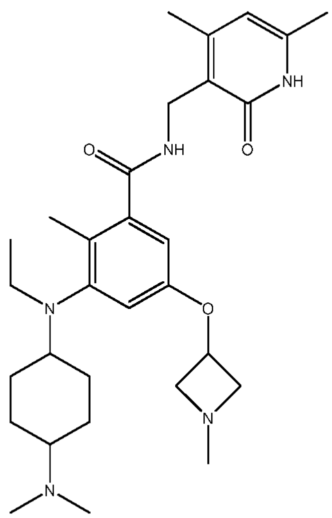 |

(fortgesetzt)

| | |
|---|---|
| 33 | 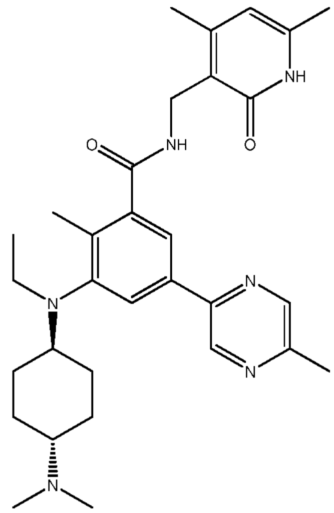 |
| 34 | 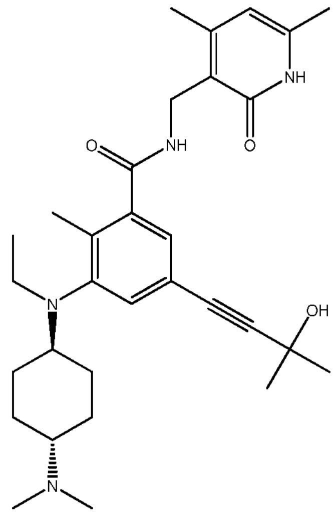 |
| 35 | 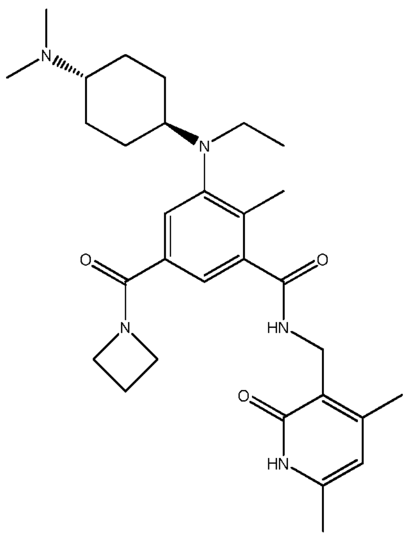 |

(fortgesetzt)

| | |
|---|---|
| 36 | 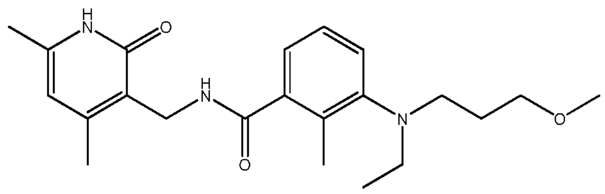 |
| 37 | 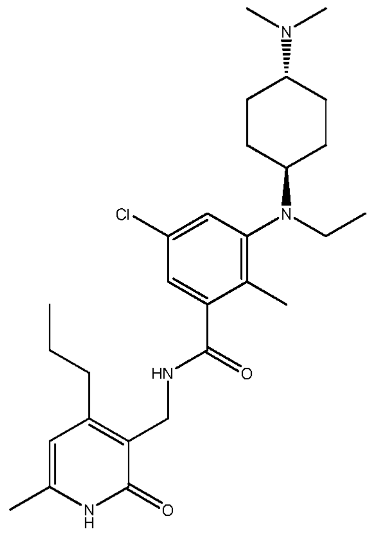 |
| 38 | 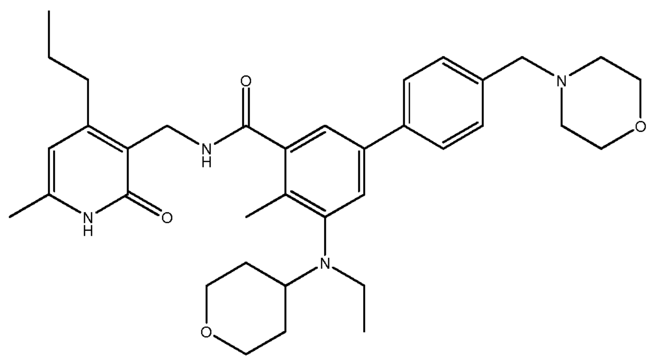 |
| 39 | 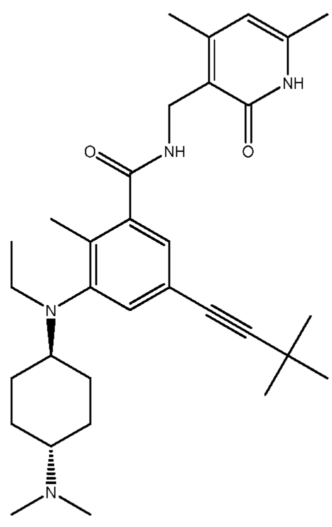 |

(fortgesetzt)

| | |
|---|---|
| 40 | 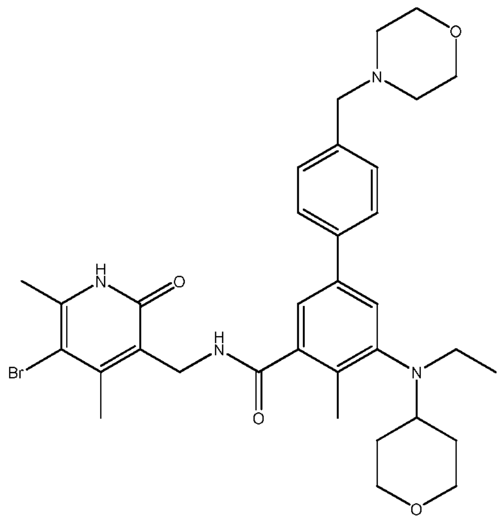 |
| 41 | 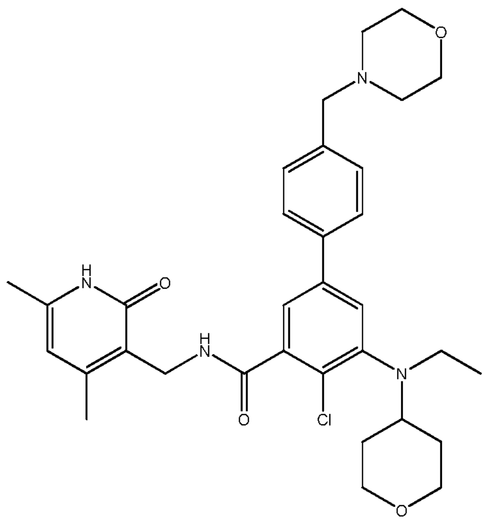 |
| 42 | 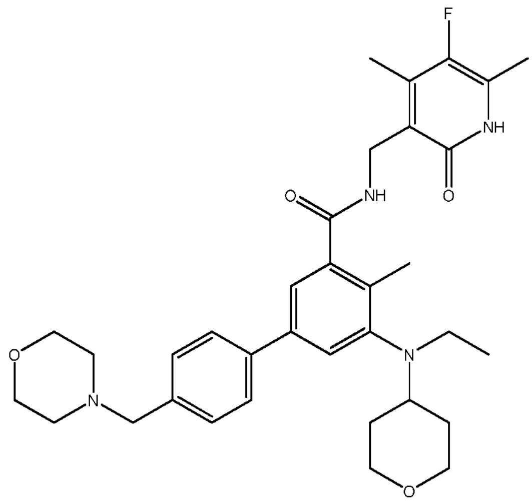 |

(fortgesetzt)

| | |
|---|---|
| 43 | 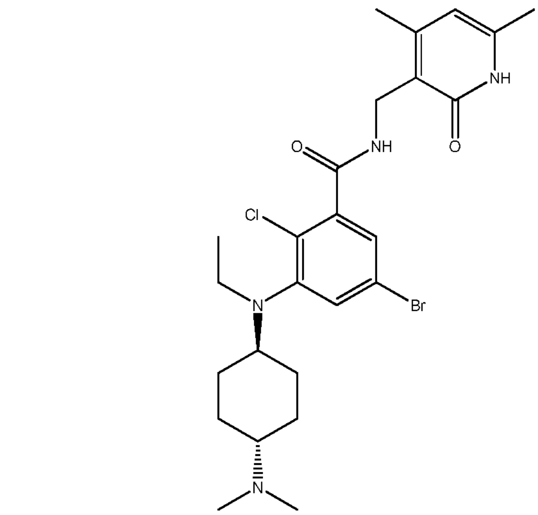 |
| 44 | 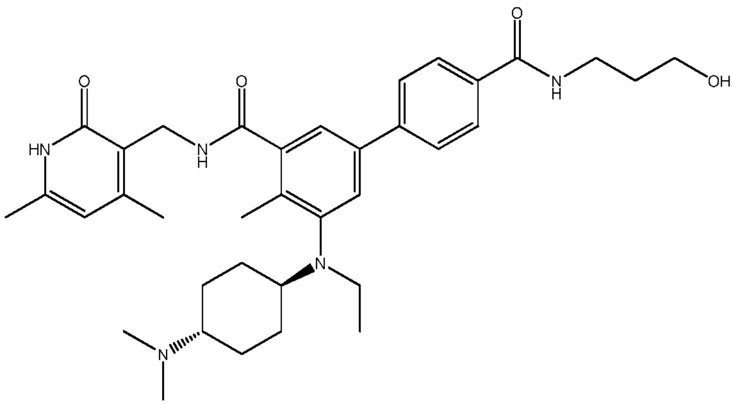 |
| 45 | 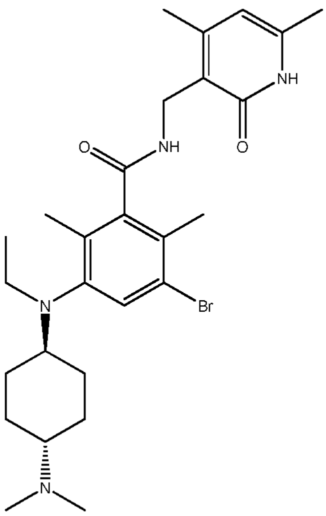 |

(fortgesetzt)

| | |
|---|---|
| 46 | 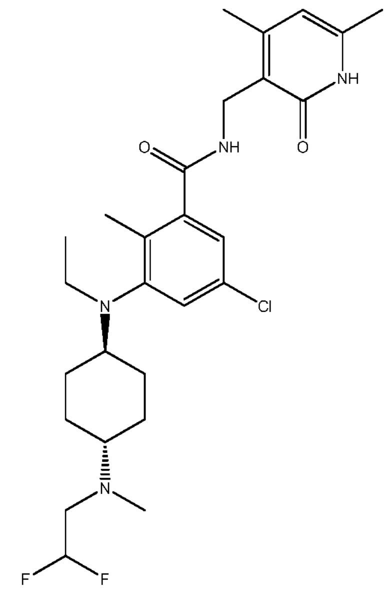 |
| 47 | 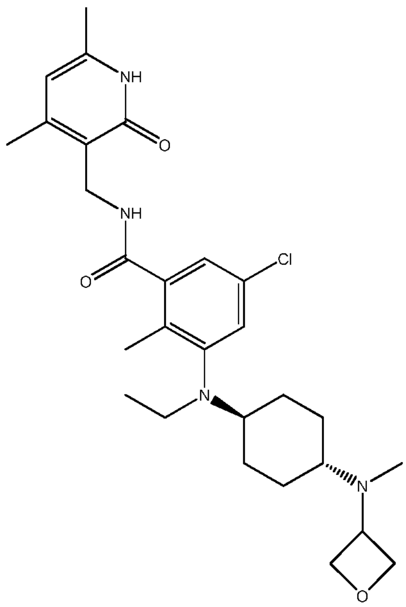 |
| 48 | 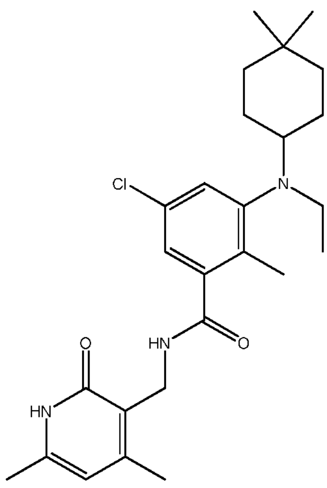 |

(fortgesetzt)

| | |
|---|---|
| 49 | 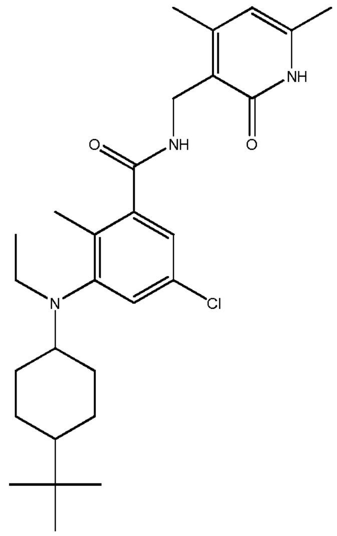 |
| 50 | 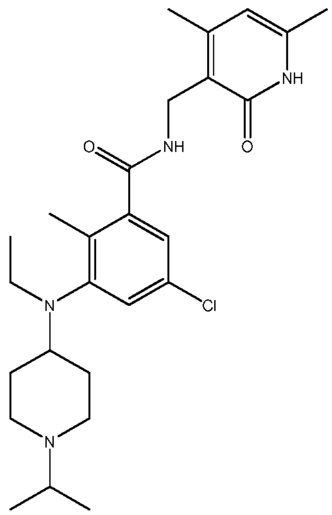 |
| 51 | 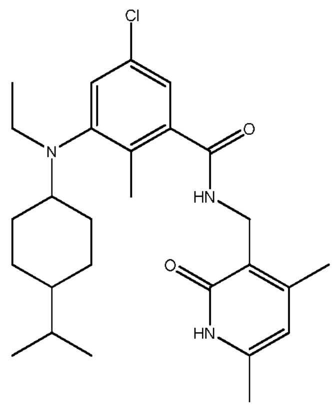 |

(fortgesetzt)

| | |
|---|---|
| 52 | 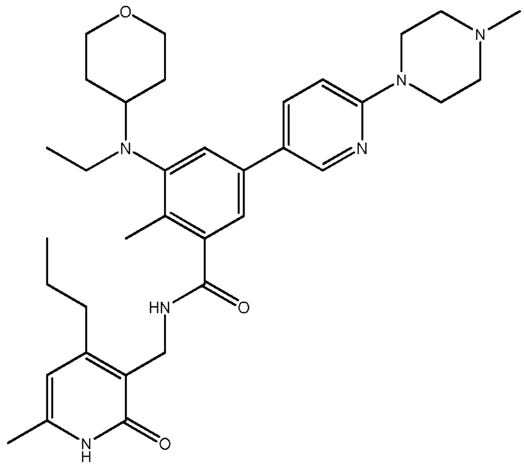 |
| 53 | 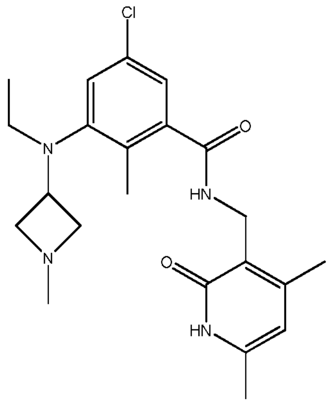 |
| 54 | 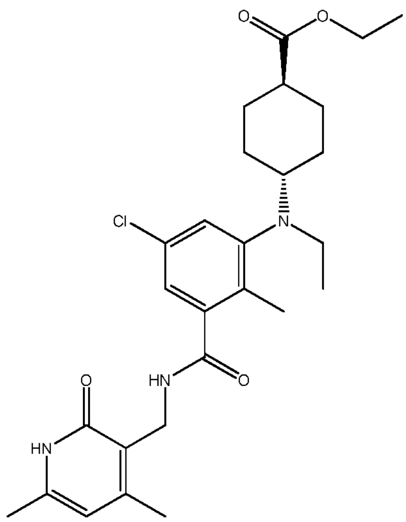 |

(fortgesetzt)

| | |
|---|---|
| 55 | 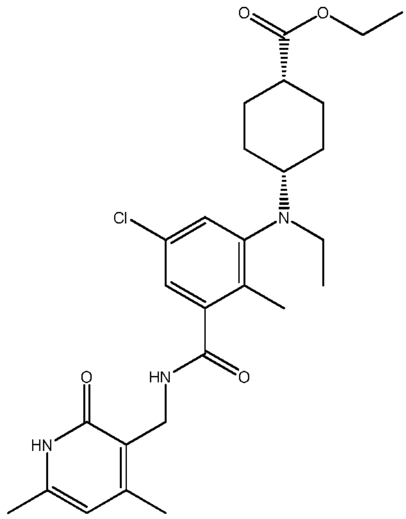 |
| 56 | 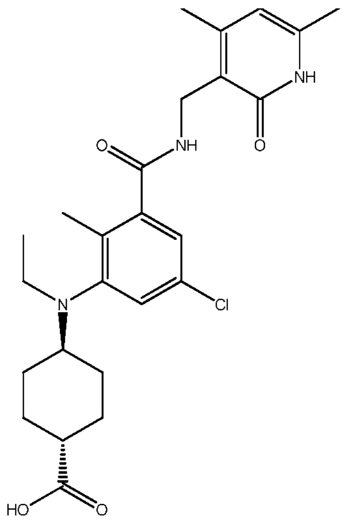 |
| 57 | 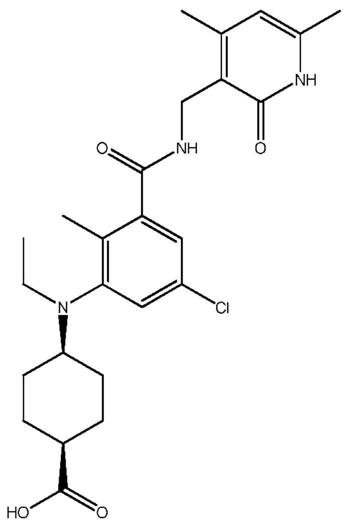 |

(fortgesetzt)

| | |
|---|---|
| 58 | 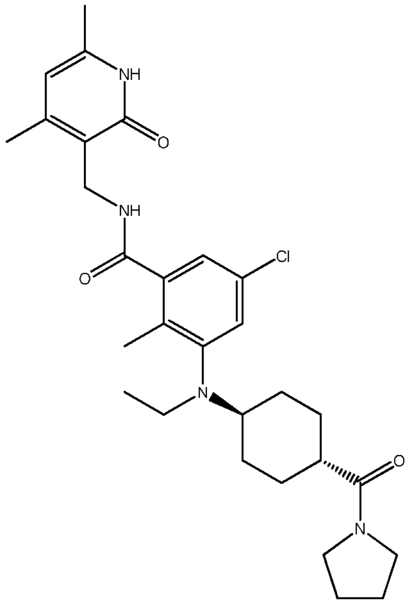 |
| 59 | 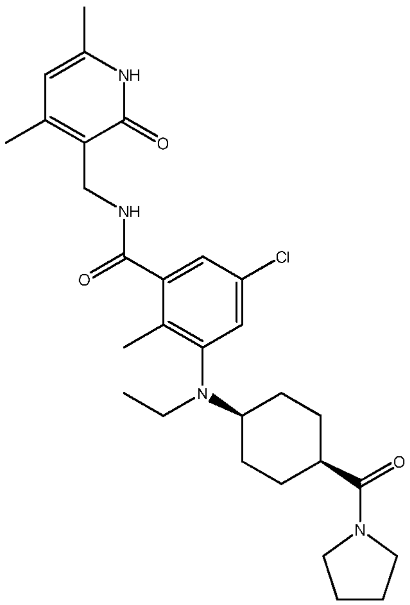 |

(fortgesetzt)

| | |
|---|---|
| 60 | 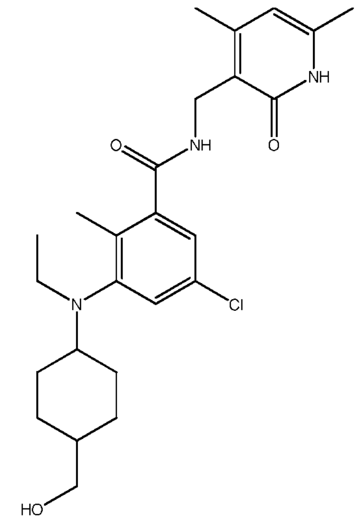 |
| 61 | 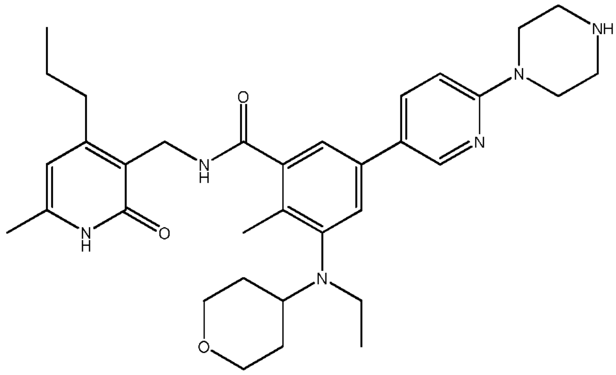 |
| 62 | 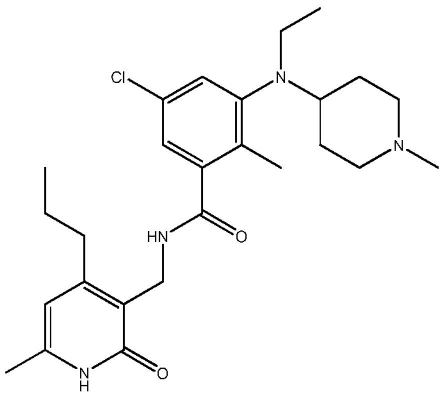 |

oder einem pharmazeutisch unbedenklichen Salz davon, zur Verwendung in einem Verfahren zum Behandeln von Krebs oder myelodysplastischen Syndromen (MDS, ehemals bekannt als Präleukämie) in einem Subjekt, das ein mutiertes EZH2 exprimiert, wobei das mutierte EZH2 eine Substitutionsmutation an der Aminosäureposition 677, 687 oder 641 der SEQ ID No: 1 umfasst.

**2.** EZH2-Hemmstoff zur Verwendung nach Anspruch 1, wobei die EZH2-Mutante mehr als eine Substitutionsmutation an der Aminosäureposition 677, 687 oder 641 der SEQ ID No: 1 umfasst.

**3.** EZH2-Hemmstoff zur Verwendung nach Anspruch 1, wobei der EZH2-Hemmstoff Folgender ist:

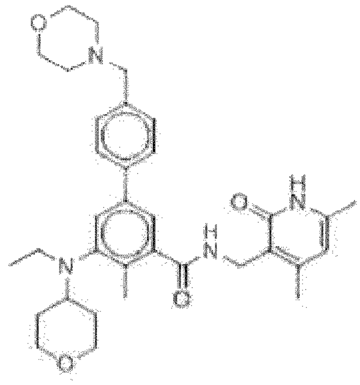

4. EZH2-Hemmstoff zur Verwendung nach Anspruch 1, wobei der Krebs aus Folgendem ausgewählt ist:

a) Lymphom, optional wobei das Lymphom ausgewählt ist aus der Gruppe bestehend aus Non-Hodgkin-Lymphom, follikulärem Lymphom und diffusem großzelligem B-Zell-Lymphom oder
b) Leukämie, optional wobei die Leukämie chronische myeloische Leukämie (CML) ist,
c) Melanom.

5. EZH2-Hemmstoff zur Verwendung nach Anspruch 1, wobei die Mutation ausgewählt ist aus der Gruppe bestehend aus einer Substitution von Glycin (G) für den Rest von Wildtyp-Alanin (A) an der Aminosäureposition 677 der SEQ ID No: 1 (A677G); einer Substitution von Valin (V) für den Rest von Wildtyp-Alanin (A) an der Aminosäureposition 687 der SEQ ID No: 1 (A687V); einer Substitution von Phenylalanin (F) für den Rest von Wildtyp-Tyrosin (Y) an der Aminosäureposition 641 der SEQ ID No: 1 (Y641F); einer Substitution von Histidin (H) für den Rest von Wildtyp-Tyrosin (Y) an der Aminosäureposition 641 der SEQ ID No: 1 (Y641H); einer Substitution von Asparagin (N) für den Rest von Wildtyp-Tyrosin (Y) an der Aminosäureposition 641 der SEQ ID No: 1 (Y641N); einer Substitution von Serin (S) für den Rest von Wildtyp-Tyrosin (Y) an der Aminosäureposition 641 der SEQ ID No: 1 (Y641S); und aus einer Substitution von Cystein (C) für den Rest von Wildtyp-Tyrosin (Y) an der Aminosäureposition 641 der SEQ ID No: 1 (Y641C).

6. Verfahren zum Bestimmen einer Empfindlichkeit eines Subjekts mit Krebs oder myelodysplastischen Syndromen (MDS, ehemals bekannt als Präleukämie) auf einen EZH2-Hemmstoff, ausgewählt aus Verbindung 2 bis Verbindung 62,

| | |
|---|---|
| 2 | 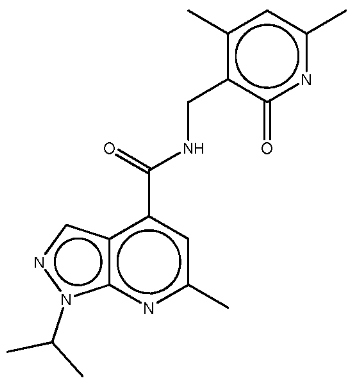 |

(fortgesetzt)

| | |
|---|---|
| 3 | 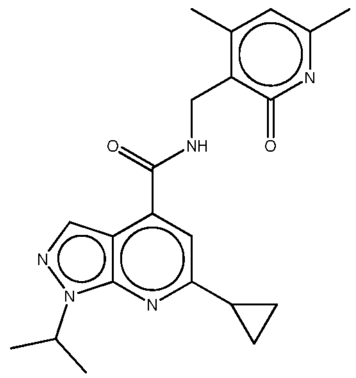 |
| 4 | 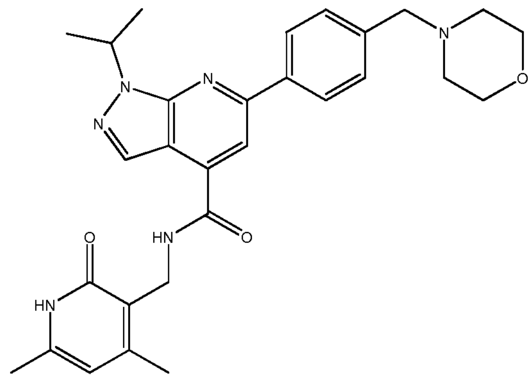 |
| 5 | 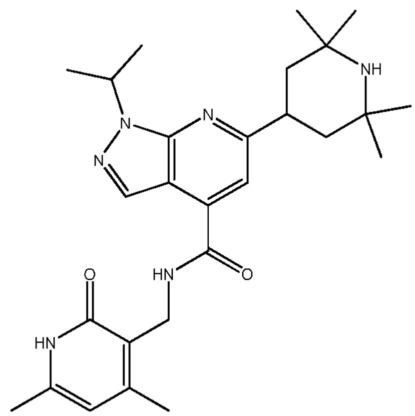 |
| 6 | 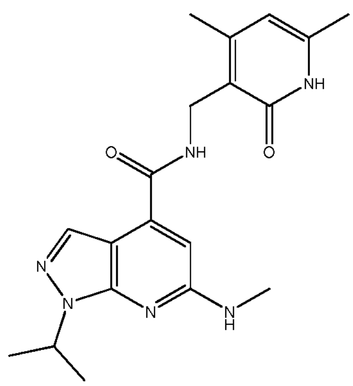 |

(fortgesetzt)

| | |
|---|---|
| 7 | 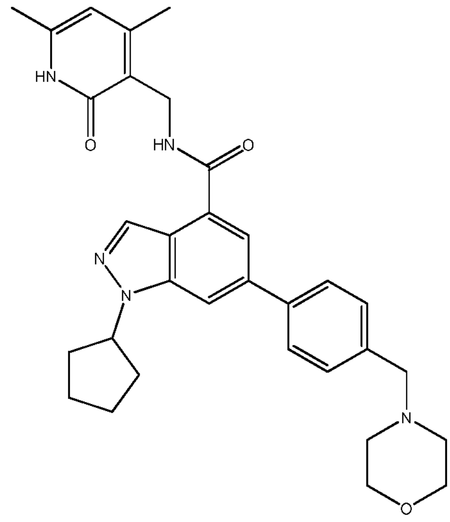 |
| 8 | 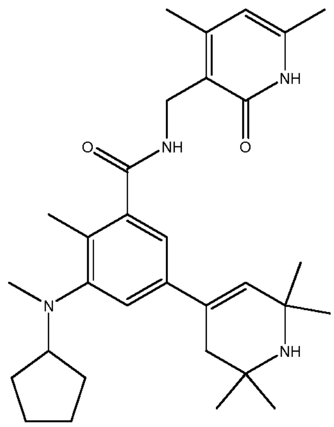 |
| 9 | 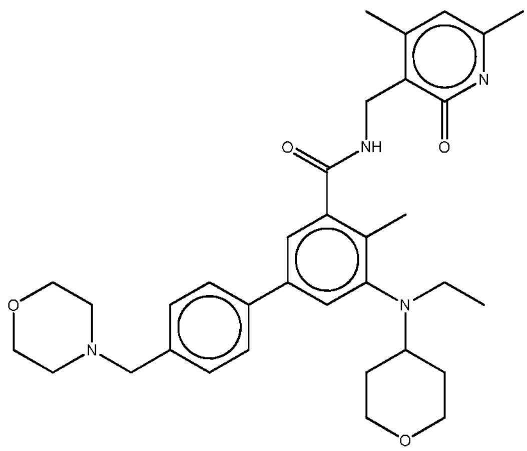 |

(fortgesetzt)

| | |
|---|---|
| 10 | 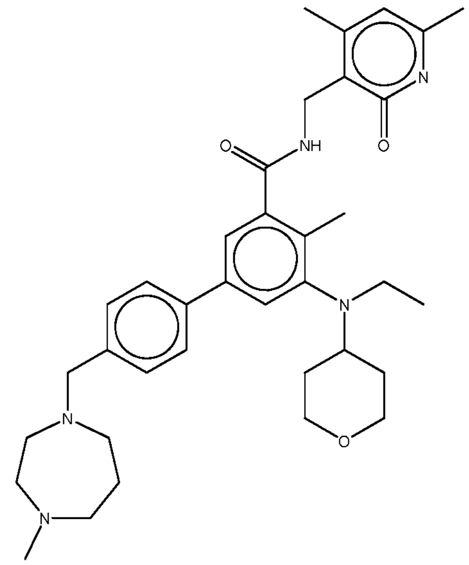 |
| 11 | 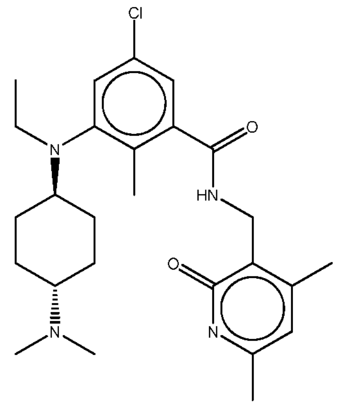 |
| 12 | 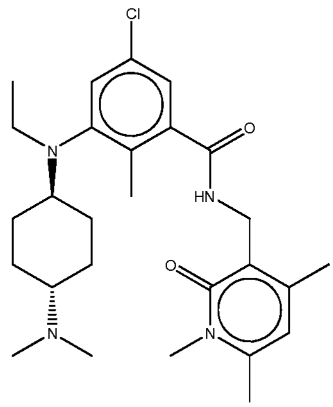 |

(fortgesetzt)

| | |
|---|---|
| 13 | 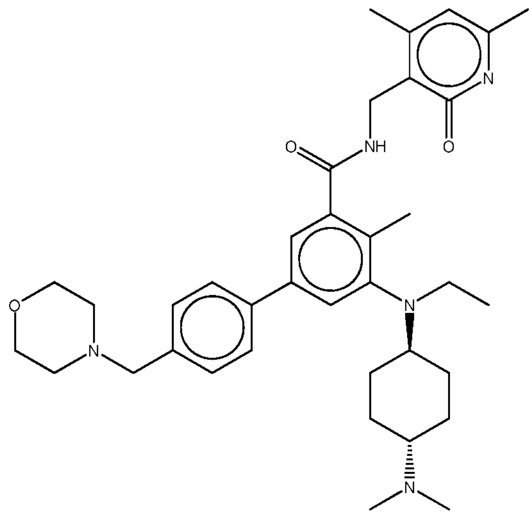 |
| 14 | 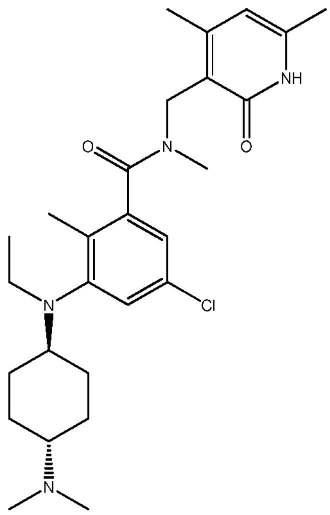 |
| 15 | 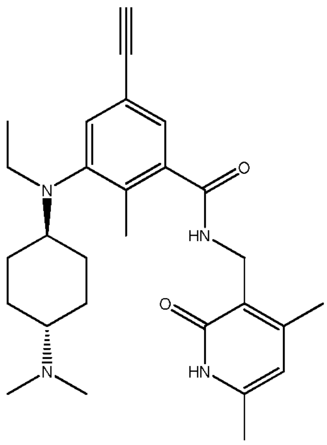 |

(fortgesetzt)

| | |
|---|---|
| 16 | 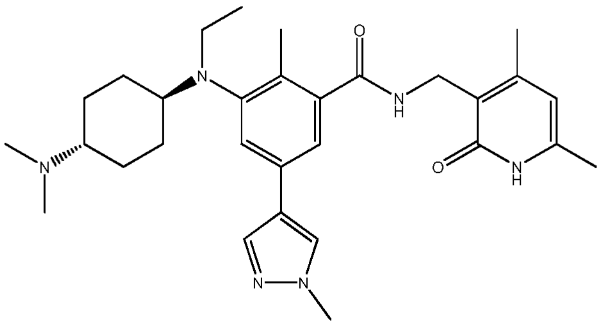 |
| 17 | 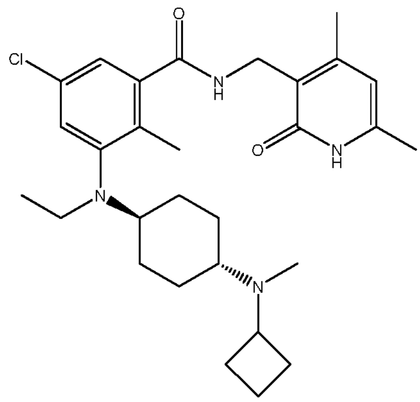 |
| 18 | 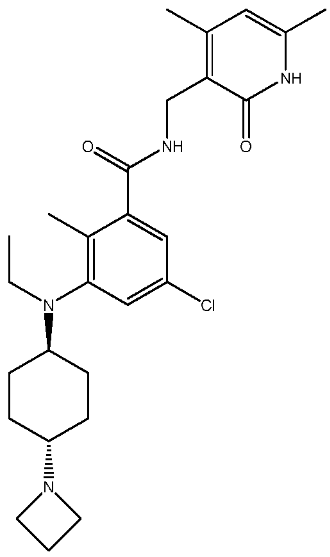 |

(fortgesetzt)

| | |
|---|---|
| 19 | 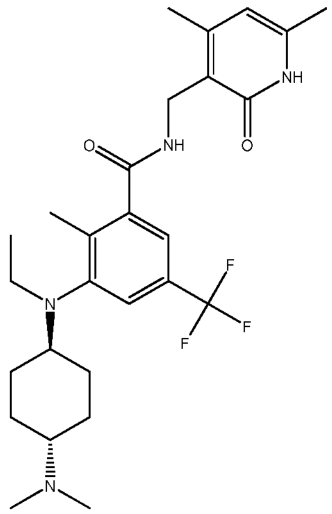 |
| 20 | 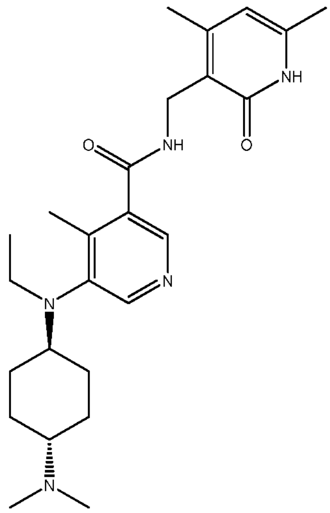 |
| 21 | 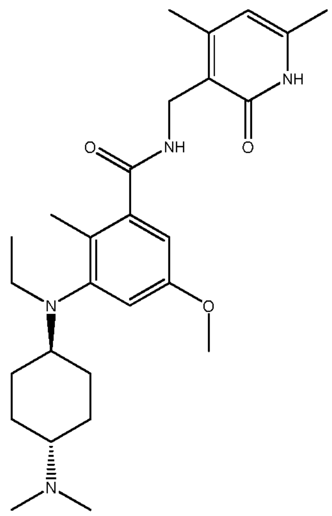 |

(fortgesetzt)

| | |
|---|---|
| 22 | 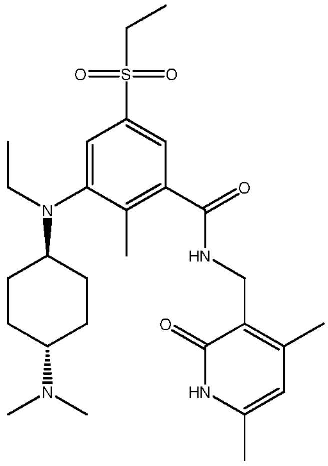 |
| 23 | 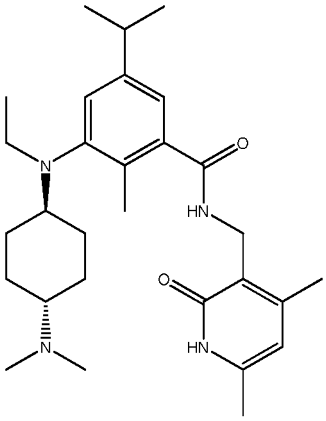 |
| 24 | 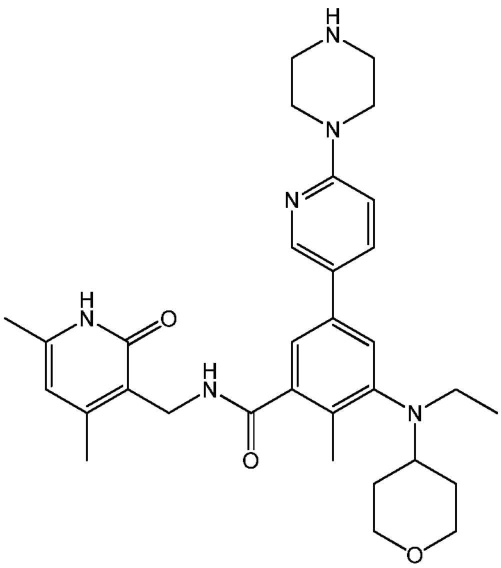 |

(fortgesetzt)

| | |
|---|---|
| 25 | 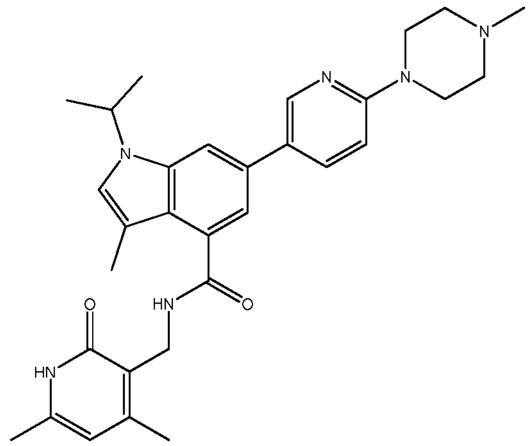 |
| 26 | 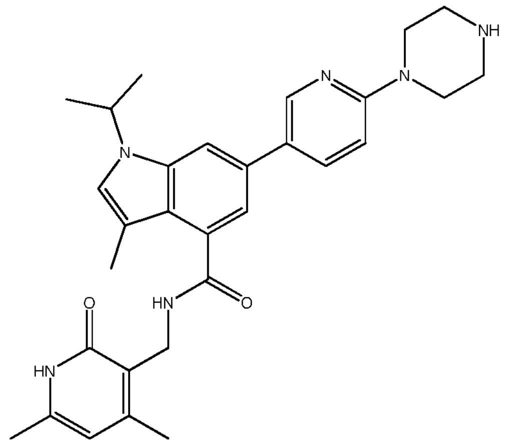 |
| 27 | 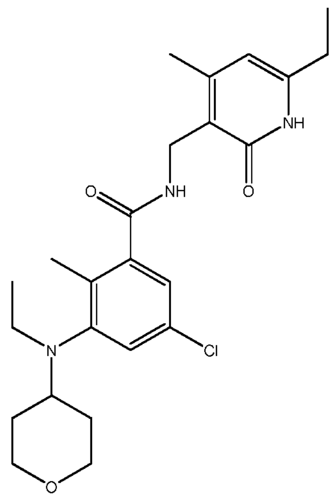 |

(fortgesetzt)

| | |
|---|---|
| 28 | 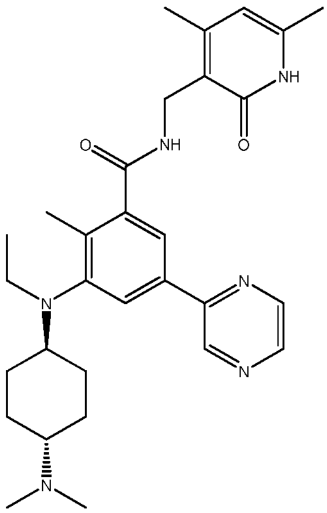 |
| 29 | 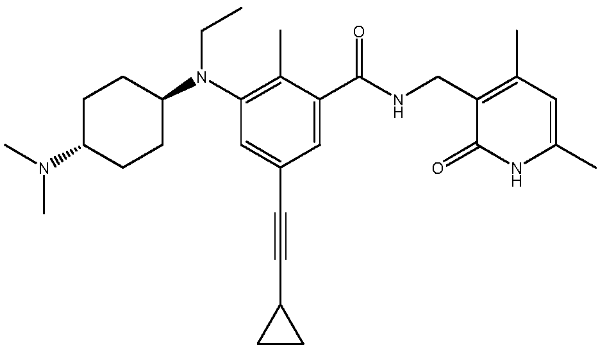 |
| 30 | 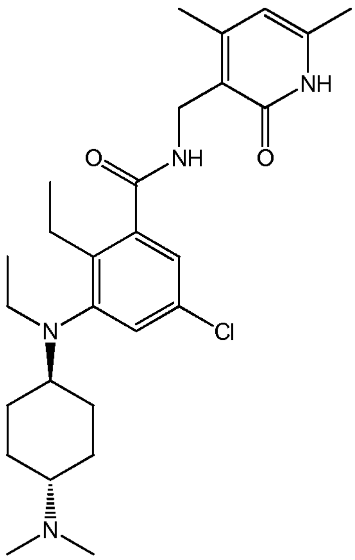 |

(fortgesetzt)

| | |
|---|---|
| 31 | 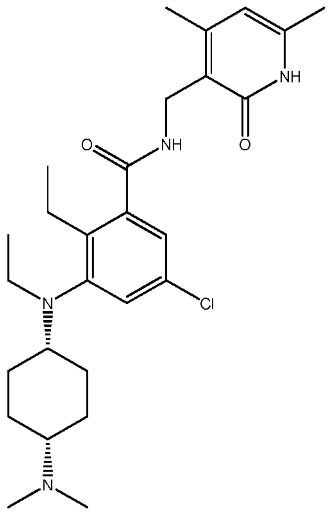 |
| 32 | 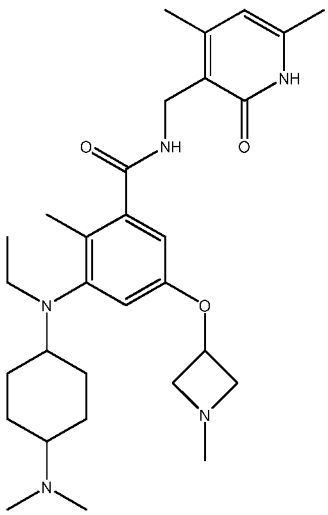 |
| 33 | 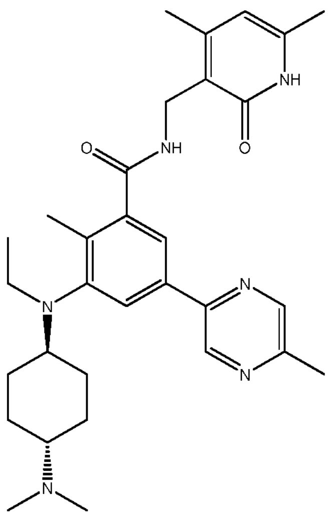 |

(fortgesetzt)

| | |
|---|---|
| 34 | 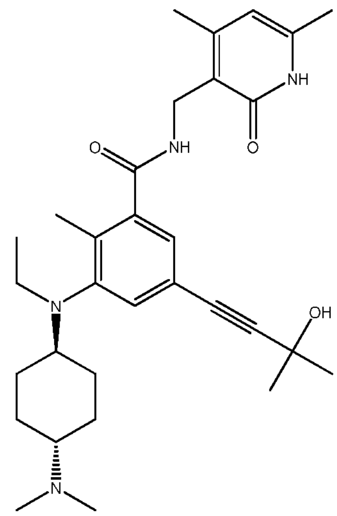 |
| 35 | 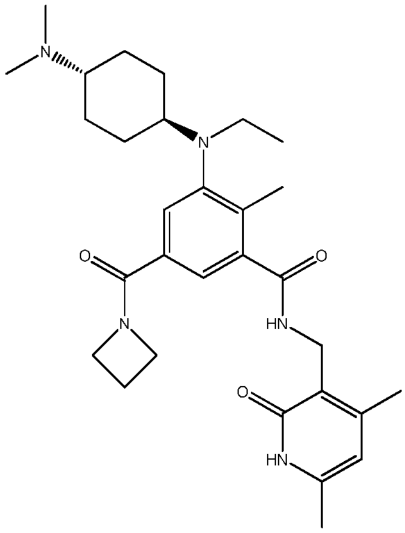 |
| 36 | 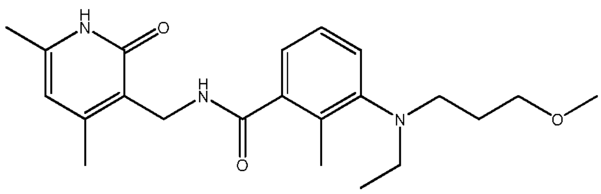 |

(fortgesetzt)

| | |
|---|---|
| 37 | 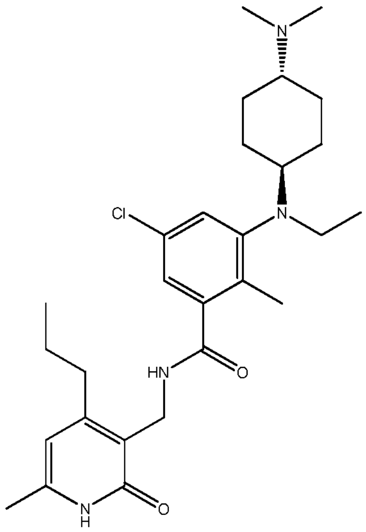 |
| 38 | 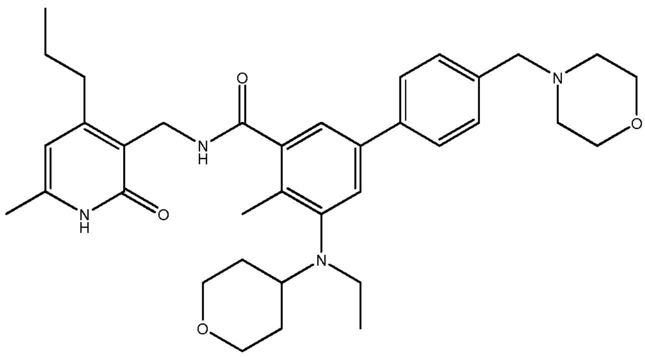 |
| 39 | 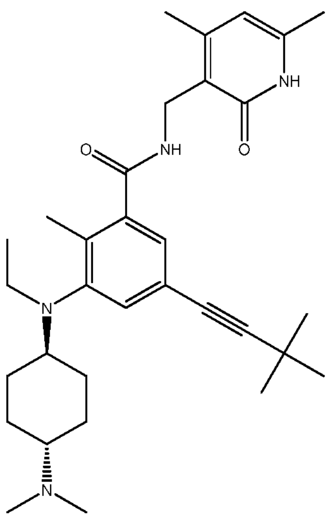 |

(fortgesetzt)

| | |
|---|---|
| 40 | 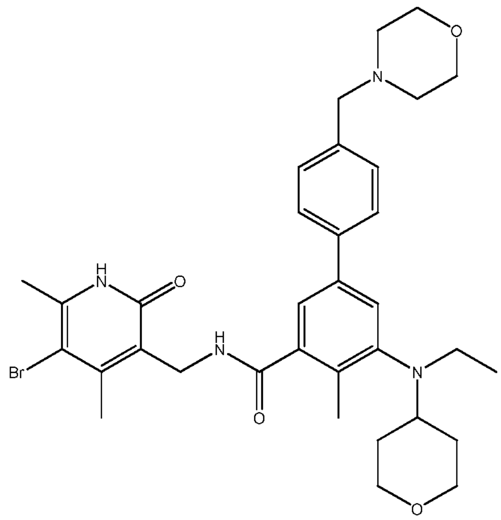 |
| 41 | 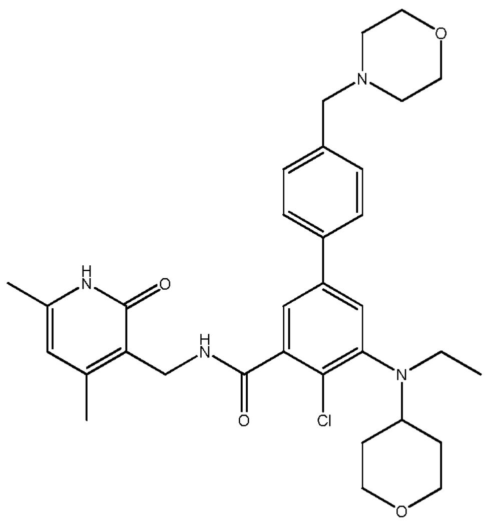 |
| 42 | 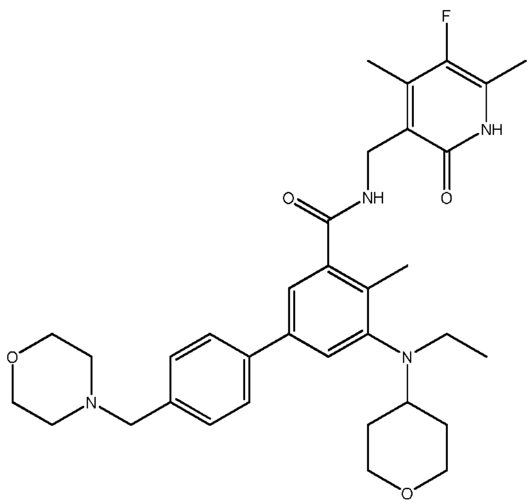 |

(fortgesetzt)

| | |
|---|---|
| 43 | 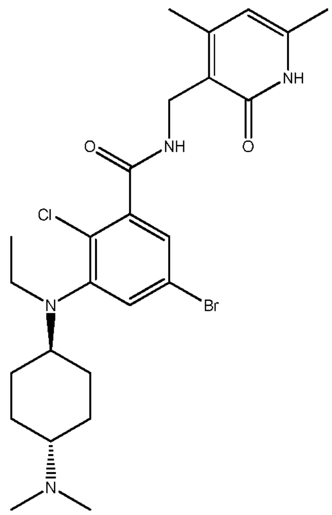 |
| 44 | 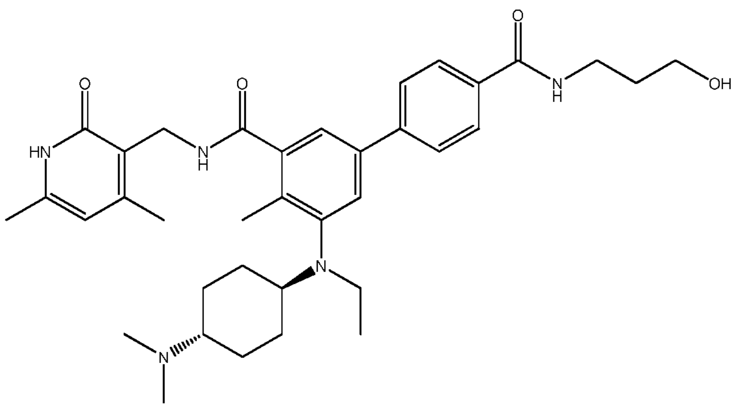 |
| 45 | 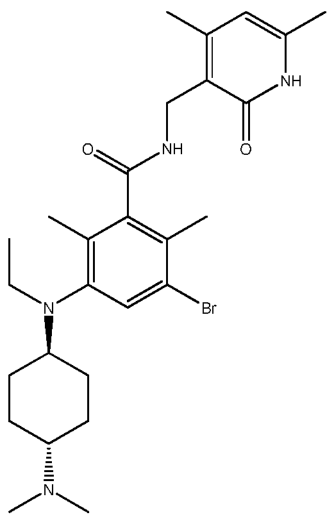 |

(fortgesetzt)

| | |
|---|---|
| 46 | 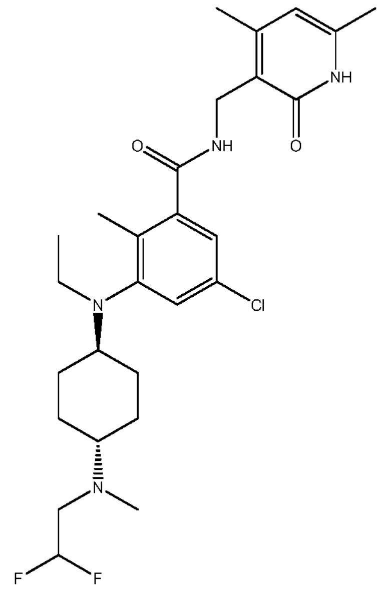 |
| 47 | 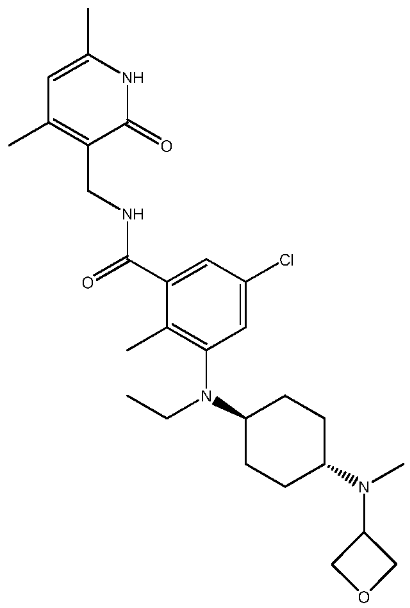 |
| 48 | 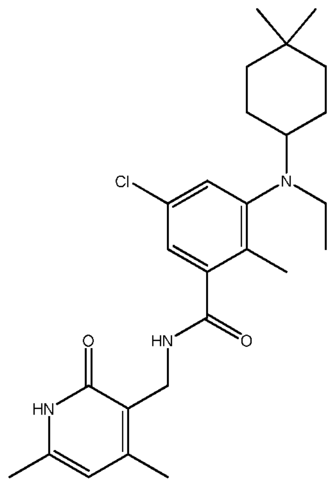 |

(fortgesetzt)

| | |
|---|---|
| 49 | 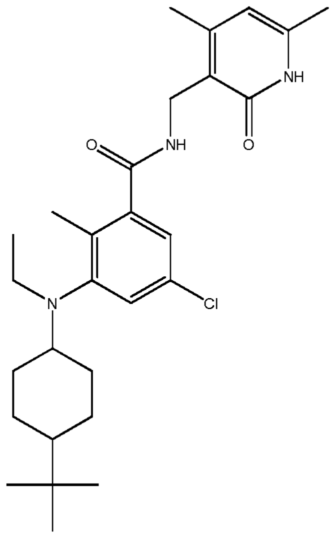 |
| 50 | 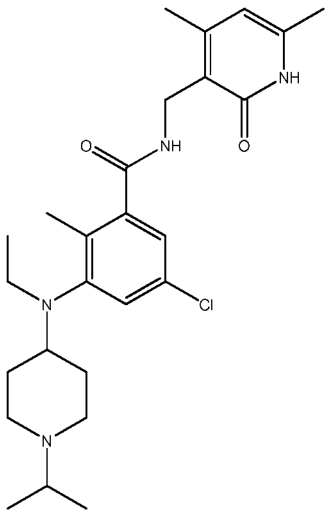 |
| 51 | 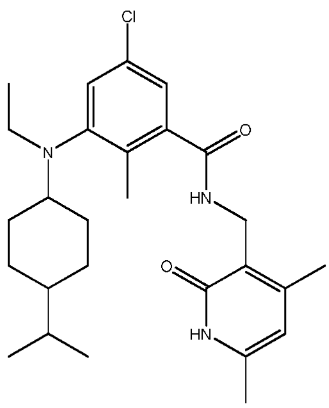 |

(fortgesetzt)

| | |
|---|---|
| 52 | 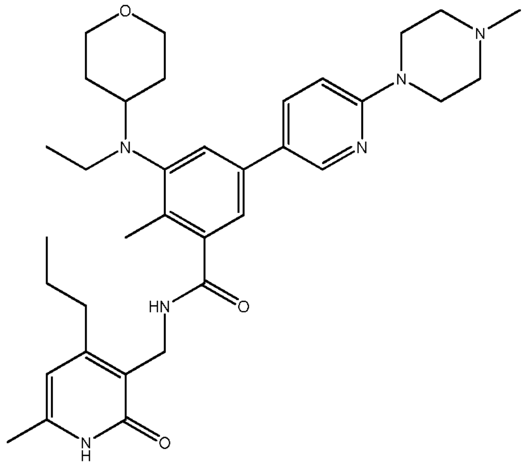 |
| 53 | 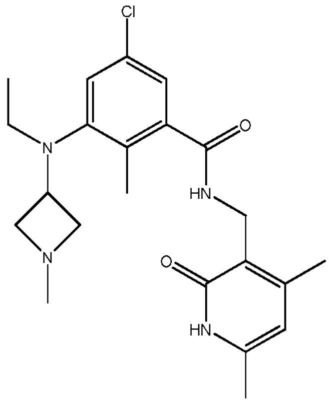 |
| 54 | 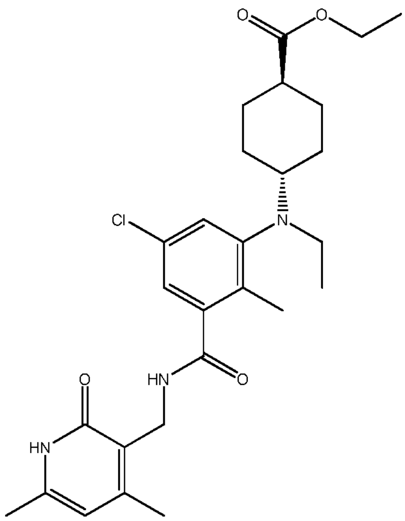 |

(fortgesetzt)

| | |
|---|---|
| 55 | 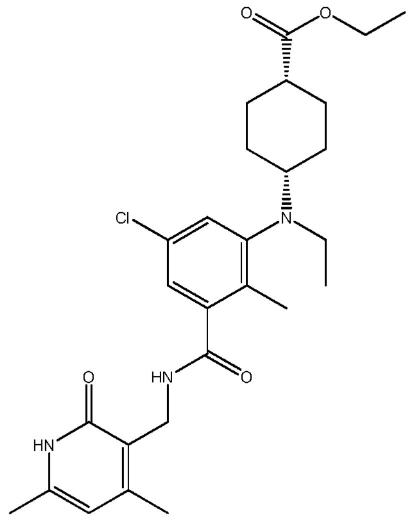 |
| 56 | 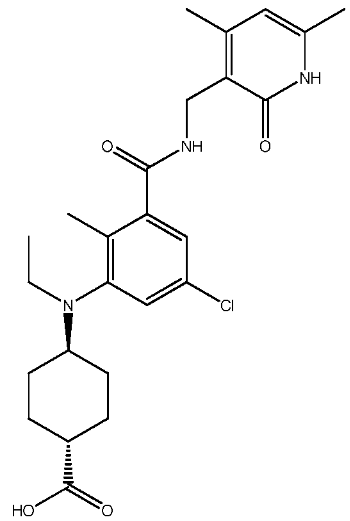 |
| 57 | 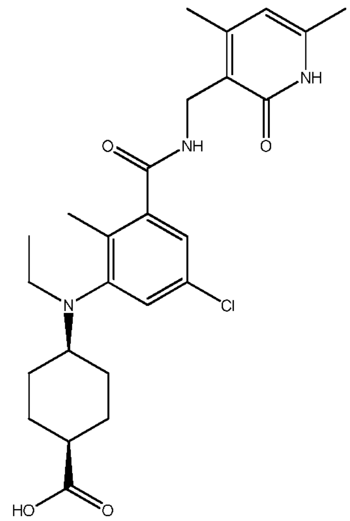 |

(fortgesetzt)

| | |
|---|---|
| 58 | 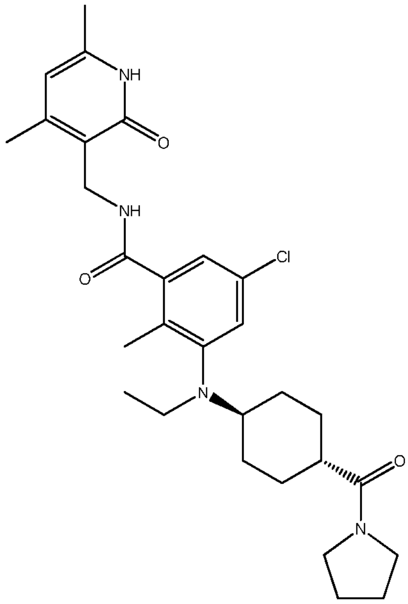 |
| 59 | 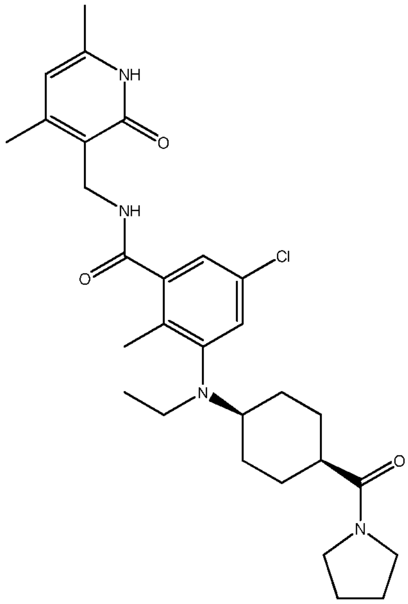 |

(fortgesetzt)

| | |
|---|---|
| 60 | 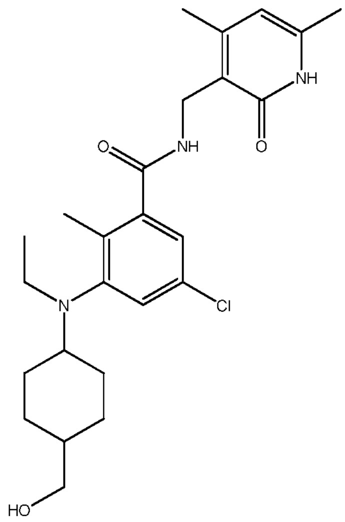 |
| 61 | 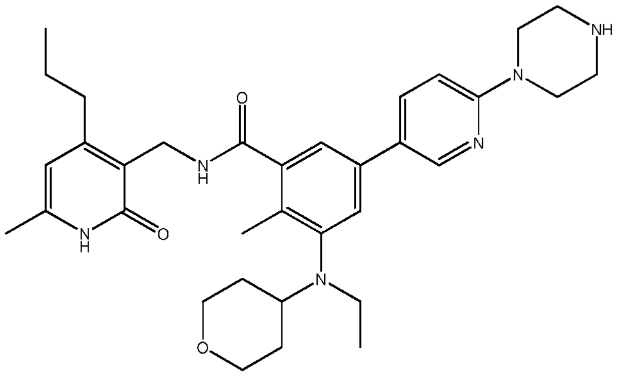 |
| 62 | 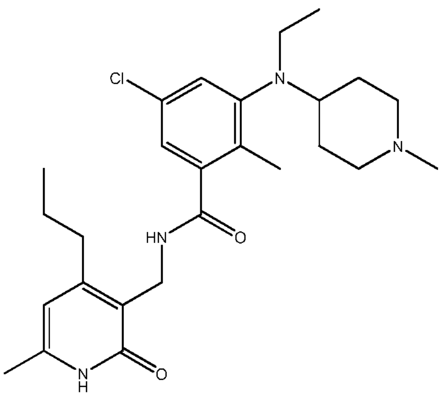 |

Folgendes umfassend: Erfassen des Vorhandenseins einer Mutation in EZH2 in einer Probe, die von dem Subjekt erhalten wurde, wobei die Mutation eine Substitutionsmutation an der Aminosäureposition 677, 687 oder 641 der SEQ ID No: 1 ist oder als diese exprimiert wird, wobei das Vorhandensein dieser Mutation anzeigt, dass das Subjekt auf den EZH2-Hemmstoff empfindlich ist.

**7.** Verfahren nach Anspruch 6, wobei das Verfahren das Erfassen des Vorhandenseins von mehr als einer Substitutionsmutation an der Aminosäureposition 677, 687 oder 641 der SEQ ID No: 1 enthält.

**8.** Verfahren nach Anspruch 6, wobei der Krebs aus Folgendem ausgewählt ist:

a) Lymphom, optional wobei das Lymphom ausgewählt ist aus der Gruppe bestehend aus Non-Hodgkin-Lymphom, follikulärem Lymphom und diffusem großzelligem B-Zell-Lymphom oder
b) Leukämie, optional wobei die Leukämie chronische myeloische Leukämie (CML) ist, oder
c) Melanom.

**9.** Verfahren nach Anspruch 6, wobei die Mutation ausgewählt ist aus der Gruppe bestehend aus einer Substitution von Glycin (G) für den Rest von Wildtyp-Alanin (A) an der Aminosäureposition 677 der SEQ ID No: 1 (A677G); einer Substitution von Valin (V) für den Rest von Wildtyp-Alanin (A) an der Aminosäureposition 687 der SEQ ID No: 1 (A687V); einer Substitution von Phenylalanin (F) für den Rest von Wildtyp-Tyrosin (Y) an der Aminosäureposition 641 der SEQ ID No: 1 (Y641F); einer Substitution von Histidin (H) für den Rest von Wildtyp-Tyrosin (Y) an der Aminosäureposition 641 der SEQ ID No: 1 (Y641H); einer Substitution von Asparagin (N) für den Rest von Wildtyp-Tyrosin (Y) an der Aminosäureposition 641 der SEQ ID No: 1 (Y641N); einer Substitution von Serin (S) für den Rest von Wildtyp-Tyrosin (Y) an der Aminosäureposition 641 der SEQ ID No: 1 (Y641S); und aus einer Substitution von Cystein (C) für den Rest von Wildtyp-Tyrosin (Y) an der Aminosäureposition 641 der SEQ ID No: 1 (Y641C).

## Revendications

**1.** Inhibiteur d'EZH2 choisi parmi le composé 2 au composé 62,

| | |
|---|---|
| 2 | 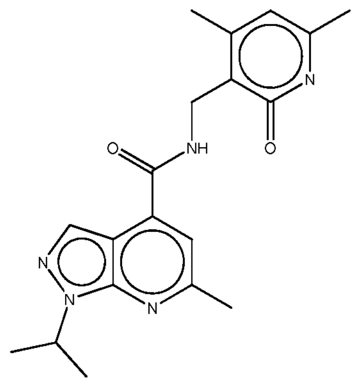 |
| 3 | 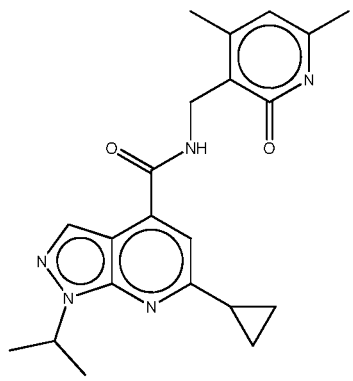 |
| 4 | 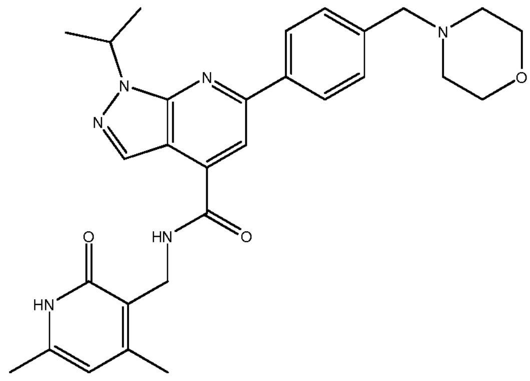 |

(suite)

| | |
|---|---|
| 5 | 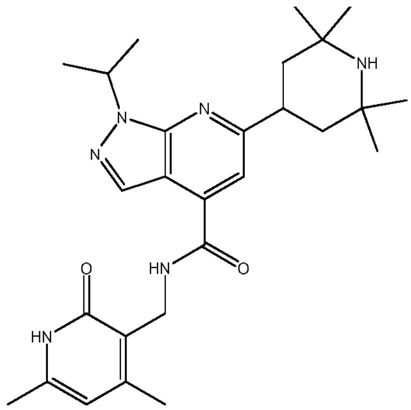 |
| 6 | 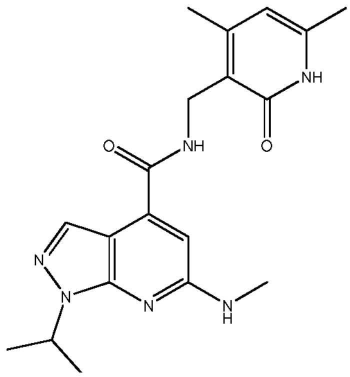 |
| 7 | 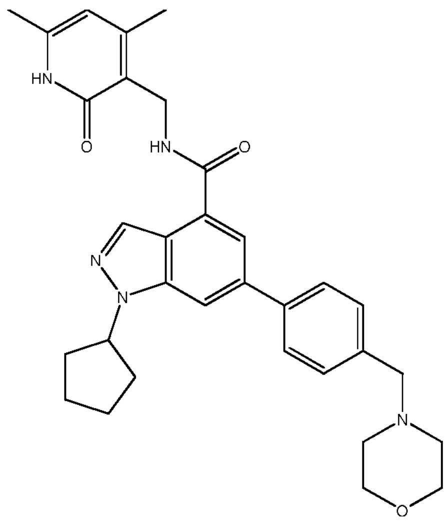 |

(suite)

| | |
|---|---|
| 8 | 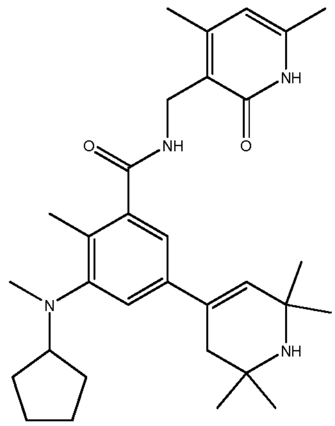 |
| 9 | 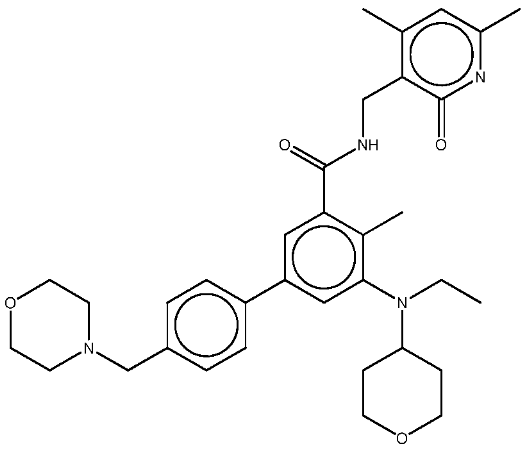 |
| 10 | 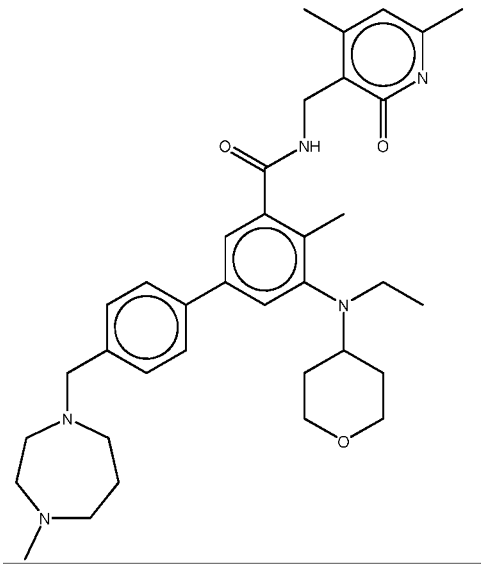 |

(suite)

| | |
|---|---|
| 11 | 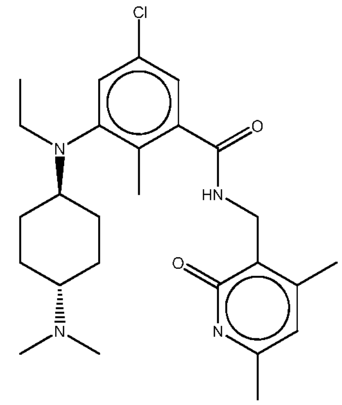 |
| 12 | 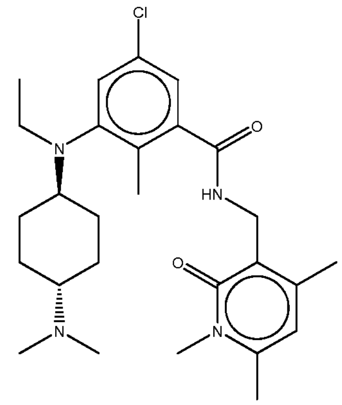 |
| 13 | 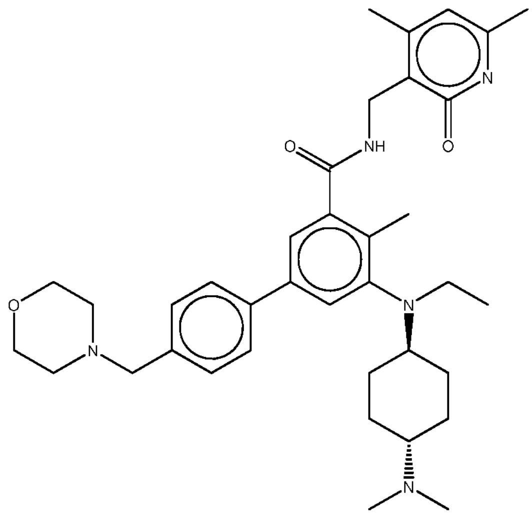 |

(suite)

| | |
|---|---|
| 14 | 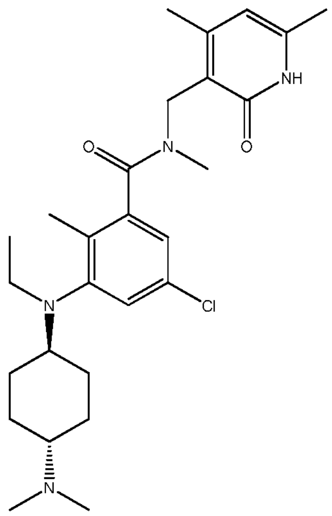 |
| 15 | 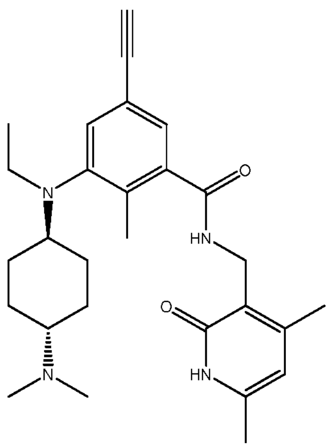 |
| 16 | 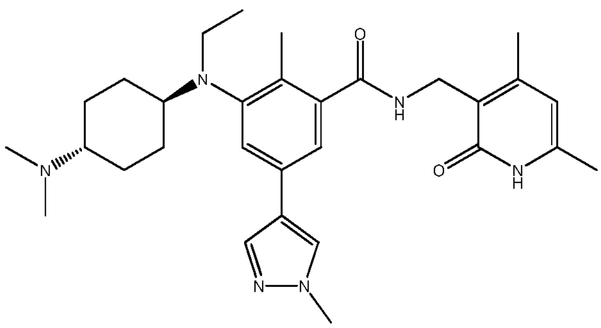 |

(suite)

| | |
|---|---|
| 17 | 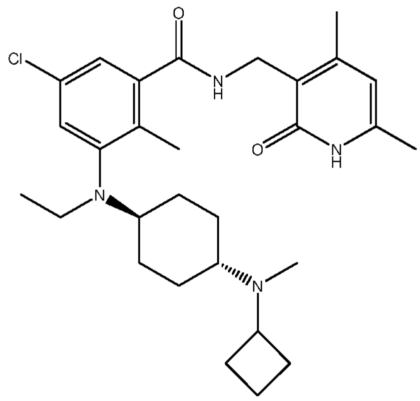 |
| 18 | 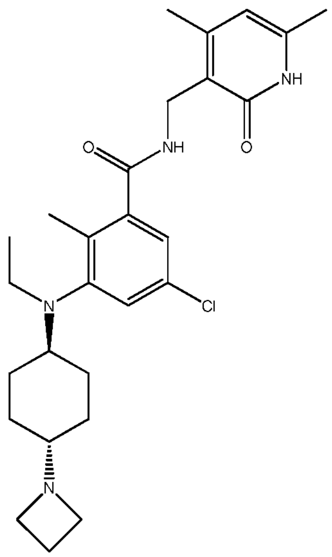 |
| 19 | 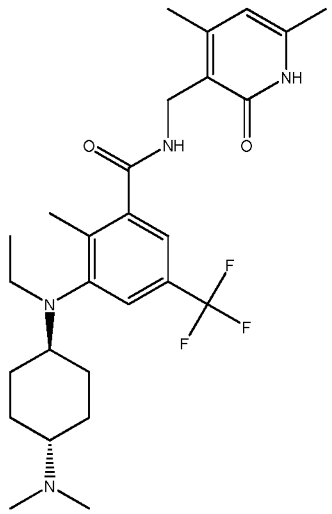 |

(suite)

| | |
|---|---|
| 20 | 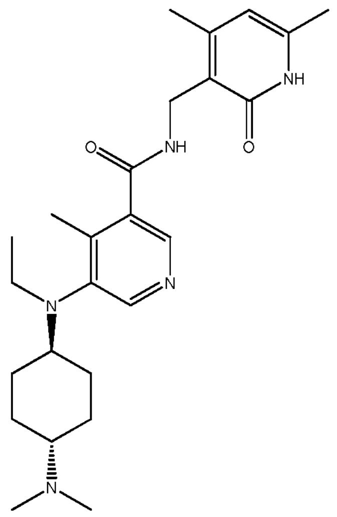 |
| 21 | 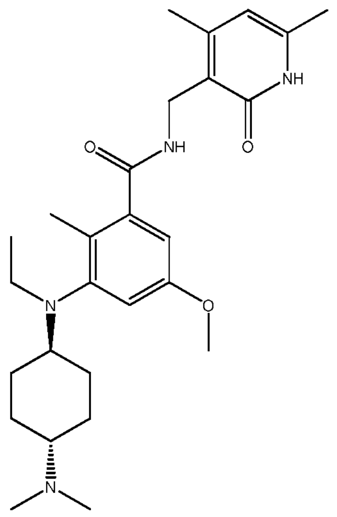 |
| 22 | 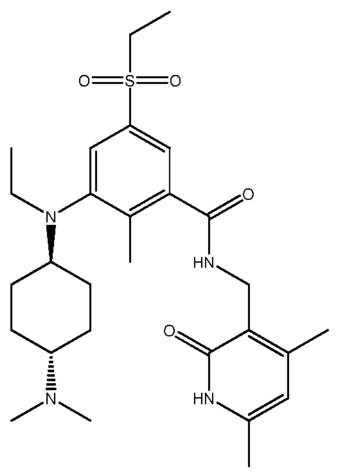 |

(suite)

| | |
|---|---|
| 23 | 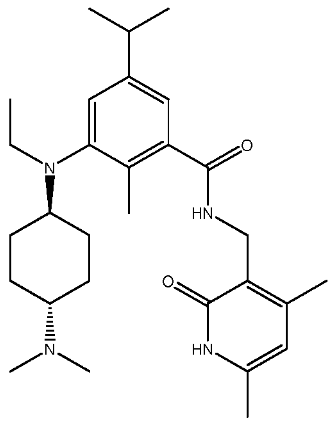 |
| 24 | 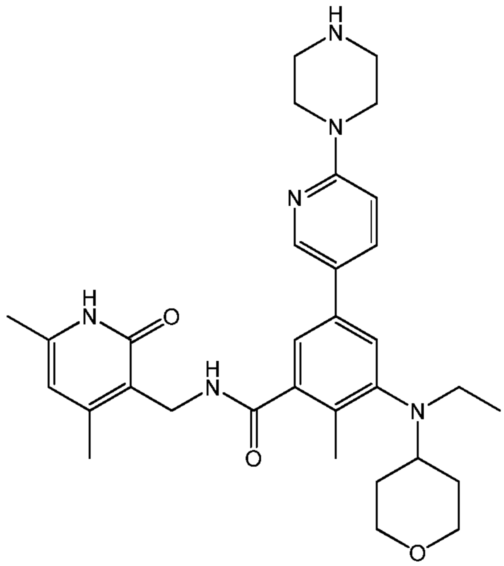 |
| 25 | 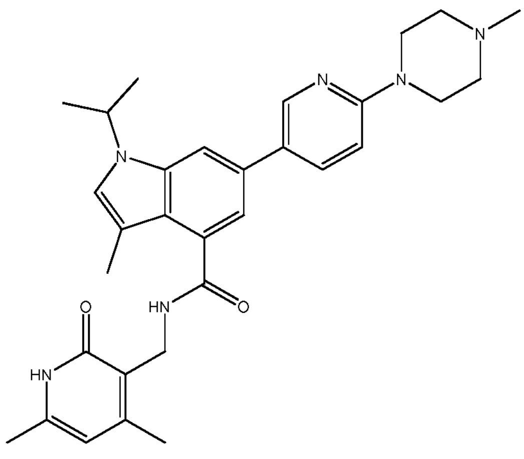 |

(suite)

| | |
|---|---|
| 26 | 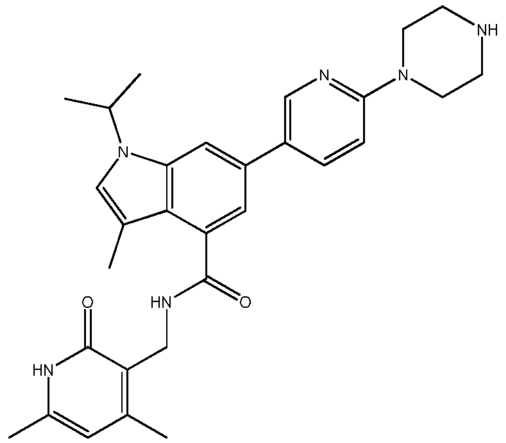 |
| 27 | 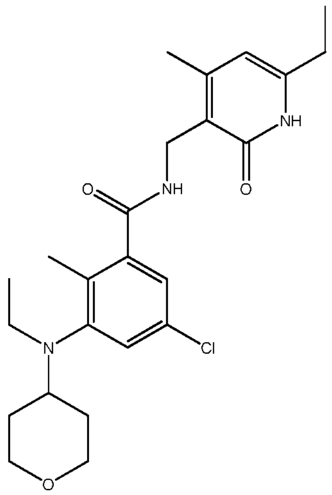 |
| 28 | 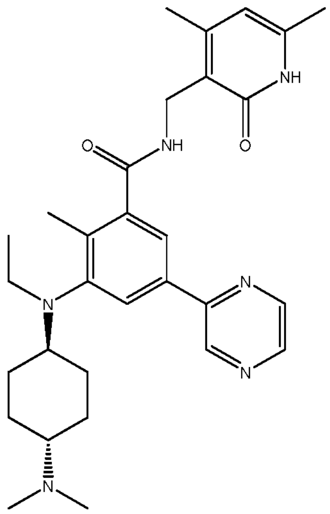 |

(suite)

| | |
|---|---|
| 29 | 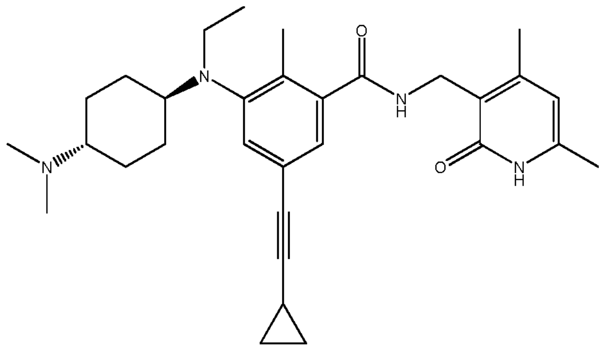 |
| 30 | 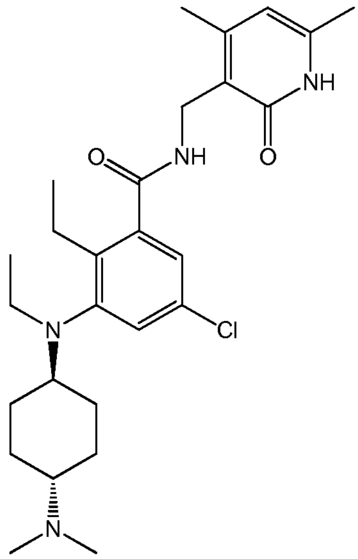 |
| 31 | 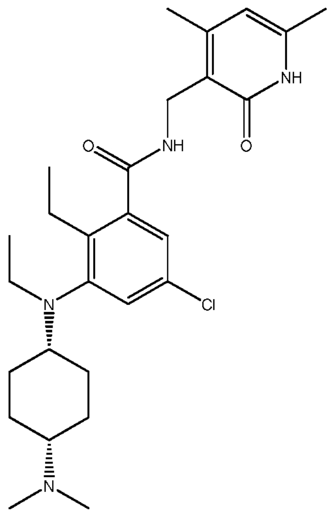 |

(suite)

| | |
|---|---|
| 32 | 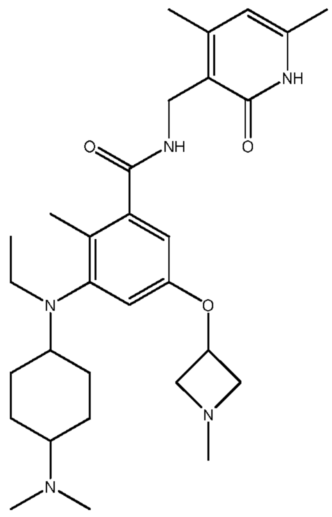 |
| 33 | 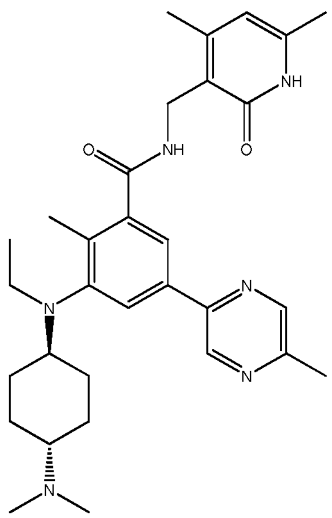 |
| 34 | 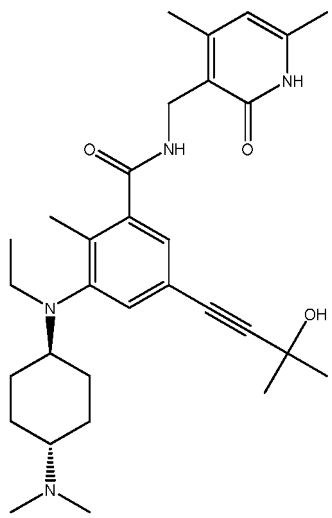 |

(suite)

| | |
|---|---|
| 35 | 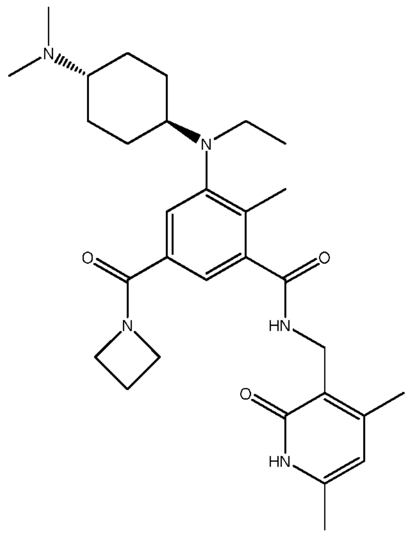 |
| 36 | 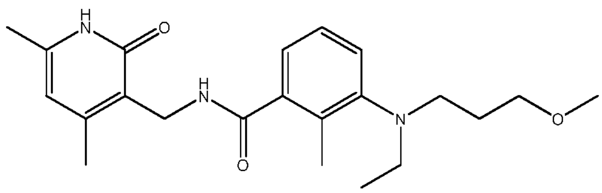 |
| 37 | 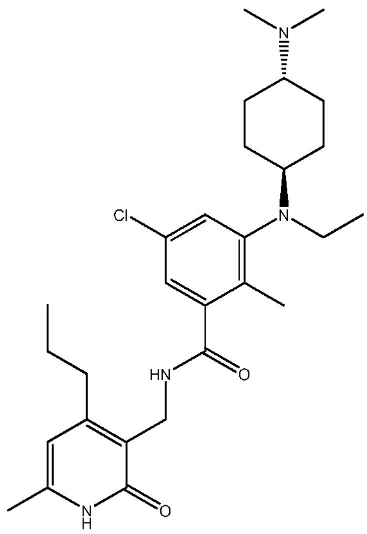 |
| 38 | 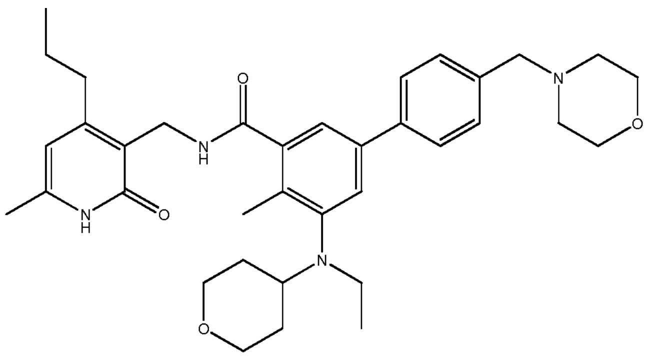 |

(suite)

| | |
|---|---|
| 39 | 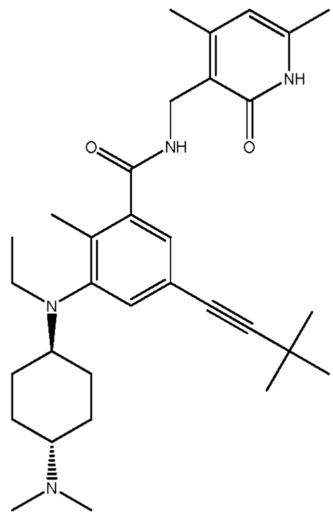 |
| 40 | 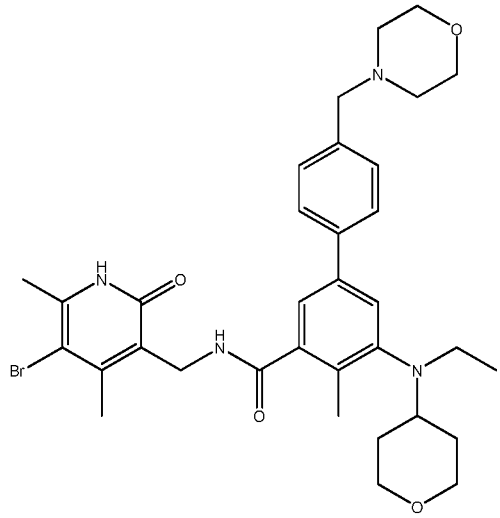 |
| 41 | 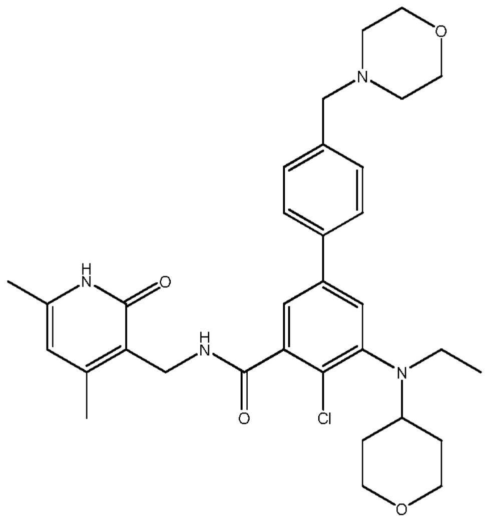 |

(suite)

| | |
|---|---|
| 42 | 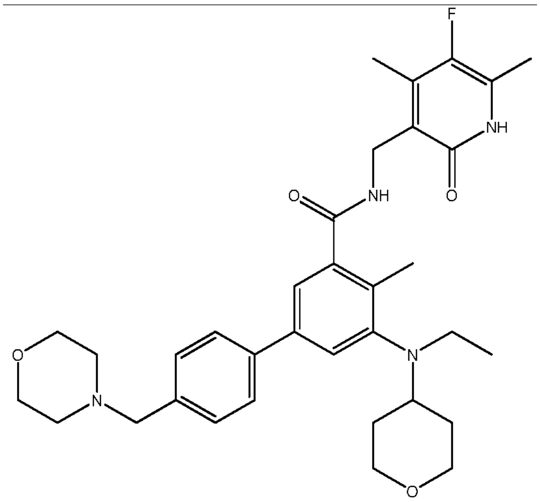 |
| 43 | 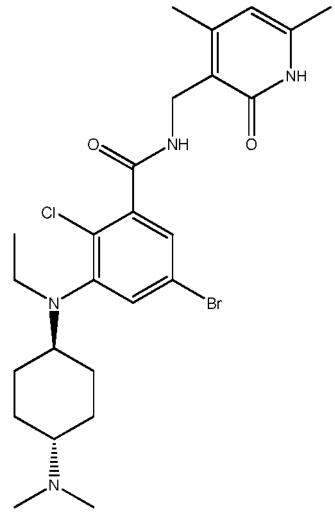 |
| 44 | 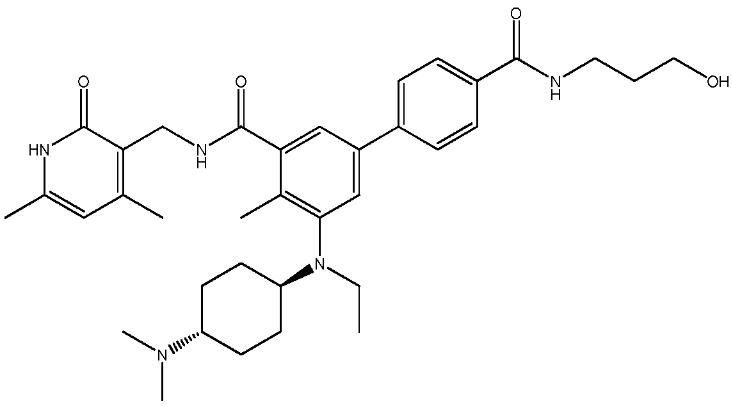 |

(suite)

| | |
|---|---|
| 45 | 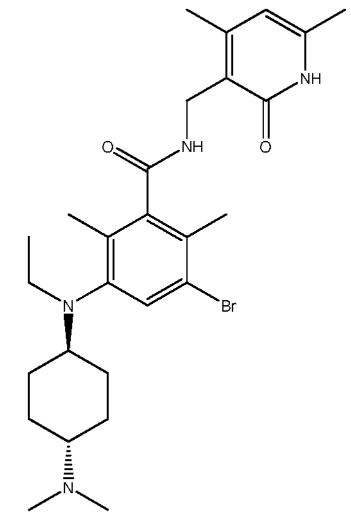 |
| 46 | 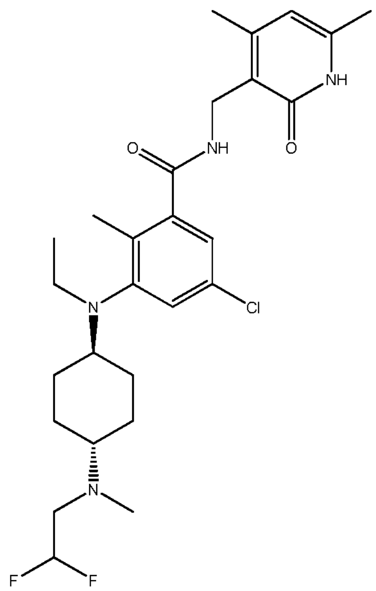 |

(suite)

| | |
|---|---|
| 47 | 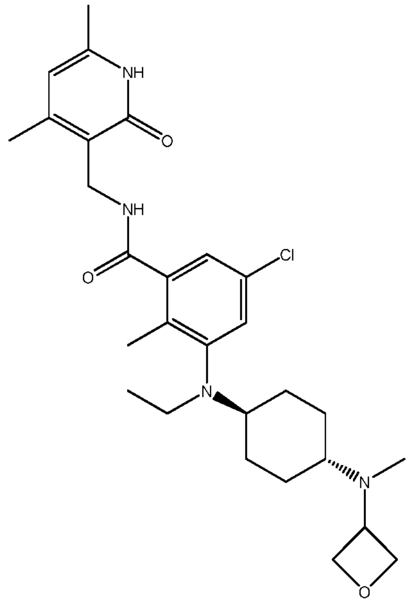 |
| 48 | 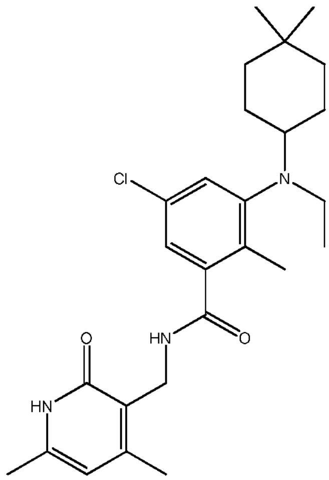 |
| 49 | 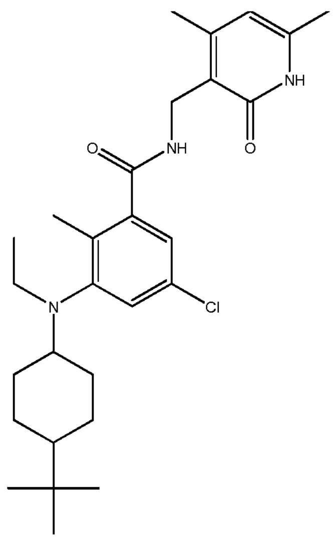 |

(suite)

| | |
|---|---|
| 50 | 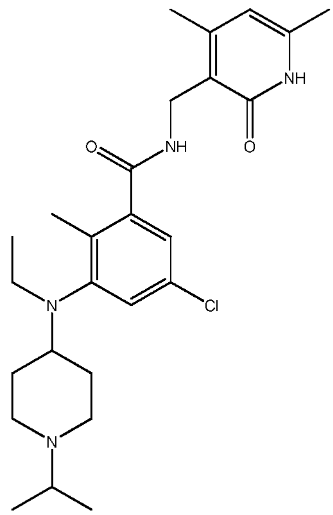 |
| 51 | 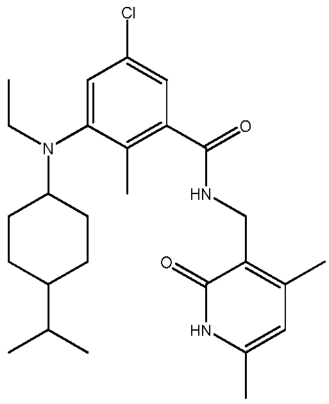 |
| 52 | 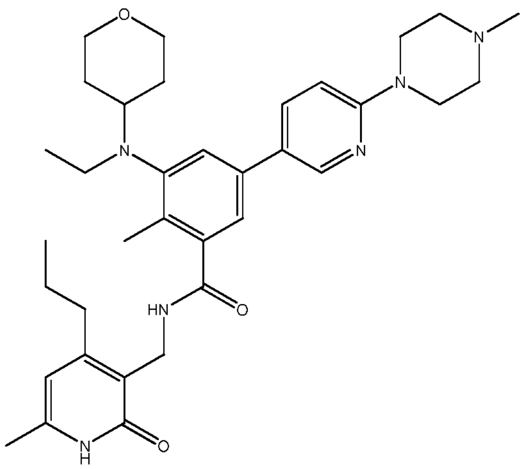 |

(suite)

| | |
|---|---|
| 53 | 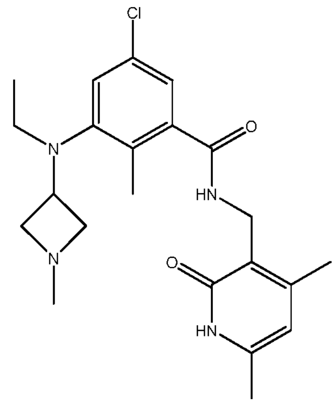 |
| 54 | 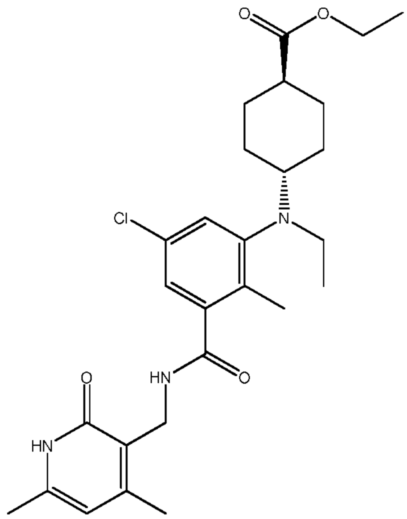 |
| 55 | 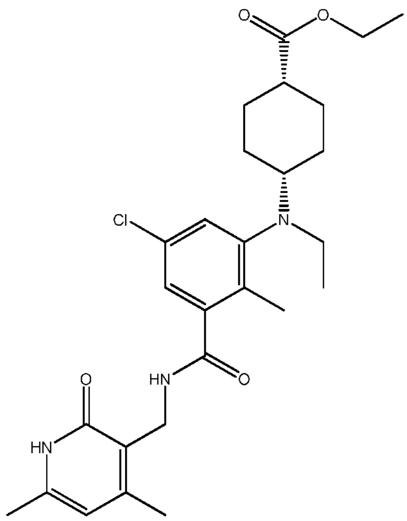 |

(suite)

| | |
|---|---|
| 56 | 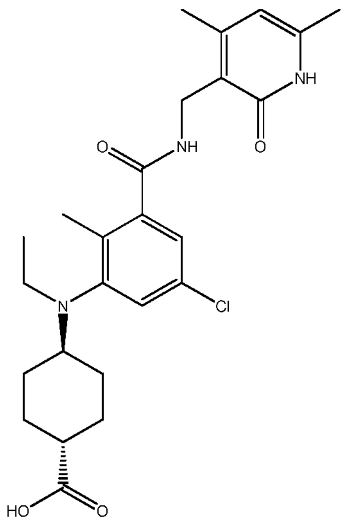 |
| 57 | 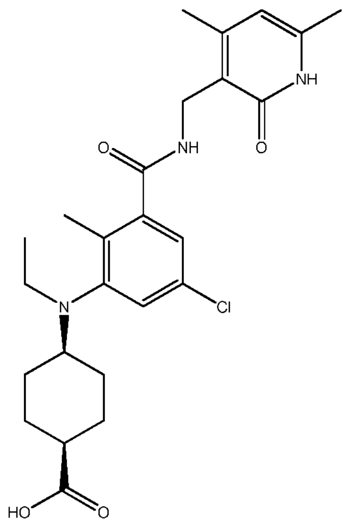 |
| 58 | 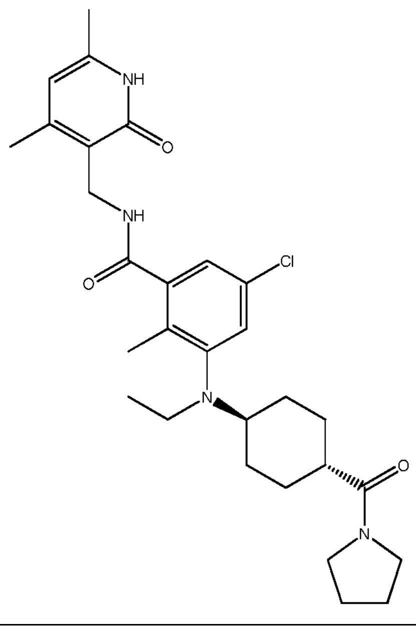 |

(suite)

| | |
|---|---|
| 59 | 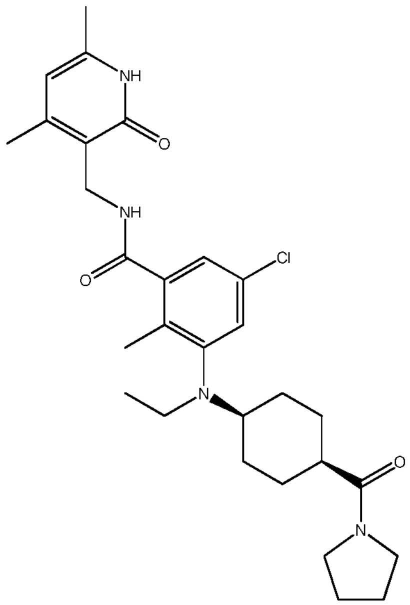 |
| 60 | 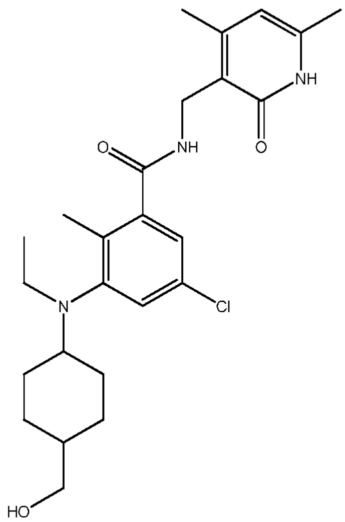 |
| 61 | 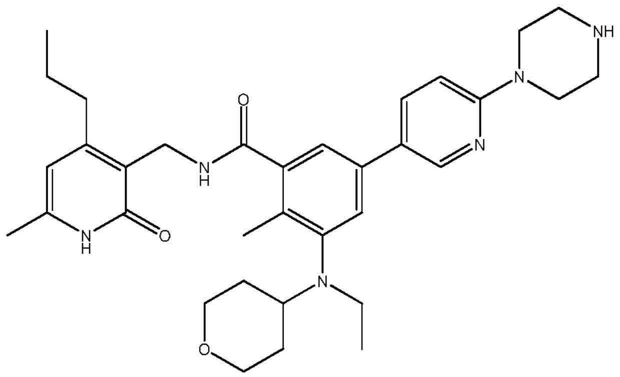 |

(suite)

| | |
|---|---|
| 62 | 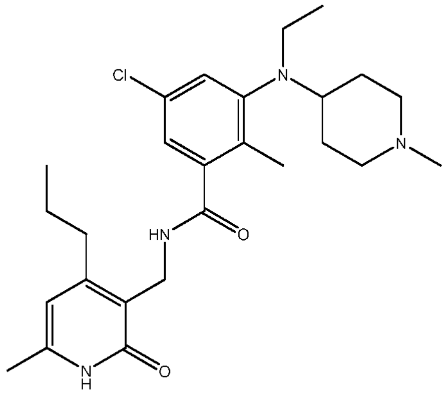 |

ou sel pharmaceutiquement acceptable de celui-ci pour une utilisation dans un procédé de traitement du cancer ou des syndromes myélodysplasiques (SMD, anciennement connus sous le nom de préleucémie) chez un sujet exprimant un EZH2 mutant, l'EZH2 mutant comprenant une mutation par substitution au niveau de la position d'acide aminé 677, 687 ou 641 de SÉQ ID n° : 1.

2. Inhibiteur d'EZH2 pour l'utilisation selon la revendication 1, l'EZH2 mutant comprenant plus d'une mutation par substitution au niveau de la position d'acide aminé 677, 687 ou 641 de SÉQ ID n° : 1.

3. Inhibiteur d'EZH2 pour l'utilisation selon la revendication 1, l'inhibiteur d'EZH2 étant

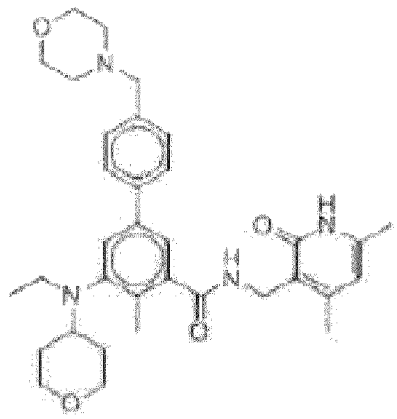

4. Inhibiteur d'EZH2 pour l'utilisation selon la revendication 1, le cancer étant choisi parmi

   a) le lymphome, le lymphome étant facultativement choisi dans le groupe constitué par le lymphome non hodgkinien, le lymphome folliculaire et le lymphome diffus à grandes cellules B, ou
   b) la leucémie, la leucémie étant facultativement la leucémie myéloïde chronique (LMC),
   c) le mélanome.

5. Inhibiteur d'EZH2 pour l'utilisation selon la revendication 1, ladite mutation étant choisie dans le groupe constitué par une substitution de glycine (G) pour le résidu alanine (A) de type sauvage au niveau de la position d'acide aminé 677 de SÉQ ID n° : 1 (A677G) ; une substitution de valine (V) pour le résidu alanine (A) de type sauvage au niveau de la position d'acide aminé 687 de SÉQ ID n° : 1 (A687V) ; une substitution de phénylalanine (F) pour le résidu tyrosine (Y) de type sauvage au niveau de la position d'acide aminé 641 de SÉQ ID n° : 1 (Y641H); une substitution d'histidine (H) pour le résidu tyrosine (Y) de type sauvage au niveau de la position d'acide aminé 641 de SÉQ ID n° : 1 (Y641H) ; une substitution d'asparagine (N) pour le résidu tyrosine (Y) de type sauvage au niveau de la position d'acide aminé 641 de SÉQ ID n° : 1 (Y641N) ; une substitution de sérine (S) pour le résidu tyrosine (Y) de type sauvage au niveau de la position d'acide aminé 641 de SÉQ ID n° : 1 (Y641S) ; et une substitution de cystéine (C)

pour le résidu tyrosine (Y) de type sauvage au niveau de la position d'acide aminé 641 de SÉQ ID n° : 1 (Y641C).

**6.** Procédé de détermination de la réactivité d'un sujet, ayant un cancer ou des syndromes myélodysplasiques (SMD, anciennement connus sous le nom de préleucémie), à un inhibiteur d'EZH2 choisi parmi le composé 2 au composé 62,

| | |
|---|---|
| 2 | 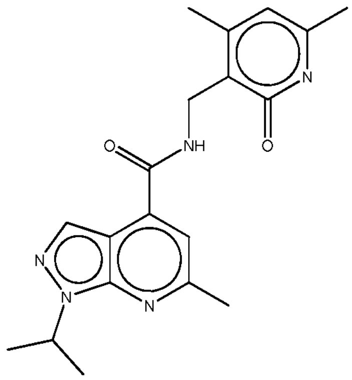 |
| 3 | 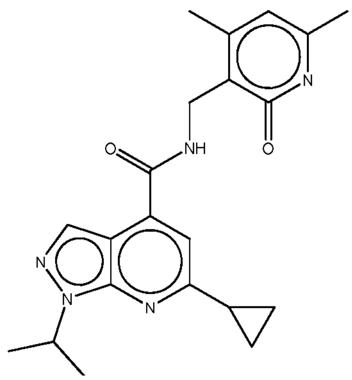 |
| 4 | 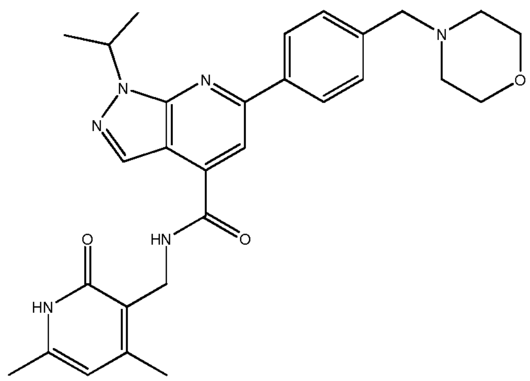 |
| 5 | 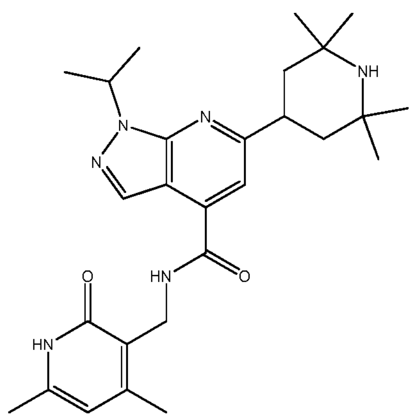 |

(suite)

| | |
|---|---|
| 6 | 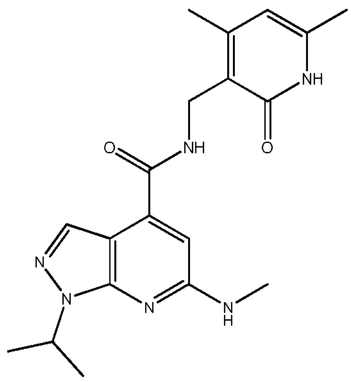 |
| 7 | 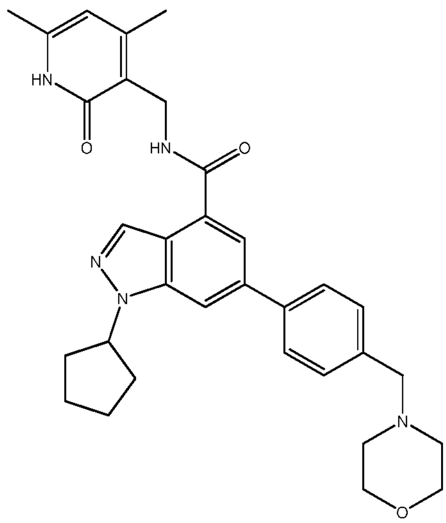 |
| 8 | 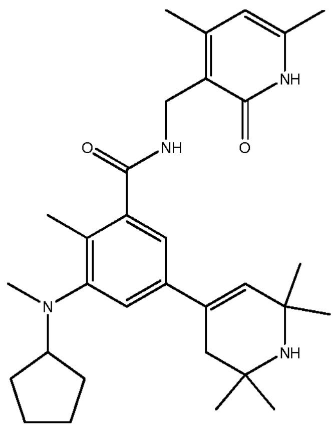 |

(suite)

| | |
|---|---|
| 9 | 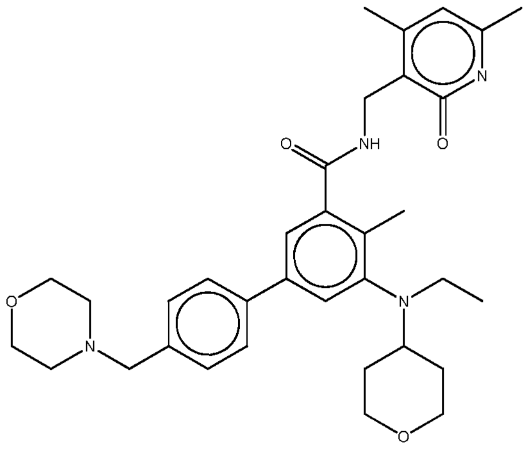 |
| 10 | 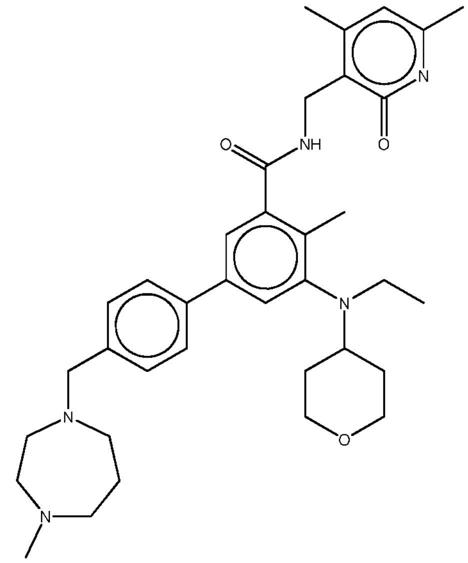 |
| 11 | 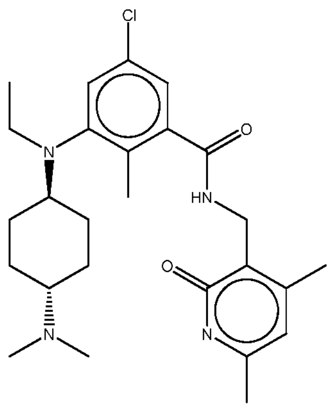 |

(suite)

| | |
|---|---|
| 12 | 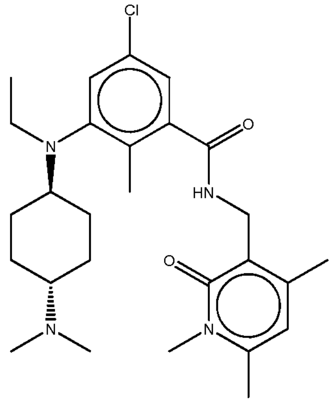 |
| 13 | 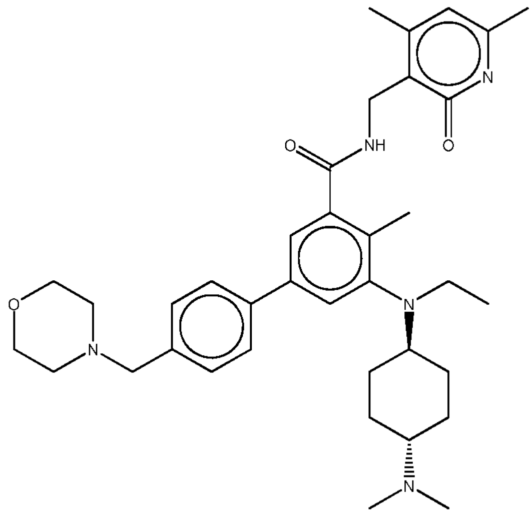 |
| 14 | 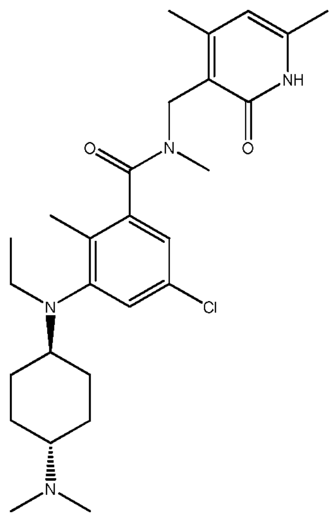 |

(suite)

| | |
|---|---|
| 15 | 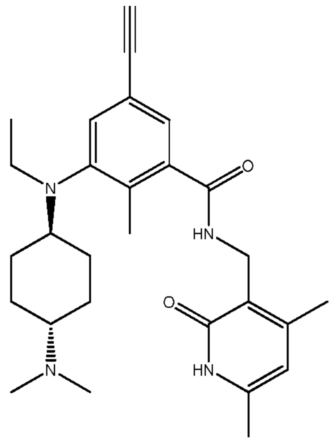 |
| 16 | 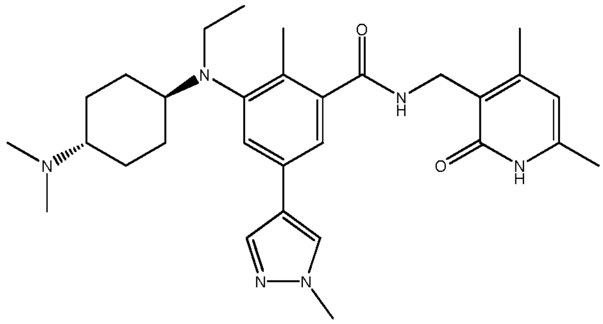 |
| 17 | 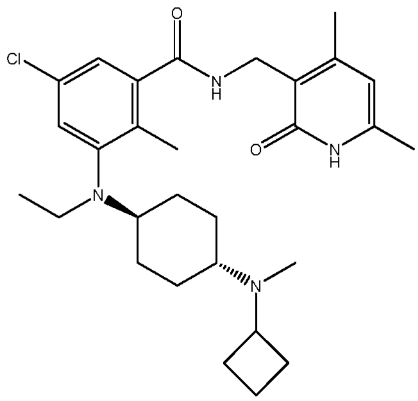 |

(suite)

| | |
|---|---|
| 18 | 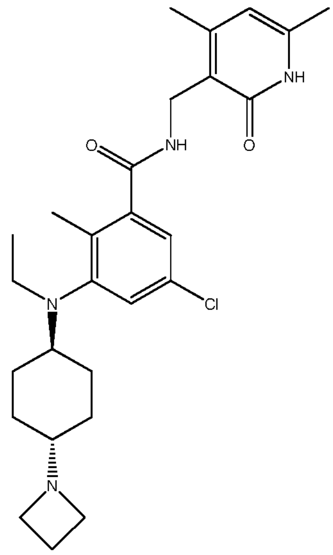 |
| 19 | 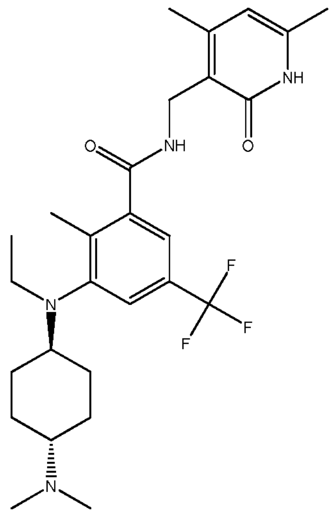 |
| 20 | 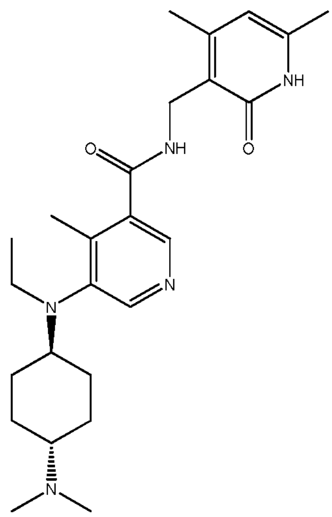 |

(suite)

| | |
|---|---|
| 21 | 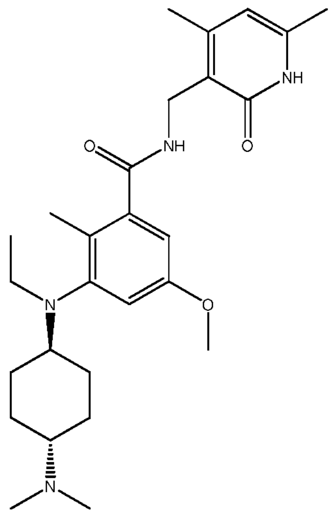 |
| 22 | 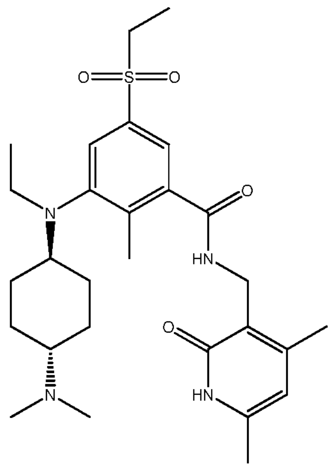 |
| 23 | 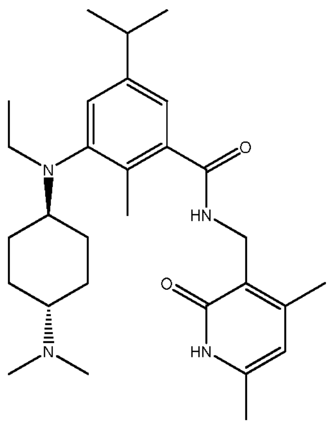 |

(suite)

| | |
|---|---|
| 24 | 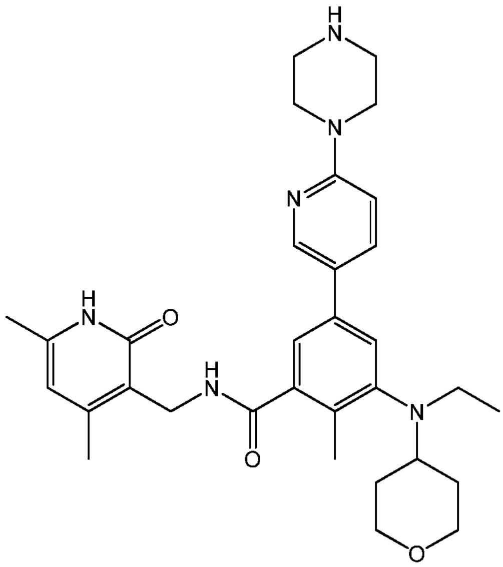 |
| 25 | 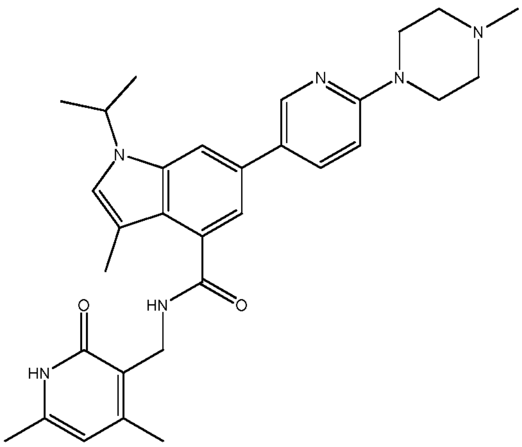 |
| 26 | 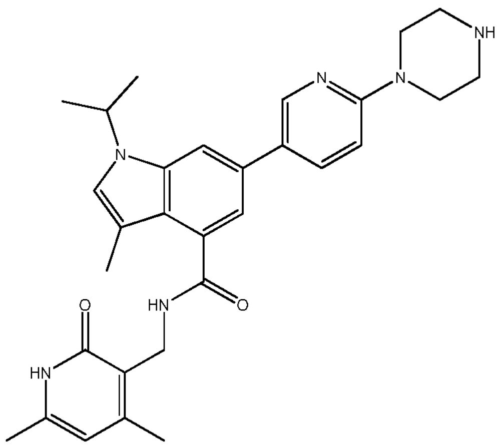 |

(suite)

| | |
|---|---|
| 27 | 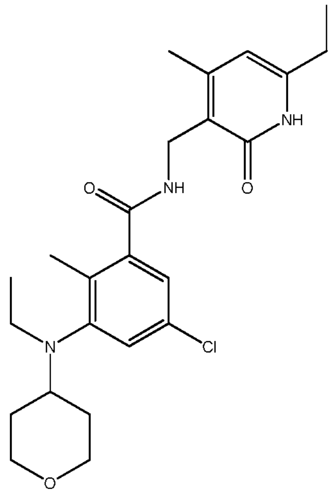 |
| 28 | 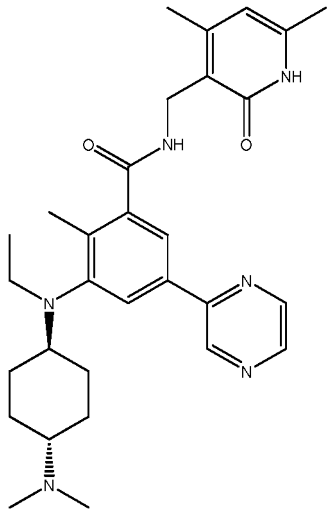 |
| 29 | 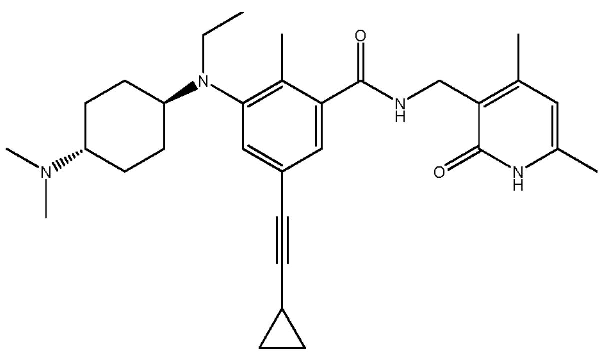 |

(suite)

| | |
|---|---|
| 30 | 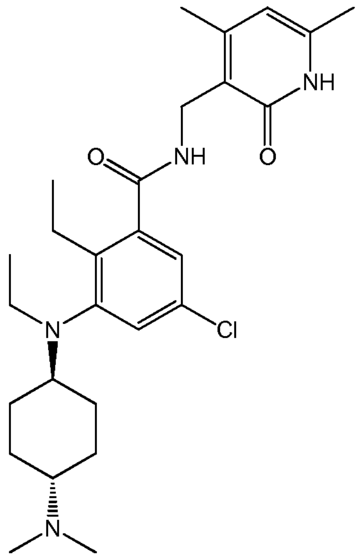 |
| 31 | 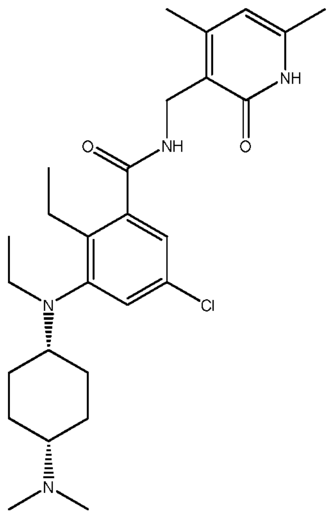 |
| 32 | 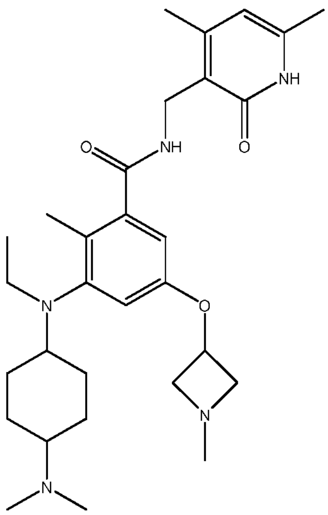 |

(suite)

| | |
|---|---|
| 33 | 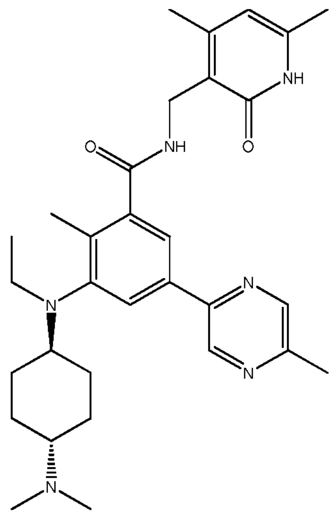 |
| 34 | 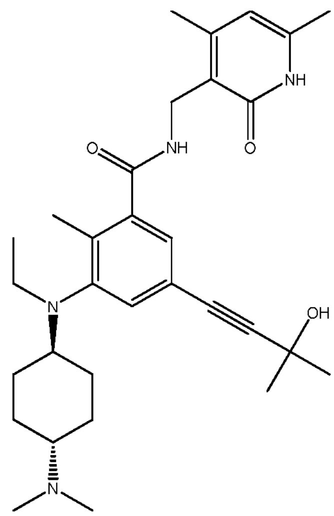 |
| 35 | 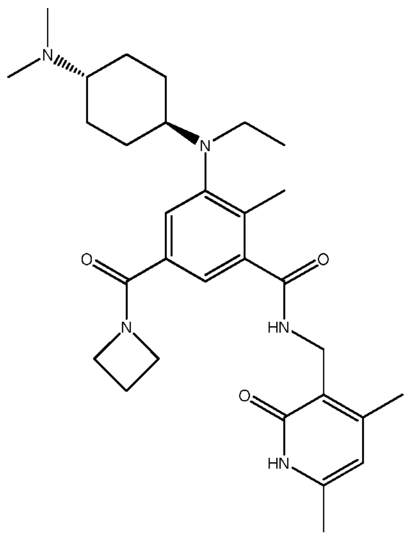 |

(suite)

| | |
|---|---|
| 36 | 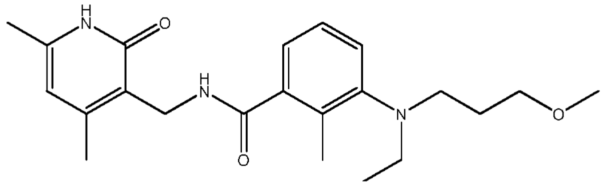 |
| 37 | 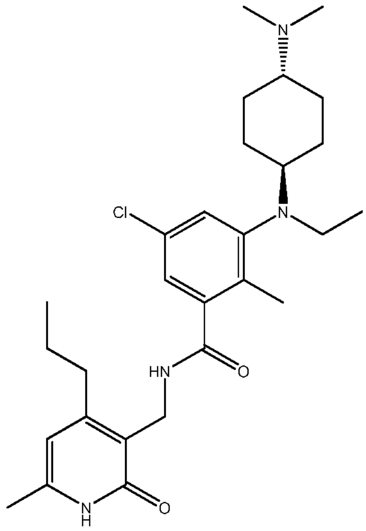 |
| 38 | 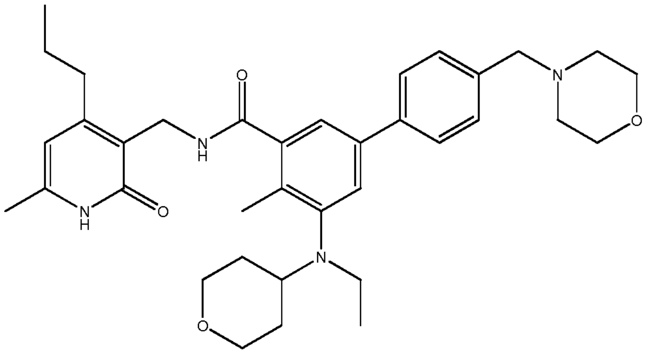 |
| 39 | 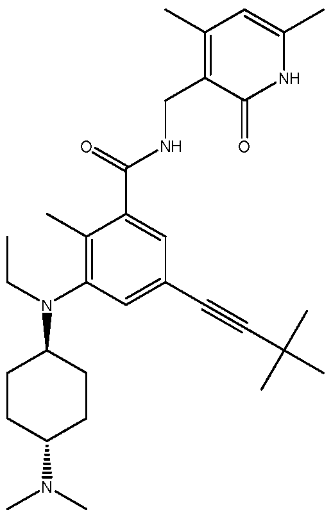 |

(suite)

| | |
|---|---|
| 40 | 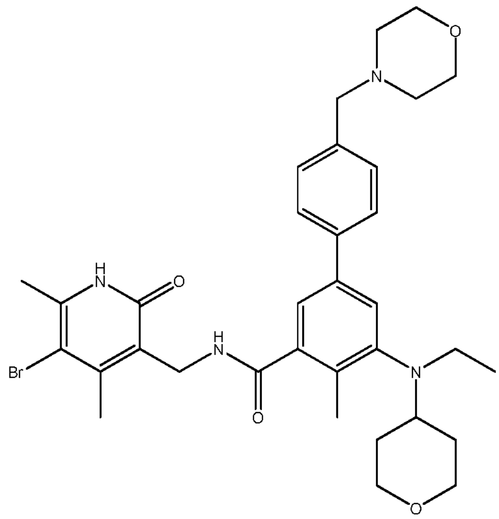 |
| 41 | 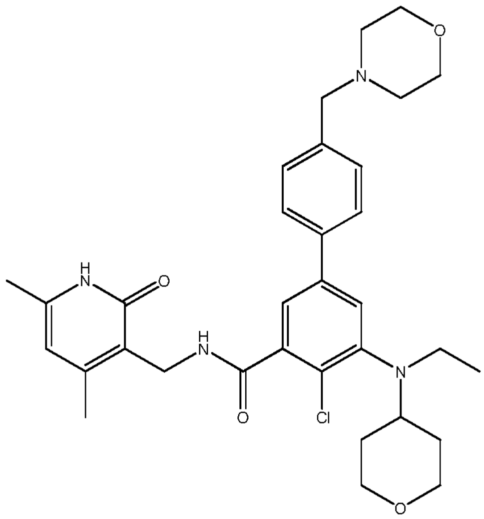 |
| 42 | 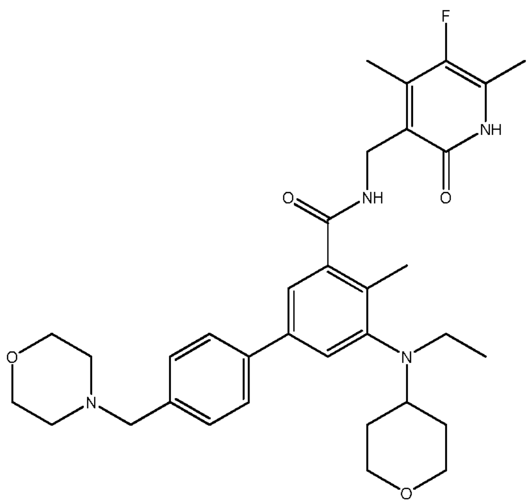 |

(suite)

| | |
|---|---|
| 43 | 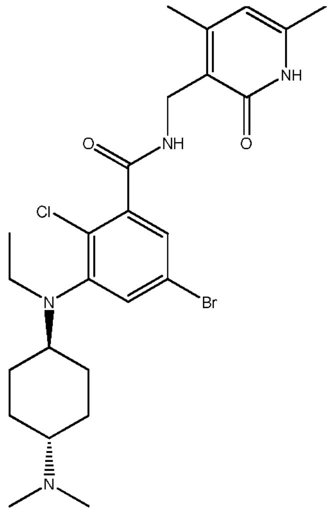 |
| 44 | 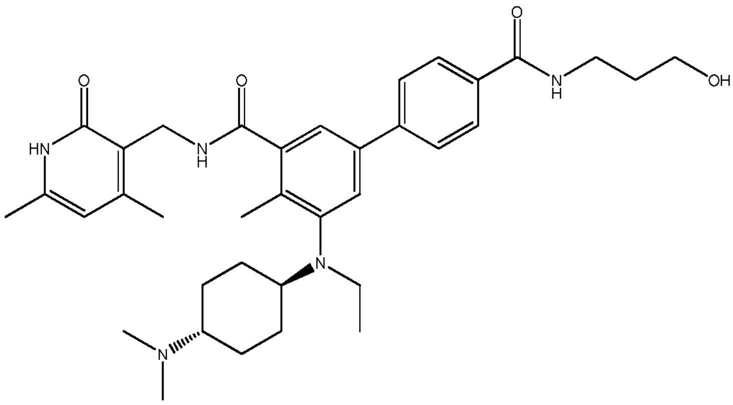 |
| 45 | 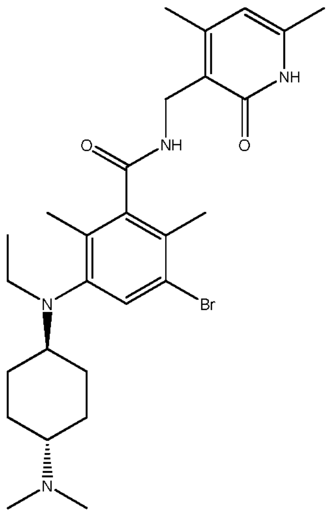 |

(suite)

| | |
|---|---|
| 46 | 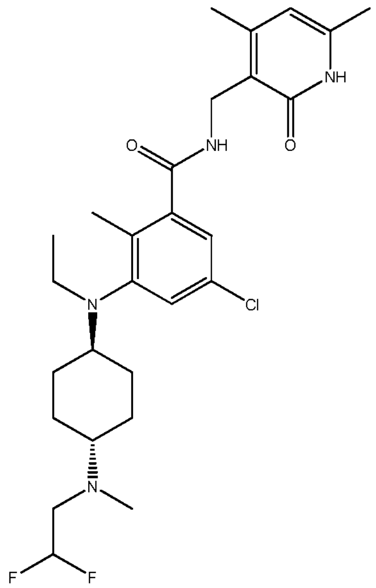 |
| 47 | 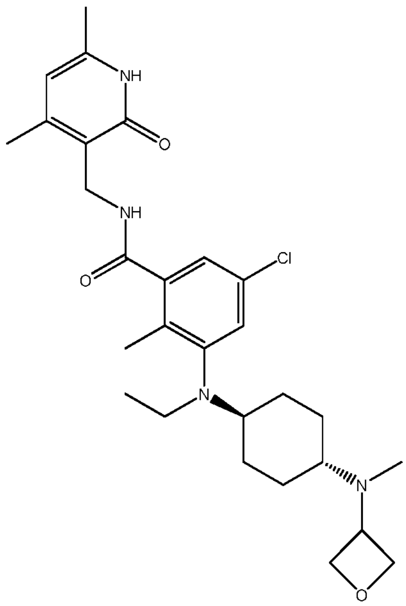 |
| 48 | 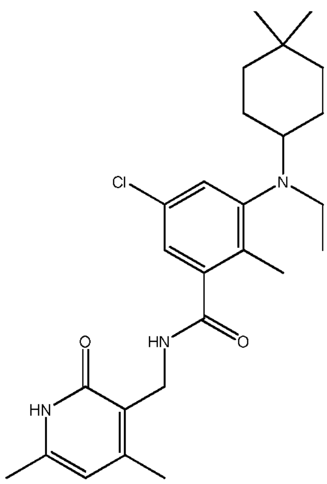 |

(suite)

| | |
|---|---|
| 49 | 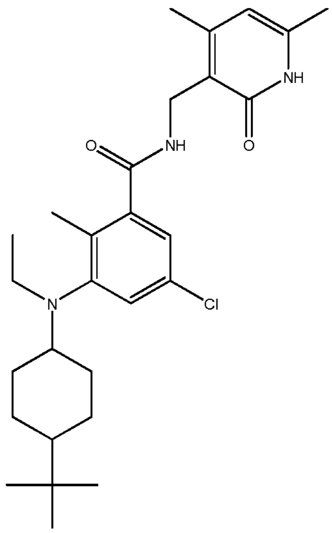 |
| 50 | 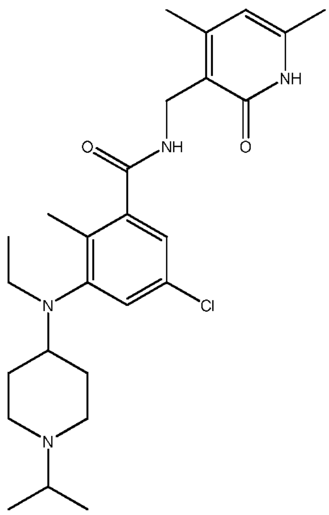 |
| 51 | 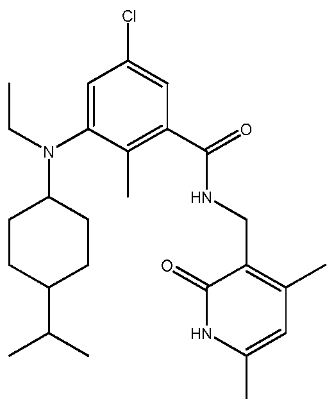 |

(suite)

| | |
|---|---|
| 52 | 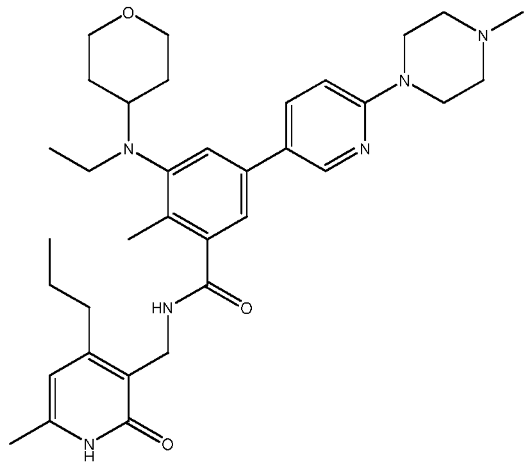 |
| 53 | 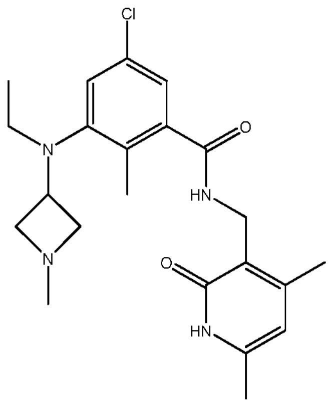 |
| 54 | 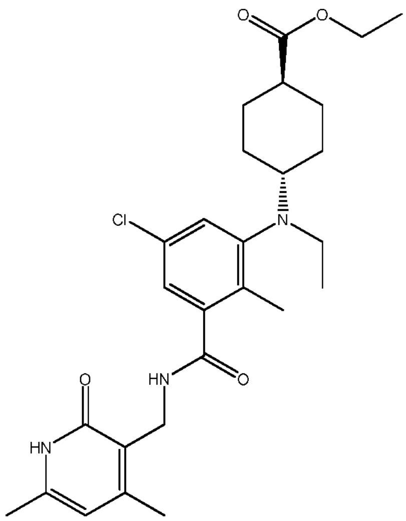 |

(suite)

| | |
|---|---|
| 55 | 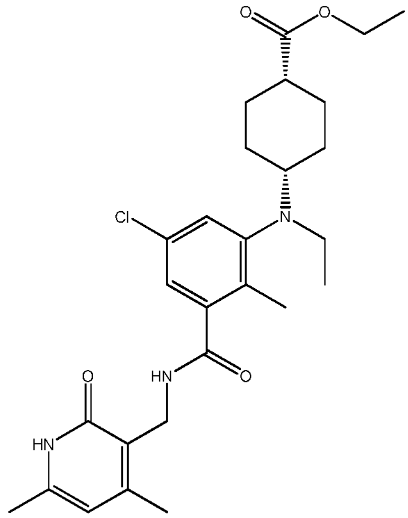 |
| 56 | 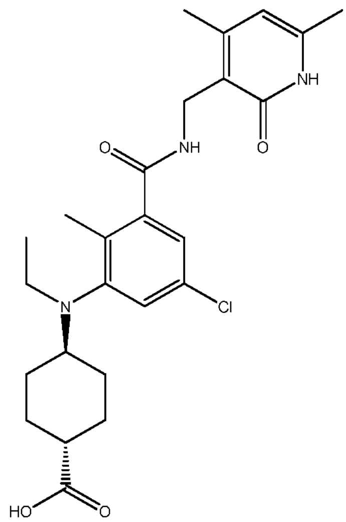 |
| 57 | 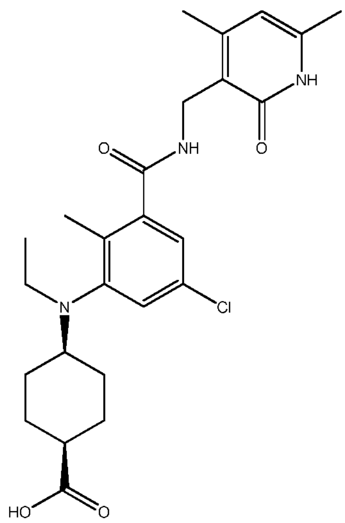 |

(suite)

| | |
|---|---|
| 58 | 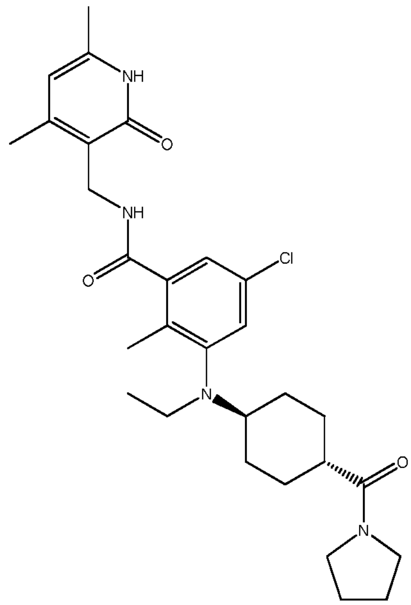 |
| 59 | 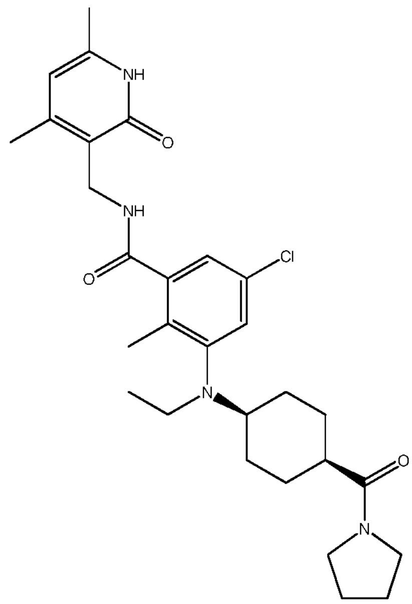 |

(suite)

| | |
|---|---|
| 60 | 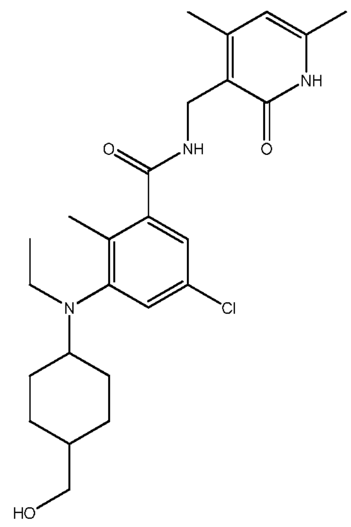 |
| 61 | 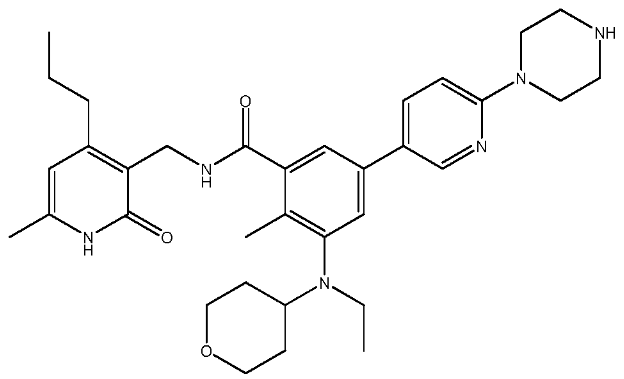 |
| 62 | 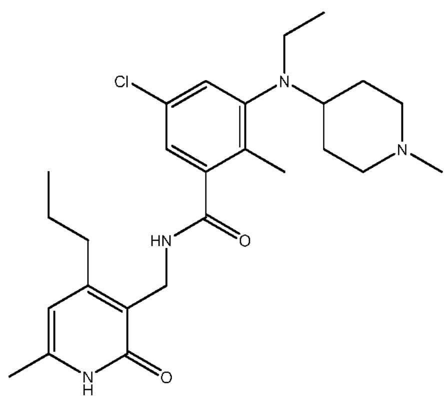 |

comprenant la détection de la présence de mutation dans EZH2 dans un échantillon prélevé sur un sujet, la mutation étant ou étant exprimée sous forme de mutation par substitution au niveau de la position d'acide aminé 677, 687 ou 641 de SÉQ ID n° : 1, la présence de ladite mutation indiquant que le sujet réagit à l'inhibiteur d'EZH2.

**7.** Procédé selon la revendication 6, le procédé comprenant la détection de la présence de plus d'une mutation par substitution au niveau de la position d'acide aminé 677, 687 ou 641 de SÉQ ID n° : 1.

**8.** Procédé selon la revendication 6, le cancer étant choisi parmi

a) le lymphome, le lymphome étant facultativement choisi dans le groupe constitué par le lymphome non hodgkinien, le lymphome folliculaire et le lymphome diffus à grandes cellules B, ou

b) la leucémie, la leucémie étant facultativement la leucémie myéloïde chronique (LMC), ou
c) le mélanome.

9. Procédé selon la revendication 6, ladite mutation étant choisie dans le groupe constitué par une substitution de glycine (G) pour le résidu alanine (A) de type sauvage au niveau de la position d'acide aminé 677 de SÉQ ID n° : 1 (A677G) ; une substitution de valine (V) pour le résidu alanine (A) de type sauvage au niveau de la position d'acide aminé 687 de SÉQ ID n° : 1 (A687V) ; une substitution de phénylalanine (F) pour le résidu tyrosine (Y) de type sauvage au niveau de la position d'acide aminé 641 de SÉQ ID n° : 1 (Y641H) ; une substitution d'histidine (H) pour le résidu tyrosine (Y) de type sauvage au niveau de la position d'acide aminé 641 de SÉQ ID n° : 1 (Y641H); une substitution d'asparagine (N) pour le résidu tyrosine (Y) de type sauvage au niveau de la position d'acide aminé 641 de SÉQ ID n° : 1 (Y641N); une substitution de sérine (S) pour le résidu tyrosine (Y) de type sauvage au niveau de la position d'acide aminé 641 de SÉQ ID n° : 1 (Y641S) ; et une substitution de cystéine (C) pour le résidu tyrosine (Y) de type sauvage au niveau de la position d'acide aminé 641 de SÉQ ID n° : 1 (Y641C).

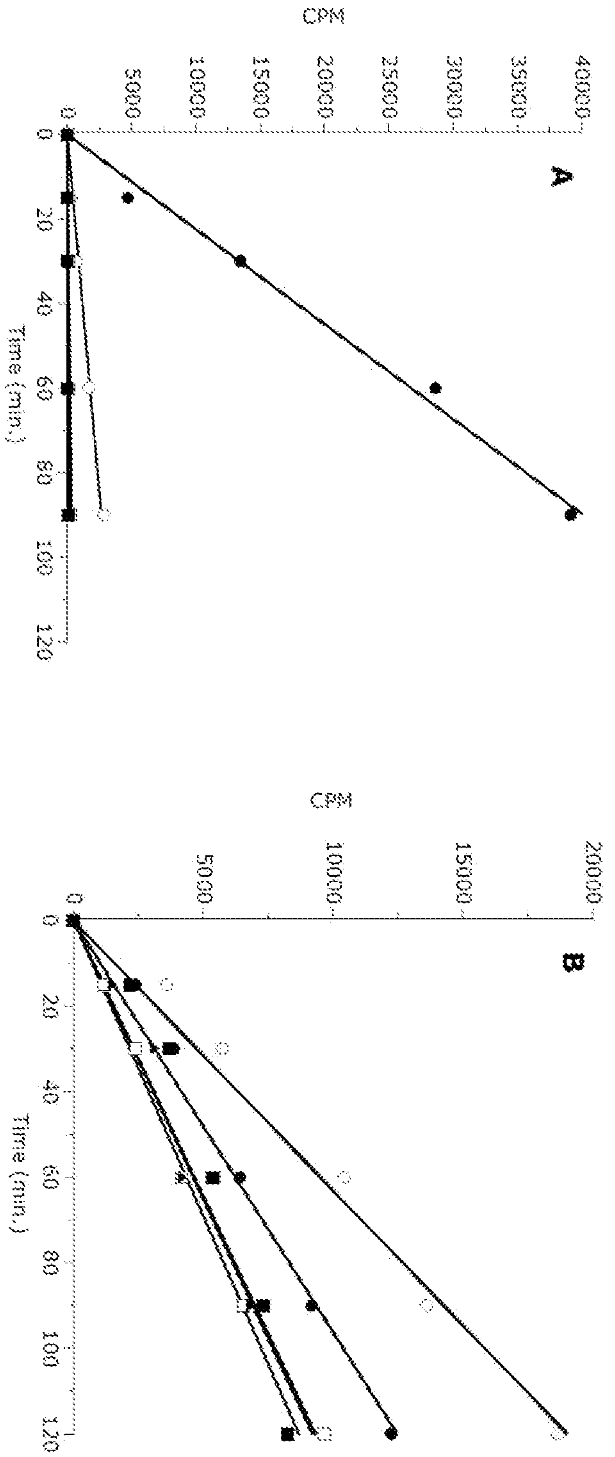
A
CPM
0
5000
10000
15000
20000
25000
30000
35000
40000
0
20
40
60
80
100
120
Time (min.)
B
CPM
0
5000
10000
15000
20000
0
20
40
60
80
100
120
Time (min.)

FIG. 1

FIG. 2

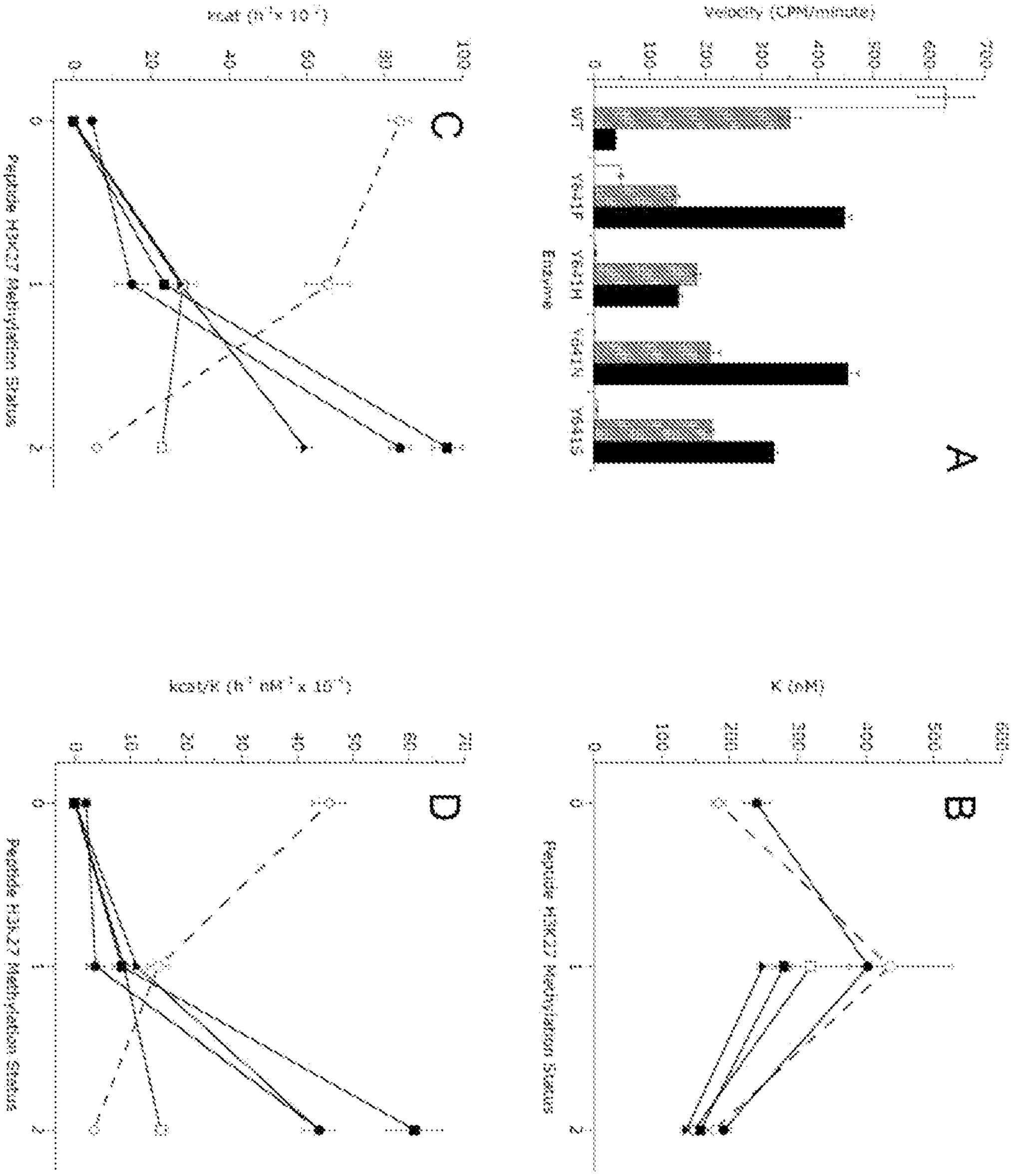

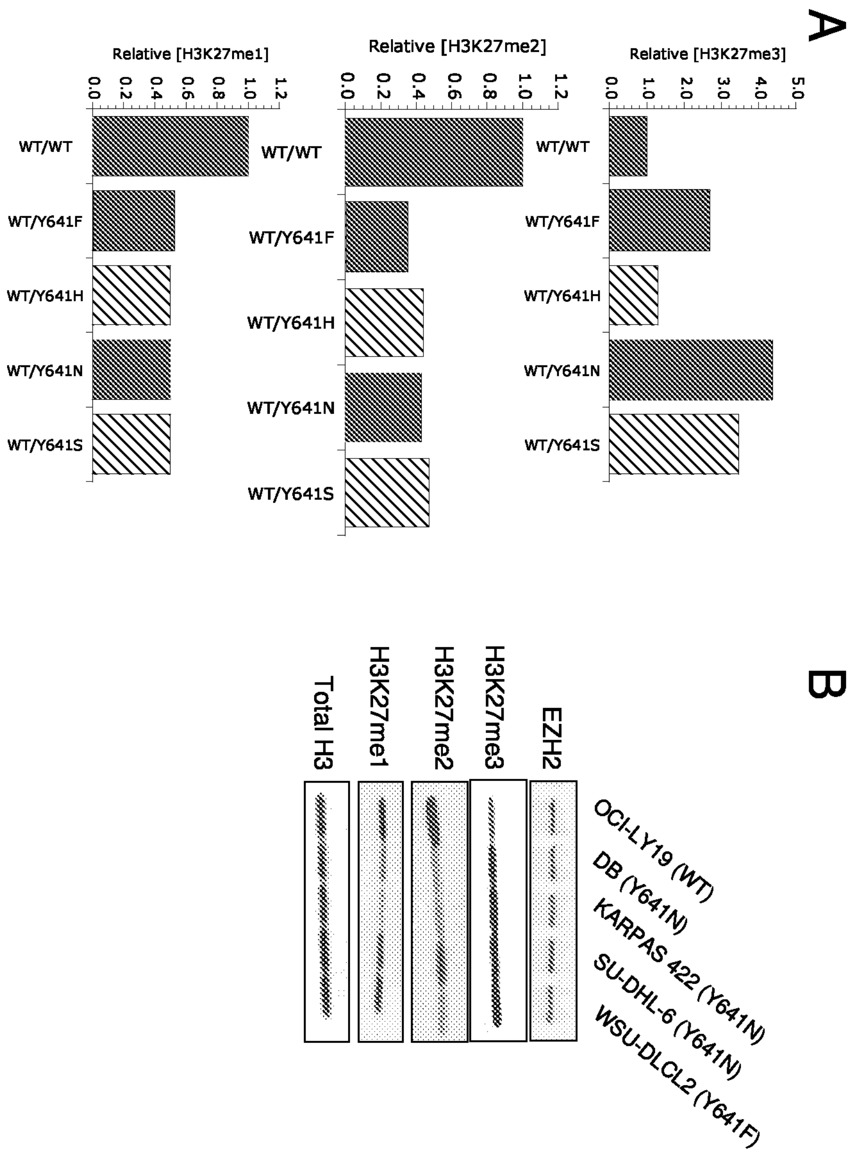
A
Relative [H3K27me1]
0.0
0.2
0.4
0.6
0.8
1.0
1.2
WT/WT
WT/Y641F
WT/Y641H
WT/Y641N
WT/Y641S
Relative [H3K27me2]
0.0
0.2
0.4
0.6
0.8
1.0
1.2
WT/WT
WT/Y641F
WT/Y641H
WT/Y641N
WT/Y641S
Relative [H3K27me3]
0.0
1.0
2.0
3.0
4.0
5.0
WT/WT
WT/Y641F
WT/Y641H
WT/Y641N
WT/Y641S
B
Total H3
H3K27me1
H3K27me2
H3K27me3
EZH2
OCI-LY19 (WT)
DB (Y641N)
KARPAS 422 (Y641N)
SU-DHL-6 (Y641N)
WSU-DLCL2 (Y641F)

FIG. 3

FIG. 4

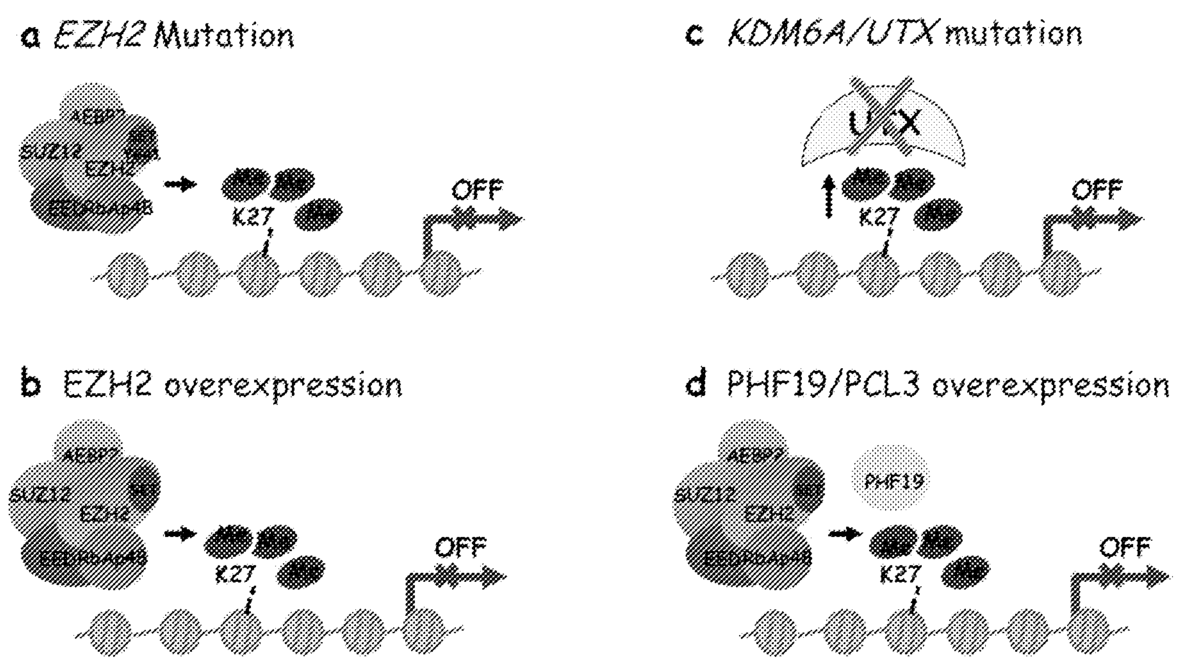
a EZH2 Mutation
AEBP2
SUZ12
EZH2
EED/RbAp48
K27
OFF
b EZH2 overexpression
AEBP2
SUZ12
SET
EZH2
EED/RbAp48
K27
OFF
c KDM6A/UTX mutation
UTX
K27
OFF
d PHF19/PCL3 overexpression
AEBP2
SUZ12
EZH2
EED/RbAp48
PHF19
K27
OFF

FIG. 5

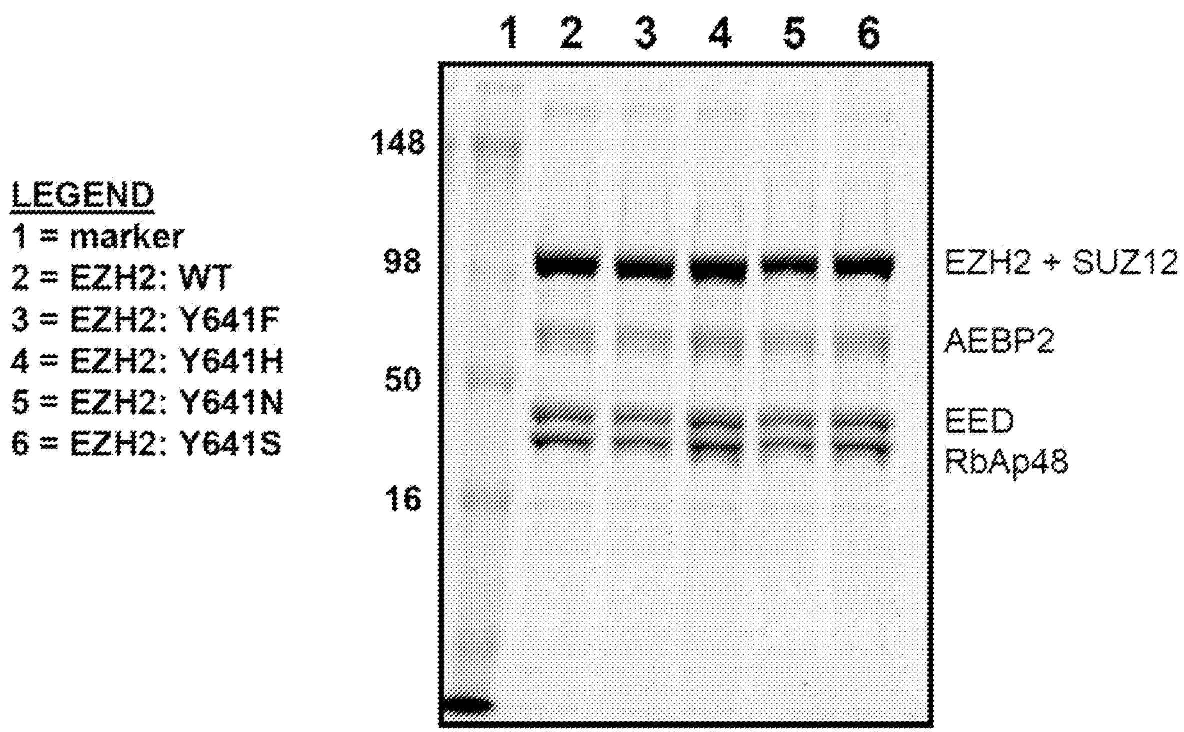
LEGEND
1 = marker
2 = EZH2: WT
3 = EZH2: Y641F
4 = EZH2: Y641H
5 = EZH2: Y641N
6 = EZH2: Y641S
1
2
3
4
5
6
148
98
50
16
EZH2 + SUZ12
AEBP2
EED
RbAp48

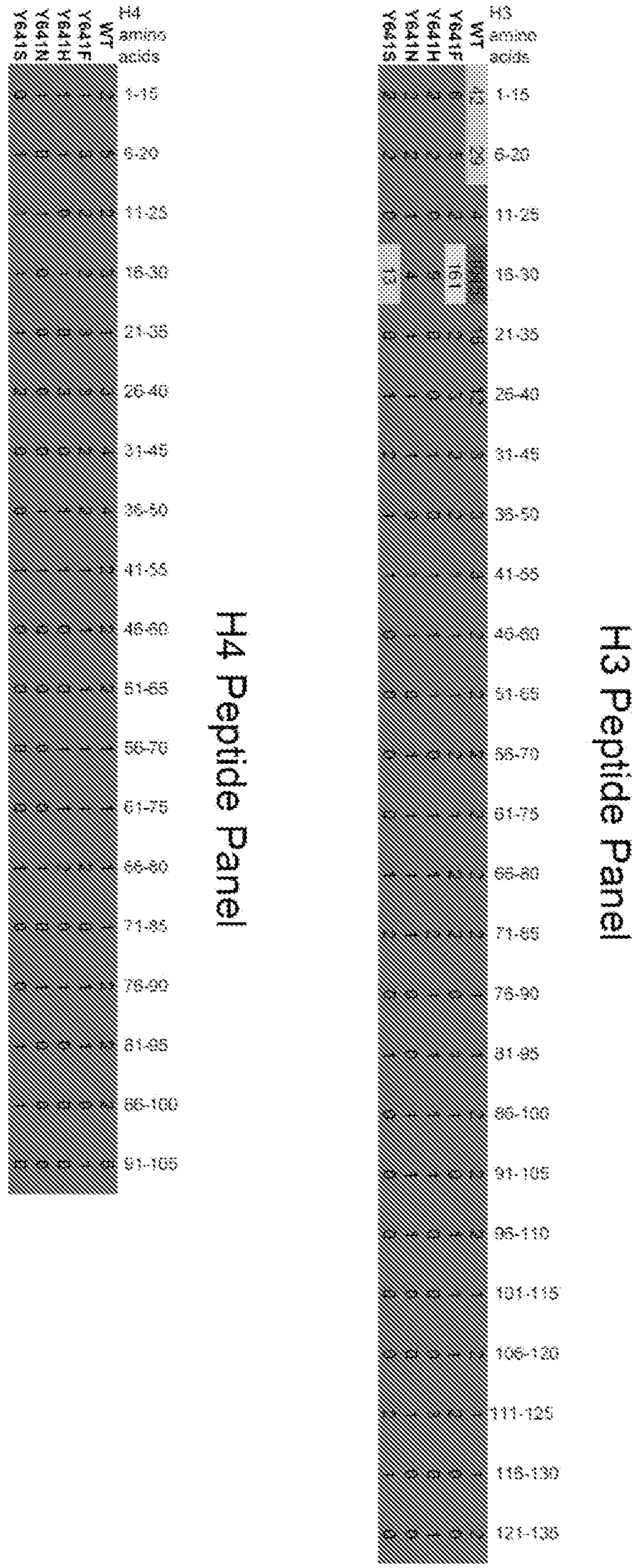
H4 amino acids
WT
Y641F
Y641H
Y641N
Y641S
1-15
6-20
11-25
16-30
21-35
26-40
31-45
36-50
41-55
46-60
51-65
56-70
61-75
66-80
71-85
76-90
81-95
86-100
91-105
H4 Peptide Panel
H3 amino acids
WT
Y641F
Y641H
Y641N
Y641S
1-15
6-20
11-25
16-30
21-35
26-40
31-45
36-50
41-55
46-60
51-65
56-70
61-75
66-80
71-85
76-90
81-95
86-100
91-105
96-110
101-115
106-120
111-125
116-130
121-135
H3 Peptide Panel

FIG. 6

FIG. 7

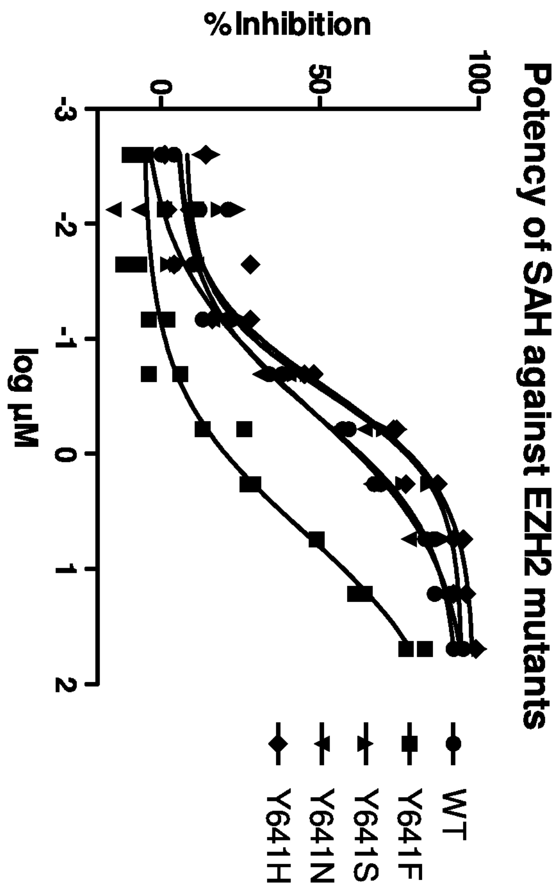
Potency of SAH against EZH2 mutants
%Inhibition
100
50
0
-3
-2
-1
0
1
2
log μM
WT
Y641F
Y641S
Y641N
Y641H

FIG. 8

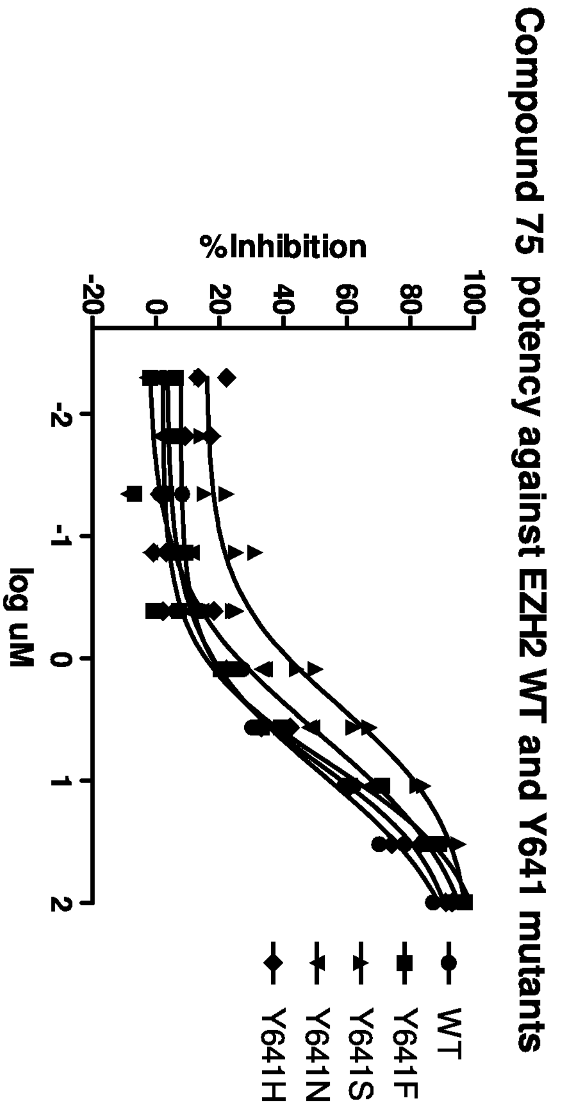
Compound 75 potency against EZH2 WT and Y641 mutants
%Inhibition
100
80
60
40
20
0
-20
-2
-1
0
1
2
log uM
WT
Y641F
Y641S
Y641N
Y641H

FIG. 9

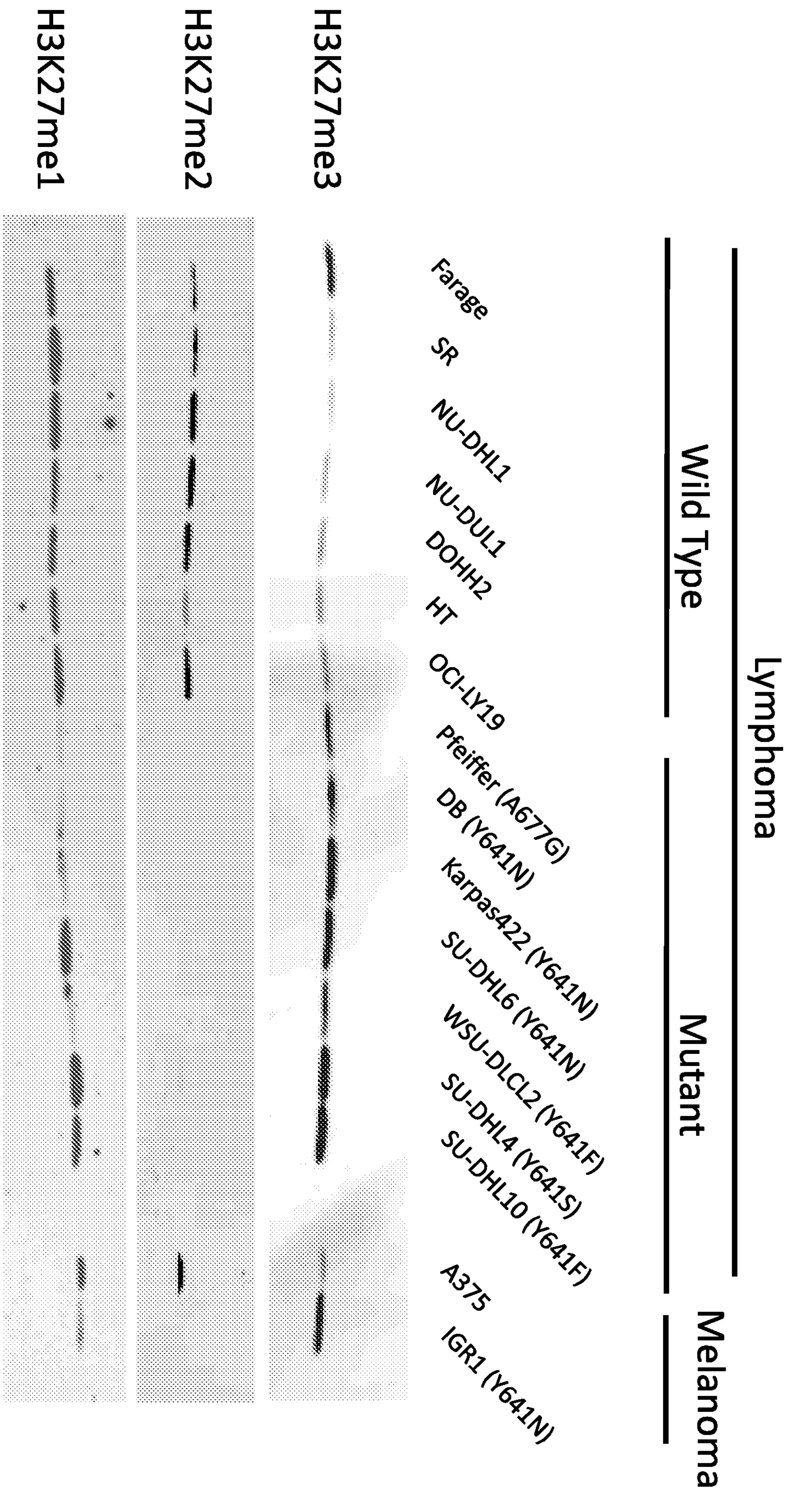
Lymphoma
Wild Type
Mutant
Melanoma
Farage
SR
NU-DHL1
NU-DUL1
DOHH2
HT
OCI-LY19
Pfeiffer (A677G)
DB (Y641N)
Karpas422 (Y641N)
SU-DHL6 (Y641N)
WSU-DLCL2 (Y641F)
SU-DHL4 (Y641S)
SU-DHL10 (Y641F)
A375
IGR1 (Y641N)
H3K27me3
H3K27me2
H3K27me1

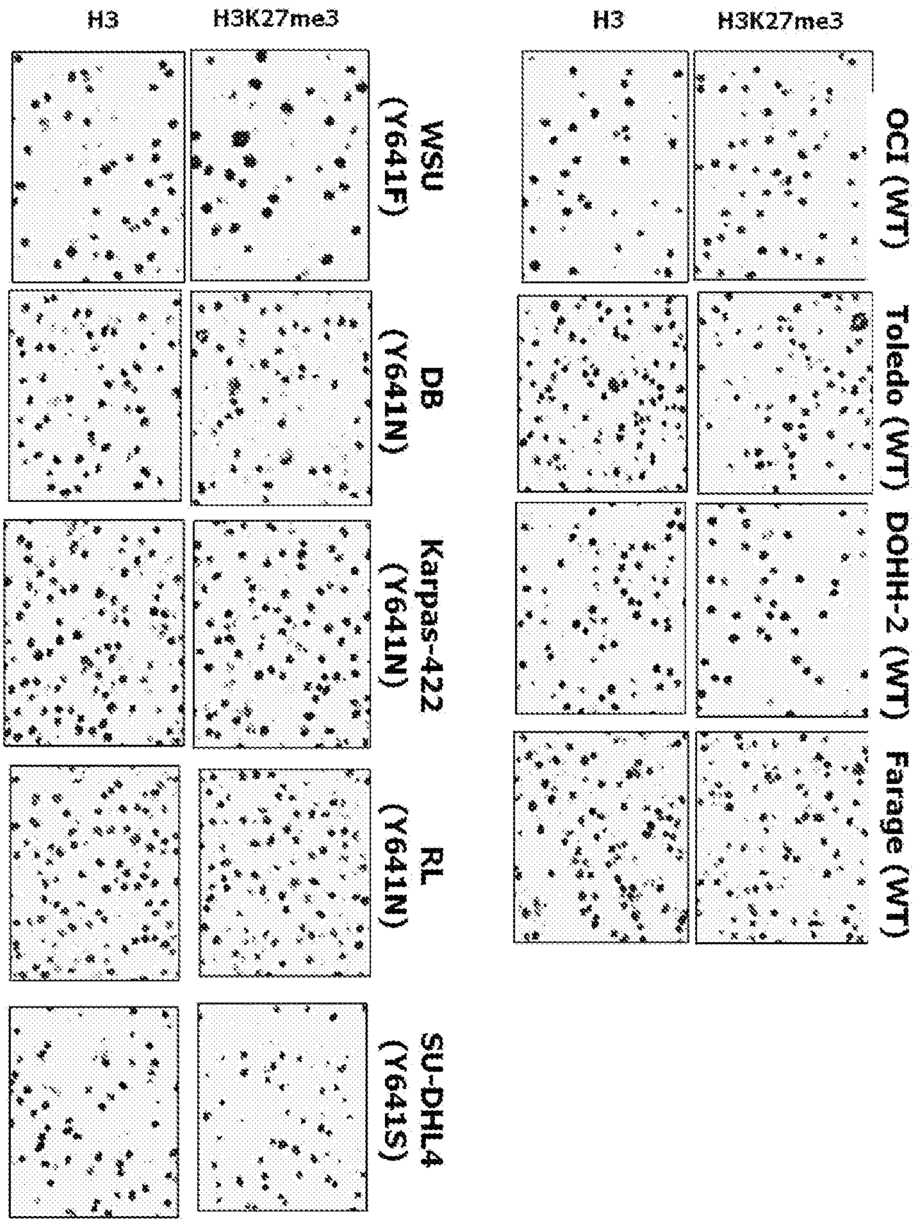
H3
H3K27me3
WSU (Y641F)
DB (Y641N)
Karpas-422 (Y641N)
RL (Y641N)
SU-DHL4 (Y641S)
H3
H3K27me3
OCI (WT)
Toledo (WT)
DOHH-2 (WT)
Farage (WT)

FIG. 10

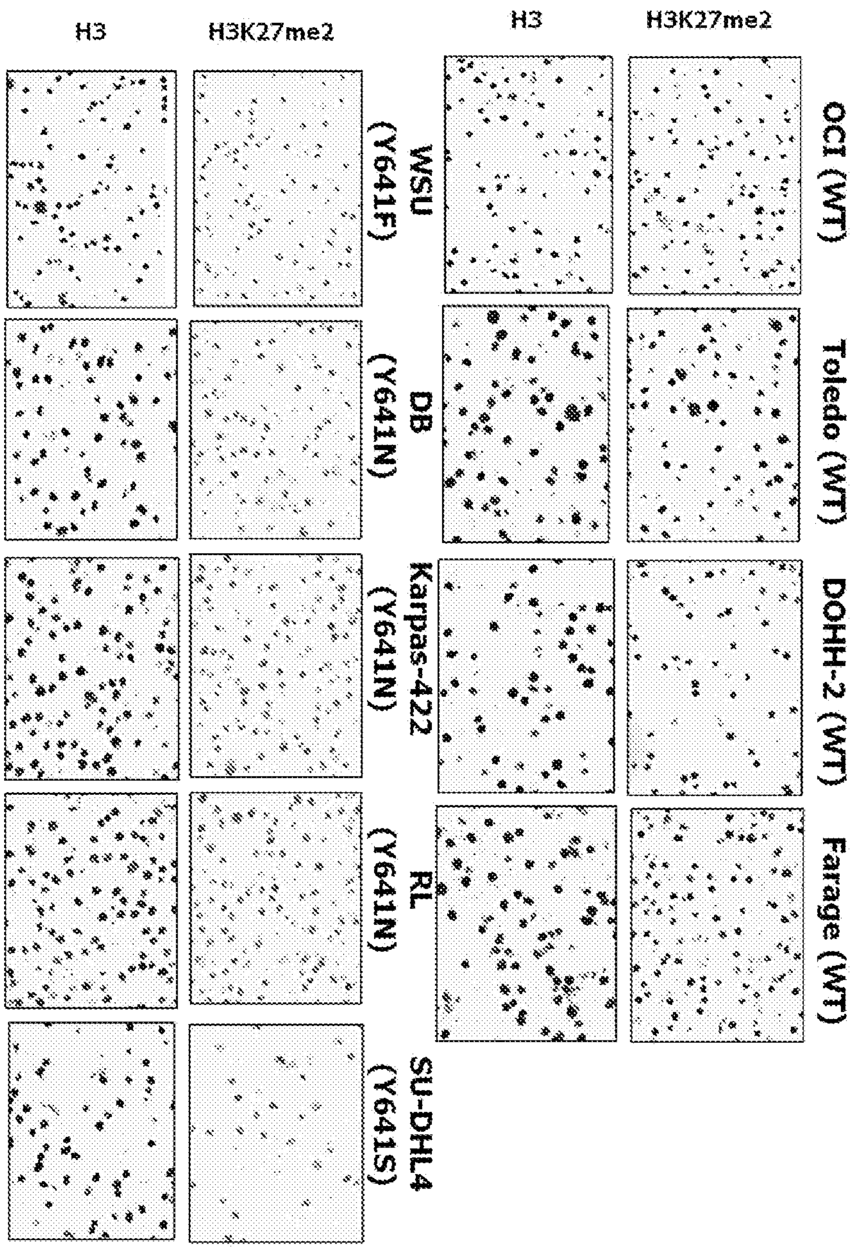
H3
H3K27me2
H3
H3K27me2
OCI (WT)
Toledo (WT)
DOHH-2 (WT)
Farage (WT)
WSU (Y641F)
DB (Y641N)
Karpas-422 (Y641N)
RL (Y641N)
SU-DHL4 (Y641S)

FIG. 11

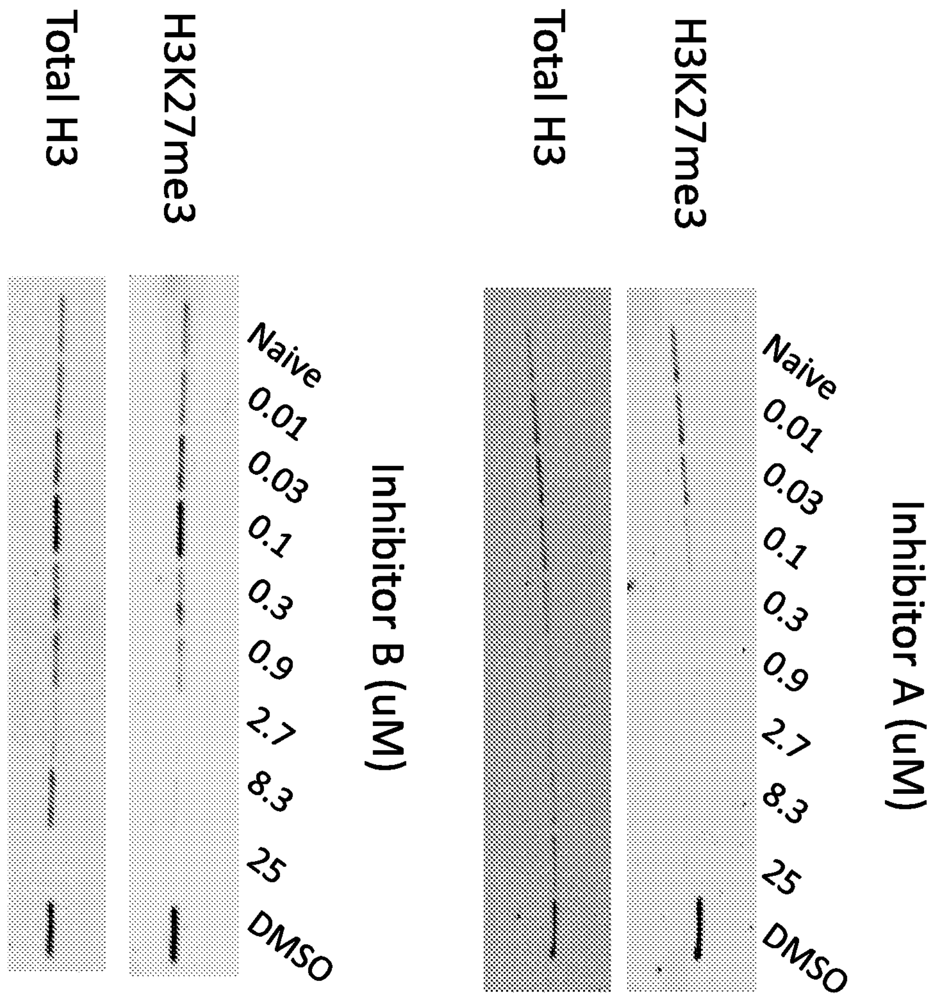
Total H3
H3K27me3
Naive
0.01
0.03
0.1
0.3
0.9
2.7
8.3
25
DMSO
Inhibitor B (uM)
Total H3
H3K27me3
Naive
0.01
0.03
0.1
0.3
0.9
2.7
8.3
25
DMSO
Inhibitor A (uM)

FIG. 12

FIG. 13

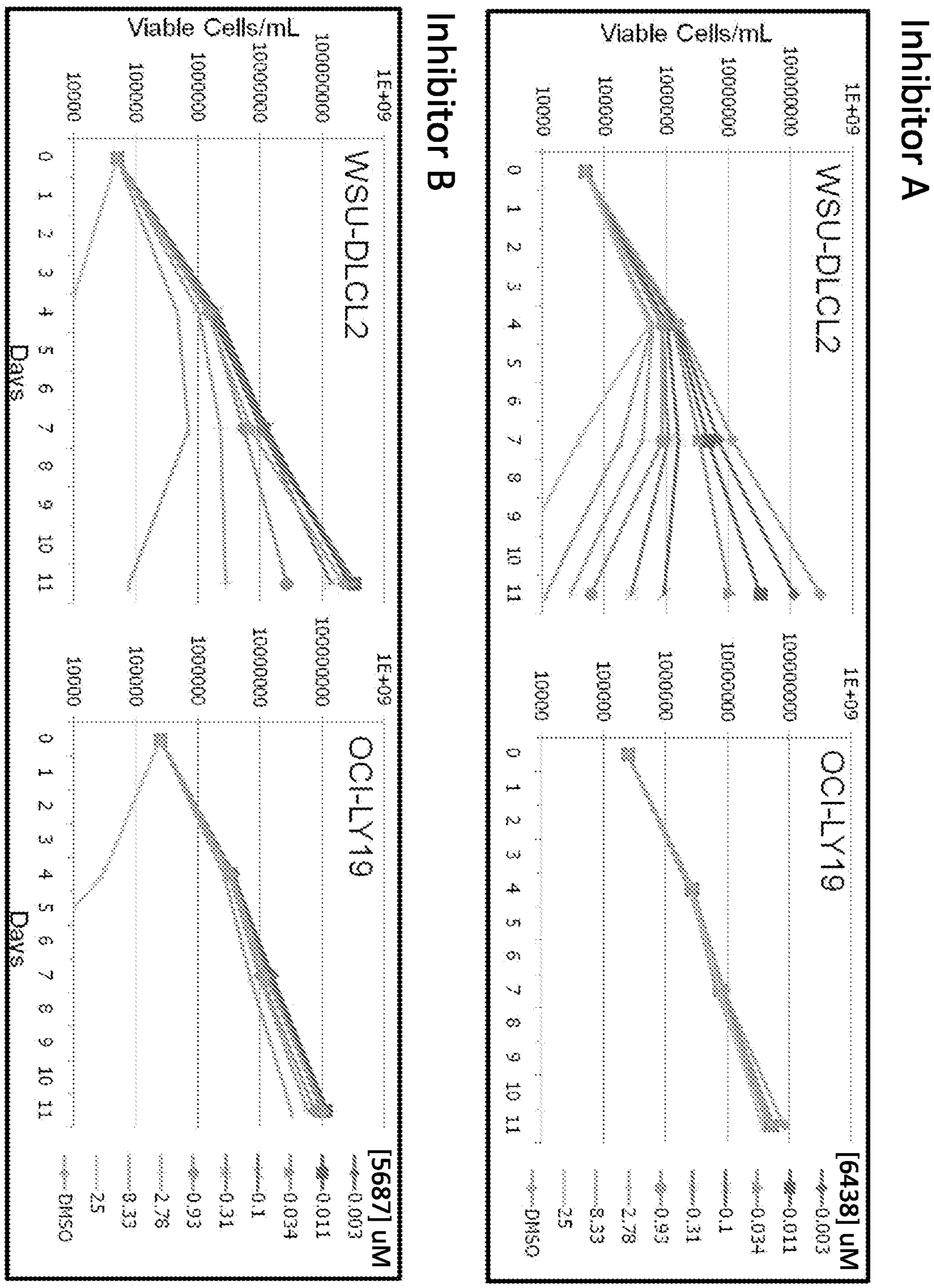
Inhibitor A
WSU-DLCL2
OCI-LY19
Viable Cells/mL
1E+09
100000000
10000000
1000000
100000
10000
0 1 2 3 4 5 6 7 8 9 10 11
[6438] uM
0.003
0.011
0.034
0.1
0.31
0.93
2.78
8.33
25
DMSO
Inhibitor B
WSU-DLCL2
OCI-LY19
Viable Cells/mL
Days
[5687] uM
0.003
0.011
0.034
0.1
0.31
0.93
2.76
8.33
25
DMSO
13

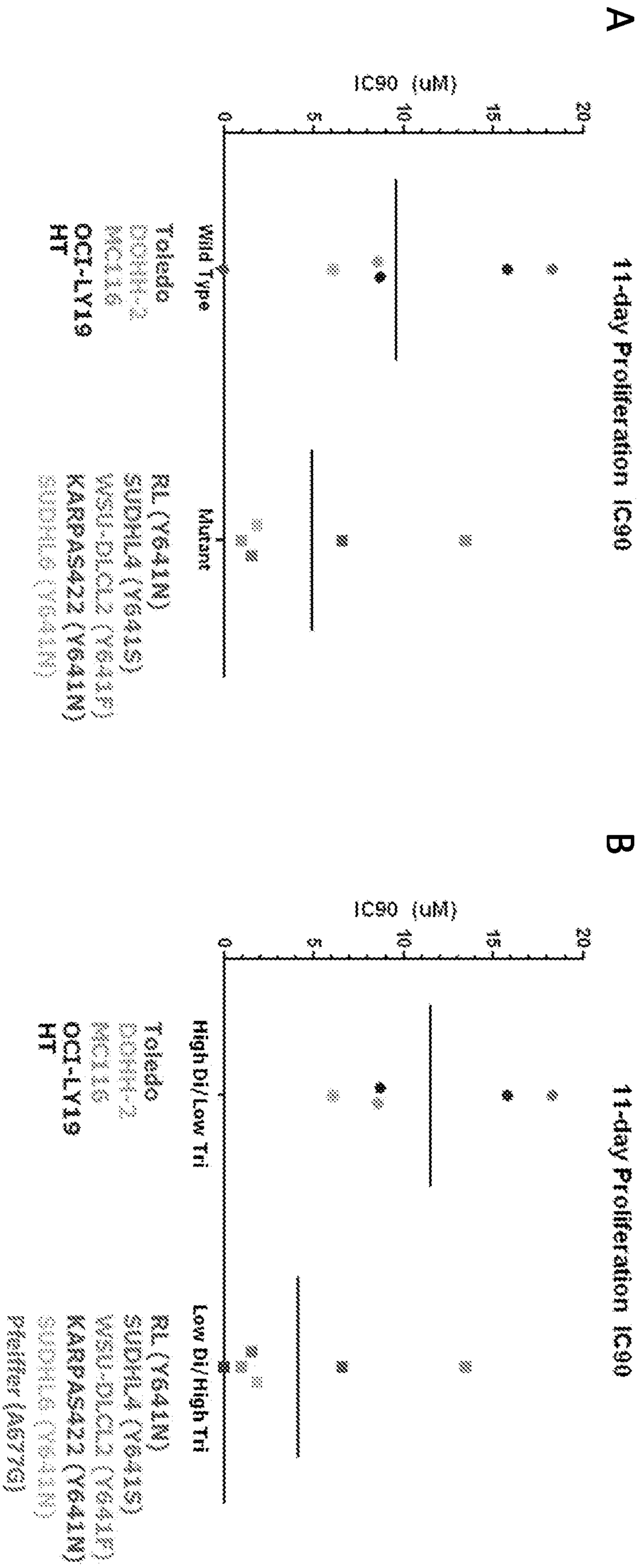
A
11-day Proliferation IC90
IC90 (uM)
0
5
10
15
20
Wild Type
Mutant
Toledo
DOHH-2
MC116
OCI-LY19
HT
RL (Y641N)
SUDHL4 (Y641S)
WSU-DLCL2 (Y641F)
KARPAS422 (Y641N)
SUDHL6 (Y641N)
B
11-day Proliferation IC90
IC90 (uM)
0
5
10
15
20
High Di/Low Tri
Low Di/High Tri
Toledo
DOHH-2
MC116
OCI-LY19
HT
RL (Y641N)
SUDHL4 (Y641S)
WSU-DLCL2 (Y641F)
KARPAS422 (Y641N)
SUDHL6 (Y641N)
Pfeiffer (A677G)

FIG. 14

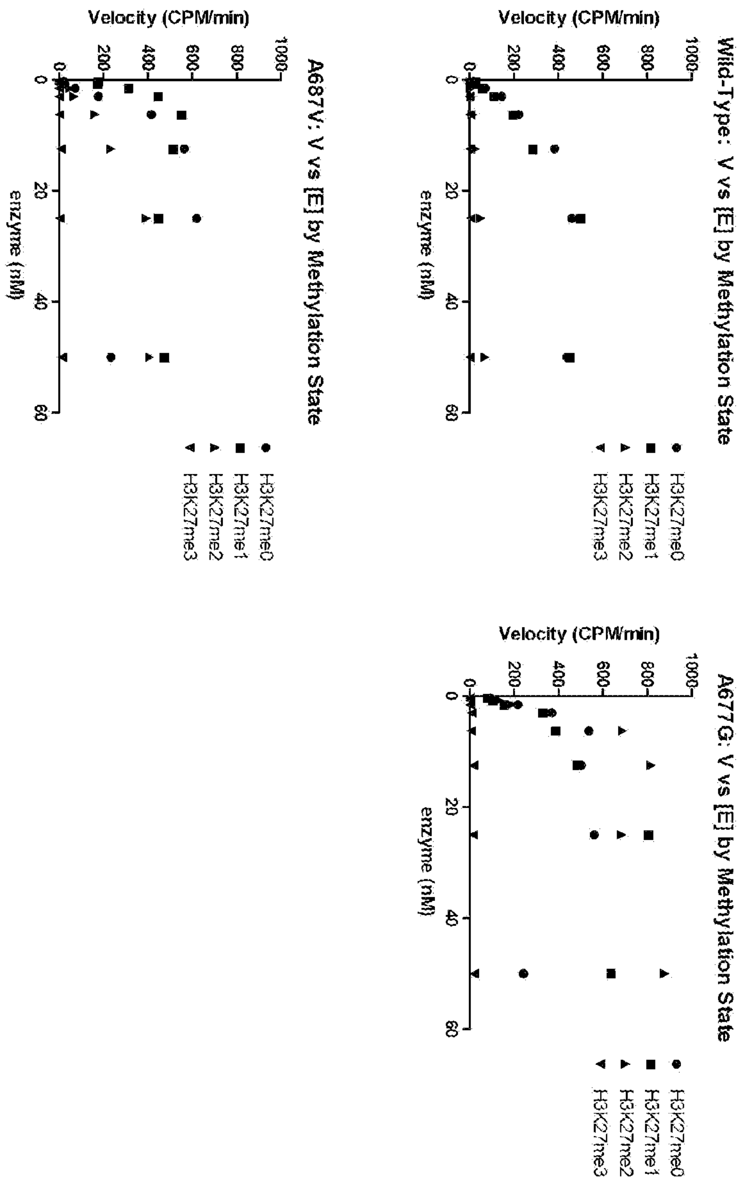
Wild-Type: V vs [E] by Methylation State
Velocity (CPM/min)
enzyme (nM)
A677G: V vs [E] by Methylation State
Velocity (CPM/min)
enzyme (nM)
A687V: V vs [E] by Methylation State
Velocity (CPM/min)
enzyme (nM)
H3K27me0
H3K27me1
H3K27me2
H3K27me3

FIG. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7563589 B
- US 2012026953 W
- US 61474821 A
- US 61474825 B
- US 61499595 B
- US 61505676 B
- US 5763263 A


### Non-patent literature cited in the description


- **FRANCIS et al.** *Nat Rev Mol Cell Biol,* 2001, vol. 2, 409-21
- **SIMON et al.** *Curr Opin Genet Dev,* 2002, vol. 12, 210-8
- **BEISEL et al.** *Nature,* 2002, vol. 419, 857-62
- **CAO et al.** *Science,* 2002, vol. 298, 1039-43
- **CZERMIN et al.** *Cell,* 2002, vol. 111, 185-96
- **KUZMICHEV et al.** *Genes Dev,* 2002, vol. 16, 2893-905
- **MILNE et al.** *Mol Cell,* 2002, vol. 10, 1107-17
- **MULLER et al.** *Cell,* 2002, vol. 111, 197-208
- **NAKAMURA et al.** *Mol Cell,* 2002, vol. 10, 1119-28
- **OTTE et al.** *Curr Opin Genet Dev,* 2003, vol. 13, 448-54
- **PLATH et al.** *Science,* 2003, vol. 300, 131-5
- **SILVA et al.** *Dev Cell,* 2003, vol. 4, 481-95
- **ERHARDT et al.** *Development,* 2003, vol. 130, 4235-48
- **BRACKEN et al.** *EMBO J,* 2003, vol. 22, 5323-35
- **KIRMIZIS et al.** *Mol Cancer Ther,* 2003, vol. 2, 113-21
- **KLEER et al.** *Proc Natl Acad Sci USA,* 2003, vol. 100, 11606-11
- **VARAMBALLY et al.** *Nature,* 2002, vol. 419, 624-9
- **MORIN et al.** *Nat Genet,* 2010, vol. 42, 181-5
- **MORIN et al.** *Nat Genet,* 2010, vol. 42 (2), 181-5
- **CHEN et al.** *Genomics,* 1996, vol. 38, 30-7
- **SHEN, X. et al.** *Mol Cell,* 2008, vol. 32, 491-502
- **CUI et al.** *Cell Stem Cell,* 2009, vol. 4, 80-93
- **BARSKI.** *Cell,* 2007, vol. 129, 823-37
- **KIRMIZIS et al.** *Genes Dev,* 2007, vol. 18, 1592-1605
- **BERNSTEIN et al.** *Cell,* 2006, vol. 125, 315-26
- **COPELAND et al.** *Nat Rev Drug Discov,* 2009, vol. 8, 724-32
- **COPELAND et al.** *Curr Opin Chem Biol,* 2010, vol. 14 (4), 505-10
- **POLLOCK et al.** *Drug Discovery Today: Therapeutic Strategies,* 2010, vol. 6 (1), 71-9
- **COPELAND, R. A.** Enzymes: A Practical Introduction to Structure, Mechanism and Data Analysis. Wiley, 2000
- **KOHLER ; MILSTEIN.** *Nature,* 1975, vol. 256 (5517), 495-7
- **BENTLEY.** *Curr Opin Genet Dev.,* 2006, vol. 16, 545-52
- **LI et al.** *Genome Res,* 2009, vol. 19, 1124-32
- Molecular Cloning: A Laboratory Manual. Cold Spring Harbor Laboratory Press, 1989
- Current Protocols in Molecular Biology. John Wiley & Sons, Inc,
- **FANG et al.** *Methods Enzymol,* 2004, vol. 377, 213-26
- Remington's "The Science and Practice of Pharmacy. Lippincott Williams & Wilkins, 2005
- **CAHN et al.** *Angew. Chem. Inter. Edit.,* 1966, vol. 5, 385
- **CAHN et al.** *Angew. Chem.,* 1966, vol. 78, 413
- **CAHN ; INGOLD.** *J. Chem. Soc.,* 1951, 612
- **CAHN et al.** *Experientia,* 1956, vol. 12, 81
- **CAHN.** *J. Chem. Educ.,* 1964, vol. 41, 116
- **PATANI ; LAVOIE.** *Chem. Rev.,* 1996, vol. 96, 3147-3176
- **SAMBROOK et al.** Molecular Cloning: A Laboratory Manual. Cold Spring Harbor, 2001
- **F.M. AUSUBEL et al.** Current Protocols in Molecular Biology. Green Publishing Associates, Inc. and John Wiley & Sons, Inc,
- **SMITH, M. B. ; MARCH, J.** March's Advanced Organic Chemistry: Reactions, Mechanisms, and Structure. John Wiley & Sons, 2001
- **GREENE, T.W. ; WUTS, P.G. M.** Protective Groups in Organic Synthesis. John Wiley & Sons, 1999

- **DEVLIN.** High Throughput Screening. Marcel Dekker, 1998
- **MARTINEZ-GARCIA et al.** *Nat Genet,* 2010, vol. 42, 100-1
- **SIMON et al.** *Mutat Res,* 2008, vol. 647, 21-9
- **VAN HAAFTEN et al.** *Nat Genet,* 2009, vol. 41, 521-3
- **WANG et al.** *Gene,* 2004, vol. 343, 69-78
- **CAO et al.** *Mol Cell Biol,* 2008, vol. 28, 1862-72
- **SARMA et al.** *Mol Cell Biol,* 2008, vol. 28, 2718-31
- **CAO et al.** *Mol Cell,* 2004, vol. 15, 57-67
- **COPELAND.** Evaluation of Enzyme Inhibitors in Drug Discovery: A Guide to Medicinal Chemists and Pharmacologists. Wiley, 2005
- **TOWNSEND, A. P. et al.** *Org. Let.,* 2009, vol. 11, 2976-2979
- **WIGLE et al.** *Febs Lett,* 03 October 2011, vol. 585 (19), 3011-4